# EUROPEAN PATENT APPLICATION

(11) **EP 3 858 840 A1**
(43) Date of publication of application: **04.08.2021**
(21) Application number: 19864112.8
(22) Date of filing: 27.09.2019
(51) Int. Cl.: C07D 515/22, A61K 31/395, A61P 35/00

(54) **INDOLE MACROCYCLIC DERIVATIVE, PREPARATION METHOD THEREFOR AND APPLICATION THEREOF IN MEDICINE**

(30) Priority: 30.09.2018 CN 201811156217; 25.03.2019 CN 201910227359
(71) Applicant: Jiangsu Hengrui Medicine Co., Ltd., Lianyungang, Jiangsu 222047 (CN); Shanghai Hengrui Pharmaceutical Co., Ltd., Shanghai 200245 (CN)
(72) Inventor: ZHANG, Guobao, Shanghai 200245 (CN); FEI, Hongbo, Shanghai 200245 (CN); ZHANG, Xiaomin, Shanghai 200245 (CN); HU, Weimin, Shanghai 200245 (CN); HE, Feng, Shanghai 200245 (CN); TAO, Weikang, Shanghai 200245 (CN)
(74) Representative: Viganò, Elena
(86) International application number: PCT/CN2019/108322
(87) International publication number: WO 2020/063792

(57) **Abstract**

The present invention relates to an indole macrocyclic derivative, a preparation method therefor and an application thereof in medicine. Specifically, the present invention relates to an indole macrocyclic derivative represented by general formula (IM), a preparation method therefor, a pharmaceutical composition containing the derivative, and a use thereof as a therapeutic agent, especially as an MCL-1 inhibitor. Each substituent of general formula (IM) is the same as those defined in the description.

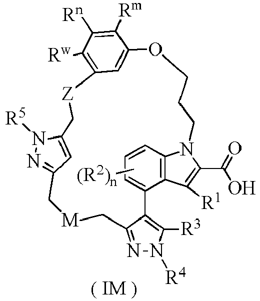

## Description

### FIELD OF THE INVENTION

The present invention belongs to the field of medicine, and relates to an indole macrocyclic derivative of formula (IM), a method for preparing the same, a pharmaceutical composition comprising the same, and a use thereof as a therapeutic agent, particularly as an MCL-1 inhibitor.

### BACKGROUND OF THE INVENTION

An important feature that distinguishes tumor cells from normal cells is that the apoptosis of tumor cells is inhibited, which gives them a greater survival advantage. Apoptosis is also known as programmed death, which can be divided into exogenous apoptosis and endogenous apoptosis. Endogenous apoptosis is an important obstacle to the development of cancer. BCL-2 family proteins are important regulators of endogenous apoptosis.

BCL-2 family proteins mainly exist on the mitochondrial membrane, and can be divided into two categories according to their functions: anti-apoptotic proteins and pro-apoptotic proteins. Anti-apoptotic proteins include BCL-2, BCL-XL, BCL-w and MCL-1. Pro-apoptotic proteins include Bax, Bak and BH3-only protein. When Bax and Bak are activated, multimer cavities will form, which increases the permeability of cell mitochondrial membrane and promotes the release of cytochrome C into the cytoplasm, thereby leading to apoptosis. The BH3-only protein contains only a BH3 domain. When the cell is alive, the BH3-only protein (such as Bim) binds to anti-apoptotic protein. When the cell is under external pressure, the balance of binding is broken, the BH3-only protein is released and binds to BAX on mitochondria, which promotes BAX/BAK to form multimers and the release of cytochrome C and SMAC into the cytoplasm, thereby activating downstream apoptosis path.

Existing clinical data indicate that MCL-1 is overexpressed in a variety of tumors. For example, overexpression of MCL-1 has been found in 55% of breast cancer samples and 84% of lung cancer samples. In multiple myeloma samples, as the degree of cancer progression raises, the expression of MCL-1 increases significantly, while the expression of BCL-2 does not change. In addition, the expression of MCL-1 is negatively correlated with the survival rate of patient. High expression of MCL-1 with lower survival rates has been observed in both breast cancer patients and multiple myeloma patients. It can be seen that MCL-1 is an important target for tumor treatment.

Novartis, Amgen and AstraZeneca have all developed small molecule inhibitors against MCL-1, but they are still in the clinical stage. Therefore, further development of MCL-1 inhibitor drugs is needed.

### SUMMARY OF THE INVENTION

The object of the present invention is to provide a compound of formula (IM) or a tautomer, mesomer, racemate, enantiomer, diastereomer thereof, or mixture thereof, or a pharmaceutically acceptable salt thereof, 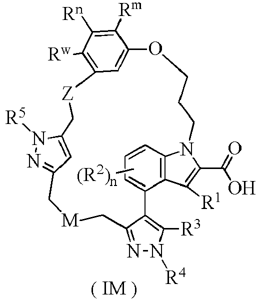 wherein:
R^{m}, Rⁿ and R^{w} are identical or different and are each independently selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or R^{m} and Rⁿ together with adjacent carbon atoms form an aryl, heteroaryl, cycloalkyl or heterocyclyl; and R^{w} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or Rⁿ and R^{w} together with adjacent carbon atoms form an aryl, heteroaryl, cycloalkyl or heterocyclyl; and R^{m} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
Z is a S atom or -CH₂-;
M is a S atom, O atom or -NR₆-;
R¹ is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
R² is selected from the group consisting of hydrogen atom, halogen, alkyl, deuterated alkyl, alkoxy, haloalkyl, hydroxy, hydroxyalkyl, cyano, amino and nitro;
R³ is selected from the group consisting of hydrogen atom, halogen, alkyl, deuterated alkyl, alkoxy, haloalkyl, hydroxy, hydroxyalkyl, cyano, amino and nitro;
R⁴ is selected from the group consisting of hydrogen atom, alkyl, deuterated alkyl and cycloalkyl;
or R³ and R⁴ together with the adjacent carbon atom and N atom form a heterocyclyl;
R⁵ is selected from the group consisting of hydrogen atom, alkyl, deuterated alkyl and cycloalkyl;
R⁶ is selected from the group consisting of hydrogen atom, alkyl and cycloalkyl;
n is 0, 1, 2 or 3.

In a preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (IM-1) or (IM-2) or a tautomer, mesomer, racemate, enantiomer, diastereomer thereof, or mixture thereof, or a pharmaceutically acceptable salt thereof: 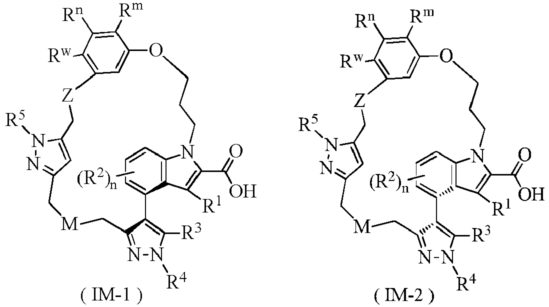 wherein
R^{m}, Rⁿ, R^{w}, Z, M, R¹∼R⁵ and n are as defined in formula (IM).

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein R^{m} and Rⁿ together with adjacent carbon atoms form an aryl, heteroaryl, cycloalkyl or heterocyclyl; R^{w} is selected from the group consisting of hydrogen atom, halogen, deuterated alkyl, alkoxy, haloalkyl, hydroxy, hydroxyalkyl, cyano, amino, nitro, cycloalkyl, cycloalkyloxy and heterocyclyl; or Rⁿ and R^{w} together with adjacent carbon atoms form an aryl, heteroaryl, cycloalkyl or heterocyclyl; and R^{m} is selected from the group consisting of hydrogen atom, halogen, deuterated alkyl, alkoxy, haloalkyl, hydroxy, hydroxyalkyl, cyano, amino, nitro, cycloalkyl, cycloalkyloxy and heterocyclyl.

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (IIM) or a tautomer, mesomer, racemate, enantiomer, diastereomer thereof, or mixture thereof, or a pharmaceutically acceptable salt thereof: 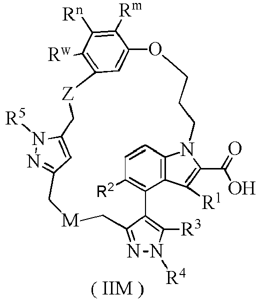 wherein:
R^{m}, Rⁿ, R^{w}, Z, M and R¹∼R⁵ are as defined in formula (IM).

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (IIM-1) or (IIM-2) or a tautomer, mesomer, racemate, enantiomer, diastereomer thereof, or mixture thereof, or a pharmaceutically acceptable salt thereof: 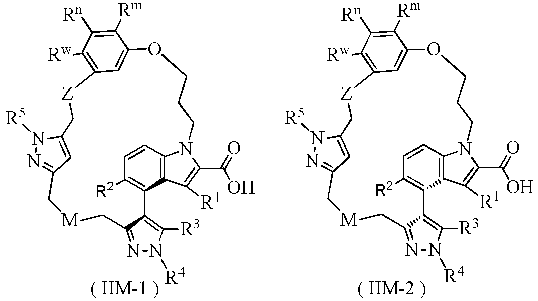 wherein:
R^{m}, Rⁿ, R^{w}, Z, M and R¹∼R⁵ are as defined in formula (IM).

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein R^{m} and Rⁿ together with adjacent carbon atoms form a phenyl or cycloalkyl; R^{w} is selected from the group consisting of hydrogen atom, halogen and alkyl; or Rⁿ and R^{w} together with adjacent carbon atoms form a cycloalkyl; and R^{m} is selected from the group consisting of hydrogen atom, halogen and alkyl.

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein R^{m} and Rⁿ together with adjacent carbon atoms form a phenyl or C_{4∼6} cycloalkyl; R^{w} is a hydrogen atom; or Rⁿ and R^{w} together with adjacent carbon atoms form a C_{4∼6} cycloalkyl; and R^{m} is a hydrogen atom.

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein 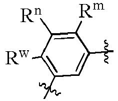 is selected from the group consisting of 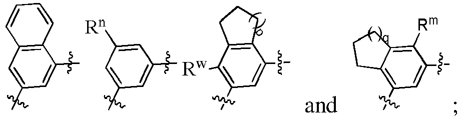 R^{m}, Rⁿ and R^{w} are identical or different and are each independently selected from the group consisting of hydrogen atom, halogen and alkyl; p is 0, 1 or 2; and q is 0, 1 or 2.

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein 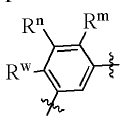 is selected from the group consisting of 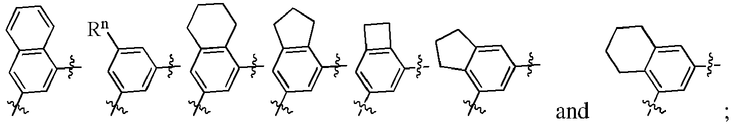 and Rⁿ is selected from the group consisting of hydrogen atom, halogen and alkyl.

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (IK) or (IL) or a tautomer, mesomer, racemate, enantiomer, diastereomer thereof, or mixture thereof, or a pharmaceutically acceptable salt thereof: 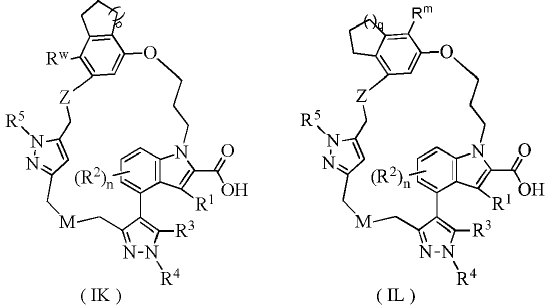 wherein:
p is 0, 1 or 2;
q is 0, 1 or 2;
R^{m} and R^{w} are identical or different and are each independently selected from the group consisting of hydrogen atom, halogen and alkyl;
Z, M, R¹∼R⁵ and n are as defined in formula (IM).

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (IK-1), (IK-2), (IL-1) or (IL-2) or a tautomer, mesomer, racemate, enantiomer, diastereomer thereof, or mixture thereof, or a pharmaceutically acceptable salt thereof: 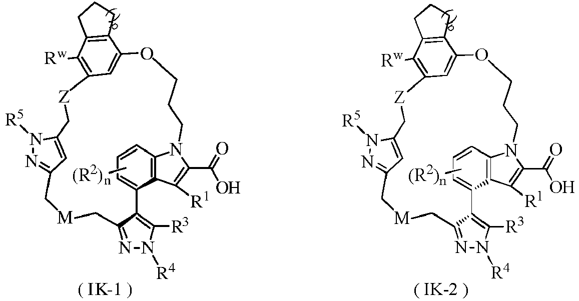 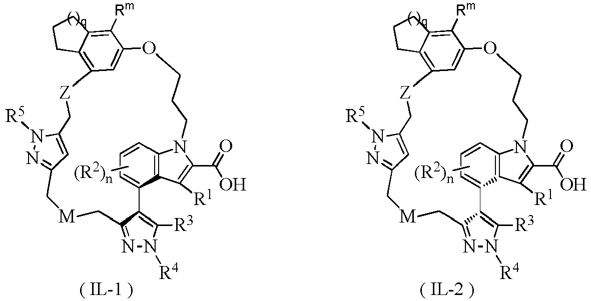 wherein:
p is 0, 1 or 2;
q is 0, 1 or 2;
R^{m} and R^{w} are identical or different and are each independently selected from the group consisting of hydrogen atom, halogen and alkyl;
Z, M, R¹∼R⁵ and n are as defined in formula (IM).

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (IIK) or (IIL) or a tautomer, mesomer, racemate, enantiomer, diastereomer thereof, or mixture thereof, or a pharmaceutically acceptable salt thereof: 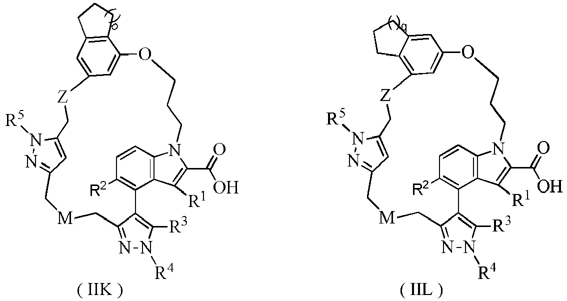 wherein:
p is 1 or 2;
q is 1 or 2;
Z, M and R¹∼R⁵ are as defined in formula (IM).

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (IIK-1), (IIK-2), (IIL-1) or (IIL-2) or a tautomer, mesomer, racemate, enantiomer, diastereomer thereof, or mixture thereof, or a pharmaceutically acceptable salt thereof: 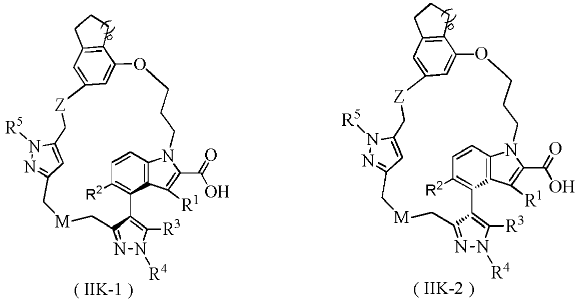 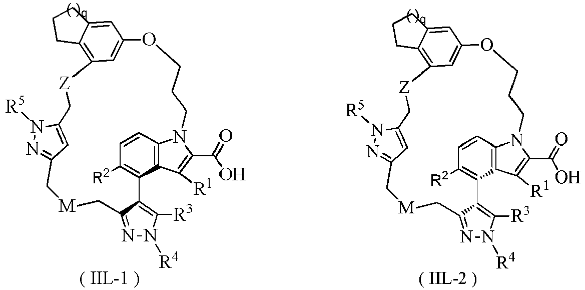 wherein:
p is 1 or 2;
q is 1 or 2;
Z, M and R¹∼R⁵ are as defined in formula (IM).

In another preferred embodiment of the present invention, the compound of formula (IM) according to the present invention is a compound of formula (I) or a tautomer, mesomer, racemate, enantiomer, diastereomer thereof, or mixture thereof, or a pharmaceutically acceptable salt thereof: 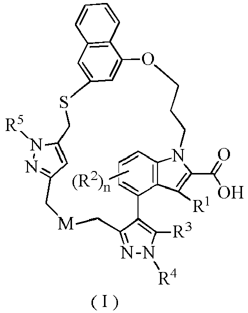 wherein:
M is a S atom, O atom or -NR₆-;
R¹ is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
R² is selected from the group consisting of hydrogen atom, halogen, alkyl, deuterated alkyl, alkoxy, haloalkyl, hydroxy, hydroxyalkyl, cyano, amino and nitro;
R³ is selected from the group consisting of hydrogen atom, halogen, alkyl, deuterated alkyl, alkoxy, haloalkyl, hydroxy, hydroxyalkyl, cyano, amino and nitro;
R⁴ is selected from the group consisting of hydrogen atom, alkyl, deuterated alkyl and cycloalkyl;
or R³ and R⁴ together with the adjacent carbon atom and N atom form a heterocyclyl;
R⁵ is selected from the group consisting of hydrogen atom, alkyl, deuterated alkyl and cycloalkyl;
R⁶ is selected from the group consisting of hydrogen atom, alkyl and cycloalkyl;
n is 0, 1, 2 or 3.

In another preferred embodiment of the present invention, the compound of formula (I) or the tautomer, mesomer, racemate, enantiomer, diastereomer thereof, or mixture thereof, or the pharmaceutically acceptable salt thereof according to the present invention is a compound of formula (I-1) or (1-2) or a tautomer, mesomer, racemate, enantiomer, diastereomer thereof, or mixture thereof, or a pharmaceutically acceptable salt thereof: 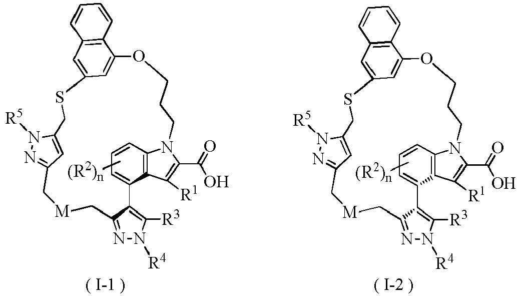 wherein
M, R¹∼R⁵ and n are as defined in formula (I).

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein R³ is an alkyl, preferably C₁₋₆ alkyl, and more preferably methyl.

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein R⁴ or R⁵ is an alkyl, preferably C₁₋₆ alkyl, and more preferably methyl.

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (II) or a tautomer, mesomer, racemate, enantiomer, diastereomer thereof, or mixture thereof, or a pharmaceutically acceptable salt thereof: 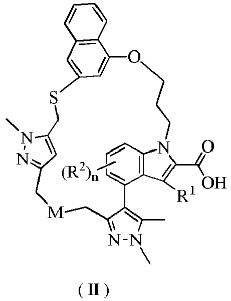 wherein
M, R¹, R² and n are as defined in formula (I).

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein M is an S atom or -N(CH₃)-, and preferably S atom.

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (III) or a tautomer, mesomer, racemate, enantiomer, diastereomer thereof, or mixture thereof, or a pharmaceutically acceptable salt thereof: 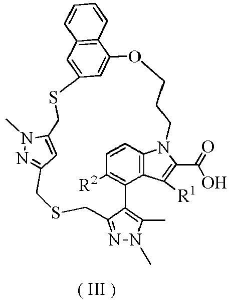 wherein
R¹ and R² are as defined in formula (I).

In another preferred embodiment of the present invention, the compound of formula (IM) or the tautomer, mesomer, racemate, enantiomer, diastereomer thereof, or mixture thereof, or the pharmaceutically acceptable salt thereof according to the present invention is a compound of formula (III-1) or (III-2) or a tautomer, mesomer, racemate, enantiomer, diastereomer thereof, or mixture thereof, or a pharmaceutically acceptable salt thereof: 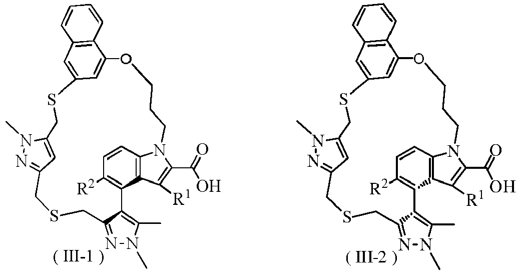 wherein
R¹ and R² are as defined in formula (I).

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein R¹ is a hydrogen atom or alkyl, preferably alkyl, and more preferably methyl.

In another preferred embodiment of the present invention, in the compound of formula (IM) or the tautomer, mesomer, racemate, enantiomer, diastereomer thereof, or mixture thereof, or the pharmaceutically acceptable salt thereof according to the present invention, wherein R² is a halogen, and preferably chlorine.

Typical compounds of the present invention include, but are not limited to:

| Example No. | Structure and name of the compound |
|---|---|
| 1 | 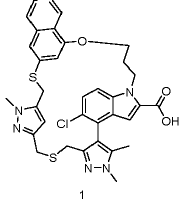 |
| | 17-Chloro-5,13-14-trimethyl-28-oxa-2,9-dithia-5,6,12,13,24-pentaazaheptacyclo[27 .7.1.1^{4,7}.0^{11,15}.0^{16,21}.0^{20,24}.0^{30,35}]octatriaconta-1(37),4(38),6,11,14,16,18,20,22,29,31,33 ,35-tridecaene-23-carboxylic acid |
| 1-1 | 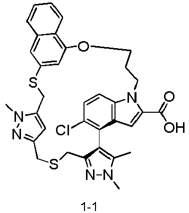 |
| | (*Ra*)-17-Chloro-5,13-14-trimethyl-28-oxa-2,9-dithia-5,6,12,13,24-pentaazaheptacyc lo[27.7.1.1^{4,7}.0^{11,15}.0^{16,21}.0^{20,24}.0^{30,35}]loctatriaconta-1(37),4(38),6,11,14,16,18,20,22,29, 31,33,35-tridecaene-23-carboxylic acid |
| 1-2 | 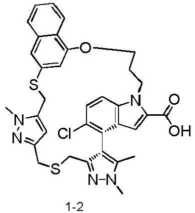 |
| | (*Sa*)-17-Chloro-5,13-14-trimethyl-28-oxa-2,9-dithia-5,6,12,13,24-pentaazaheptacyc lo[27.7.1.1^{4,7}.0^{11,15}.0^{16,21}.0^{20,24}.0^{30,35}]octatriaconta-1(37),4(38),6,11,14,16,18,20,22,29, 31,33,35-tridecaene-23-carboxylic acid |
| 2 | 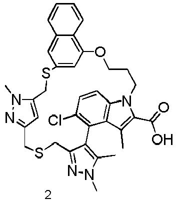 |
| | 17-Chloro-5,13,14,22-tetramethyl-28-oxa-2,9-dithia-5,6,12,13,24-pentaazaheptacyc lo[27.7.1.1^{4,7}.0^{11,15}.0^{16,21}.0^{20,24}.0^{30,35}]octatriaconta-1(37),4(38),6,11,14,16,18,20,22,29, 31,33,35-tridecaene-23-carboxylic acid |
| 2-1 | 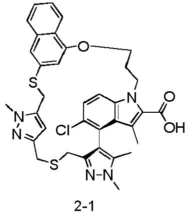 |
| | (*Ra*)-17-Chloro-5,13,14,22-tetramethyl-28-oxa-2,9-dithia-5,6,12,13,24-pentaazahep tacyclo[27.7.1.1^{4,7}.0^{11,15}.0^{16,21}.0^{20,24}.0^{10,15}]octatriaconta-1(37),4(38),6,11,14,16,18,20,2 2,29,31,33,35-tridecaene-23-carboxylic acid |
| 2-2 | 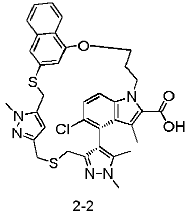 |
| | (*Sa*)-17-Chloro-5,13,14,22-tetramethyl-28-oxa-2,9-dithia-5,6,12,13,24-pentaazahep tacyclo[27.7.1.1^{4,7}.0^{11,15}.0^{16,21}.0^{20,24}.0^{30,35}]octatriaconta-1(37),4(38),6,11,14,16,18,20,2 2,29,31,33,35-tridecaene-23-carboxylic acid |
| 3 | 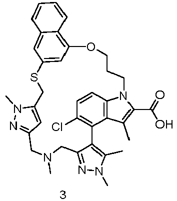 |
| | 17-Chloro-5,9,13,14,22-pentamethyl-28-oxa-2-thia-5,6,9,12,13,24-hexaazaheptacyc lo[27.7.1.1^{4,7}.0^{11,15}.0^{16,21}.0^{20,24}.0^{30,35}]octatriaconta-1(37),4(38),6,11,14,16,18,20,22,29, 31,33,35-tridecaene-23-carboxylic acid |
| 3-1 | 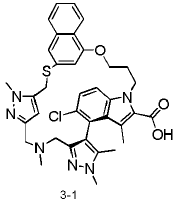 |
| | (*Ra*)-17-Chloro-5,9,13,14,22-pentamethyl-28-oxa-2-thia-5,6,9,12,13,24-hexaazahep tacyclo[27.7.1.1^{4,7}.0^{11,15}.0^{16,21}.0^{20,24}.0^{30,35}]octatriaconta-1(37),4(38),6,11,14,16,18,20,2 2,29,31,33,35-tridecaene-23-carboxylic acid |
| 3-2 | 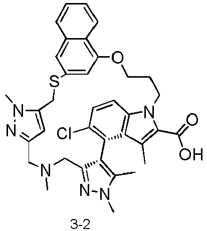 |
| | (*Sa*)-17-Chloro-5,9,13,14,22-pentamethyl-28-oxa-2-thia-5,6,9,12,13,24-hexaazahep tacyclo[27.7.1.1^{4,7}.0^{11,15}.0^{16,21}.0^{20,24}.0^{30,35}]octatriaconta-1(37),4(38),6,11,14,16,18,20,2 2,29,31,33,35-tridecaene-23-carboxylic acid |
| 4 | 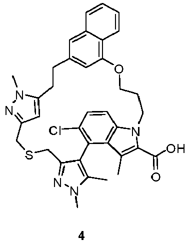 |
| | 17-Chloro-5,13,14,22-tetramethyl-28-oxa-9-thia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,3 3,35-tridecaene-23-carboxylic acid |
| 4-1 | 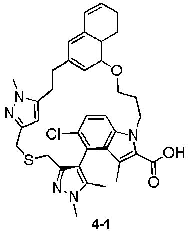 |
| | (*Ra*)-17-Chloro-5,13,14,22-tetramethyl-28-oxa-9-thia-5,6,12,13,24-pentaazaheptac yclo[27.7.1.1^{4,7}.0^{11,15}.0^{16,21}.0^{20,24}.0^{30,35}]octatriaconta-1(37),4(38),6,11,14,16,18,20,22,2 9,31,33,35-tridecaene-23-carboxylic acid |
| 4-2 | 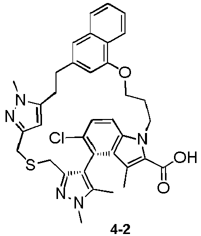 |
| | (*Sa*)-17-Chloro-5,13,14,22-tetramethyl-28-oxa-9-thia-5,6,12,13,24-pentaazaheptacy clo[27.7.1.1^{1,7}.0^{11,15}.0^{16,21}.0^{20,24}.0^{30,35}]octatriaconta-1(37),4(38),6,11,14,16,18,20,22,29 ,31,33,35-tridecaene-23-carboxylic acid |
| 5 | 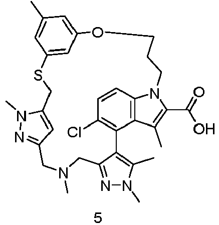 |
| | 17-Chloro-5,9,13,14,22,31-hexamethyl-28-oxa-2-thia-5,6,9,12,13,24-hexaazahexac yclo[27.3.1.1^{4,7}.0^{11,15}.0^{16,21}.0^{20,24}]tetratriaconta-1(33),4(34),6,11,14,16,18,20,22,29,31-undecaene-23-carboxylic acid |
| 6 | 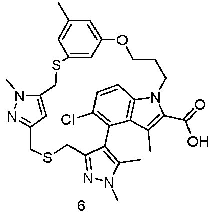 |
| | 17-Chloro-5,13,14,22,31-pentamethyl-28-oxa-2,9-dithia-5,6,12,13,24-pentaazahexa cyclo[27.3.1.1^{4,7}.0^{11,15}.0^{16,21}.0^{20,24}]tetratriaconta-1(33),4(34),6,11,14,16,18,20,22,29,3 1-undecaene-23-carboxylic acid |
| 7 | 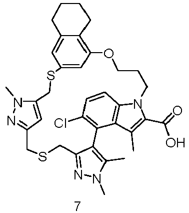 |
| | 17-Chloro-5,13,14,22-tetramethyl-28-oxa-2,9-dithia-5,6,12,13,24-pentaazaheptacyc lo[27.7.1.1^{4,7}.0^{11,15}.0^{16,21}.0^{20,24}.0^{30,35}]octatriaconta-1(37),4(38),6,11,14,16,18,20,22,29, 35-undecaene-23-carboxylic acid |
| 7-1 | 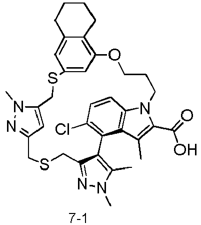 |
| | (*Ra*)-17-Chloro-5,13,14,22-tetramethyl-28-oxa-2,9-dithia-5,6,12,13,24-pentaazahep tacyclo[27.7.1.1^{4,7}.0^{11,15}.0^{16,21}.0^{20,24}.0^{30,15}]octatriaconta-1(37),4(38),6,11,14,16,18,20,2 2,29,35-undecaene-23-carboxylic acid |
| 7-2 | 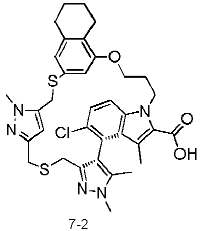 |
| | (*Sa*)-17-Chloro-5,13,14,22-tetramethyl-28-oxa-2,9-dithia-5,6,12,13,24-pentaazahep tacyclo[27.7.1.1^{4,7}.0^{11,15}.0^{16,21}.0^{20,24}.0^{30,35}]octatriaconta-1(37),4(38),6,11,14,16,18,20,2 2,29,35-undecaene-23-carboxylic acid |
| 8 | 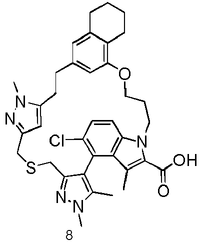 |
| | 17-Chloro-5,13,14,22-tetramethyl-28-oxa-9-thia-5,6,12,13,24-pentaazaheptacyclo[27 .7.1.1^{4,7}.0^{11,15}.0^{16,21}.0^{20,24}.0^{30,35}]octatriaconta-1(37),4(38),6,11,14,16,18,20,22,29,35-un decaene-23-carboxylic acid |
| 8-1 | 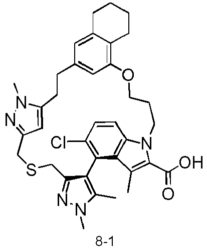 |
| | (*Ra*)-17-Chloro-5,13,14,22-tetramethyl-28-oxa-9-thia-5,6,12,13,24-pentaazaheptac yclo[27.7.1.1^{4,7}.0^{11,15}.0^{16,21}.0^{20,24}.0^{30,35}]octatriaconta-1(37),4(38),6,11,14,16,18,20,22,2 9,35-undecaene-23-carboxylic acid |
| 8-2 | 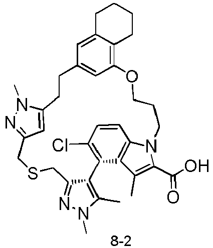 |
| | (*Sa*)-17-Chloro-5,13,14,22-tetramethyl-28-oxa-9-thia-5,6,12,13,24-pentaazaheptacy clo[27.7.1.1^{4,7}.0^{11,15}.0^{16,21}.0^{20,24}.0^{30,35}]octatriaconta-1(37),4(38),6,11,14,16,18,20,22,29 |
| | ,35-undecaene-23-carboxylic acid |
| 9 | 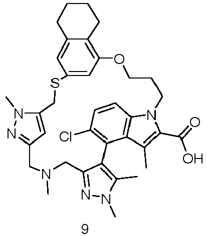 |
| | 17-Chloro-5,9,13,14,22-pentamethyl-28-oxa-2-thia-5,6,9,12,13,24-hexaazaheptacyc lo[27.7.1.1^{4,7}.0^{11,15}.0^{16,21}.0^{20,24}.0^{30,35}]octatriaconta-1(37),4(38),6,11,14,16,18,20,22,29, 35-undecaene-23-carboxylic acid |
| 10 | 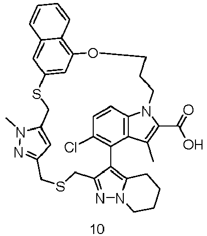 |
| | 21-Chloro-5,26-dimethyl-32-oxa-2,9-dithia-5,6,12,13,28-pentaazaoctacyclo[3 1.7 .1.1^{4,7}.0^{11,19}.0^{13,18}.0^{20,25}.0^{24,28}.0^{34,39}]dotetraconta-1(41),4(42),6,11,18,20,22,24,26,33,35 ,37,39-tridecaene-27-carboxylic acid |
| 11 | 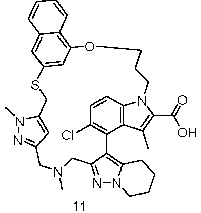 |
| | 21-Chloro-5,9,26-trimethyl-32-oxa-2-thia-5,6,9,12,13,28-hexaazaoctacyclo[31.7. 1.1^{4,7}.0^{11,19}.0^{13,18}.0^{20,25}.0^{24,28}.0^{34,39}]dotetraconta-1(41),4(42),6,11,18,20,22,24,26,33,35, 37,39-tridecaene-27-carboxylic acid |
| 12 | 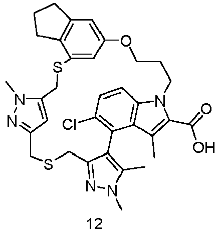 |
| | 17-Chloro-5,13,14,22-tetramethyl-28-oxa-2,9-dithia-5,6,12,13,24-pentaazaheptacyc lo[27.6.1.1^{4,7}.0^{11,15}.0^{16,21}.0^{20,24}.0^{30,35}]heptatriaconta-1(36),4(37),6,11,14,16,18,20,22,2 9,31(35)-undecaene-23-carboxylic acid |
| 12-1 | 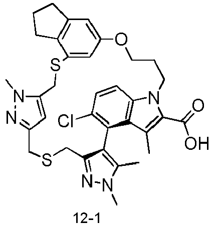 |
| | (*Ra*)-17-Chloro-5,13,14,22-tetramethyl-28-oxa-2,9-dithia-5,6,12,13,24-pentaazahep tacyclo[27.6.1.1^{4,7}.0^{11,15}.01^{6,21}.0^{20,24}.0^{30,15}]heptatriaconta-1(36),4(37),6,11,14,16,18,20, 22,29,31(35)-undecaene-23-carboxylic acid |
| 12-2 | 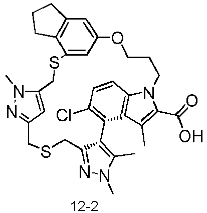 |
| | (*Sa*)-17-Chloro-5,13,14,22-tetramethyl-28-oxa-2,9-dithia-5,6,12,13,24-pentaazahep tacyclo[27.6.1.1^{4,7}.0^{11,15}.0^{16,21}.0^{20,24}.0^{30,35}]heptatriaconta-1(36),4(37),6,11,14,16,18,20, 22,29,31(35)-undecaene-23-carboxylic acid |
| 13 | 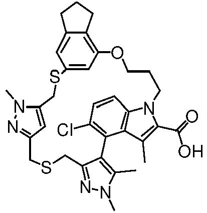 |
| | 17-Chloro-5,13,14,22-tetramethyl-28-oxa-2,9-dithia-5,6,12,13,24-pentaazaheptacyc lo[27.6.1.1^{4,7}.0^{11,15}.0^{16,21}.0^{20,24}.0^{30,34}]heptatriaconta-1(36),4(37),6,11,14,16,18,20,22,2 9,34-undecaene-23-carboxylic acid |
| 13-1 | 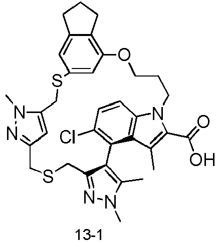 |
| | (*Ra*)-17-Chloro-5,13,14,22-tetramethyl-28-oxa-2,9-dithia-5,6,12,13,24-pentaazahep tacyclo[27.6.1.1^{4,7}.0^{11,15}.0^{16,21}.0^{20,24}.0^{30,34}]heptatriaconta-1(36),4(37),6,11,14,16,18,20, 22,29,34-undecaene-23-carboxylic acid |
| 13-2 | 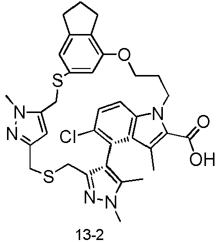 |
| | (*Sa*)-17-Chloro-5,13,14,22-tetramethyl-28-oxa-2,9-dithia-5,6,12,13,24-pentaazahep tacyclo[27.6.1.1^{4,7}.0^{11,15}.0^{16,21}.0^{20,24}.0^{30,34}]heptatriaconta-1(36),4(37),6,11,14,16,18,20, 22,29,34-undecaene-23-carboxylic acid |
| 14 | 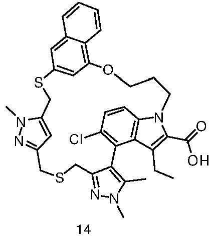 |
| | 17-Chloro-22-ethyl-5,13-14-trimethyl-28-oxa-2,9-dithia-5,6,12,13,24-pentaazahept acyclo[27.7.1.1^{4,7}.0^{11,15}.0^{16,21}.0^{20,24}.0^{30,15}]octatriaconta-1(37),4(38),6,11,14,16,18,20,2 2,29,31,33,35-tridecaene-23-carboxylic acid |
| 14-1 | 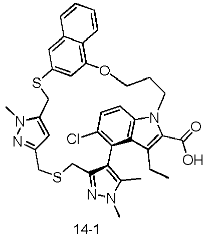 |
| | (*Ra*)-17-Chloro-22-ethyl-5,13-14-trimethyl-28-oxa-2,9-dithia-5,6,12,13,24-pentaaz aheptacyclo[27.7.1.1^{4,7}.0^{11,15}.0^{16,21}.0^{20,24}.0^{30,35}]octatriaconta-1(37),4(38),6,11,14,16,18 ,20,22,29,31,33,35-tridecaene-23-carboxylic acid |
| 14-2 | 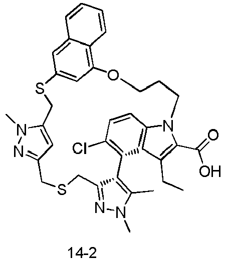 |
| | (*Sa*)-17-Chloro-22-ethyl-5,13-14-trimethyl-28-oxa-2,9-dithia-5,6,12,13,24-pentaaza heptacyclo[27.7.1.1^{4,7}.0^{11,15}.0^{16,21}.0^{20,24}.0^{30,35}]octatriaconta-1(37),4(38),6,11,14,16,18,20,22,29,31,33,35-tridecaene-23-carboxylic acid |

or a tautomer, mesomer, racemate, enantiomer, diastereomer thereof, or mixture thereof,
or a pharmaceutically acceptable salt thereof.

In another aspect, the present invention relates to a compound of formula (IMA) or a tautomer, mesomer, racemate, enantiomer, diastereomer thereof, or mixture thereof, or a pharmaceutically acceptable salt thereof: 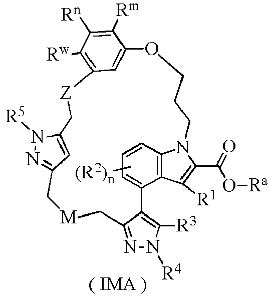 wherein:
R^{m}, Rⁿ and R^{w} are identical or different and are each independently selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or R^{m} and Rⁿ together with adjacent carbon atoms form an aryl, heteroaryl, cycloalkyl or heterocyclyl; and R^{w} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or Rⁿ and R^{w} together with adjacent carbon atoms form an aryl, heteroaryl, cycloalkyl or heterocyclyl; and R^{m} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
Z is a S atom or -CH₂-;
M is a S atom, O atom or -NR₆-;
R¹ is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
R² is selected from the group consisting of hydrogen atom, halogen, alkyl, deuterated alkyl, alkoxy, haloalkyl, hydroxy, hydroxyalkyl, cyano, amino and nitro;
R³ is selected from the group consisting of hydrogen atom, halogen, alkyl, deuterated alkyl, alkoxy, haloalkyl, hydroxy, hydroxyalkyl, cyano, amino and nitro;
R⁴ is selected from the group consisting of hydrogen atom, alkyl, deuterated alkyl and cycloalkyl;
or R³ and R⁴ together with the adjacent carbon atom and N atom form a heterocyclyl;
R⁵ is selected from the group consisting of hydrogen atom, alkyl, deuterated alkyl and cycloalkyl;
R⁶ is selected from the group consisting of hydrogen atom, alkyl and cycloalkyl;
R^{a} is an alkyl;
n is 0, 1, 2 or 3.

In a preferred embodiment, the compound of formula (IMA) or the tautomer, mesomer, racemate, enantiomer, diastereomer thereof, or mixture thereof, or the pharmaceutically acceptable salt thereof according to the present invention is a compound of formula (IMA-1) or (IMA-2) or a tautomer, mesomer, racemate, enantiomer, diastereomer thereof, or mixture thereof, or a pharmaceutically acceptable salt thereof: 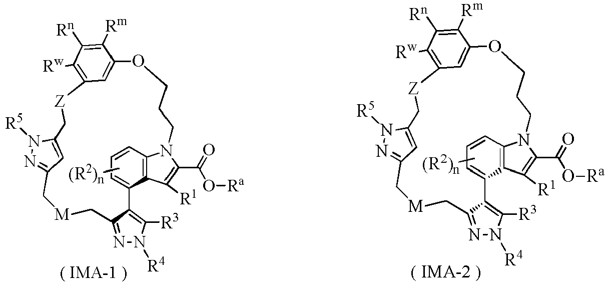 wherein:
R^{a}, R^{m}, Rⁿ, R^{w}, Z, M, R¹∼R⁵ and n are as defined in formula (IMA).

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IIMA): 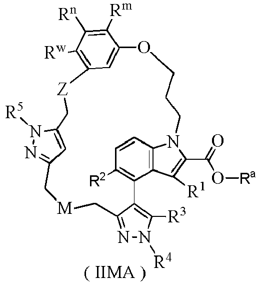 wherein:
R^{a}, R^{m}, Rⁿ, R^{w}, Z, M and R¹∼R⁵ are as defined in formula (IMA).

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IIMA-1) or (IIMA-2): 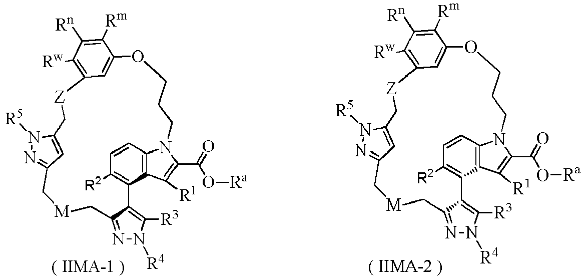 wherein:
R^{a}, R^{m}, Rⁿ, R^{w}, Z, M and R¹∼R⁵ are as defined in formula (IMA).

In another preferred embodiment of the present invention, in the compound of formula (IMA) or the tautomer, mesomer, racemate, enantiomer, diastereomer thereof, or mixture thereof, or the pharmaceutically acceptable salt thereof according to the present invention, wherein R^{m} and Rⁿ together with adjacent carbon atoms form a phenyl or cycloalkyl; R^{w} is selected from the group consisting of hydrogen atom, halogen and alkyl; or Rⁿ and R^{w} together with adjacent carbon atoms form a cycloalkyl; and R^{m} is selected from the group consisting of hydrogen atom, halogen and alkyl.

In another preferred embodiment of the present invention, in the compound of formula (IMA) or the tautomer, mesomer, racemate, enantiomer, diastereomer thereof, or mixture thereof, or the pharmaceutically acceptable salt thereof according to the present invention, wherein R^{m} and Rⁿ together with adjacent carbon atoms form a phenyl or C_{4∼6} cycloalkyl; R^{w} is a hydrogen atom; or Rⁿ and R^{w} together with adjacent carbon atoms form a C_{4∼6} cycloalkyl; and R^{m} is a hydrogen atom.

In another preferred embodiment of the present invention, in the compound of formula (IMA) or the tautomer, mesomer, racemate, enantiomer, diastereomer thereof, or mixture thereof, or the pharmaceutically acceptable salt thereof according to the present invention, wherein 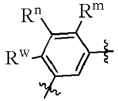 is selected from the group consisting of 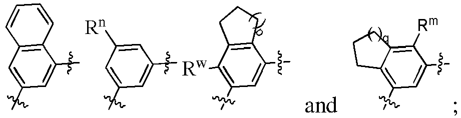 R^{m}, Rⁿ and R^{w} are identical or different and are each independently selected from the group consisting of hydrogen atom, halogen and alkyl; p is 0, 1 or 2; and q is 0, 1 or 2.

In another preferred embodiment of the present invention, in the compound of formula (IMA) or the tautomer, mesomer, racemate, enantiomer, diastereomer thereof, or mixture thereof, or the pharmaceutically acceptable salt thereof according to the present invention, wherein 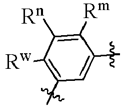 is selected from the group consisting of 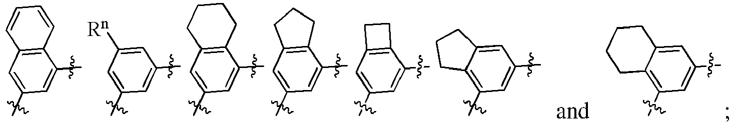 and Rⁿ is selected from the group consisting of hydrogen atom, halogen and alkyl.

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IKA) or (ILA) or a tautomer, mesomer, racemate, enantiomer, diastereomer thereof, or mixture thereof, or a pharmaceutically acceptable salt thereof: 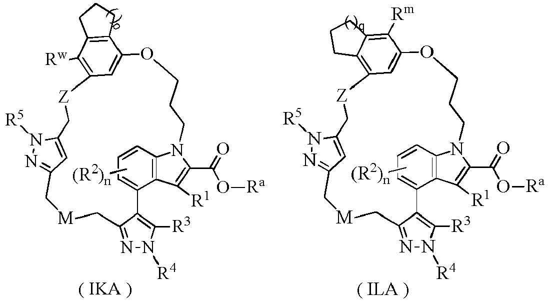 wherein:
R^{a} is an alkyl;
p is 0, 1 or 2;
q is 0, 1 or 2;
R^{m} and R^{w} are identical or different and are each independently selected from the group consisting of hydrogen atom, halogen and alkyl;
Z, M, R¹∼R⁵ and n are as defined in formula (IMA).

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IKA-1), (IKA-2), (ILA-1) or (ILA-2) or a tautomer, mesomer, racemate, enantiomer, diastereomer thereof, or mixture thereof, or a pharmaceutically acceptable salt thereof: 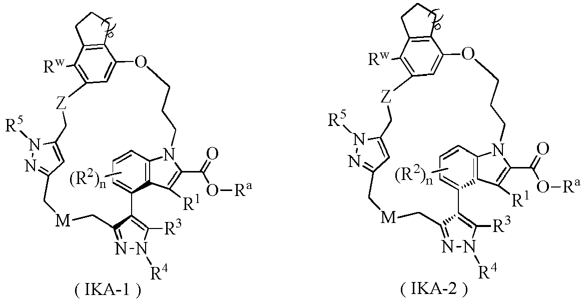 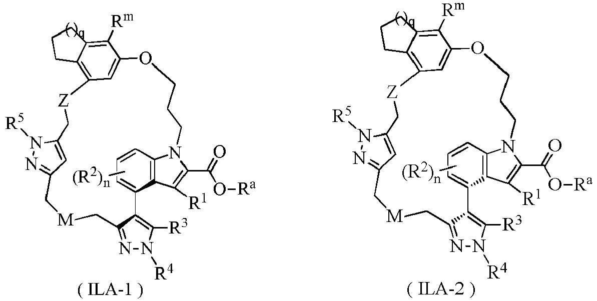 wherein:
R^{a} is an alkyl;
p is 0, 1 or 2;
q is 0, 1 or 2;
R^{m} and R^{w} are identical or different and are each independently selected from the group consisting of hydrogen atom, halogen and alkyl;
Z, M, R¹∼R⁵ and n are as defined in formula (IMA).

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IIKA) or (IILA) or a tautomer, mesomer, racemate, enantiomer, diastereomer thereof, or mixture thereof, or a pharmaceutically acceptable salt thereof: 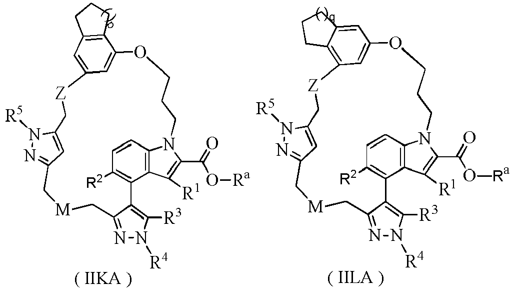 wherein:
R^{a} is an alkyl;
p is 1 or 2;
q is 1 or 2;
Z, M and R¹∼R⁵ are as defined in formula (IMA).

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IIKA-1), (IIKA-2), (IILA-1) or (IILA-2) or a tautomer, mesomer, racemate, enantiomer, diastereomer thereof, or mixture thereof, or a pharmaceutically acceptable salt thereof: 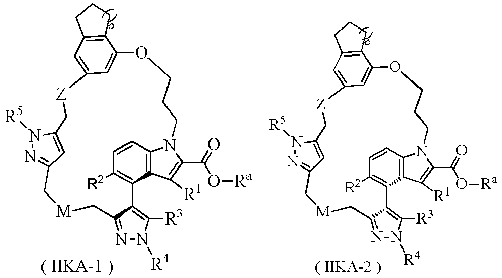 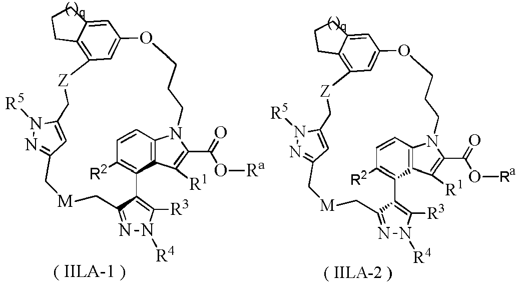 wherein:
R^{a} is an alkyl;
p is 1 or 2;
q is 1 or 2;
Z, M and R¹∼R⁵ are as defined in formula (IMA).

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IA) or a tautomer, mesomer, racemate, enantiomer, diastereomer thereof, or mixture thereof, or a pharmaceutically acceptable salt thereof: 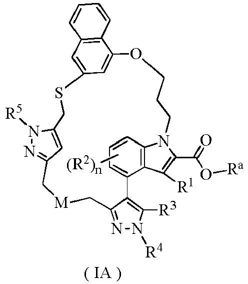 wherein:
R^{a} is an alkyl;
M, R¹∼R⁵ and n are as defined in formula (IMA).

In another preferred embodiment of the present invention, the compound of formula (IMA) or the tautomer, mesomer, racemate, enantiomer, diastereomer thereof, or mixture thereof, or the pharmaceutically acceptable salt thereof according to the present invention is a compound of formula (IA-1) or (IA-2) or a tautomer, mesomer, racemate, enantiomer, diastereomer thereof, or mixture thereof, or a pharmaceutically acceptable salt thereof: 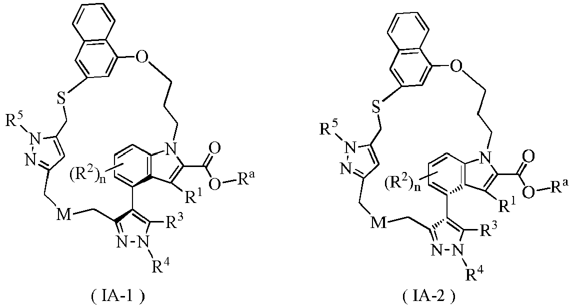 wherein:
R^{a}, M, R¹∼R⁵ and n are as defined in formula (IA).

Typical compounds of formula (IA) of the present invention include, but are not limited to:

| Example No. | Structure and name of the compound |
|---|---|
| 1o | 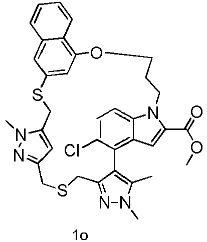 |
| | Methyl 17-chloro-5,13-14-trimethyl-28-oxa-2,9-dithia-5,6,12,13,24-pentaazaheptacyclo[27.7. 1.1^{4,7}.0^{11,15}.0^{16,21}.0^{20,24}.0^{30,35}]octatriaconta-1(37),4(38),6,11,14,16,18,20,22,29,31,33,3 5-tridecaene-23-carboxylate |
| 2o | 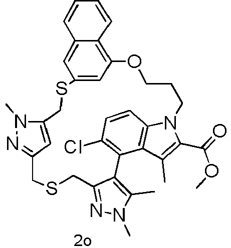 |
| | Methyl 17-chloro-5,13-14,22-tetramethyl-28-oxa-2,9-dithia-5,6,12,13,24-pentaazaheptacyclo [27.7.1.1^{4,7}.0^{11,15}.0^{16,21}.0^{20,24}.0^{30,35}]octatriaconta-1(37),4(38),6,11,14,16,18,20,22,29,31 ,33,35-tridecaene-23-carboxylate |
| 3h | 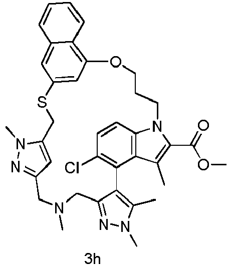 |
| | Methyl 17-chloro-5,9,13,14,22-pentamethyl-28-oxa-2-thia-5,6,9,12,13,24-hexaazaheptacyclo [27.7.1.1^{4,7}.0^{11,15}.0^{16,21}.0^{20,24}.0^{30,35}]octatriaconta-1(37),4(38),6,11,14,16,18,20,22,29,31 ,33,35-tridecaene-23-carboxylate |
| 3h-1 | 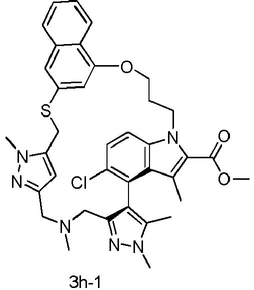 |
| | Methyl (*Ra*)-17-chloro-5,9,13,14,22-pentamethyl-28-oxa-2-thia-5,6,9,12,13,24-hexaazahepta cyclo[27.7.1.1^{4,7}.0^{11,15}.0^{16,21}.0^{20,24}.0^{30,35}]octatriaconta-1(37),4(38),6,11,14,16,18,20,22, 29,31,33,35-tridecaene-23-carboxylate |
| 3h-2 | 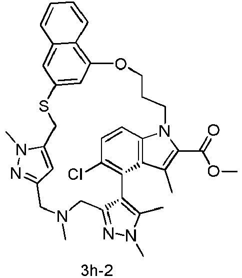 |
| | Methyl (*Sa*)-17-chloro-5,9,13,14,22-pentamethyl-28-oxa-2-thia-5,6,9,12,13,24-hexaazaheptac yclo[27.7.1.1^{4,7}.0^{11,15}.0^{16,21}.0^{20,24}.0^{30,35}]octatriaconta-1(37),4(38),6,11,14,16,18,20,22,2 9,31,33,35-tridecaene-23-carboxylate |
| 4d | 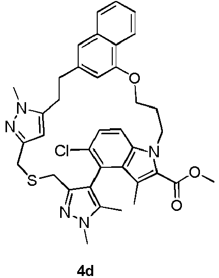 |
| | Methyl 17-chloro-5,13,14,22-tetramethyl-28-oxa-9-thia-5,6,12,13,24-pentaazaheptacyclo[27. 7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylate |
| 4d-1 | 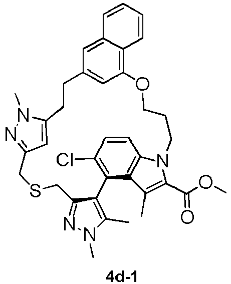 |
| | Methyl (*Ra*)-17-chloro-5,13,14,22-tetramethyl-28-oxa-9-thia-5,6,12,13,24-pentaazaheptacycl o[27.7.1.1^{4,7}.0^{11,15}.0^{16,21}.0^{20,24}.0^{30,35} ]octatriaconta-1(37),4(3 8),6,11,14,16,18,20,22,29,3 1,33,35-tridecaene-23-carboxylate |
| 4d-2 | 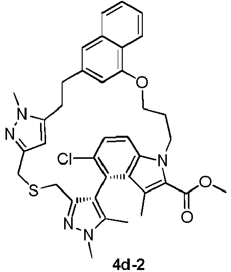 |
| | Methyl (*Sa*)-17-chloro-5,13,14,22-tetramethyl-28-oxa-9-thia-5,6,12,13,24-pentaazaheptacycl o[27.7.1.1^{4,7}.0^{11,15}.0^{16,21}.0^{20,24}.0^{30,35}]octatriaconta-1(37),4(3 8),6,11,14,16,18,20,22,29,3 1,33,35-tridecaene-23-carboxylate |
| 5d | 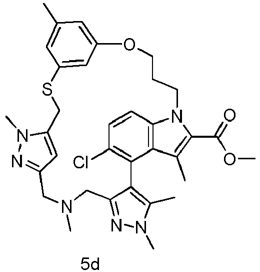 |
| | Methyl 17-chloro-5,9,13,14,22,31-hexamethyl-28-oxa-2-thia-5,6,9,12,13,24-hexaazahexacycl o[27.3.1.1^{4,7}.0^{11,15}.0^{16,21}.0^{20,24}]tetratriaconta-1(33),4(34),6,11,14,16,18,20,22,29,31-un decaene-23-carboxylate |
| 6c | 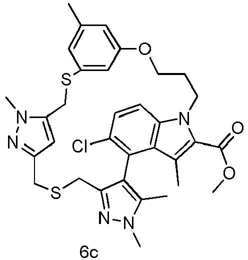 |
| | Methyl 17-chloro-5,13,14,22,31-pentamethyl-28-oxa-2,9-dithia-5,6,12,13,24-pentaazahexacy clo[27.3.1.1^{4,7}.0^{11,15}.0^{16,21}.0^{20,24}]tetratriaconta-1(33),4(34),6,11,14,16,18,20,22,29,31-u ndecaene-23-carboxylate |
| 7n | 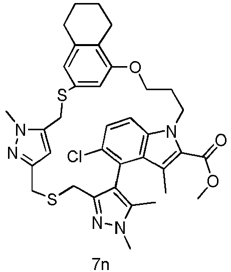 |
| | Methyl 17-chloro-5,13,14,22-tetramethyl-28-oxa-2,9-dithia-5,6,12,13,24-pentaazaheptacyclo[ 27.7.1.1^{4,7}.0^{11,15}.0^{16,21}.0^{20,24}.0^{30,35}]octatriaconta-1(37),4(38),6,11,14,16,18,20,22,29,35-undecaene-23-carboxylate |
| 7n-1 | 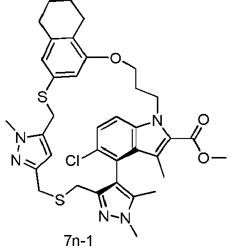 |
| | Methyl (*Ra*)-17-chloro-5,13,14,22-tetramethyl-28-oxa-2,9-dithia-5,6,12,13,24-pentaazahepta cyclo[27.7.1.1^{4,7}.0^{11,15}.0^{16,21}.0^{20,24}.0^{10,15}]octatriaconta-1(37),4(38),6,11,14,16,18,20,22, 29,35-undecaene-23-carboxylate |
| 7n-2 | 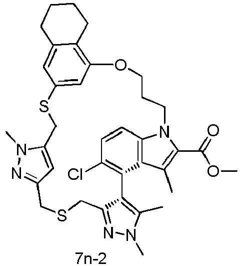 |
| | Methyl (*Sa*)-17-chloro-5,13,14,22-tetramethyl-28-oxa-2,9-dithia-5,6,12,13,24-pentaazaheptac yclo[27.7.1.1^{4,7}.0^{11,15}.0^{16,21}.0^{20,24}.0^{30,35}]octatriaconta-1(37),4(38),6,11,14,16,18,20,22,2 9,35-undecaene-23-carboxylate |
| 8n | 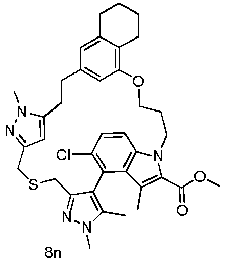 |
| | Methyl 17-chloro-5,13,14,22-tetramethyl-28-oxa-9-thia-5,6,12,13,24-pentaazaheptacyclo[27. 7.1.1^{4,7}.0^{11,15}.0^{16,21}.0^{20,24}.0^{30,35}]octatriaconta-1(37),4(38),6,11,14,16,18,20,22,29,35-un decaene-23-carboxylate |
| 8n-1 | 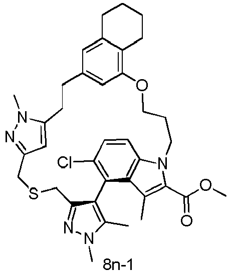 |
| | Methyl (*Ra*)-17-chloro-5,13,14,22-tetramethyl-28-oxa-9-thia-5,6,12,13,24-pentaazaheptacycl o[27.7.1.1^{4,7}.0^{11,15}.0^{16,21}.0^{20,24}.0^{30,35}]octatriaconta-1(37),4(3 8),6,11,14,16,18,20,22,29,3 5-undecaene-23-carboxylate |
| 8n-2 | 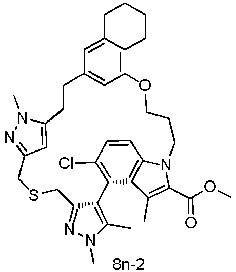 |
| | Methyl (*Sa*)-17-chloro-5,13,14,22-tetramethyl-28-oxa-9-thia-5,6,12,13,24-pentaazaheptacycl o[27.7.1.1^{4,7}.0^{11,15}.0^{16,21}.0^{20,24}.0^{30,35}]octatriaconta-1(37),4(38),6,11,14,16,18,20,22,29,3 5-undecaene-23-carboxylate |
| 9c | 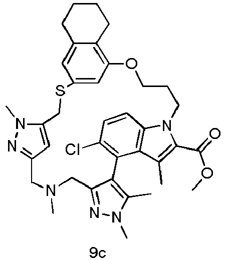 |
| | Methyl 17-chloro-5,9,13,14,22-pentamethyl-28-oxa-2-thia-5,6,9,12,13,24-hexaazaheptacyclo [27.7.1.1^{4,7}.0^{11,15}.0^{16,21}.0^{20,24}.0^{30,35}]octatriaconta-1(37),4(38),6,11,14,16,18,20,22,29,35 -undecaene-23-carboxylate |
| 101 | 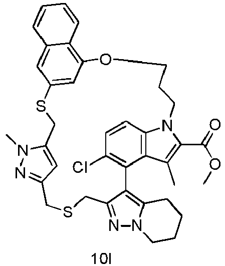 |
| | Methyl 21-chloro-5,26-dimethyl-32-oxa-2,9-dithia-5,6,12,13,28-pentaazaoctacyclo[31.7.1.1^{4, 7}.0^{11,19}.0^{13,18}.0^{20,25}.0^{24,28}.0^{34,39}]dotetraconta-1(41),4(42),6,11,18,20,22,24,26,33,35,37,3 9-tridecaene-27-carboxylate |
| 11f | 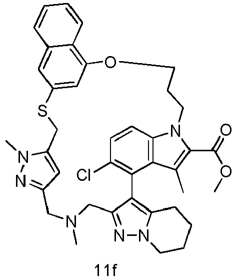 |
| | Methyl 21-chloro-5,9,26-trimethyl-32-oxa-2-thia-5,6,9,12,13,28-hexaazaoctacyclo[31.7. 1.1^{4,7}.0^{11,19}.0^{13,18}.0^{20,25}.0^{24,28}.0^{34,39}]dotetraconta-1(41),4(42),6,11,18,20,22,24,26,33,35, 37,39-tridecaene-27-carboxylate |
| 121 | 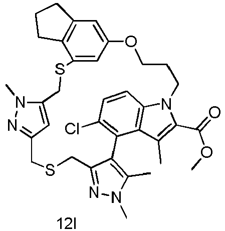 |
| | Methyl 17-chloro-5,13,14,22-tetramethyl-28-oxa-2,9-dithia-5,6,12,13,24-pentaazaheptacyclo[ 27.6.1.1^{1,7}.0^{11,15}.0^{16,21}.0^{20,24}.0^{31,35}]heptatriaconta-1(36),4(37),6,11,14,16,18,20,22,29,3 1(35)-undecaene-23-carboxylate |
| 121-1 | 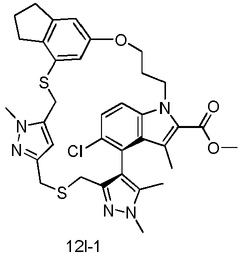 |
| | Methyl (*Ra*)-17-chloro-5,13,14,22-tetramethyl-28-oxa-2,9-dithia-5,6,12,13,24-pentaazahepta cyclo[27.6.1.1^{4,7}.0^{11,15}.0^{16,21}.0^{20,24}.0^{31,35}]heptatriaconta-1(36),4(37),6,11,14,16,18,20,2 2,29,31(35)-undecaene-23-carboxylate |
| 121-2 | 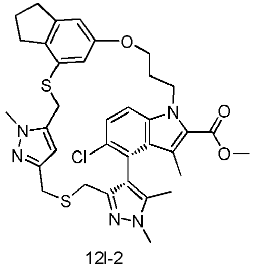 |
| | Methyl (*Sa*)-17-chloro-5,13,14,22-tetramethyl-28-oxa-2,9-dithia-5,6,12,13,24-pentaazaheptac yclo[27.6.1.1^{4,7}.0^{11,15}.0^{16,21}.0^{20,24}.0^{31,35}]heptatriaconta-l(36),4(37),6,11,14,16,18,20,22, 29,31(35)-undecaene-23-carboxylate |
| 13g | 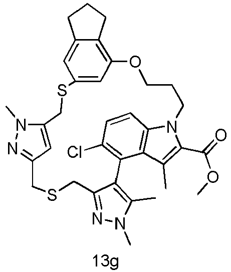 |
| | Methyl 17-chloro-5,13,14,22-tetramethyl-28-oxa-2,9-dithia-5,6,12,13,24-pentaazaheptacyclo[ 27.6.1.1^{1,7}.0^{11,15}.0^{16,21}.0^{20,24}.0^{30,34}]heptatriaconta-1(36),4(37),6,11,14,16,18,20,22,29,3 4-undecaene-23-carboxylate |
| 13g-1 | 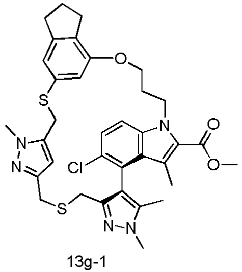 |
| | Methyl (*Ra*)-17-chloro-5,13,14,22-tetramethyl-28-oxa-2,9-dithia-5,6,12,13,24-pentaazahepta cyclo[27.6.1.1^{4,7}.0^{11,15}.0^{16,21}.0^{20,24}.0^{30,34}]heptatriaconta-1(36),4(37),6,11,14,16,18,20,2 2,29,34-undecaene-23-carboxylate |
| 13g-2 | 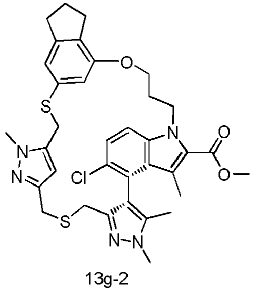 |
| | Methyl (*Sa*)-17-chloro-5,13,14,22-tetramethyl-28-oxa-2,9-dithia-5,6,12,13,24-pentaazaheptac yclo[27.6.1.1^{4,7}.0^{11,15}.0^{16,21}.0^{20,24}.0^{30,34}]heptatriaconta-1(36),4(37),6,11,14,16,18,20,22, 29,34-undecaene-23-carboxylate |
| 14m | 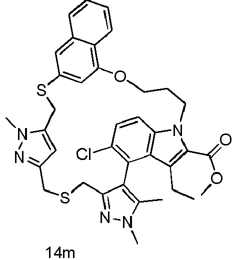 |
| | Methyl 17-chloro-22-ethyl-5,13-14-trimethyl-28-oxa-2,9-dithia-5,6,12,13,24-pentaazaheptac yclo[27.7.1.1^{4,7}.0^{11,15}.0^{16,21}.0^{20,24}.0^{30,35}]octatriaconta-1(37),4(38),6,11,14,16,18,20,22,2 9,31,33,35-tridecaene-23-carboxylate |
| 14m-1 | 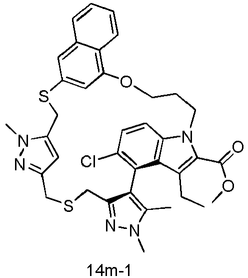 |
| | Methyl (*Ra*)-17-chloro-22-ethyl-5,13-14-trimethyl-28-oxa-2,9-dithia-5,6,12,13,24-pentaazahe ptacyclo[27.7.1.1^{4,7}.0^{11,15}.0^{16,21}.0^{20,24}.0^{10,15}]octatriaconta-1(37),4(38),6,11,14,16,18,20, 22,29,31,33,35-tridecaene-23-carboxylate |
| 14m-2 | 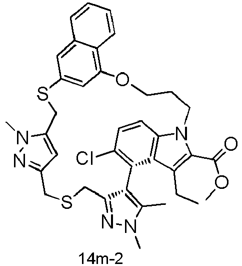 |
| | Methyl (*Sa*)-17-chloro-22-ethyl-5,13-14-trimethyl-28-oxa-2,9-dithia-5,6,12,13,24-pentaazahe ptacyclo[27.7.1.1^{4,7}.0^{11,15}.0^{16,21}.0^{20,24}.0^{30,35}]octatriaconta-1(37),4(38),6,11,14,16,18,20, 22,29,31,33,35-tridecaene-23-carboxylate |

or a tautomer, mesomer, racemate, enantiomer, diastereomer thereof, or mixture thereof,
or a pharmaceutically acceptable salt thereof.

In another aspect, the present invention relates to a method for preparing the compound of formula (IM) or the tautomer, mesomer, racemate, enantiomer, diastereomer thereof, or mixture thereof, or the pharmaceutically acceptable salt thereof according to the present invention, comprising the following step of: 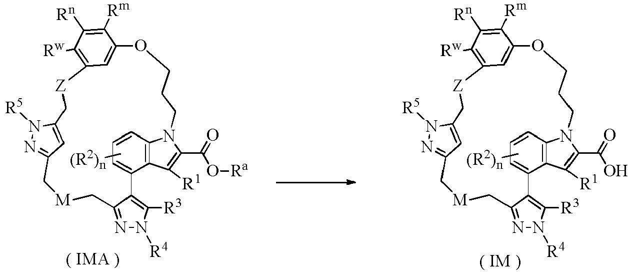 removing the protecting group R^{a} from the compound of formula (IMA) to obtain the compound of formula (IM),
wherein:
R^{a} is an alkyl;
R^{m}, Rⁿ, R^{w}, Z, M, R¹∼R⁵ and n are as defined in formula (IM).

In another aspect, the present invention relates to a method for preparing the compounds of formula (IMA-1) and formula (IMA-2) or the tautomer, mesomer, racemate, enantiomer, diastereomer thereof, or mixture thereof, or the pharmaceutically acceptable salt thereof according to the present invention, comprising the following step: 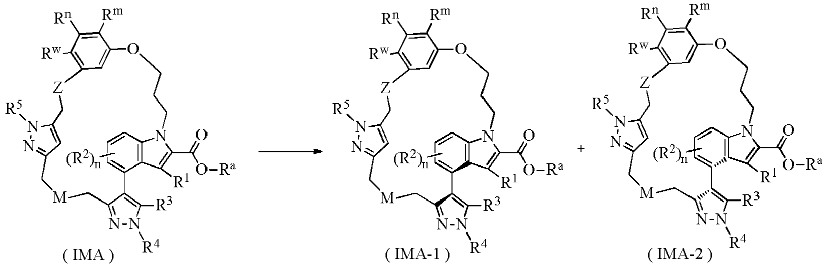 the compound of formula (IMA) is subjected to chiral separation to obtain the compounds of formula (IMA-1) and formula (IMA-2),
wherein:
R^{a}, R^{m}, Rⁿ, R^{w}, Z, M, R¹∼R⁵ and n are as defined in formula (IMA).

In another aspect, the present invention relates to a method for preparing the compound of formula (IM-1) or the tautomer, mesomer, racemate, enantiomer, diastereomer thereof, or mixture thereof, or the pharmaceutically acceptable salt thereof according to the present invention, comprising the following step of: 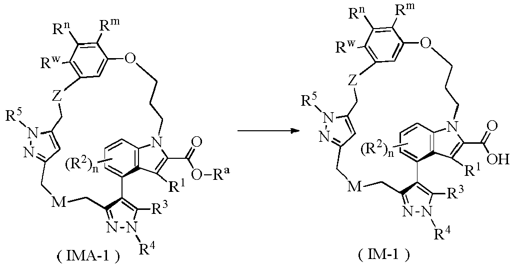 removing the protecting group R^{a} from the compound of formula (IMA-1) to obtain the compound of formula (IM-1), wherein:
R^{a} is an alkyl;
R^{m}, Rⁿ, R^{w}, Z, M, R¹∼R⁵ and n are as defined in formula (IM).

In another aspect, the present invention relates to a method for preparing the compound of formula (IM-2) or the tautomer, mesomer, racemate, enantiomer, diastereomer thereof, or mixture thereof, or the pharmaceutically acceptable salt thereof according to the present invention comprising the following step of: 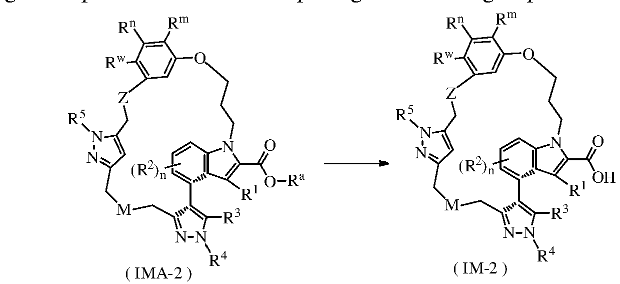 removing the protecting group R^{a} from the compound of formula (IMA-2) to obtain the compound of formula (IM-2),
wherein:
R^{a} is an alkyl;
R^{m}, Rⁿ, R^{w}, Z, M, R¹∼R⁵ and n are as defined in formula (IM).

In another aspect, the present invention relates to a method for preparing the compound of formula (IIM) or the tautomer, mesomer, racemate, enantiomer, diastereomer thereof, or mixture thereof, or the pharmaceutically acceptable salt thereof according to the present invention, comprising the following step of: 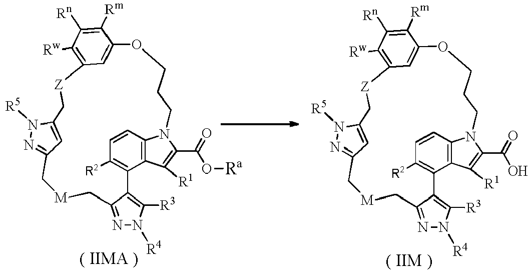 removing the protecting group R^{a} from the compound of formula (IIMA) to obtain the compound of formula (IIM),
wherein:
R^{a} is an alkyl;
R^{m}, Rⁿ, R^{w}, Z, M and R¹∼R⁵ are as defined in formula (IIM).

In another aspect, the present invention relates to a method for preparing the compounds of formula (IIMA-1) and formula (IIMA-2) or the tautomer, mesomer, racemate, enantiomer, diastereomer thereof, or mixture thereof, or the pharmaceutically acceptable salt thereof according to the present invention, comprising the following step : 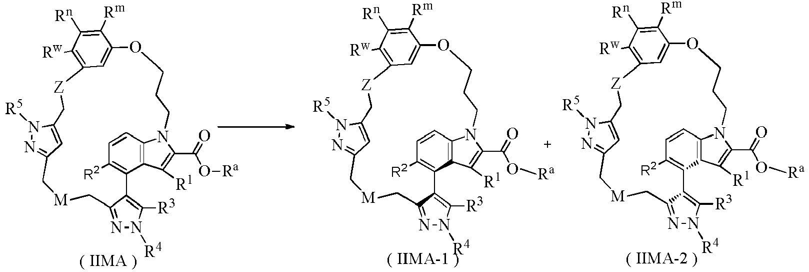 the compound of formula (IIMA) is subjected to a chiral separation to obtain the compounds of formula (IIMA-1) and formula (IIMA-2),
wherein:
R^{a}, R^{m}, Rⁿ, R^{w}, Z, M and R¹∼R⁵ are as defined in formula (IIMA).

In another aspect, the present invention relates to a method for preparing the compound of formula (IIM-1) or the tautomer, mesomer, racemate, enantiomer, diastereomer thereof, or mixture thereof, or the pharmaceutically acceptable salt thereof according to the present invention, comprising the following step of: 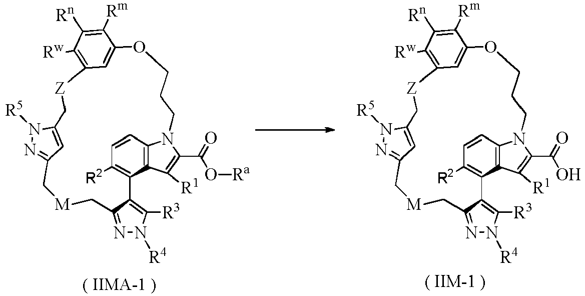 removing the protecting group R^{a} from the compound of formula (IIMA-1) to obtain the compound of formula (IIM-1),
wherein:
R^{a} is an alkyl;
R^{m}, Rⁿ, R^{w}, Z, M and R¹∼R⁵ are as defined in formula (IIM).

In another aspect, the present invention relates to a method for preparing the compound of formula (IIM-2) or the tautomer, mesomer, racemate, enantiomer, diastereomer thereof, or mixture thereof, or the pharmaceutically acceptable salt thereof according to the present invention, comprising the following step of: 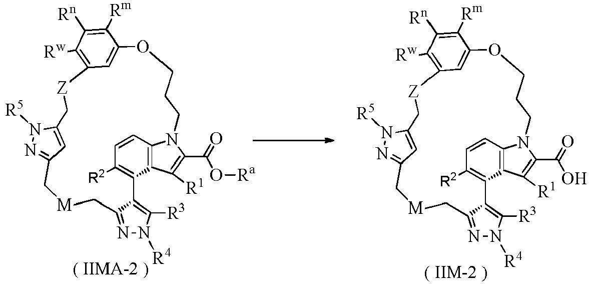 removing the protecting group R^{a} from the compound of formula (IIMA-2) to obtain the compound of formula (IIM-2),
wherein:
R^{a} is an alkyl;
R^{m}, Rⁿ, R^{w}, Z, M and R¹∼R⁵ are as defined in formula (IIM).

In another aspect, the present invention relates to a method for preparing the compound of formula (IK) or the tautomer, mesomer, racemate, enantiomer, diastereomer thereof, or mixture thereof, or the pharmaceutically acceptable salt thereof according to the present invention, comprising the following step of: 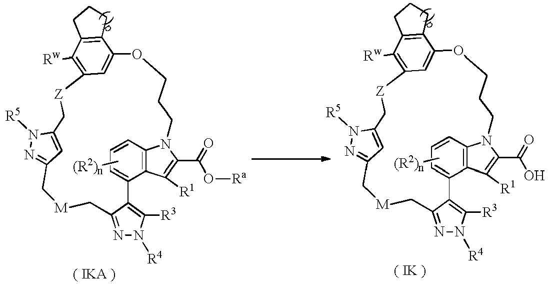 removing the protecting group R^{a} from the compound of formula (IKA) to obtain the compound of formula (IK),
wherein:
R^{a} is an alkyl;
p, R^{w}, Z, M, R¹∼R⁵ and n are as defined in formula (IK).

In another aspect, the present invention relates to a method for preparing the compounds of formula (IKA-1) and formula (IKA-2) or the tautomer, mesomer, racemate, enantiomer, diastereomer thereof, or mixture thereof, or the pharmaceutically acceptable salt thereof according to the present invention, comprising the following step : 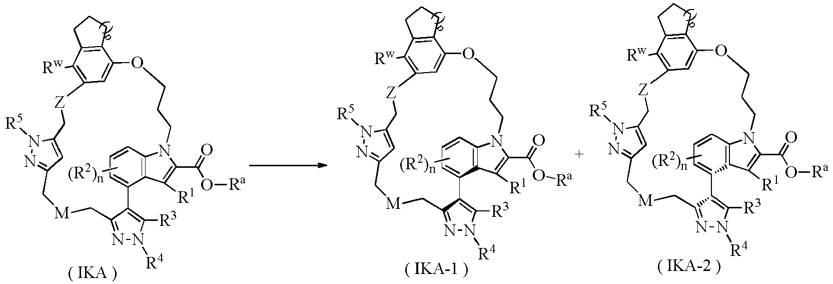 the compound of formula (IKA) is subjected to a chiral separation to obtain the compounds of formula (IKA-1) and formula (IKA-2),
wherein:
R^{a} , p, R^{w}, Z, M, R¹∼R⁵ and n are as defined in formula (IKA).

In another aspect, the present invention relates to a method for preparing the compound of formula (IK-1) or the tautomer, mesomer, racemate, enantiomer, diastereomer thereof, or mixture thereof, or the pharmaceutically acceptable salt thereof according to the present invention, comprising the following step of: 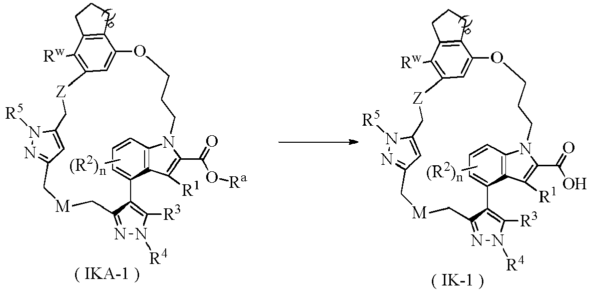 removing the protecting group R^{a} from the compound of formula (IKA-1) to obtain the compound of formula (IK-1),
wherein:
R^{a} is an alkyl;
p, R^{w}, Z, M, R¹∼R⁵ and n are as defined in formula (IK).

In another aspect, the present invention relates to a method for preparing the compound of formula (IK-2) or the tautomer, mesomer, racemate, enantiomer, diastereomer thereof, or mixture thereof, or the pharmaceutically acceptable salt thereof according to the present invention, comprising the following step of: 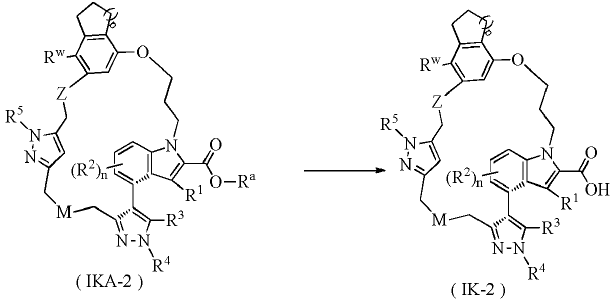 removing the protecting group R^{a} from the compound of formula (IKA-2) to obtain the compound of formula (IK-2),
wherein:
R^{a} is an alkyl;
p, R^{w}, Z, M, R¹∼R⁵ and n are as defined in formula (IK).

In another aspect, the present invention relates to a method for preparing the compound of formula (IL) or the tautomer, mesomer, racemate, enantiomer, diastereomer thereof, or mixture thereof, or the pharmaceutically acceptable salt thereof according to the present invention, comprising the following step of: 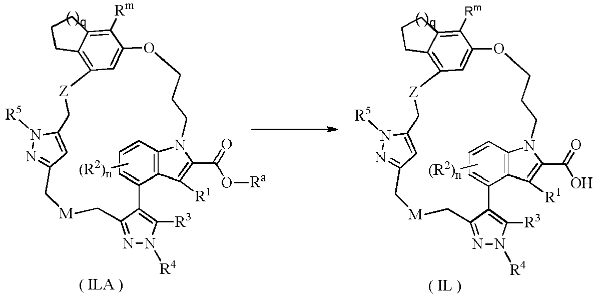 removing the protecting group R^{a} from the compound of formula (ILA) to obtain the compound of formula (IL),
wherein:
R^{a} is an alkyl;
q, R^{m}, Z, M, R¹∼R⁵ and n are as defined in formula (IL).

In another aspect, the present invention relates to a method for preparing the compounds of formula (ILA-1) and formula (ILA-2) or the tautomer, mesomer, racemate, enantiomer, diastereomer thereof, or mixture thereof, or the pharmaceutically acceptable salt thereof according to the present invention, comprising the following step : 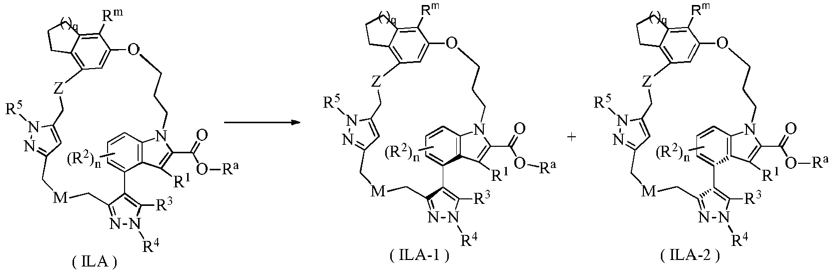 the compound of formula (ILA) is subjected to a chiral separation to obtain the compounds of formula (ILA-1) and formula (ILA-2),
wherein:
R^{a}, q, R^{m}, Z, M, R¹∼R⁵ and n are as defined in formula (ILA).

In another aspect, the present invention relates to a method for preparing the compound of formula (IL-1) or the tautomer, mesomer, racemate, enantiomer, diastereomer thereof, or mixture thereof, or the pharmaceutically acceptable salt thereof according to the present invention, comprising the following step of: 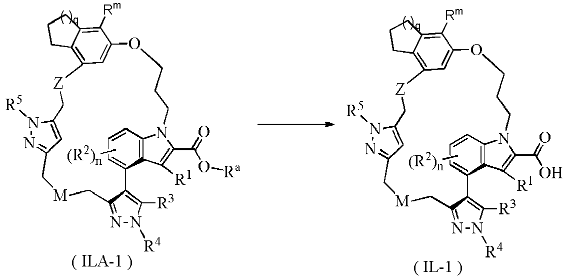 removing the protecting group R^{a} from the compound of formula (ILA-1) to obtain the compound of formula (IL-1),
wherein:
R^{a} is an alkyl;
q, R^{m}, Z, M, R¹∼R⁵ and n are as defined in formula (IL).

In another aspect, the present invention relates to a method for preparing the compound of formula (IL-2) or the tautomer, mesomer, racemate, enantiomer, diastereomer thereof, or mixture thereof, or the pharmaceutically acceptable salt thereof according to the present invention, comprising the following step of: 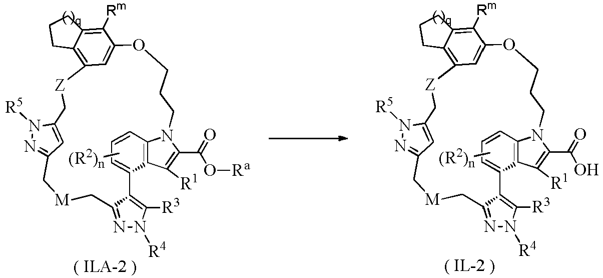 removing the protecting group R^{a} from the compound of formula (ILA-2) to obtain the compound of formula (IL-2),
wherein:
R^{a} is an alkyl;
q, R^{m}, Z, M, R¹∼R⁵ and n are as defined in formula (IL).

In another aspect, the present invention relates to a method for preparing the compound of formula (IIK) or the tautomer, mesomer, racemate, enantiomer, diastereomer thereof, or mixture thereof, or the pharmaceutically acceptable salt thereof according to the present invention, comprising the following step of: 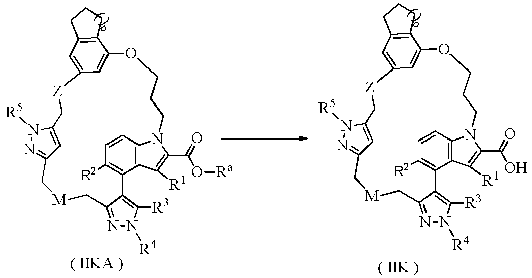 removing the protecting group R^{a} from the compound of formula (IIKA) to obtain the compound of formula (IIK),
wherein:
R^{a} is an alkyl;
p, Z, M and R¹∼R⁵ are as defined in formula (IIK).

In another aspect, the present invention relates to a method for preparing the compounds of formula (IIKA-1) and formula (IIKA-2) or the tautomer, mesomer, racemate, enantiomer, diastereomer thereof, or mixture thereof, or the pharmaceutically acceptable salt thereof according to the present invention, comprising the following step : 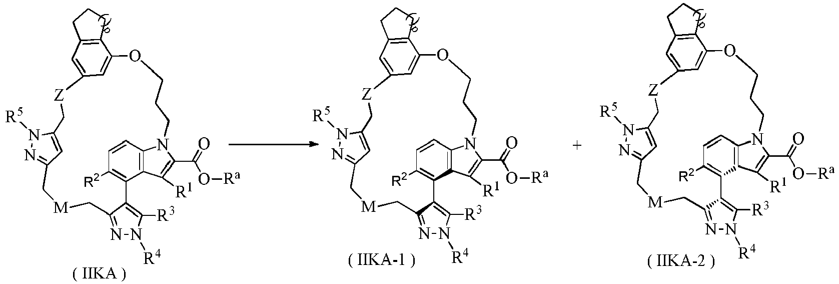 the compound of formula (IIKA) is subjected to a chiral separation to obtain the compounds of formula (IIKA-1) and formula (IIKA-2),
wherein:
R^{a}, p, Z, M and R¹∼R⁵ are as defined in formula (IIKA).

In another aspect, the present invention relates to a method for preparing the compound of formula (IIK-1) or the tautomer, mesomer, racemate, enantiomer, diastereomer thereof, or mixture thereof, or the pharmaceutically acceptable salt thereof according to the present invention, comprising the following step of: 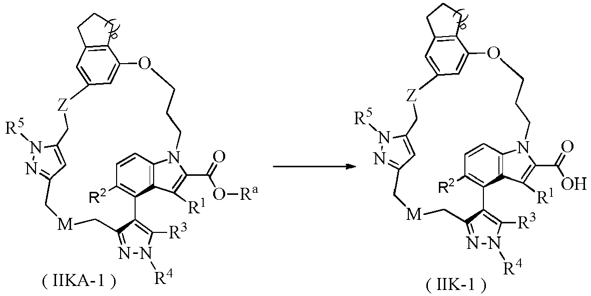 removing the protecting group R^{a} from the compound of formula (IIKA-1) to obtain the compound of formula (IIK-1),
wherein:
R^{a} is an alkyl;
p, Z, M and R¹∼R⁵ are as defined in formula (IIK).

In another aspect, the present invention relates to a method for preparing the compound of formula (IIK-2) or the tautomer, mesomer, racemate, enantiomer, diastereomer thereof, or mixture thereof, or the pharmaceutically acceptable salt thereof according to the present invention, comprising the following step of: 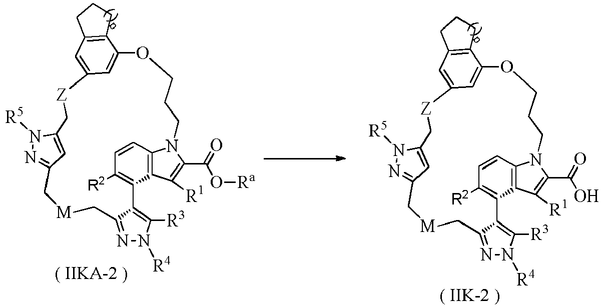 removing the protecting group R^{a} from the compound of formula (IIKA-2) to obtain the compound of formula (IIK-2),
wherein:
R^{a} is an alkyl;
p, Z, M and R¹∼R⁵ are as defined in formula (IIK).

In another aspect, the present invention relates to a method for preparing the compound of formula (IIL) or the tautomer, mesomer, racemate, enantiomer, diastereomer thereof, or mixture thereof, or the pharmaceutically acceptable salt thereof according to the present invention, comprising the following step of: 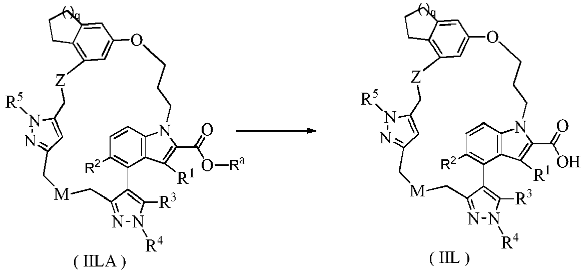 removing the protecting group R^{a} from the compound of formula (IILA) to obtain the compound of formula (IIL),
wherein:
R^{a} is an alkyl;
q, Z, M and R¹∼R⁵ are as defined in formula (IIL).

In another aspect, the present invention relates to a method for preparing the compounds of formula (IILA-1) and formula (IILA-2) or the tautomer, mesomer, racemate, enantiomer, diastereomer thereof, or mixture thereof, or the pharmaceutically acceptable salt thereof according to the present invention, comprising the following step : 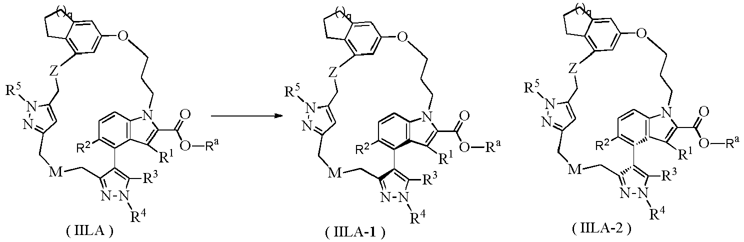 the compound of formula (IILA) is subjected to a chiral separation to obtain the compounds of formula (IILA-1) and formula (IILA-2),
wherein:
R^{a}, q, Z, M and R¹∼R⁵ are as defined in formula (IILA).

In another aspect, the present invention relates to a method for preparing the compound of formula (IIL-1) or the tautomer, mesomer, racemate, enantiomer, diastereomer thereof, or mixture thereof, or the pharmaceutically acceptable salt thereof according to the present invention, comprising the following step of: 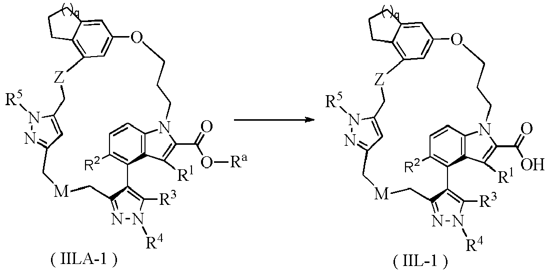 removing the protecting group R^{a} from the compound of formula (IILA-1) to obtain the compound of formula (IIL-1),
wherein:
R^{a} is an alkyl;
q, Z, M and R¹∼R⁵ are as defined in formula (IIL).

In another aspect, the present invention relates to a method for preparing the compound of formula (IIL-2) or the tautomer, mesomer, racemate, enantiomer, diastereomer thereof, or mixture thereof, or the pharmaceutically acceptable salt thereof according to the present invention, comprising the following step of: 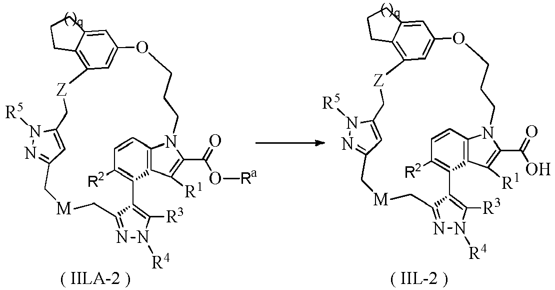 removing the protecting group R^{a} from the compound of formula (IILA-2) to obtain the compound of formula (IIL-2),
wherein:
R^{a} is an alkyl;
q, Z, M and R¹∼R⁵ are as defined in formula (IIL).

In another aspect, the present invention relates to a method for preparing the compound of formula (I) or the tautomer, mesomer, racemate, enantiomer, diastereomer thereof, or mixture thereof, or the pharmaceutically acceptable salt thereof according to the present invention, comprising the following step of: 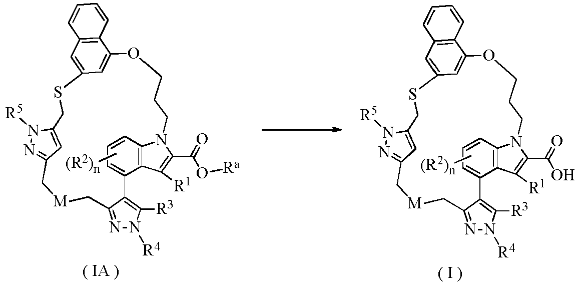 removing the protecting group R^{a} from the compound of formula (IA) to obtain the compound of formula (I),
wherein:
R^{a} is an alkyl;
M, R¹∼R⁵ and n are as defined in formula (I).

In another aspect, the present invention relates to a method for preparing the compounds of formula (IA-1) and formula (IA-2) or the tautomer, mesomer, racemate, enantiomer, diastereomer thereof, or mixture thereof, or the pharmaceutically acceptable salt thereof according to the present invention, comprising the following step: 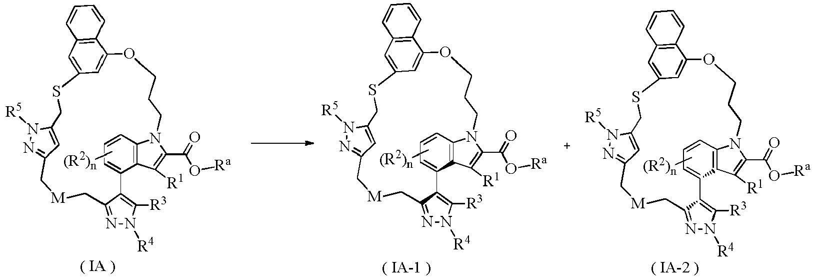 the compound of formula (IA) is subjected to a chiral separation to obtain the compounds of formula (IA-1) and formula (IA-2),
wherein:
R^{a}, M, R¹∼R⁵ and n are as defined in formula (IA).

In another aspect, the present invention relates to a method for preparing the compound of formula (I-1) or the tautomer, mesomer, racemate, enantiomer, diastereomer thereof, or mixture thereof, or the pharmaceutically acceptable salt thereof according to the present invention, comprising the following step of: 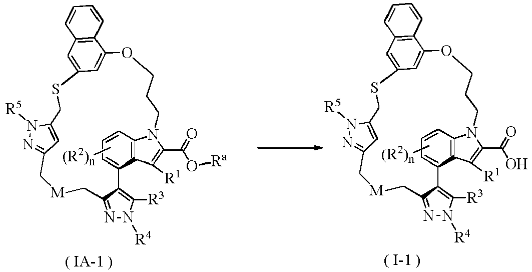 removing the protecting group R^{a} from the compound of formula (IA-1) to obtain the compound of formula (I-1),
wherein:
R^{a} is an alkyl;
M, R¹∼R⁵ and n are as defined in formula (I).

In another aspect, the present invention relates to a method for preparing the compound of formula (1-2) or the tautomer, mesomer, racemate, enantiomer, diastereomer thereof, or mixture thereof, or the pharmaceutically acceptable salt thereof according to the present invention, comprising the following step of: 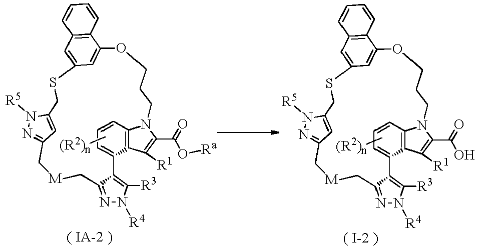 removing the protecting group R^{a} from the compound of formula (IA-2) to obtain the compound of formula (1-2),
wherein:
R^{a} is an alkyl;
M, R¹∼R⁵ and n are as defined in formula (I).

In another aspect, the present invention relates to a method for preparing the compound of formula (II) or the tautomer, mesomer, racemate, enantiomer, diastereomer thereof, or mixture thereof, or the pharmaceutically acceptable salt thereof according to the present invention, comprising the following step of: 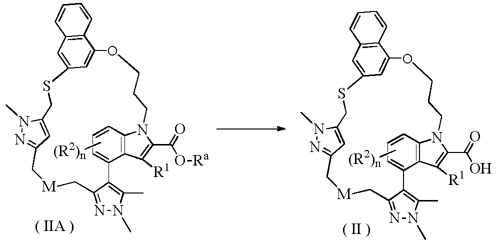 removing the protecting group R^{a} from the compound of formula (IIA) to obtain the compound of formula (II),
wherein:
R^{a} is an alkyl;
M, R¹, R² and n are as defined in formula (II).

In another aspect, the present invention relates to a method for preparing the compound of formula (III) or the tautomer, mesomer, racemate, enantiomer, diastereomer thereof, or mixture thereof, or the pharmaceutically acceptable salt thereof according to the present invention, comprising the following step of: 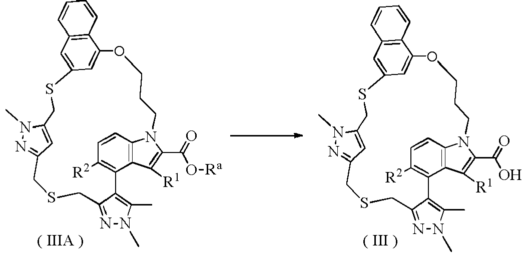 removing the protecting group R^{a} from the compound of formula (IIIA) to obtain the compound of formula (III),
wherein:
R^{a} is an alkyl;
R¹ and R² are as defined in formula (III).

In another aspect, the present invention relates to a pharmaceutical composition comprising the compound of formula (IM), or the tautomer, mesomer, racemate, enantiomer, diastereomer thereof, or mixture thereof, or the pharmaceutically acceptable salt thereof according to the present invention, and one or more pharmaceutically acceptable carrier(s), diluent(s) or excipient(s).

The present invention further relates to a use of the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention in the preparation of a medicament for inhibiting MCL-1.

The present invention further relates to a use of the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention in the preparation of a medicament for the prevention or treatment of MCL-1 mediated diseases.

The present invention further relates to a use of the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention in the preparation of a medicament for the treatment of tumors, autoimmune diseases or immune system diseases, wherein the tumor is preferably selected from the group consisting of bladder cancer, brain tumor, breast cancer, uterine cancer, cervical cancer, endometrial cancer, ovarian cancer, leukemia (such as chronic myelogenous leukemia, chronic lymphocytic leukemia, lymphoblastic leukemia or acute myeloid leukemia), kidney cancer, colon cancer, rectal cancer, colorectal cancer, esophageal cancer, liver cancer, stomach cancer, head and neck cancer, skin cancer, lymphoma, pancreatic cancer, melanoma, myeloma (such as multiple myeloma), bone cancer, neuroblastoma, glioma, sarcoma, lung cancer (such as non-small cell lung cancer or small cell lung cancer), thyroid cancer and prostate cancer.

The present invention also relates to a method for inhibiting MCL-1, comprising a step of administrating to a patient in need thereof a therapeutically effective dose of the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention.

The present invention also relates to a method for preventing or treating MCL-1 mediated diseases, comprising a step of administrating to a patient in need thereof a preventively or therapeutically effective dose of the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention.

The present invention also relates to a method for treating tumors, autoimmune diseases or immune system diseases, comprising a step of administrating to a patient in need thereof a therapeutically effective dose of the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention, wherein the tumor is preferably selected from the group consisting of bladder cancer, brain tumor, breast cancer, uterine cancer, cervical cancer, endometrial cancer, ovarian cancer, leukemia (such as chronic myelogenous leukemia, chronic lymphocytic leukemia, lymphoblastic leukemia or acute myeloid leukemia), kidney cancer, colon cancer, rectal cancer, colorectal cancer, esophageal cancer, liver cancer, stomach cancer, head and neck cancer, skin cancer, lymphoma, pancreatic cancer, melanoma, myeloma (such as multiple myeloma), bone cancer, neuroblastoma, glioma, sarcoma, lung cancer (such as non-small cell lung cancer or small cell lung cancer), thyroid cancer and prostate cancer.

The present invention further relates to the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention, for use as a medicament.

The present invention also relates to the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention, for use as a MCL-1 inhibitor.

The present invention also relates to the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention, for use as a medicament for treating or preventing MCL-1 mediated diseases.

The present invention also relates to the compound of formula (IM), or the tautomer, mesomer, racemate, enantiomer, diastereomer thereof, or mixture thereof, or the pharmaceutically acceptable salt thereof, or the pharmaceutical composition comprising the same according to the present invention, for use as a medicament for treating tumors, autoimmune diseases or immune system diseases, wherein the tumor is preferably selected from the group consisting of bladder cancer, brain tumor, breast cancer, uterine cancer, cervical cancer, endometrial cancer, ovarian cancer, leukemia (such as chronic myelogenous leukemia, chronic lymphocytic leukemia, lymphoblastic leukemia or acute myeloid leukemia), kidney cancer, colon cancer, rectal cancer, colorectal cancer, esophageal cancer, liver cancer, stomach cancer, head and neck cancer, skin cancer, lymphoma, pancreatic cancer, melanoma, myeloma (such as multiple myeloma), bone cancer, neuroblastoma, glioma, sarcoma, lung cancer (such as non-small cell lung cancer or small cell lung cancer), thyroid cancer and prostate cancer.

The active compound can be formulated into a form suitable for administration by any appropriate route, and the active compound is preferably in the form of a unit dose, or in a form in which the patient can self-administer in a single dose. The form of the unit dose of the compound or composition of the present invention can be tablet, capsule, cachet, bottled potion, powder, granule, lozenge, suppository, regenerating powder or liquid preparation.

The dosage of the compound or composition used in the treatment method of the present invention will generally vary according to the severity of the disease, the weight of the patient, and the relative efficacy of the compound. However, as a general guide, a suitable unit dose can be 0.1 to 1000 mg.

In addition to the active compound, the pharmaceutical composition of the present invention can also comprise one or more auxiliaries including filler (diluent), binder, wetting agent, disintegrant, excipient and the like. Depending on the administration mode, the composition can comprise 0.1 to 99% by weight of the active compound.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such composition can also comprise one or more components selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the manufacture of tablets.

An aqueous suspension comprises an active ingredient in admixture with excipients suitable for the manufacture of an aqueous suspension. The aqueous suspension can also comprise one or more preservative(s) such as ethyl paraben or n-propyl paraben, one or more colorant(s), one or more flavoring agent(s), and one or more sweetener(s).

An oil suspension can be formulated by suspending the active ingredient in a vegetable oil. The oil suspension can comprise a thickener. The aforementioned sweeteners and flavoring agents can be added to provide a palatable formulation.

The dispersible powders or granules suitable for the preparation of an aqueous suspension can provide the active ingredient in admixture with the dispersants or wetting agents, suspending agent or one or more preservatives by adding water. Suitable dispersants or wetting agents and suspending agents are exemplified by those already mentioned above. Additional excipients, such as sweeteners, flavoring agents and colorants, can also be added. These compositions can be preserved by adding an antioxidant, such as ascorbic acid.

The pharmaceutical composition of the present invention can also be in the form of an oil-in-water emulsion.

The pharmaceutical composition can be in the form of a sterile injectable aqueous solution. Acceptable vehicles or solvents that can be used are water, Ringer's solution or isotonic sodium chloride solution. The sterile injectable formulation can be a sterile injectable oil-in-water micro-emulsion in which the active ingredient is dissolved in an oil phase. For example, the active ingredient is dissolved in a mixture of soybean oil and lecithin. The oil solution is then added to a mixture of water and glycerin, and processed to form a micro-emulsion. The injectable solution or micro-emulsion can be introduced into a patient's bloodstream by local bolus injection. Alternatively, the solution and micro-emulsion are preferably administrated in a manner that maintains a constant circulating concentration of the compound of the present invention. In order to maintain this constant concentration, a continuous intravenous delivery device can be used. An example of such a device is Deltec CADD-PLUS. TM. 5400 intravenous injection pump.

The pharmaceutical composition can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent. Moreover, sterile fixed oils can easily be used as a solvent or suspending medium.

The compound of the present invention can be administrated in the form of a suppository for rectal administration. These pharmaceutical compositions can be prepared by mixing the drug with a suitable non-irritating excipient that is solid at ordinary temperatures, but liquid in the rectum, thereby melting in the rectum to release the drug. Such materials include cocoa butter, glycerin gelatin, hydrogenated vegetable oil, a mixture of polyethylene glycols of various molecular weights and fatty acid esters thereof.

It is well known to those skilled in the art that the dosage of a drug depends on a variety of factors including, but not limited to the following factors: activity of a specific compound, age of the patient, weight of the patient, general health of the patient, behavior of the patient, diet of the patient, administration time, administration route, excretion rate, drug combination and the like. In addition, the optimal treatment, such as treatment mode, daily dose of the compound of formula (IM) or the type of pharmaceutically acceptable salt thereof can be verified according to traditional therapeutic regimens.

### Definition

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently optionally selected from the group consisting of H atom, D atom, halogen, alkyl, alkoxy, haloalkyl, hydroxy, hydroxyalkyl, cyano, amino, nitro, cycloalkyl, heterocyclyl, aryl and heteroaryl.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more groups independently selected from the group consisting of H atom, D atom, halogen, alkyl, alkoxy, haloalkyl, hydroxy, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent group having 3 to 20 carbon atoms, preferably 3 to 12 carbon atoms, preferably 3 to 8 carbon atoms, and more preferably 4 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably a 6 to 14 membered spiro cycloalkyl, and more preferably a 7 to 10 membered spiro cycloalkyl (such as 7, 8, 9 or 10 membered spiro cycloalkyl). According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5 -membered, 4-membered/6-membered, 5-membered/5-membered, or 5-membered/6-membered mono-spiro cycloalkyl. Non-limiting examples of spiro cycloalkyl include:

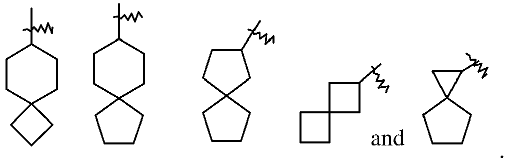

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

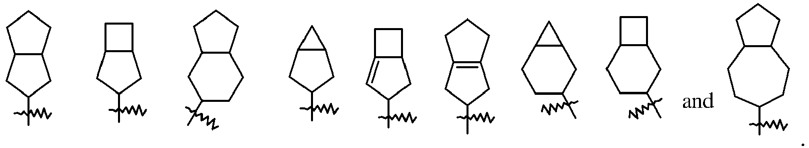

The term "bridged cycloalkyl" refers to a 5 to 20 membered all-carbon polycyclic group, wherein every two rings in the system share two disconnected carbon atoms, the rings can have one or more double bonds, but none of the rings has a completely conjugated π-electron system. The bridged cycloalkyl is preferably a 6 to 14 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

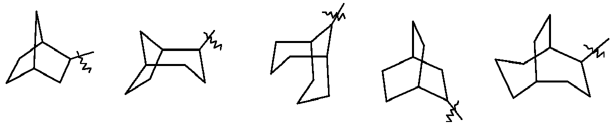

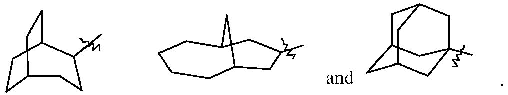

The cycloalkyl (including monocycloalkyl, spiro cycloalkyl, fused cycloalkyl and bridged cycloalkyl) ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like, and preferably benzocyclopentyl, tetrahydronaphthyl.

The cycloalkyl can be substituted or unsubstituted. When substituted, the substituent group(s) can be substituted at any available connection point. The substituent group(s) is preferably one or more groups independently optionally selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably 3 to 8 ring atoms wherein 1 to 3 atoms are heteroatoms; more preferably 3 to 6 ring atoms wherein 1 to 3 atoms are heteroatoms; and most preferably 5 or 6 ring atoms wherein 1 to 3 atoms are heteroatoms. Non-limiting examples of monocyclic heterocyclyl include pyrrolidinyl, tetrahydropyranyl, 1,2,3,6-tetrahydropyridyl, piperidinyl, piperazinyl, morpholinyl, thiomorpholinyl, homopiperazinyl and the like. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, where the rings can contain one or more double bonds, but none of the rings has a completely conjugated π-electron system. The spiro heterocyclyl is preferably a 6 to 14 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5 -membered, 4-membered/6-membered, 5-membered/5-membered, or 5-membered/6-membered mono-spiro heterocyclyl. Non-limiting examples of spiro heterocyclyl include:

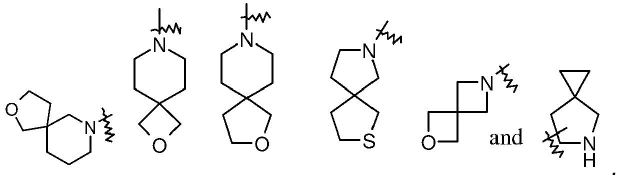

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and the fused heterocyclyl is preferably a bicyclic or tricyclic fused heterocyclyl, and more preferably a 5-membered/5-membered or 5-membered/6-membered bicyclic fused heterocyclyl. Non-limiting examples of fused heterocyclyl include:

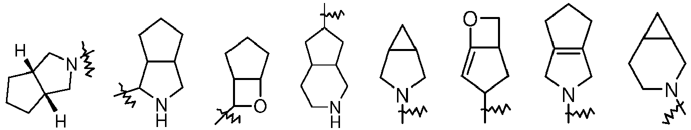

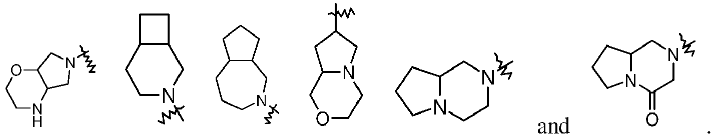

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably a 6 to 14 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

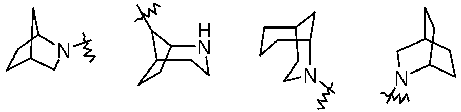

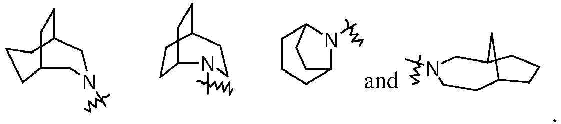

The heterocyclyl (including monocyclic heterocyclyl, spiro heterocyclyl, fused heterocyclyl and bridged heterocyclyl) ring can be fused to the ring of aryl, heteroaryl or cycloalkyl, wherein the ring bound to the parent structure is heterocyclyl. Non-limiting examples thereof include: 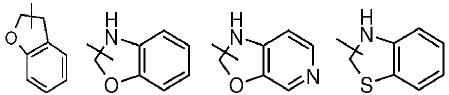 and the like.

The heterocyclyl can be substituted or unsubstituted. When substituted, the substituent group(s) can be substituted at any available connection point. The substituent group(s) is preferably one or more groups independently optionally selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "aryl" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl ring can be fused to the ring of heteroaryl, heterocyclyl or cycloalkyl, wherein the ring bound to the parent structure is aryl ring. Non-limiting examples thereof include:

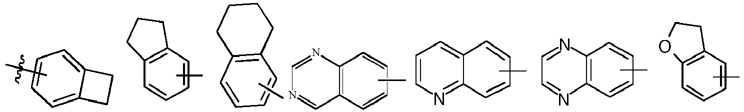

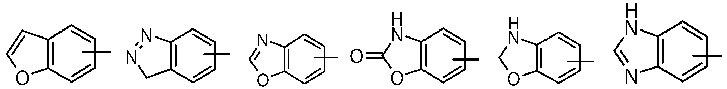

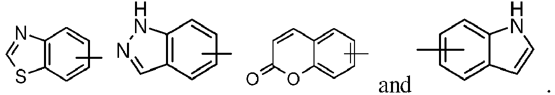

The aryl can be substituted or unsubstituted. When substituted, the substituent group(s) can be substituted at any available connection point. The substituent group(s) is preferably one or more groups independently optionally selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "heteroaryl" refers to a 5 to 14 membered heteroaromatic system having 1 to 4 heteroatoms selected from the group consisting of O, S and N. The heteroaryl is preferably a 5 to 10 membered heteroaryl, more preferably a 5 or 6 membered heteroaryl, for example furyl, thienyl, pyridyl, pyrrolyl, N-alkylpyrrolyl, pyrimidinyl, pyrazinyl, pyridazinyl, imidazolyl, pyrazolyl, triazolyl, tetrazolyl and the like. The heteroaryl ring can be fused to the ring of aryl, heterocyclyl or cycloalkyl, wherein the ring bound to the parent structure is heteroaryl ring. Non-limiting examples thereof include:

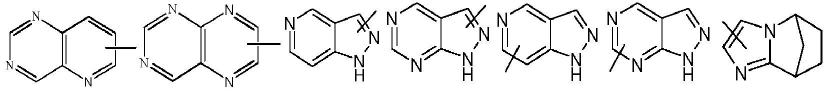

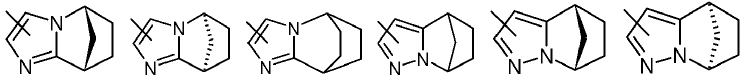

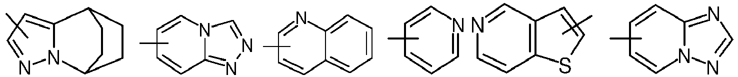

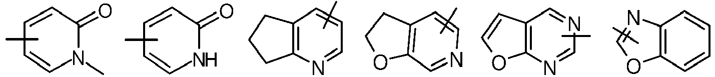

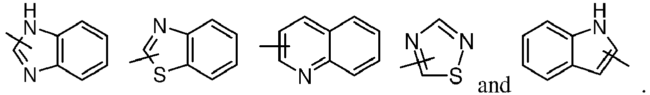

The heteroaryl can be substituted or unsubstituted. When substituted, the substituent group(s) can be substituted at any available connection point. The substituent group(s) is preferably one or more groups independently optionally selected from the group consisting of hydrogen atom, halogen, alkyl, alkoxy, haloalkyl, hydroxy, hydroxyalkyl, cyano, amino, nitro, cycloalkyl, heterocyclyl, aryl and heteroaryl.

The term "cycloalkyloxy" refers to a cycloalkyl-O- group, wherein the cycloalkyl is as defined above.

The term "haloalkyl" refers to an alkyl group substituted by one or more halogen(s), wherein the alkyl is as defined above.

The term "deuterated alkyl" refers to an alkyl group substituted by one or more deuterium atom(s), wherein the alkyl is as defined above.

The term "hydroxy" refers to an -OH group.

The term "hydroxyalkyl" refers to an alkyl group substituted by hydroxy(s), wherein the alkyl is as defined above.

The term "halogen" refers to fluorine, chlorine, bromine or iodine.

The term "hydroxy" refers to an -OH group.

The term "amino" refers to a -NH₂ group.

The term "cyano" refers to a -CN group.

The term "nitro" refers to a -NO₂ group.

The term "carbonyl" refers to a C=O group.

The term "carboxy" refers to a -C(O)OH group.

The term "alkoxycarbonyl" refers to a -C(O)O(alkyl) or -C(O)O(cycloalkyl) group, wherein the alkyl and cycloalkyl are as defined above.

The present invention also comprises the compounds of formula (IM) in various deuterated forms. Each of the available hydrogen atoms attached to the carbon atom can be independently replaced by a deuterium atom. Those skilled in the art can synthesize a compound of formula (IM) in a deuterated form with reference to the relevant literatures. The compound of formula (IM) in deuterated form can be prepared by employing commercially available deuterated raw materials, or they can be synthesized by conventional techniques with deuterated reagents including, but not limited to, deuterated borane, trideuterated borane in tetrahydrofuran, deuterated lithium aluminum hydride, deuterated iodoethane, deuterated iodomethane and the like.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

The term "pharmaceutical composition" refers to a mixture of one or more of the compounds described herein or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

A "pharmaceutically acceptable salt" refers to a salt of the compound of the present invention, which is safe and effective in mammals and has the desired biological activity.

### Synthesis Method of the Compounds of the Present Invention

In order to achieve the object of the present invention, the present invention applies the following schemes:

### Scheme I

A method for preparing the compound of formula (IM) or a tautomer, mesomer, racemate, enantiomer, diastereomer thereof, or mixture thereof, or a pharmaceutically acceptable salt thereof according to the present invention, comprises the following step of: 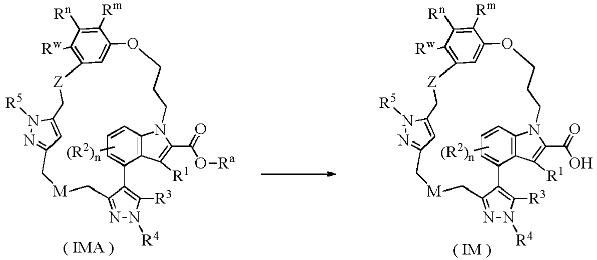 removing the protecting group R^{a} from the compound of formula (IMA) under an alkaline condition to obtain the compound of formula (IM),
wherein:
R^{a} is an alkyl;
R^{m}, Rⁿ, R^{w}, Z, M, R¹∼R⁵ and n are as defined in formula (IM).

### Scheme II

A method for preparing the compound of formula (IM-1) or (IM-2) according to the present invention, comprises the following steps: 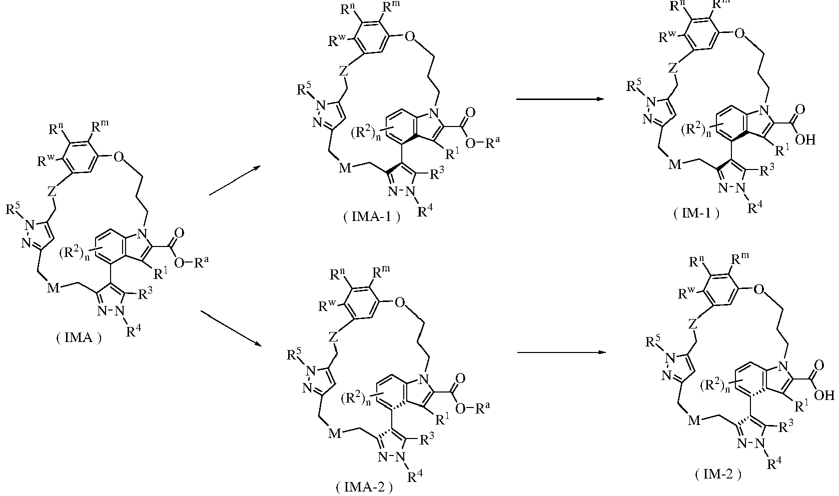 the compound of formula (IMA) is subjected to a chiral preparation to obtain the compounds of formula (IMA-1) and formula (IMA-2),
the protecting group R^{a} is removed from the compound of formula (IMA-1) under an alkaline condition to obtain the compound of formula (IM-1); and the protecting group R^{a} is removed from the compound of formula (IMA-2) under an alkaline condition to obtain the compound of formula (IM-2);
wherein:
R^{a} is an alkyl;
R^{m}, Rⁿ, R^{w}, Z, M, R¹∼R⁵ and n are as defined in formula (IM).

### Scheme III

A method for preparing the compound of formula (IIM) or a tautomer, mesomer, racemate, enantiomer, diastereomer thereof, or mixture thereof, or a pharmaceutically acceptable salt thereof according to the present invention, comprises the following step of: 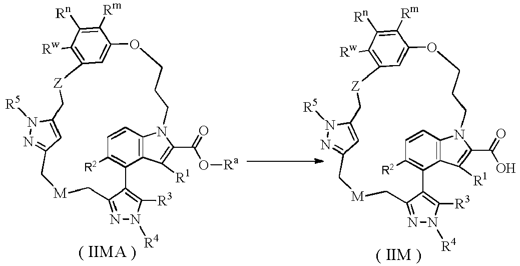 removing the protecting group R^{a} from the compound of formula (IIMA) under an alkaline condition to obtain the compound of formula (IIM),
wherein:
R^{a} is an alkyl;
R^{m}, Rⁿ, R^{w}, Z, M and R¹∼R⁵ are as defined in formula (IIM).

### Scheme IV

A method for preparing the compound of formula (IIM-1) or (IIM-2) according to the present invention, comprises the following steps: 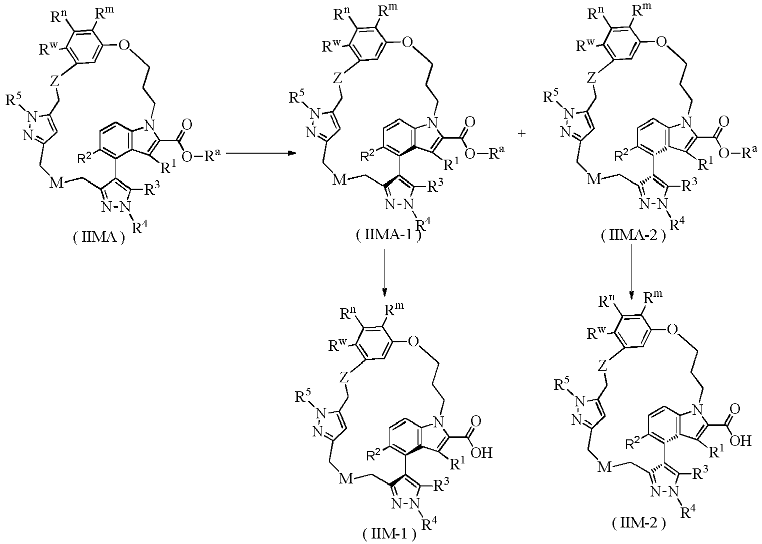 the compound of formula (IIMA) is subjected to a chiral preparation to obtain the compounds of formula (IIMA-1) and formula (IIMA-2);
the protecting group R^{a} is removed from the compound of formula (IIMA-1) under an alkaline condition to obtain the compound of formula (IIM-1); and the protecting group R^{a} is removed from the compound of formula (IIMA-2) under an alkaline condition to obtain the compound of formula (IIM-2);
wherein:
R^{a} is an alkyl;
R^{m}, Rⁿ, R^{w}, Z, M and R¹∼R⁵ are as defined in formula (IIM).

### Scheme V

A method for preparing the compound of formula (IK) or a tautomer, mesomer, racemate, enantiomer, diastereomer thereof, or mixture thereof, or a pharmaceutically acceptable salt thereof according to the present invention, comprises the following step of: 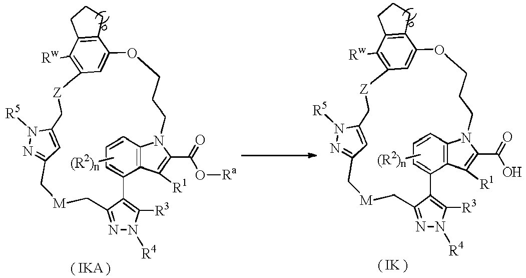 removing the protecting group R^{a} from the compound of formula (IKA) under an alkaline condition to obtain the compound of formula (IK),
wherein:
R^{a} is an alkyl;
p, R^{w}, Z, M, R¹∼R⁵ and n are as defined in formula (IK).

### Scheme VI

A method for preparing the compound of formula (IK-1) or (IK-2) according to the present invention, comprises the following steps: 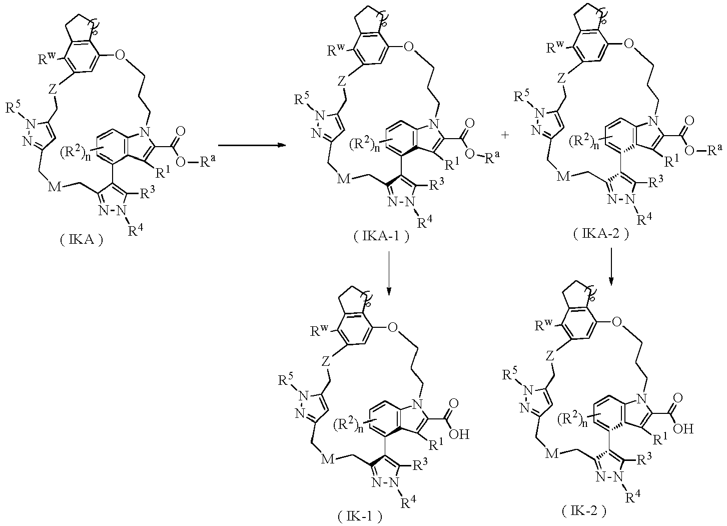 the compound of formula (IKA) is subjected to a chiral preparation to obtain the compounds of formula (IKA-1) and formula (IKA-2);
the protecting group R^{a} is removed from the compound of formula (IKA-1) under an alkaline condition to obtain the compound of formula (IK-1); and the protecting group R^{a} is removed from the compound of formula (IKA-2) under an alkaline condition to obtain the compound of formula (IK-2);
wherein:
R^{a} is an alkyl;
p, R^{w}, Z, M, R¹∼R⁵ and n are as defined in formula (IK).

### Scheme VII

A method for preparing the compound of formula (IL) or a tautomer, mesomer, racemate, enantiomer, diastereomer thereof, or mixture thereof, or a pharmaceutically acceptable salt thereof according to the present invention, comprises the following step of: 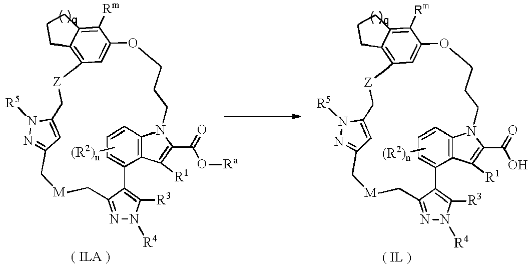 removing the protecting group R^{a} from the compound of formula (ILA) under an alkaline condition to obtain the compound of formula (IL),
wherein:
R^{a} is an alkyl;
q, R^{m}, Z, M, R¹∼R⁵ and n are as defined in formula (IL).

### Scheme VIII

A method for preparing the compound of formula (IL-1) or (IL-2) according to the present invention, comprises the following steps: 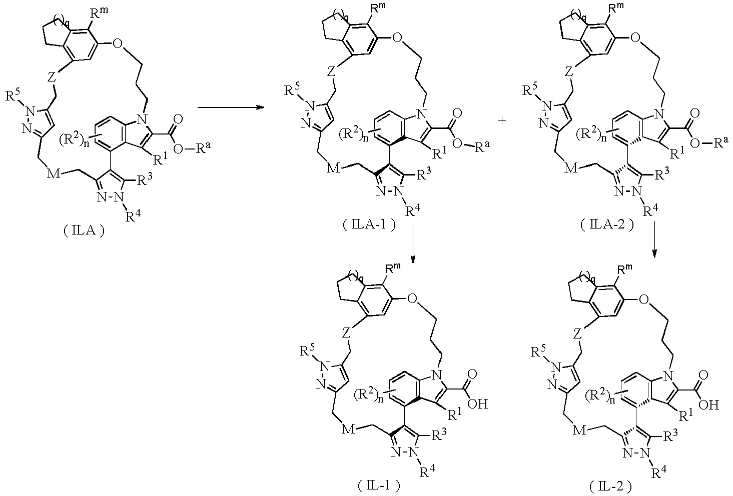 the compound of formula (ILA) is subjected to a chiral preparation to obtain the compounds of formula (ILA-1) and formula (ILA-2);
the protecting group R^{a} is removed from the compound of formula (ILA-1) under an alkaline condition to obtain the compound of formula (IL-1); and the protecting group R^{a} is removed from the compound of formula (ILA-2) under an alkaline condition to obtain the compound of formula (IL-2);
wherein:
R^{a} is an alkyl;
q, R^{m}, Z, M, R¹∼R⁵ and n are as defined in formula (IL).

### Scheme IX

A method for preparing the compound of formula (IIK) or a tautomer, mesomer, racemate, enantiomer, diastereomer thereof, or mixture thereof, or a pharmaceutically acceptable salt thereof according to the present invention, comprises the following step of: 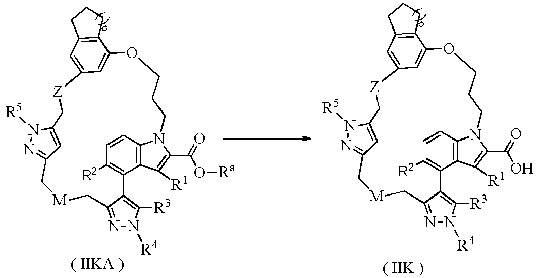 removing the protecting group R^{a} from the compound of formula (IIKA) under an alkaline condition to obtain the compound of formula (IIK),
wherein:
R^{a} is an alkyl;
p, Z, M and R¹∼R⁵ are as defined in formula (IIK).

### Scheme X

A method for preparing the compound of formula (IIK-1) or (IIK-2) according to the present invention, comprises the following steps: 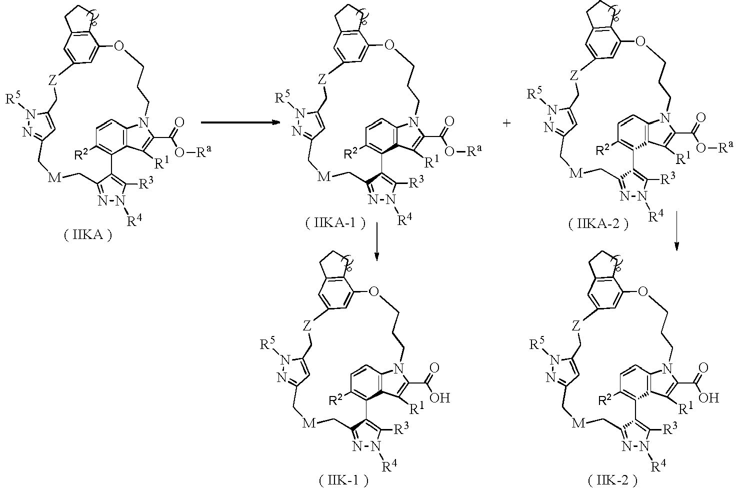 the compound of formula (IIKA) is subjected to a chiral preparationg to obtain the compounds of formula (IIKA-1) and formula (IIKA-2);
the protecting group R^{a} is removed from the compound of formula (IIKA-1) under an alkaline condition to obtain the compound of formula (IIK-1); and the protecting group R^{a} is removed from the compound of formula (IIKA-2) under an alkaline condition to obtain the compound of formula (IIK-2);
wherein:
R^{a} is an alkyl;
p, Z, M and R¹∼R⁵ are as defined in formula (IIK).

### Scheme XI

A method for preparing the compound of formula (IIL) or a tautomer, mesomer, racemate, enantiomer, diastereomer thereof, or mixture thereof, or a pharmaceutically acceptable salt thereof according to the present invention, comprises the following step of: 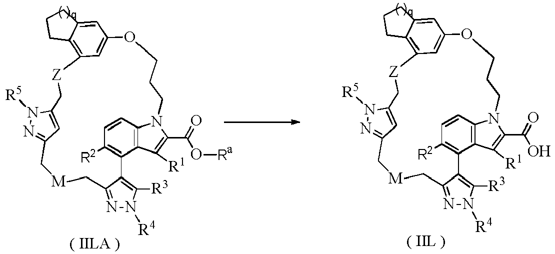 removing the protecting group R^{a} from the compound of formula (IILA) under an alkaline condition to obtain the compound of formula (IIL),
wherein:
R^{a} is an alkyl;
q, Z, M and R¹∼R⁵ are as defined in formula (IIL).

### Scheme XII

A method for preparing the compound of formula (IIL-1) or (IIL-2) according to the present invention, comprises the following steps: 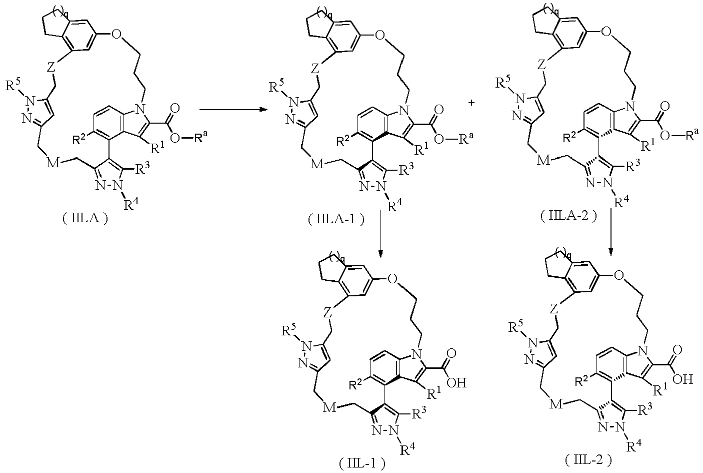 the compound of formula (IILA) is subjected to a chiral preparation to obtain the compounds of formula (IILA-1) and formula (IILA-2);
the protecting group R^{a} is removed from the compound of formula (IILA-1) under an alkaline condition to obtain the compound of formula (IIL-1); and the protecting group R^{a} is removed from the compound of formula (IILA-2) under an alkaline condition to obtain the compound of formula (IIL-2);
wherein:
R^{a} is an alkyl;
q, Z, M and R¹∼R⁵ are as defined in formula (IIL).

### Scheme XIII

A method for preparing the compound of formula (I) or a tautomer, mesomer, racemate, enantiomer, diastereomer thereof, or mixture thereof, or a pharmaceutically acceptable salt thereof according to the present invention, comprises the following step of: 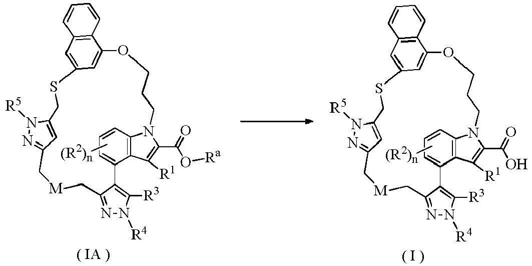 removing the protecting group R^{a} from the compound of formula (IA) under an alkaline condition to obtain the compound of formula (I),
wherein:
R^{a} is an alkyl;
M, R¹∼R⁵ and n are as defined in formula (I).

### Scheme XIV

A method for preparing the compound of formula (I-1) or (1-2) according to the present invention, comprises the following steps: 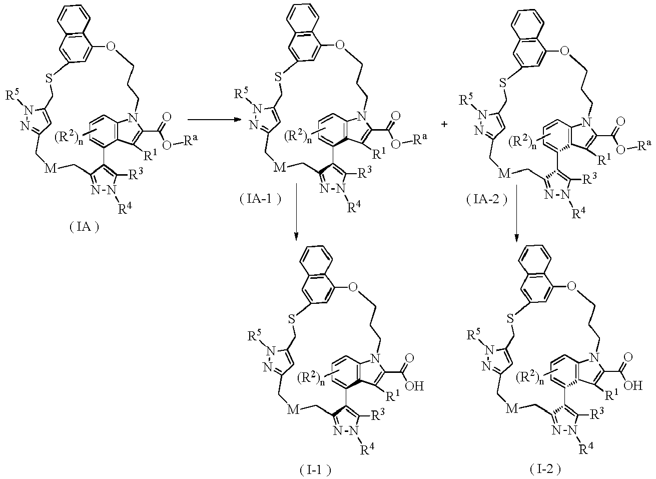 the compound of formula (IA) is subjected to a chiral preparation to obtain the compounds of formula (IA-1) and formula (IA-2);
the protecting group R^{a} is removed from the compound of formula (IA-1) under an alkaline condition to obtain the compound of formula (I-1); and the protecting group R^{a} is removed from the compound of formula (IA-2) under an alkaline condition to obtain the compound of formula (1-2);
wherein:
R^{a} is an alkyl;
M, R¹∼R⁵ and n are as defined in formula (I).

### Scheme XV

A method for preparing the compound of formula (II) or a tautomer, mesomer, racemate, enantiomer, diastereomer thereof, or mixture thereof, or a pharmaceutically acceptable salt thereof according to the present invention, comprises the following step of: 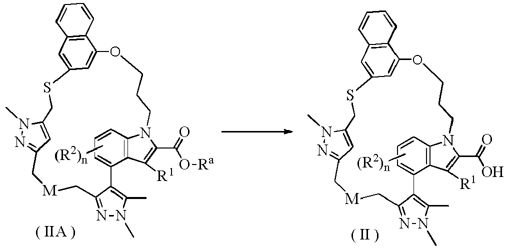 removing the protecting group R^{a} from the compound of formula (IIA) under an alkaline condition to obtain the compound of formula (II),
wherein:
R^{a} is an alkyl;
M, R¹, R² and n are as defined in formula (II).

### Scheme XVI

A method for preparing the compound of formula (III) or a tautomer, mesomer, racemate, enantiomer, diastereomer thereof, or mixture thereof, or a pharmaceutically acceptable salt thereof according to the present invention, comprises the following step of: 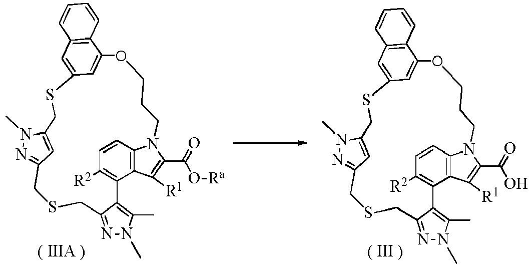 removing the protecting group R^{a} from the compound of formula (IIIA) under an alkaline condition to obtain the compound of formula (III),
wherein:
R^{a} is an alkyl;
R¹ and R² are as defined in formula (III).

### Scheme XVII

A method for preparing the compound of formula (III-1) or a tautomer, mesomer, racemate, enantiomer, diastereomer thereof, or mixture thereof, or a pharmaceutically acceptable salt thereof according to the present invention, comprises the following step of: 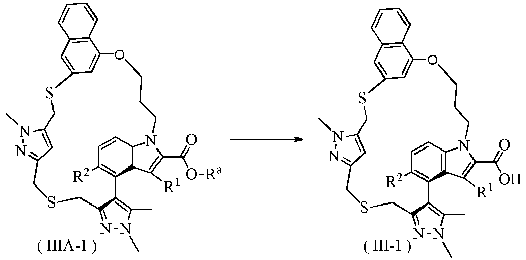 removing the protecting group R^{a} from the compound of formula (IIIA-1) under an alkaline condition to obtain the compound of formula (III-1),
wherein:
R^{a} is an alkyl;
R¹ and R² are as defined in formula (III).

In the above Scheme I to Scheme XVII, the reagent that provides an alkaline condition includes organic bases and inorganic bases. The organic bases include, but are not limited to, triethylamine, *N*,*N*-diisopropylethylamine, *n*-butyllithium, lithium diisopropylamide, lithium bistrimethylsilylamide, potassium acetate, potassium acetate, sodium *tert*-butoxide, potassium *tert*-butoxide and sodium *n*-butoxide. The inorganic bases include, but are not limited to, sodium bicarbonate, potassium bicarbonate, sodium hydride, potassium phosphate, sodium carbonate, potassium carbonate, potassium acetate, cesium carbonate, sodium hydroxide, lithium hydroxide and hydrates thereof, and preferably lithium hydroxide monohydrate.

The reactions in the above Scheme I to Scheme XVII are preferably carried out in a solvent. The solvents used include, but are not limited to, acetic acid, methanol, ethanol, *n*-butanol, *tert*-butanol, toluene, tetrahydrofuran, dichloromethane, petroleum ether, ethyl acetate, *n*-hexane, dimethyl sulfoxide, 1,4-dioxane, ethylene glycol dimethyl ether, water or *N*,*N*-dimethylformamide and mixtures thereof.

### DETAILED DESCRIPTION OF THE INVENTION

The present invention will be further described with reference to the following examples, but the examples should not be considered as limiting the scope of the present invention.

### EXAMPLES

The structures of the compounds were identified by nuclear magnetic resonance (NMR) and/or mass spectrometry (MS). NMR shifts (δ) are given in 10⁻⁶ (ppm). NMR is determined by a Bruker AVANCE-400 machine. The solvents for determination are deuterated-dimethyl sulfoxide (DMSO-*d₆*), deuterated-chloroform (CDCl₃) and deuterated-methanol (CD₃OD), and the internal standard is tetramethylsilane (TMS).

MS is determined by a FINNIGAN LCQAd (ESI) mass spectrometer (manufacturer: Thermo, type: Finnigan LCQ advantage MAX).

High performance liquid chromatography (HPLC) analysis is determined on an Agilent HPLC 1200DAD, Agilent HPLC 1200VWD and Waters HPLC e2695-2489 high pressure liquid chromatograph.

Chiral HPLC analysis is determined on an Agilent 1260 DAD high performance liquid chromatograph.

Preparative high performance liquid chromatography is carried out on Waters 2767, Waters 2767-SQ Detecor2, Shimadzu LC-20AP and Gilson-281 preparative chromatographs.

Chiral preparation is carried out on a Shimadzu LC-20AP preparative chromatograph.

CombiFlash rapid preparation instrument used is Combiflash Rf200 (TELEDYNE ISCO).

Yantai Huanghai HSGF254 or Qingdao GF254 silica gel plate is used as the thin-layer silica gel chromatography (TLC) plate. The dimension of the silica gel plate used in TLC is 0.15 mm to 0.2 mm, and the dimension of the silica gel plate used in product purification is 0.4 mm to 0.5 mm.

Yantai Huanghai 200 to 300 mesh silica gel is generally used as a carrier for silica gel column chromatography.

The average kinase inhibition rates and IC₅₀ values are determined by a NovoStar ELISA (BMG Co., Germany).

The known starting materials of the present disclosure can be prepared by the known methods in the art, or can be purchased from ABCR GmbH & Co. KG, Acros Organnics, Aldrich Chemical Company, Accela ChemBio Inc., Chembee Company etc.

Unless otherwise stated, the reactions are carried out under argon atmosphere or nitrogen atmosphere.

"Argon atmosphere" or "nitrogen atmosphere" means that a reaction flask is equipped with an argon or nitrogen balloon (about1 L).

"Hydrogen atmosphere" means that a reaction flask is equipped with a hydrogen balloon (about1 L).

Pressurized hydrogenation reaction is performed on a Parr 3916EKX hydrogenation instrument and a Qinglan QL-500 hydrogen generator or HC2-SS hydrogenation instrument.

In hydrogenation reactions, the reaction system is generally vacuumed and filled with hydrogen, which is repeated three times.

CEM Discover-S 908860 type microwave reactor is used in microwave reactions.

Unless otherwise stated, the solution refers to an aqueous solution.

Unless otherwise stated, the reaction temperature is room temperature from 20°C to 30°C.

The reaction process in the examples is monitored by thin layer chromatography (TLC). The developing solvent used in the reactions, the eluent system in column chromatography and the developing solvent system in thin layer chromatography for purification of the compounds include: A: n-hexane/ethyl acetate system, and B: dichloromethane/methanol system. The ratio of the volume of the solvent is adjusted according to the polarity of the compounds, and a small quantity of alkaline reagent such as triethylamine or acidic reagent such as acetic acid could also be added for adjustment.

### Example 1

### 17-Chloro-5,13-14-trimethyl-28-oxa-2,9-dithia-5,6,12,13,24-pentaazaheptacyclo[27.7.1. 1^{4,7}.0^{11,15}.0^{16,21}.0^{20,24}.0^{30,35}]octatriaconta-1(37),4(38),6,11,14,16,18,20,22,29,31,33,35-tri decaene-23-carboxylic acid 1

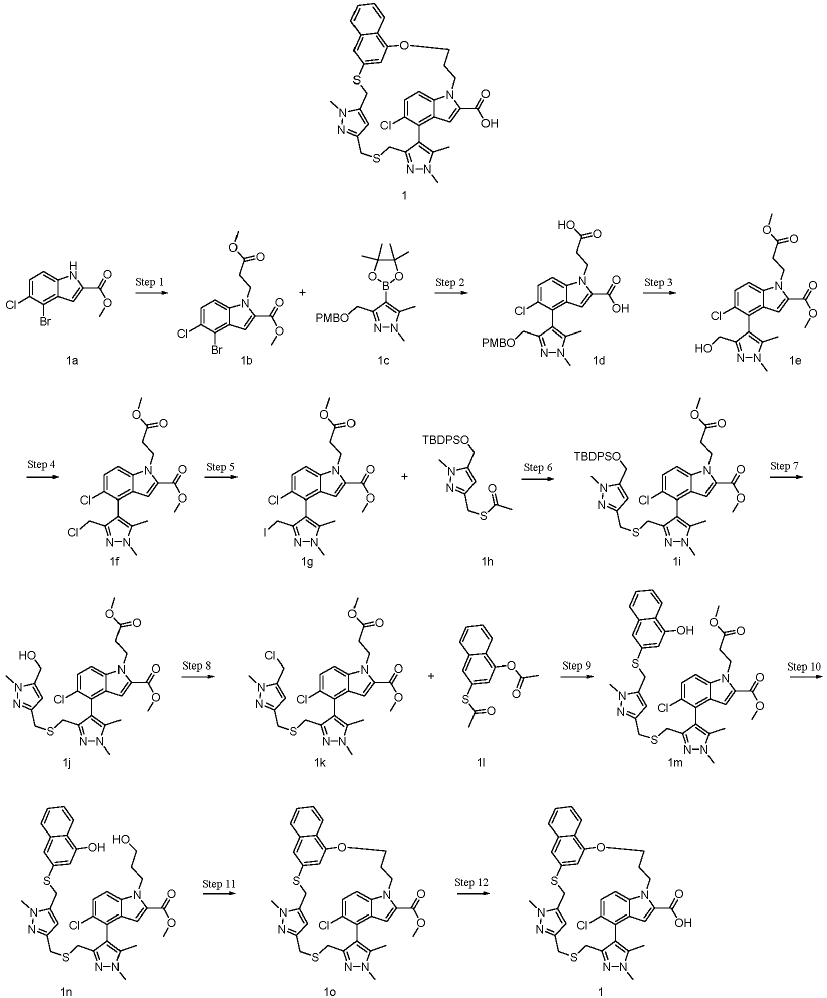

### Step 1

### Methyl 4-bromo-5-chloro-1-(3-methoxy-3-oxopropyl)-1H-indole-2-carboxylate 1b

Methyl 4-bromo-5-chloro-1*H*-indole-2-carboxylate **1a** (3.50 g, 12.13 mmol, prepared according to the method disclosed in the patent application "WO2017156181A1") was added to 40 mL of acetonitrile, followed by the addition of 1,8-diazabicyclo[5.4.0]undec-7-ene (1.53 g, 6.07 mmol) under an ice bath. Methyl acrylate (1.56 g, 18.12 mmol) was added dropwise, and the reaction solution was heated to reflux and stirred for 16 hours. The reaction solution was cooled to room temperature, to which 30 mL of water and 30 mL of ethyl acetate were added, and the resulting solution was partitioned. The organic phase was washed with 1*N* hydrochloric acid (20 ml×2), water (20 ml×2) and saturated sodium chloride solution (20 ml×2) successively, dried over anhydrous sodium sulfate and filtered. The filtrate was concentrated under reduced pressure, and the resulting residues were purified by silica gel column chromatography with eluent system A to obtain the title product **1b** (4.00 g, yield: 88.1%).
MS m/z (ESI): 373.9 375.9 [M+1].

### Step 2

### 1-(2-Carboxyethyl)-5-chloro-4-(3-(((4-methoxybenzyl)oxy)methyl)-1,5-dimethyl-1H-p yrazol-4-yl)-1H-indole-2-carboxylic acid 1d

**1b** (2.60 g, 6.94 mmol) and 3-(((4-methoxybenzyl)oxy)methyl)-1,5-dimethyl-4-(4,4,5,5-tetramethyl-1,3,2-dioxabor olan-2-yl)-1*H*-pyrazole **1c** (2.58 g, 6.93 mmol, prepared according to the method disclosed in the patent application "WO2017182625A1") were dissolved in 24 mL of 1,4-dioxane and 6 mL of water. The solution was purged with argon three times, and added with 1,1'-bis(di-*tert*-butylphosphine)ferrocaene dichloropalladium (229 mg, 0.35 mmol) and cesium carbonate (4.52 g, 13.87 mmol). The reaction solution was purged with argon three times, heated to 95°C under an argon atmosphere, and stirred for 16 hours. The reaction solution was cooled to room temperature, and concentrated under reduced pressure to remove most of the solvent. 40 mL of water was added to the reaction solution, and extracted with ethyl acetate (20 mL×3). The aqueous phase was adjusted to pH=1-2 with 1*N* HCl, and extracted with dichloromethane (containing a small amount of methanol, 20 mL×3). The organic phase was washed with water (30 mL) and saturated sodium chloride solution (30 mL) successively, dried over anhydrous sodium sulfate, and filtered to remove the desiccant. The filtrate was concentrated under reduced pressure to obtain the title product **1d** (2.20 g, yield: 61.9%). The product was used directly in the next step without purification.
MS m/z (ESI):512.2 [M+1].

### Step 3

### Methyl 5-chloro-4-(3-(hydroxymethyl)-1,5-dimethyl-1H-pyrazol-4-yl)-1-(3-methoxy-3-oxopro pyl)-1H-indole-2-carboxylate 1e

The crude product **1d** (2.20 g, 4.30 mmol) was dissolved in 30 mL of methanol. Concentrated sulfuric acid (2.00 g, 20.39 mmol) was added dropwise under an ice bath, and the reaction solution was heated to reflux and stirred for 16 hours. The reaction solution was cooled under an ice bath, and added dropwise with saturated aqueous sodium bicarbonate solution under an ice bath to adjust pH to 7-8. The solution was extracted with dichloromethane (30 mL×3). The organic phases were combined, washed with water (20 mL) and saturated sodium chloride solution (20 mL) successively, dried over anhydrous sodium sulfate, and filtered to remove the desiccant. The filtrate was concentrated under reduced pressure, and the resulting residues were purified by silica gel column chromatography with eluent system B to obtain the title product **1e** (850 mg, yield: 47.11%).
MS m/z (ESI): 420.2 [M+1].

### Step 4

### Methyl 5-chloro-4-(3-(chloromethyl)-1,5-dimethyl-1H-pyrazol-4-yl)-1-(3-methoxy-3-oxopropy 1)-1H-indole-2-carboxylate 1f

**1e** (850 mg, 2.02 mmol) was dissolved in 15 mL of dichloromethane, and the solution was purged with argon three times. Thionyl chloride (361 mg, 3.03 mmol) was added dropwise under an ice bath, and the reaction solution was reacted at room temperature for 2 hours. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate, and filtered to remove the desiccant. The filtrate was concentrated under reduced pressure to obtain the title product **1f** (850 mg, yield: 95.79%), which was used directly in the next step without purification.
MS m/z (ESI): 438.1 [M+1].

### Step 5

### Methyl 5-chloro-4-(3-(iodomethyl)-1,5-dimethyl-1H-pyrazol-4-yl)-1-(3-metboxy-3-oxopropyl)-1H-indole-2-carboxylate 1g

The crude product **1f** (850 mg, 1.94 mmol) was dissolved in 10 mL of acetonitrile, followed by the addition of sodium iodide (581mg, 3.88 mmol). The reaction solution was heated to 80°C and stirred for 2 hours. The reaction solution was cooled to room temperature, and added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure to obtain the title product **1g** (1.00 g, yield: 97.34%), which was used directly in the next step without purification.
MS m/z (ESI): 530.0 [M+1].

### Step 6

### Methyl 4-(3-((((5-(((tert-butyldiphenylsilyl)oxy)methyl)-1-methyl-1H-pyrazol-3-yl)methyl)thio )methyl)-1,5-dimethyl-1H-pyrazol-4-yl)-5-chloro-1-(3-methoxy-3-oxopropyl)-1H-indol e-2-carboxylate 1i

The crude product **1g** (850 mg, 1.94 mmol) was dissolved in 10 mL of methanol and 5 mL of tetrahydrofuran, followed by the addition of potassium carbonate (313 mg, 2.27 mmol). The solution was purged with argon three times, and added dropwise with a solution of *S*-((5-(((*tert*-butyldiphenylsilyl)oxy)methyl)-1-methyl-1*H*-pyrazol-3-yl)methyl) ethanethioate **1h** (994 mg, 2.27 mmol, prepared according to the method disclosed in the patent application "WO2017182625A1") in methanol (5 mL) at room temperature. The reaction solution was reacted at room temperature for 2 hours. The reaction solution was concentrated under reduced pressure to remove most of the solvent, and then added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **1i** (1.30 g, yield: 86.25%).
MS m/z (ESI): 798.2 [M+1].

### Step 7

### Methyl 5-chloro-4-(3-((((5-(bydroxymethyl)-1-methyl-1H-pyrazol-3-yl)methyl)thio)methyl)-1, 5-dimethyl-1H-pyrazol-4-yl)-1-(3-methoxy-3-oxopropyl)-1H-indole-2-carboxylate 1j

**1i** (1.30 g, 1.63 mmol) was dissolved in 10 mL of tetrahydrofuran. 1.0M tetrabutylammonium fluoride (1.95 mL, 1.95 mmol) was added dropwise, and the reaction solution was reacted at room temperature for 1 hour. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **1j** (560 mg, yield: 61.41%).
MS m/z (ESI): 560.2 [M+1].

### Step 8

### Methyl 5-chloro-4-(3-((((5-(chloromethyl)-1-methyl-1H-pyrazol-3-yl)methyl)thio)methyl)-1,5-dimethyl-1H-pyrazol-4-yl)-1-(3-methoxy-3-oxopropyl)-1H-indole-2-carboxylate 1k

**1j** (560 mg, 1.00 mmol) was dissolved in 10 mL of dichloromethane, and the solution was purged with argon three times. Thionyl chloride (143 mg, 1.20 mmol) was added dropwise under an ice bath, and the reaction solution was reacted at room temperature for 2 hours. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate, and filtered to remove the desiccant. The filtrate was concentrated under reduced pressure to obtain the title product **1k** (600 mg, yield: 103.73%), which was used directly in the next step without purification.
MS m/z (ESI): 578.1 [M+1].

### Step 9

### Methyl 5-chloro-4-(3-((((5-(((4-hydroxynaphthalen-2-yl)thio)methyl)-1-methyl-1H-pyrazol-3-y l)methyl)thio)methyl)-1,5-dimethyl-1H-pyrazol-4-yl)-1-(3-methoxy-3-oxopropyl)-1H-i ndole-2-carboxylate 1m

The crude product **1k** (600 mg, 1.04 mmol) and 3-(acetylthio)naphthalen-1-yl acetate **1l** (324 mg, 1.47 mmol, prepared according to the method disclosed in the patent application "WO2017182625A1") were dissolved in 10 mL of methanol. Potassium carbonate (401 mg, 2.91 mmol) was added at room temperature, and the reaction solution was reacted at room temperature for 2 hours. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **1m** (700 mg, yield: 93.96%).
MS m/z (ESI): 718.1 [M+1].

### Step 10

### Methyl 5-chloro-4-(3-((((5-(((4-hydroxynaphthalen-2-yl)thio)methyl)-1-methyl-1H-pyrazol-3-y l)methyl)thio)methyl)-1,5-dimethyl-1H-pyrazol-4-yl)-1-(3-hydroxypropyl)-1H-indole-2 -carboxylate 1n

**1m** (700 mg, 0.97 mmol) was dissolved in 10 mL of tetrahydrofuran. 1.0 M solution of borane in tetrahydrofuran (9.7 mL, 9.70 mmol) was added dropwise under an ice bath, and the reaction solution was reacted at room temperature for 5 hours. 4.25 mL of methanol and 8.5 mL of hydrochloric acid (6M) were added dropwise under an ice bath, and the reaction solution was stirred for 1 hour. 50 mL of water was added to the reaction solution, which was extracted with a mixed solution of dichloromethane and methanol (V:V=10:1) (50 mL×2). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system B to obtain the title product **1n** (550 mg, yield: 81.76%).
MS m/z (ESI): 690.2 [M+1].

### Step 11

### Methyl 17-chloro-5,13-14-trimethyl-28-oxa-2,9-dithia-5,6,12,13,24-pentaazaheptacyclo[27.7.1. 1^{4,7}.0^{11,15}.0^{16,21}.0^{20,24}.0^{30,35}]octatriaconta-1(37),4(38),6,11,14,16,18,20,22,29,31,33,35-tri decaene-23-carboxylate 1o

Triphenylphosphine (228 mg, 0.87 mmol) and dibenzyl azodicarboxylate (200 mg, 0.87 mmol) were dissolved in 10 mL of toluene, and the solution was purged with argon three times. 6 mL of solution of **1n** (300 mg, 0.43 mmol) in toluene and tetrahydrofuran (V:V=5:1) was added dropwise, and the reaction solution was reacted at room temperature for 16 hours. 10 mL of hydrochloric acid (2M) was added, and the reaction solution was stirred for 10 minutes. 50 mL of water was added to the reaction solution, and extracted with ethyl acetate (50 mL×2). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system B to obtain the title product **1o** (230 mg, yield: 78.72%).
MS m/z (ESI): 672.2 [M+1].

### Step 12

### 17-Chloro-5,13-14-trimethyl-28-oxa-2,9-dithia-5,6,12,13,24-pentaazaheptacyclo[27.7.1. 1^{4,7}.0^{11,15}.0^{16,21}.0^{20,24}.0^{30,35}]octatriaconta-1(37),4(38),6,11,14,16,18,20,22,29,31,33,35-tri decaene-23-carboxylic acid 1

**1l** (300 mg, 0.45 mmol) was dissolved in 15 mL of a mixed solvent of methanol, tetrahydrofuran and water (V:V:V=1:1:1). Lithium hydroxide monohydrate (225 mg, 5.36 mmol) was added, and the reaction solution was heated to 50°C and reacted for 0.5 hour. The reaction solution was cooled to room temperature, and concentrated under reduced pressure to remove most of the solvent. 2M hydrochloric acid was added to adjust pH to 1-2, and the solution was extracted with a mixed solvent (50 mL×2) of dichloromethane and methanol (V:V=10:1). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system B to obtain the title product **1** (15 mg, yield: 5.11%).
MS m/z (ESI): 658.1 [M+1].
¹H NMR (400 MHz, CDCl₃) δ 8.37-8.39 (m, 1H), 7.78-7.79 (m, 1H), 7.66 (s, 1H), 7.54-7.60 (m, 2H), 7.27-7.29 (m, 1H), 7.12 (s, 1H), 7.01-7.03 (m, 1H), 6.11 (s, 1H), 5.23-5.26 (m, 1H), 5.10 (s, 1H), 4.62-4.67 (m, 1H), 3.77-3.88 (m, 6H), 3.50-3.63 (m, 5H), 3.30-3.40 (m, 1H), 3.11-3.15 (m, 1H), 2.62-2.65(m, 1H), 2.34-2.45 (m, 2H), 2.06 (s,3H).

### Examples 1-1 and 1-2

### (Ra)-17-Chloro-5,13-14-trimethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylic acid 1-1

### (Sa)-17-Chloro-5,13-14-trimethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylic acid 1-2

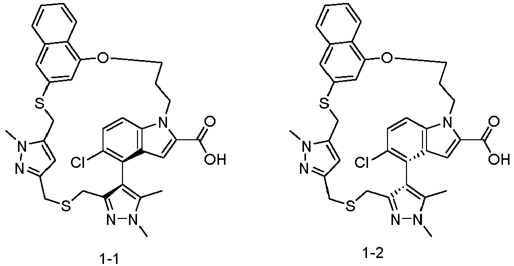

**1** (60 mg, 0.48 mmol) was separated chirally (separation conditions: CHIRALPAK IE chiral preparative column, 5.0 cm I.D. × 25 cm L; mobile phase: Hexane/EtOH/HAc=70/30/0.1(V/V/V); flow rate: 60 mL/min). The corresponding fractions were collected and concentrated under reduced pressure to obtain the title products (22 mg, 28 mg).

Compound **1-1** with single configuration (having shorter retention time):
MS m/z (ESI):658.2 [M+1].

Chiral HPLC analysis: retention time 6.892 minutes, chiral purity: 100% (chromatographic column: CHIRALPAK IE 150^{∗}4.6 mm, 5 µm; mobile phase: *n*-hexane/ethanol/diethylamine= 70/30/0.1 (v/v/v)).
¹H NMR (400 MHz, CDCl₃) ¹H NMR (400 MHz, CDCl₃) δ 8.37-8.39 (m, 1H), 7.78-7.79 (m, 1H), 7.66 (s, 1H), 7.54-7.60 (m, 2H), 7.27-7.29 (m, 1H), 7.12 (s, 1H), 7.01-7.03 (m, 1H), 6.11 (s, 1H), 5.23-5.26 (m, 1H), 5.10 (s, 1H), 4.62-4.67 (m, 1H), 3.77-3.88 (m, 6H), 3.50-3.63 (m, 5H), 3.30-3.40 (m, 1H), 3.11-3.15 (m, 1H), 2.62-2.65(m, 1H), 2.34-2.45 (m, 2H), 2.06 (s,3H).

Compound **1-2** with single configuration (having longer retention time):
MS m/z (ESI):658.2 [M+1].

Chiral HPLC analysis: retention time 8.887 minutes, chiral purity: 100% (chromatographic column: CHIRALPAK IE 150^{∗}4.6 mm, 5 µm; mobile phase: *n*-hexane/ethanol/diethylamine= 70/30/0.1(v/v/v)).
¹H NMR (400 MHz, CDCl₃) ¹H NMR (400 MHz, CDCl₃) δ 8.37-8.39 (m, 1H), 7.78-7.79 (m, 1H), 7.66 (s, 1H), 7.54-7.60 (m, 2H), 7.27-7.29 (m, 1H), 7.12 (s, 1H), 7.01-7.03 (m, 1H), 6.11 (s, 1H), 5.23-5.26 (m, 1H), 5.10 (s, 1H), 4.62-4.67 (m, 1H), 3.77-3.88 (m, 6H), 3.50-3.63 (m, 5H), 3.30-3.40 (m, 1H), 3.11-3.15 (m, 1H), 2.62-2.65(m, 1H), 2.34-2.45 (m, 2H), 2.06 (s,3H).

### Example 2

### 17-Chloro-5,13,14,22-tetramethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylic acid 2

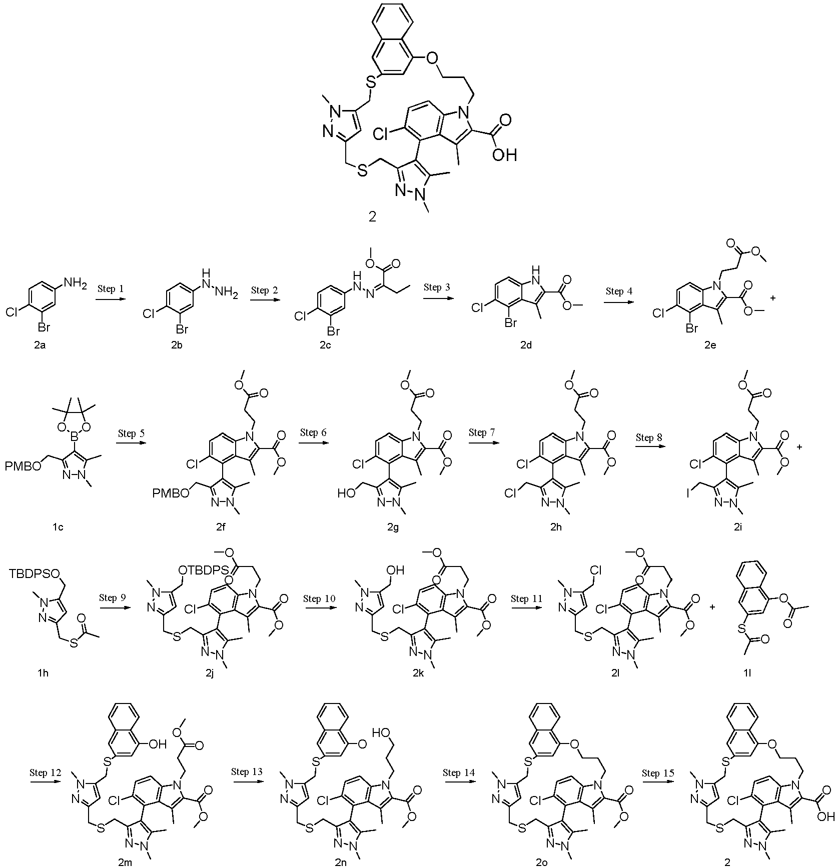

### Step 1

### (3-Bromo-4-chlorophenyl)hydrazine 2b

3-Bromo-4-chloroaniline **2a** (20 g, 62.58 mmol, purchased from Shanghai Bide Pharmatech Ltd.) was dissolved in 96 mL of 25% hydrochloric acid. 60 mL of aqueous solution of sodium nitrite (7.69 g, 111.46 mmol) was added dropwise under an ice bath, and the temperature was maintained below 10°C. The reaction solution was reacted at 0°C for 1 hour. The above solution was added dropwise to 144 mL of solution of stannous chloride dihydrate (98.00 g, 434.30 mmol) in 25% hydrochloric acid, and the temperature was maintained below 10°C. The reaction solution was reacted at 0°C for 1 hour. After completion of the reaction, 420 mL of 32% sodium hydroxide solution was added dropwise under an ice bath to alkalize the reaction solution, and 960 mL of water was added. The solution was extracted with dichloromethane (800 mL×3) and partitioned. The organic phase was washed with water (200 mL×2) and saturated sodium chloride solution (200 mL×2) successively, dried over anhydrous sodium sulfate and filtered. The filtrate was concentrated under reduced pressure to obtain the title product **2b** (19.40 g, yield: 90.42%).
MS m/z (ESI): 220.7 223.0 [M+1].

### Step 2

### Methyl (Z)-2-(2-(3-bromo-4-chlorophenyl)hydrazono)butanoate 2c

**2b** (19.40 g, 87.59 mmol) was dissolved in 60 mL of ethanol. 20 mL of a solution of methyl 2-oxobutyrate (10.58 g, 91.12 mmol) in ethanol was added dropwise under an ice bath, and the reaction solution was reacted at room temperature for 1 hour. The reaction solution was concentrated under reduced pressure, and then added with 50 mL of *n*-hexane to pulp. The mixture was filtered, and the filter cake was collected and dried under vacuum to obtain the title product **2c** (20.00 g, yield: 71.45%).
MS m/z (ESI): 318.7 320.9 [M+1].

### Step 3

### Methyl 4-bromo-5-chloro-3-methyl-1H-indole-2-carboxylate

**2c** (20.00 g, 62.58 mmol) was dissolved in 200 mL of glacial acetic acid. Zinc chloride (47.00 g, 344.84 mmol) was added, and the reaction solution was heated to 120°C and reacted for 1 hour. The reaction solution was poured into 500 mL of ice water, and a white solid was precipitated. The solid was filtered, and dried under vacuum to obtain the crude title product **2d** (18.50 g, yield: 97.70%), which was used directly in the next step without purification.
MS m/z (ESI): 299.9 301.9 [M-1].

### Step 4

### Methyl 4-bromo-5-chloro-1-(3-methoxy-3-oxopropyl)-3-methyl-1H-indole-2-carboxylate 2e

The crude product **2d** (5.00 g, 16.53 mmol) was added to 40 mL of acetonitrile, followed by the addition of 1,8-diazabicyclo[5.4.0]undec-7-ene (20.81 g, 82.62 mmol, purchased from Accela ChemBio Co., Ltd.) under an ice bath. Methyl acrylate (2.13 g, 24.74 mmol) was added dropwise, and the reaction solution was heated to reflux and stirred for 30 minutes. Additional methyl acrylate (2.13 g, 24.74 mol) was added four times. After completion of the reaction, 100 mL of water and 100 mL of ethyl acetate were added, and the resulting solution was partitioned. The organic phase was washed with 1N HCl (30 ml×2), water (30 ml×2) and saturated sodium chloride solution (30 ml×2) successively, dried over anhydrous sodium sulfate and filtered. The filtrate was concentrated under reduced pressure, and the resulting residues were purified by silica gel column chromatography with eluent system A to obtain the title product **2e** (900 mg, yield: 14.01%).
MS m/z (ESI): 388.0 390.0 [M+1].

### Step 5

### Methyl 5-chloro-1-(3-methoxy-3-oxopropyl)-4-(3-(((4-methoxybenzyl)oxy)methyl)-1,5-dimeth yl-1H-pyrazol-4-yl)-3-methyl-1H-indole-2-carboxylate 2f

**2e** (780 mg, 2.01 mmol) and **1c** (747 mg, 2.01 mmol) were dissolved in 20 mL of a mixed solution of 1,4-dioxane and water (V:V=4:1). The solution was purged with argon three times, and added with 1,1'-bis(di-*tert*-butylphosphine)ferrocaene dichloropalladium (71 mg, 0.10 mmol) and cesium carbonate (1.31 g, 4.02 mmol). The reaction solution was heated to 95°C and stirred for 16 hours. The reaction solution was concentrated under reduced pressure to remove most of the solvent, and added with 40 mL of water and extracted with ethyl acetate (20 mL×3). The organic phase was washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system B to obtain the title product **2f** (720 mg, yield: 64.75%).
MS m/z (ESI):554.2 [M+1].

### Step 6

### Methyl 5-chloro-4-(3-(hydroxymethyl)-1,5-dimethyl-1H-pyrazol-4-yl)-1-(3-methoxy-3-oxopro pyl)-3-methyl-1H-indole-2-carboxylate 2g

**2f** (720 mg, 1.30 mmol) was dissolved in 4 mL of dichloromethane. Trifluoroacetic acid (1.48 g, 12.98 mmol) was added dropwise, and the reaction solution was reacted at room temperature for 30 minutes. Saturated aqueous sodium bicarbonate solution was added dropwise to the reaction solution under an ice bath to adjust pH to 7-8. The solution was extracted with dichloromethane (30 mL×3). The organic phases were combined, washed with water (20 mL) and saturated sodium chloride solution (20 mL) successively, dried over anhydrous sodium sulfate, and filtered to remove the desiccant. The filtrate was concentrated under reduced pressure, and the resulting residues were purified by silica gel column chromatography with eluent system B to obtain the title product **2g** (320 mg, yield: 56.75%).
MS m/z (ESI): 434.1 [M+1].

### Step 7

### Methyl 5-chloro-4-(3-(chloromethyl)-1,5-dimethyl-1H-pyrazol-4-yl)-1-(3-methoxy-3-oxopropy 1)-3-methyl-1H-indole-2-carboxylate 2h

**2g** (320 mg, 0.74 mmol) was dissolved in 10 mL of dichloromethane, and the solution was purged with argon three times. Thionyl chloride (132 mg, 1.11 mmol) was added dropwise under an ice bath, and the reaction solution was reacted at room temperature for 30 minutes. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate, and filtered to remove the desiccant. The filtrate was concentrated under reduced pressure to obtain the crude title product **2h** (350 mg), which was used directly in the next step without purification.
MS m/z (ESI): 452.1 [M+1].

### Step 8

### Methyl 5-chloro-4-(3-(iodomethyl)-1,5-dimethyl-1H-pyrazol-4-yl)-1-(3-methoxy-3-oxopropyl)-3-methyl-1H-indole-2-carboxylate 2i

The crude product **2h** (350 mg, 0.77 mmol) was dissolved in 10 mL of acetonitrile, followed by the addition of sodium iodide (232 mg, 1.55 mmol). The reaction solution was heated to 80°C and stirred for 2 hours. The reaction solution was cooled to room temperature, and added with 50 mL of water was added. The solution was stirred for 30 minutes, and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure to obtain the title product **2i** (370 mg, yield: 87.94%).
MS m/z (ESI): 544.1 [M+1].

### Step 9

### Methyl 4-(3-((((5-(((tert-butyldiphenylsilyl)oxy)methyl)-1-methyl-1H-pyrazol-3-yl)methyl)thio )methyl)-1,5-dimethyl-1H-pyrazol-4-yl)-5-chloro-1-(3-methoxy-3-oxopropyl)-3-methyl -1H-indole-2-carboxylate 2j

**2i** (370 mg, 0.68 mmol) was dissolved in 10 mL of methanol and 2 mL of tetrahydrofuran, followed by the addition of potassium carbonate (113 mg, 0.82 mmol). The solution was purged with argon three times, and added dropwise with a solution of **1h** (358 mg, 0.82 mmol) in methanol (5 mL) at room temperature. The reaction solution was reacted at room temperature for 2 hours. The reaction solution was concentrated under reduced pressure to remove most of the solvent, and added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **2j** (640 mg, yield: 115.77%).
MS m/z (ESI): 812.3 [M+1].

### Step 10

### Methyl 5-chloro-4-(3-((((5-(hydroxymethyl)-1-methyl-1H-pyrazol-3-yl)methyl)thio)methyl)-1, 5-dimethyl-1H-pyrazol-4-yl)-1-(3-methoxy-3-oxopropyl)-3-methyl-1H-indole-2-carbox ylate 2k

**2j** (640 mg, 0.79 mmol) was dissolved in 10 mL of tetrahydrofuran. 1.0M tetrabutylammonium fluoride (0.95 mL, 0.95 mmol) was added dropwise, and the reaction solution was stirred at room temperature for 1 hour. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **2k** (240 mg, yield: 53.07%).
MS m/z (ESI): 574.2 [M+1].

### Step 11

### Methyl 5-chloro-4-(3-((((5-(chloromethyl)-1-methyl-1H-pyrazol-3-yl)methyl)thio)methyl)-1,5-dimethyl-1H-pyrazol-4-yl)-1-(3-methoxy-3-oxopropyl)-3-methyl-1H-indole-2-carboxyl ate 2l

**2k** (240 mg, 0.42 mmol) was dissolved in 5 mL of dichloromethane, and the solution was purged with argon three times. Thionyl chloride (60 mg, 0.50 mmol) was added dropwise under an ice bath, and the reaction solution was reacted at room temperature for 30 minutes. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate, and filtered to remove the desiccant. The filtrate was concentrated under reduced pressure to obtain the title product **2l** (270 mg), which was used directly in the next step without purification.
MS m/z (ESI): 592.2 [M+1].

### Step 12

### Methyl 5-chloro-4-(3-((((5-(((4-hydroxynaphthalen-2-yl)thio)methyl)-1-methyl-1H-pyrazol-3-y 1)methyl)thio)methyl)-1,5-dimethyl-1H-pyrazol-4-yl)-1-(3-methoxy-3-oxopropyl)-3-me thyl-1H-indole-2-carboxylate 2m

The crude product **2l** (270 mg, 0.46 mmol) and **1l** (142 mg, 0.55 mmol) were dissolved in 10 mL of methanol. Potassium carbonate (176 mg, 1.28 mmol) was added at room temperature, and the reaction solution was reacted at room temperature for 1 hour. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **2m** (300 mg, yield: 89.90%).
MS m/z (ESI): 732.2 [M+1].

### Step 13

### Methyl 5-chloro-4-(3-((((5-(((4-hydroxynaphthalen-2-yl)thio)methyl)-1-methyl-1H-pyrazol-3-y 1)methyl)thio)methyl)-1,5-dimethyl-1H-pyrazol-4-yl)-1-(3-hydroxypropyl)-3-methyl-1 H-indole-2-carboxylate 2n

**2m** (300 mg, 0.41 mmol) was dissolved in 5 mL of tetrahydrofuran. 1.0 M solution of borane in tetrahydrofuran (4.10 mL, 4.10 mmol) was added dropwise under an ice bath, and the reaction solution was stirred at room temperature for 6 hours. 2.7 mL of methanol and 5.3 mL of diluted hydrochloric acid (6M) were added dropwise under an ice bath, and the reaction solution was stirred for 1 hour. 50 mL of water was added to the reaction solution, which was extracted with a mixed solution of dichloromethane and methanol (V:V=10:1) (50 mL×2). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system B to obtain the title product **2n** (150 mg, yield: 51.99%).
MS m/z (ESI):704.2 [M+1].

### Step 14

### Methyl 17-chloro-5,13,14,22-tetramethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylate 2o

Triphenylphosphine (112 mg, 0.43 mmol) and dibenzyl azodicarboxylate (98 mg, 0.43 mmol) were dissolved in 5 mL of toluene, and the solution was purged with argon three times. 6 mL of a solution of **2n** (150 mg, 0.21 mmol) in toluene and tetrahydrofuran (V:V=5:1) was added dropwise, and the reaction solution was stirred at room temperature for 16 hours. 10 mL of diluted hydrochloric acid (2M) was added, and the reaction solution was stirred for 10 minutes. 50 mL of water was added to the reaction solution, and extracted with ethyl acetate (50 mL×2). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system B to obtain the title product **2o** (300 mg), which contained triphenylphosphine oxide and was used directly in the next step.
MS m/z (ESI): 686.2 [M+1].

### Step 15

### 17-Chloro-5,13,14,22-tetramethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylic acid 2

**2o** (300 mg, 0.44 mmol) was dissolved in 6 mL of a mixed solvent of methanol, tetrahydrofuran and water (V:V:V=1:1:1). Lithium hydroxide monohydrate (183 mg, 4.36 mmol) was added, and the reaction solution was heated to 50°C and stirred for 0.5 hour. The reaction solution was concentrated under reduced pressure to remove most of the solvent. 2*M* hydrochloric acid was added to adjust pH to 1-2, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **2** (10 mg, yield: 3.40%).
MS m/z (ESI): 672.2 [M+1].
¹H NMR (400 MHz, CDCl₃) δ 8.34-8.36 (m, 1H), 7.75-7.77 (m, 1H), 7.62 (s, 1H), 7.51-7.59 (m, 2H), 7.30-7.33 (m, 1H), 7.02-7.04 (m, 1H), 6.11 (s, 1H), 5.17-5.20 (m, 1H), 5.09 (s, 1H), 4.58-4.62 (m, 1H), 3.81-3.95 (m, 5H), 3.71-3.74 (m, 1H), 3.64 (s, 3H), 3.45-3.49 (m, 2H), 3.24-3.28 (m, 1H), 3.12-3.16 (m, 1H), 2.75-2.78 (m, 1H), 2.34-2.41 (m, 2H), 2.22 (s, 3H), 2.01 (s, 3H).

### Examples 2-1 and 2-2

### (Ra)-17-Chloro-5,13,14,22-tetramethyl-28-oxa-2,9-dithia-5,6,12,13,24-pentaazaheptacy clo[27.7.1.1^{1,7}.0^{11,15}.0^{16,21}.0^{20,24}.0^{30,35}]octatriaconta-1(37),4(38),6,11,14,16,18,20,22,29,3 1,33,35-tridecaene-23-carboxylic acid 2-1

### (Sa)-17-Chloro-5,13,14,22-tetramethyl-28-oxa-2,9-dithia-5,6,12,13,24-pentaazaheptacy clo[27.7.1.1^{4,7}.0^{11,15}.0^{16,21}.0^{20,24}.0^{30,35}]octatriaconta-1(37),4(38),6,11,14,16,18,20,22,29,3 1,33,35-tridecaene-23-carboxylic acid 2-2

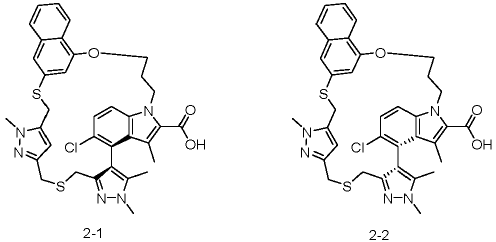

**2** (20 mg, 0.48 mmol) was separated chirally (separation conditions: CHIRALPAK IG chiral preparative column, 5.0 cm I.D. × 25 cm L; mobile phase: Hexane/EtOH/HAc=60/40/0.1(V/V/V); flow rate: 60 mL/min). The corresponding fractions were collected and concentrated under reduced pressure to obtain the title products (5 mg, 5 mg).

Compound **2-1** with single configuration (having shorter retention time):
MS m/z (ESI): 672.2 [M+1].

Chiral HPLC analysis: retention time 7.978 minutes, chiral purity: 100% (chromatographic column: CHIRALPAK IG 150^{∗}4.6 mm, 5 µm, equipped with a guard column; mobile phase: n-hexane/ethanol/trifluoroacetic acid =75/25/0.1(v/v/v)).
¹H NMR (400 MHz, CDCl₃) δ8.34-8.36 (m, ¹H ), 7.75-7.77 (m, 1H), 7.62 (s, 1H), 7.51-7.59 (m, 2H), 7.30-7.33 (m, 1H), 7.02-7.04 (m, 1H), 6.11 (s, 1H), 5.17-5.20 (m, 1H), 5.09 (s, 1H), 4.58-4.62 (m, 1H), 3.81-3.95 (m, 5H), 3.71-3.74 (m, 1H), 3.64 (s, 3H), 3.45-3.49 (m, 2H), 3.24-3.28 (m, 1H), 3.12-3.16 (m, 1H), 2.75-2.78 (m, 1H), 2.34-2.41 (m, 2H), 2.22 (s, 3H), 2.01 (s, 3H).

Compound **2-2** with single configuration (having longer retention time):
MS m/z (ESI):672.2 [M+1].

Chiral HPLC analysis: retention time 11.297 minutes, chiral purity: 100% (chromatographic column: CHIRALPAK IG 150^{∗}4.6 mm, 5 µm, equipped with a guard column; mobile phase: n-hexane/ethanol/trifluoroacetic acid =75/25/0.1(v/v/v)).
¹H NMR (400 MHz, CDCl₃) δ 8.34-8.36 (m, 1H), 7.75-7.77 (m, 1H), 7.62 (s, 1H), 7.51-7.59 (m, 2H), 7.30-7.33 (m, 1H), 7.02-7.04 (m, 1H), 6.11 (s, 1H), 5.17-5.20 (m, 1H), 5.09 (s, 1H), 4.58-4.62 (m, 1H), 3.81-3.95 (m, 5H), 3.71-3.74 (m, 1H), 3.64 (s, 3H), 3.45-3.49 (m, 2H), 3.24-3.28 (m, 1H), 3.12-3.16 (m, 1H), 2.75-2.78(m, 1H), 2.34-2.41 (m, 2H), 2.22 (s, 3H), 2.01 (s, 3H).

### Example 3

### 17-Chloro-5,9,13,14,22-pentamethyl-28-oxa-2-thia-5,6,9,12,13,24-hexaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylic acid 3

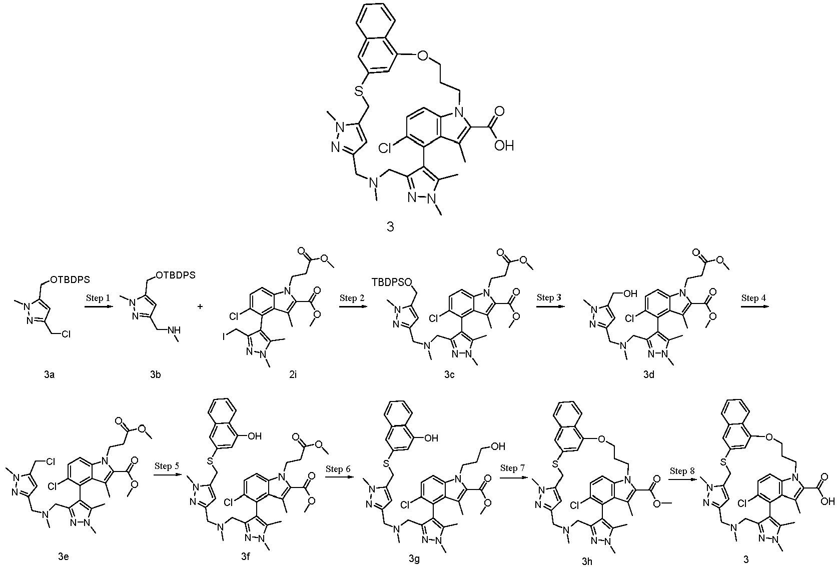

### Step 1

### 1-[5-[[Tert-butyl(diphenyl)silyl]oxymethyl]-1-methyl-pyrazol-3-yl]-N-methyl-methana mine 3b

5-((*Tert*-butyldiphenylsilyl)oxy)-3-(chloromethyl)-1-methyl-1*H*-pyrazole **3a** (7 g, 17.54 mmol, prepared according to the method disclosed in the patent application "WO2017182625A1") was dissolved in a solution of methylamine in ethanol (∼30wt%, 80 mL). The reaction solution was warmed up to 50°C, and stirred for 1 hour. The reaction solution was cooled to room temperature, and concentrated under reduced pressure. The resulting residues were purified by silica gel column chromatography with eluent system A to obtain the title product **3b** (5.0 g, yield: 72.41%).

### Step 2

### Methyl 4-(3-((((5-(((tert-butyldiphenylsilyl)oxy)methyl)-1-methyl-1H-pyrazol-3-yl)methyl)(me thyl)amino)methyl)-1,5-dimethyl-1H-pyrazol-4-yl)-5-chloro-1-(3-methoxy-3-oxopropyl )-3-methyl-1H-indole-2-carboxylate 3c

**2i** (1.0 g, 1.84 mmol) was dissolved in *N*,*N*-dimethylformamide (10 mL), and then added with potassium carbonate (634 mg, 4.59 mmol) and **3b** (869 mg, 2.21 mmol) successively. The reaction solution was warmed up to 60°C, and stirred for 1 hour. The reaction solution was cooled to room temperature, diluted with ethyl acetate (300 mL), and washed with water (100 ml×2) and saturated sodium chloride solution (100 ml×2) successively. The organic phase was dried over anhydrous sodium sulfate and filtered. The filtrate was concentrated under reduced pressure, and the resulting residues were purified by silica gel column chromatography with eluent system A to obtain the title product **3c** (1.10 g, yield: 73.9%).
MS m/z (ESI): 809.2 [M+1].

### Step 3

### Methyl 5-chloro-4-(3-((((5-(hydroxymethyl)-1-methyl-1H-pyrazol-3-yl)methyl)(methyl)amino) methyl)-1,5-dimethyl-1H-pyrazol-4-yl)-1-(3-methoxy-3-oxopropyl)-3-methyl-1H-indol e-2-carboxylate 3d

**3c** (1.10 g, 1.36 mmol) was dissolved in tetrahydrofuran (10 mL) at room temperature. Tetrabutylammonium fluoride (1.63 mL, 1.63 mmol, 1 *M* solution in tetrahydrofuran) was added dropwise, and the reaction solution was stirred at room temperature for 1 hour. The reaction solution was concentrated under reduced pressure. The resulting residues were dissolved in ethyl acetate (50 mL), and washed with water (30 ml×3) and saturated sodium chloride solution (30 ml×2) successively. The organic phase was dried over anhydrous sodium sulfate and filtered. The filtrate was concentrated under reduced pressure, and the resulting residues were purified by silica gel column chromatography with eluent system A to obtain the title product **3d** (470 mg, yield: 60.6%).
MS m/z (ESI): 571.2 [M+1].

### Step 4

### Methyl 5-chloro-4-(3-((((5-(chloromethyl)-1-methyl-1H-pyrazol-3-yl)methyl)(methyl)amino)m ethyl)-1,5-dimethyl-1H-pyrazol-4-yl)-1-(3-methoxy-3-oxopropyl)-3-methyl-1H-indole-2-carboxylate 3e

**3d** (470 mg, 0.82 mmol) was dissolved in dichloromethane (10 mL) at room temperature, and the solution was cooled to 0-5°C. Thionyl chloride (117 mg, 0.98 mmol) was slowly added dropwise, and the reaction solution was stirred at room temperature for 30 minutes. The reaction solution was concentrated under reduced pressure to obtain the title product **3e** (400 mg, yield: 82.4%), which was used directly in the next step without purification.
MS m/z (ESI): 589.1[M+1].

### Step 5

### Methyl 5-chloro-4-(3-((((5-(((4-hydroxynaphthalen-2-yl)thio)methyl)-1-methyl-1H-pyrazol-3-y 1)methyl)(methyl)amino)methyl)-1,5-dimethyl-1H-pyrazol-4-yl)-1-(3-methoxy-3-oxopr opyl)-3-methyl-1H-indole-2-carboxylate 3f

The crude product **3e** (525 mg, 0.89 mmol) was dissolved in methanol (20 mL) at room temperature. **1l** (278 mg, 1.07 mmol) and potassium carbonate (344 mg, 2.49 mmol) were added successively, and the reaction solution was stirred at room temperature for 2 hours. The reaction solution was diluted with ethyl acetate (50 mL), and washed with water (30 ml×3) and saturated sodium chloride solution (30 ml×2) successively. The organic phase was dried over anhydrous sodium sulfate and filtered. The filtrate was concentrated under reduced pressure, and the resulting residues were purified by silica gel column chromatography with eluent system A to obtain the title product **3f** (580 mg, yield: 90%).
MS m/z (ESI): 729.3[M+1].

### Step 6

### Methyl 5-chloro-4-(3-((((5-(((4-hydroxynaphthalen-2-yl)thio)methyl)-1-methyl-1H-pyrazol-3-y 1)methyl)(methyl)amino)methyl)-1,5-dimethyl-1H-pyrazol-4-yl)-1-(3-hydroxypropyl)-3 -methyl-1H-indole-2-carboxylate 3g

**3f** (580 mg, 1.23 mmol) was dissolved in tetrahydrofuran (8 mL) at room temperature, and cooled to 0-5°C. 1.0 M solution of borane in tetrahydrofuran (8 mL) was slowly added dropwise, and the reaction solution was warmed up to room temperature and stirred for 16 hours. The reaction solution was cooled to 0-5°C and quenched by methanol. The solution was warmed up to room temperature and stirred for 30 minutes. Hydrochloric acid (10.6 mL, 6.0 *N*) was added and stirred for 30 minutes. Saturated sodium bicarbonate solution was added to adjust pH to 7, and the solution was extracted with a mixed solvent (30 mL×3) of dichloromethane and methanol (V:V=10:1). The organic phases were combined, washed with saturated sodium chloride solution (30 mL), dried over anhydrous sodium sulfate and filtered. The filtrate was concentrated under reduced pressure, and the resulting residues were purified by silica gel column chromatography with eluent system A to obtain the title product **3g** (400 mg, yield: 71.7%).
MS m/z (ESI): 701.1 [M+1].

### Step 7

### Methyl 17-chloro-5,9,13,14,22-pentamethyl-28-oxa-2-thia-5,6,9,12,13,24-hexaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylate 3h

**3g** (400 mg, 570 µmol) was dissolved in toluene (10 mL) and tetrahydrofuran (5 ml) at room temperature, followed by the addition of tri-n-butylphosphine (576 mg, 2.85 mmol). The solution was purged with argon three times, and added dropwise with a solution (3 mL) of azodicarbonyl dipiperidine (720 mg, 2.85 mmol) in toluene. The reaction solution was heated to 60°C and stirred for 2 hours. The reaction solution was concentrated under reduced pressure, and the resulting residues were purified by silica gel column chromatography with eluent system A to obtain the title product **3h** (360 mg, yield: 92.4%).
MS m/z (ESI): 683.1 [M+1].

### Step 8

### 17-Chloro-5,9,13,14,22-pentamethyl-28-oxa-2-thia-5,6,9,12,13,24-hexaazaheptacyclo[2 7.7.1.1^{4,7}.0^{11,15}.0^{16,21}.0^{20,24}.0^{30,35}]octatriaconta-1(37),4(38),6,11,14,16,18,20,22,29,31,33, 35-tridecaene-23-carboxylic acid 3

**3h** (30 mg, 43.9 µmol) was dissolved in 10 mL of a mixed solution of tetrahydrofuran and methanol (V:V = 1:1) at room temperature. A solution of lithium hydroxide monohydrate (18 mg, 0.43 mmol) in water (2 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 *N*) was added dropwise until pH = 6-7, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title product **3** (25 mg, yield: 85.1%).
MS m/z (ESI): 669.0 [M+1].
¹H NMR (400 MHz, CDCl₃) δ 8.31-8.34 (m, 1H), 7.70-7.72 (m, 1H), 7.55 (s, 1H), 7.50-7.53 (m, 2H), 7.25-7.27 (m, 1H), 6.91-6.93 (m, 1H), 6.23 (s, 1H), 5.44 (s, 1H), 5.10-5.13 (m, 1H), 4.44-4.50 (m, 1H), 3.91-3.97 (m, 2H), 3.74-3.89 (m, 5H), 3.68-3.71 (m, 1H), 3.62 (s, 3H), 3.47-3.49 (m, 1H), 3.20-3.24 (m, 1H), 3.09-3.12 (m, 1H), 3.28-3.57 (m, 5H), 2.02-2.06 (m, 6H).

### Examples 3-1 and 3-2

### (Ra)-17-Chloro-5,9,13,14,22-pentamethyl-28-oxa-2-thia-5,6,9,12,13,24-hexaazaheptacy clo[27.7.1.1^{4,7}.0^{11,15}.0^{16,21}.0^{20,24}.0^{30,35}]octatriaconta-1(37),4(38),6,11,14,16,18,20,22,29,3 1,33,35-tridecaene-23-carboxylic acid 3-1

### (Sa)-17-Chloro-5,9,13,14,22-pentamethyl-28-oxa-2-thia-5,6,9,12,13,24-hexaazaheptacy clo[27.7.1.1^{4,7}.0^{11,15}.0^{16,21}.0^{20,24}.0^{30,35}]octatriaconta-1(37),4(38),6,11,14,16,18,20,22,29,3 1,33,35-tridecaene-23-carboxylic acid 3-2

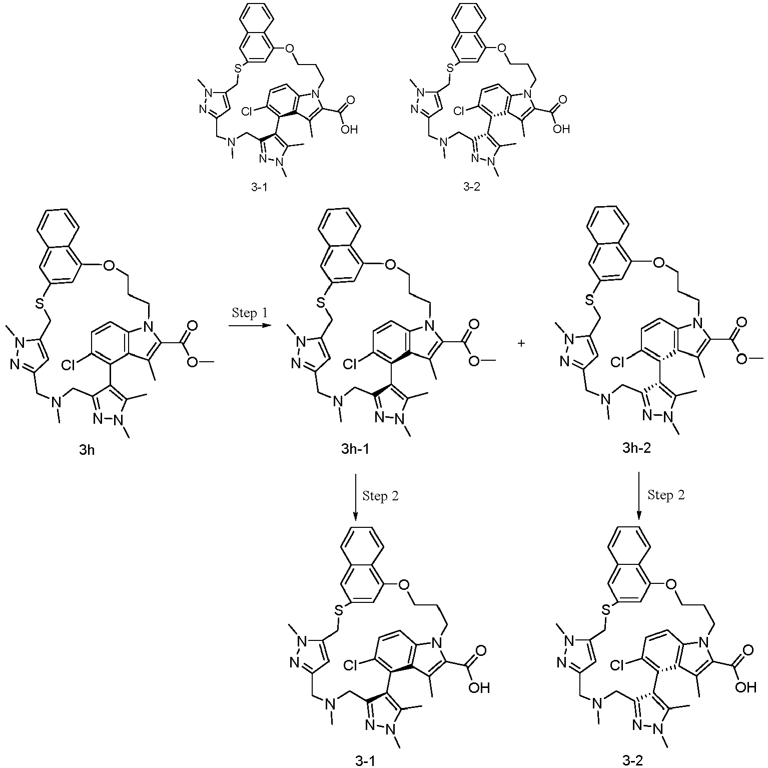

### Step 1

### Methyl (Ra)-17-chloro-5,9,13,14,22-pentamethyl-28-oxa-2-thia-5,6,9,12,13,24-hexaazaheptacy clo[27.7.1.1^{4,7}.0^{11,15}.0^{16,21}.0^{20,24}.0^{30,35}]octatriaconta-l(37),4(38),6,11,14,16,18,20,22,29,3 1,33,35-tridecaene-23-carboxylate 3h-1

### Methyl (Sa)-17-chloro-5,9,13,14,22-pentamethyl-28-oxa-2-thia-5,6,9,12,13,24-hexaazaheptacy clo[27.7.1.1^{4,7}.0^{11,15}.0^{16,21}.0^{20,24}.0^{30,35}]octatriaconta-1(37),4(38),6,11,14,16,18,20,22,29,3 1,33,35-tridecaene-23-carboxylate 3h-2

**3h** (330 mg, 0.48 mmol) was separated chirally (separation conditions: AY Phenomenex Lux Amylose-2 250^{∗}21.2mm, 5 µm; mobile phase: Hexane/EtOH/DEA=85/15/0.1(V/V/V); flow rate: 20 mL/min). The corresponding fractions were collected and concentrated under reduced pressure to obtain the title products (120 mg, 130 mg).

Compound **3h-1** with single configuration (having shorter retention time):
MS m/z (ESI): 683.1 [M+1].

Chiral HPLC analysis: retention time 8.551 minutes, chiral purity: 100% (chromatographic column: AY Phenomenex Lux Amylose-2 150^{∗}4.6mm, 5 µm, equipped with a guard column; mobile phase: *n*-hexane/ethanol/diethylamine=85/15/0.1 (v/v/v)).

Compound **3h-2** with single configuration (having longer retention time):
MS m/z (ESI):683.1 [M+1].

Chiral HPLC analysis: retention time 11.798 minutes, chiral purity: 100% (chromatographic column: AY Phenomenex Lux Amylose-2 150^{∗}4.6mm, 5 µm, equipped with a guard column; mobile phase: *n*-hexane/ethanol/diethylamine=85/15/0.1 (v/v/v)).

### Step 2

### (Ra)-17-Chloro-5,9,13,14,22-pentamethyl-28-oxa-2-thia-5,6,9,12,13,24-hexaazaheptacy clo[27.7.1.1^{4,7}.0^{11,15}.0^{16,21}.0^{20,24}.0^{30,35}]octatriaconta-1(37),4(38),6,11,14,16,18,20,22,29,3 1,33,35-tridecaene-23-carboxylic acid 3-1

### (Sa)-17-Chloro-5,9,13,14,22-pentamethyl-28-oxa-2-thia-5,6,9,12,13,24-hexaazaheptacy clo[27.7.1.1^{4,7}.0^{11,15}.0^{16,21}.0^{20,24}.0^{30,35}]octatriaconta-1(37),4(38),6,11,14,16,18,20,22,29,3 1,33,35-tridecaene-23-carboxylic acid 3-2

Compound **3h-1** (having shorter retention time)/compound **3h-2** (having longer retention time) (120 mg/120 mg, 176 µmol/176 µmol) was dissolved in 10 mL of a mixed solution of tetrahydrofuran and methanol (V:V = 1:1) at room temperature, respectively. A solution of lithium hydroxide monohydrate (74 mg/74 mg, 1.76 mmol/1.76 mmol) in water (2 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 *N*) was added dropwise until pH = 6-7, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title products **3-1** and **3-2** (80 mg, 80 mg), respectively.

Compound **3-1** with single configuration (having shorter retention time):
MS m/z (ESI): 669.0 [M+1].

Chiral HPLC analysis: retention time 10.354 minutes, chiral purity: 100% (chromatographic column: CHIRALPAK ID 250^{∗}4.6mm, 5 µm, equipped with a guard column; mobile phase: Hexane/IPA/EtOH/HAC/DEA=70/15/15/0.1/0.1(V/V/V/V/V)).
¹H NMR (400 MHz, CDCl₃) δ 8.31-8.34 (m, 1H), 7.70-7.72 (m, 1H), 7.55 (s, 1H), 7.50-7.53 (m, 2H), 7.25-7.27 (m, 1H), 6.91-6.93 (m, 1H), 6.23 (s, 1H), 5.44 (s, 1H), 5.10-5.13 (m, 1H), 4.44-4.50 (m, 1H), 3.91-3.97 (m, 2H), 3.74-3.89 (m, 5H), 3.68-3.71 (m, 1H), 3.62 (s, 3H), 3.47-3.49 (m, 1H), 3.20-3.24 (m, 1H), 3.09-3.12 (m, 1H), 3.28-3.57 (m, 5H), 2.02-2.06 (m, 6H).

Compound **3-2** with single configuration (having longer retention time):
MS m/z (ESI): 669.0 [M+1].

Chiral HPLC analysis: retention time 11.662 minutes, chiral purity: 98.3% (chromatographic column: CHIRALPAK ID 250^{∗}4.6mm, 5 µm, equipped with a guard column; mobile phase: Hexane/IPA/EtOH/HAC/DEA=70/15/15/0.1/0.1(V/V/V/V/V)).
¹H NMR (400 MHz, CDCl₃) δ 8.31-8.34 (m, 1H), 7.70-7.72 (m, 1H), 7.55 (s, 1H), 7.50-7.53 (m, 2H), 7.25-7.27 (m, 1H), 6.91-6.93 (m, 1H), 6.23 (s, 1H), 5.44 (s, 1H), 5.10-5.13 (m, 1H), 4.44-4.50 (m, 1H), 3.91-3.97 (m, 2H), 3.74-3.89 (m, 5H), 3.68-3.71 (m, 1H), 3.62 (s, 3H), 3.47-3.49 (m, 1H), 3.20-3.24 (m, 1H), 3.09-3.12 (m, 1H), 3.28-3.57 (m, 5H), 2.02-2.06 (m, 6H).

### Example 4

### 17-Chloro-5,13,14,22-tetramethyl-28-oxa-9-thia-5,6,12,13,24-pentaazaheptacyclo[27.7. 1.1^{4,7}.0^{11,15}.0^{16,21}.0^{20,24}.0^{30,35}]octatriaconta-1(37),4(38),6,11,14,16,18,20,22,29,31,33,35-t ridecaene-23-carboxylic acid 4

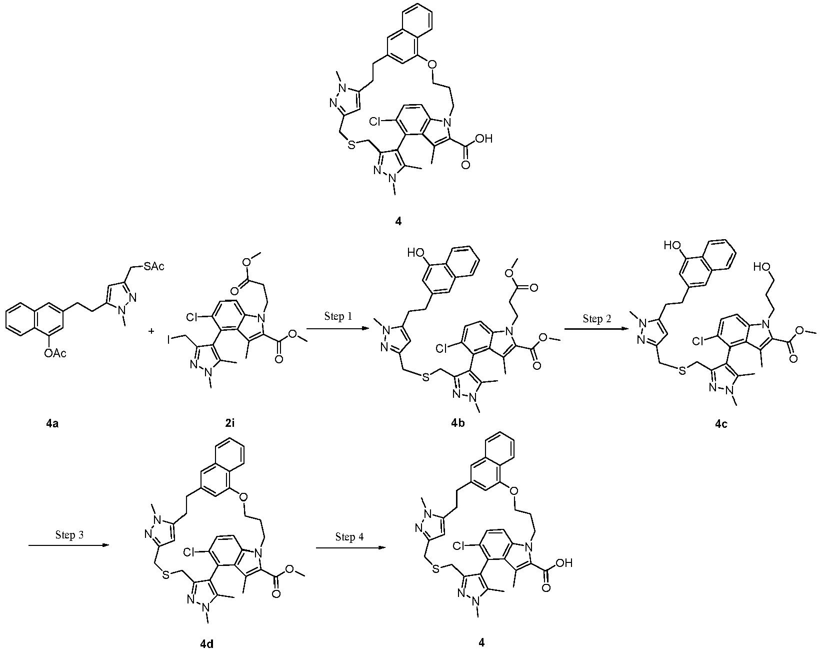

### Step 1

### Methyl 5-chloro-4-(3-((((5-(2-(4-hydroxynaphthalen-2-yl)ethyl)-1-methyl-1H-pyrazol-3-yl)met hyl)thio)methyl)-1,5-dimethyl-1H-pyrazol-4-yl)-1-(3-methoxy-3-oxopropyl)-3-methyl-1H-indole-2-carboxylate 4b

3-(2-(3-((Acetylthio)methyl)-1-methyl-1*H*-pyrazol-5-yl)ethyl)naphthalen-1-yl acetate **4a** (1.1 g, 2.88 mmol, prepared according to the method disclosed in the intermediate 46 on page 76 of the description of the patent application "WO2018178226A1") was dissolved in 20 mL of methanol. Potassium carbonate (660 mg, 4.78 mmol) and **2i** (1.3 g, 2.39 mmol) were added, and the reaction solution was reacted under an argon atmosphere at room temperature for 4 hours. The reaction solution was concentrated under reduced pressure to remove most of the solvent, and then added with 50 mL of water and extracted with ethyl acetate (50 mL×2). The organic phases were combined, washed with 30 mL of saturated sodium chloride solution, dried over anhydrous sodium sulfate and filtered. The filtrate was concentrated under reduced pressure, and the resulting residues were purified by silica gel column chromatography with eluent system B to obtain the title product **4b** (1.0 g, yield: 58.6%).
MS m/z (ESI): 714.2 [M+1].

### Step 2

### Methyl 5-chloro-4-(3-((((5-(2-(4-hydroxynaphthalen-2-yl)ethyl)-1-methyl-1H-pyrazol-3-yl)met hyl)thio)methyl)-1,5-dimethyl-1H-pyrazol-4-yl)-1-(3-hydroxypropyl)-3-methyl-1H-ind ole-2-carboxylate 4c

**4b** (1 g, 1.4 mmol) was dissolved in 10 mL of tetrahydrofuran. 1.0 *M* solution of borane in tetrahydrofuran (14 mL, 14 mmol) was added dropwise under an ice bath, and the reaction solution was stirred at room temperature for 16 hours. 10 mL of methanol and 10 mL of 6 N hydrochloric acid were added dropwise under an ice bath, and the reaction solution was stirred in the ice bath for 5 minutes. The reaction solution was warmed up to 50°C, stirred for 30 minutes, and cooled to room temperature. The reaction solution was neutralized to neutral with sodium bicarbonate solution, and the organic phase was separated. The aqueous phase was extracted with 50 mL of a mixed solvent of dichloromethane and methanol (V:V=10:1) twice. The organic phases were combined, washed with 30 mL of saturated sodium chloride solution, dried over anhydrous sodium sulfate, and filtered to remove the desiccant. The filtrate was concentrated under reduced pressure, and the resulting residues were purified by silica gel column chromatography with eluent system B to obtain the title product **4c** (850 mg, yield: 88.5%).
MS m/z (ESI):686.3 [M+1].

### Step 3

### Methyl 17-chloro-5,13,14,22-tetramethyl-28-oxa-9-thia-5,6,12,13,24-pentaazaheptacyclo[27.7. 1.1^{4,7}.0^{11,15}.0^{16,21}.0^{20,24}.0^{30,35}]octatriaconta-1(37),4(38),6,11,14,16,18,20,22,29,31,33,35-t ridecaene-23-carboxylate 4d

**4c** (500.0 mg, 0.73 mmol) was dissolved in 6 mL of anhydrous tetrahydrofuran and 30 mL of anhydrous toluene. Tri-n-butylphosphine (974.0 mg, 4.8 mmol) and azodicarbonyl dipiperidine (1.2 g, 4.8 mmol) were added at room temperature, and the reaction solution was reacted at 60°C under an argon atmosphere for 3 hours. The reaction solution was concentrated under reduced pressure to remove the solvent, and the resulting residues were purified by silica gel column chromatography with eluent system B to obtain the title product **4d** (105 mg, yield: 21.6%).
MS m/z (ESI): 668.3 [M+1].

### Step 4

### 17-Chloro-5,13,14,22-tetramethyl-28-oxa-9-thia-5,6,12,13,24-pentaazaheptacyclo[27.7. 1.1^{4,7}.0^{11,15}.0^{16,21}.0^{20,24}.0^{30,35}]octatriaconta-1(37),4(38),6,11,14,16,18,20,22,29,31,33,35-t ridecaene-23-carboxylic acid 4

**4d** (40 mg, 0.06 mmol) was dissolved in a mixed solvent of 1 mL of methanol, 0.5 mL of tetrahydrofuran and 0.5 mL of water. Lithium hydroxide monohydrate (42 mg, 1 mmol) was added, and the reaction solution was stirred at 50°C for 1 hour. The reaction solution was neutralized to neutral with 2*N* hydrochloric acid, and concentrated under reduced pressure to remove most of the solvent. The resulting residues were purified by high performance liquid chromatography (Sharpsil-T C18 Column 21.2^{∗}150mm 5 µm; eluent system: 10 mmoL/L ammonium acetate, water, acetonitrile) to obtain the title product **4** (7.8 mg, yield: 19.9%).
MS m/z (ESI): 654.2 [M+1].
¹H NMR (400 MHz, DMSO-*d₆*) δ 8.17-8.15 (m, 1H), 7.72-7.70 (m, 1H), 7.52-7.49 (m, 1H), 7.45-7.43 (m, 2H), 7.19 (s, 1H), 7.02-7.00 (m, 1H), 6.09 (s, 1H), 4.99-4.95 (m, 1H), 4.90(s, 1H), 4.59-4.58 (m, 1H), 3.72-3.68 (m, 4H), 3.53-3.50 (m, 1H), 3.36 (s, 3H), 3.26 (s, 2H), 3.00-2.96 (m, 3H), 2.80-2.72 (m, 3H), 2.29-2.20 (m, 2H), 2.01 (s, 3H), 1.88 (s, 3H).

### Examples 4-1 and 4-2

### (Ra)-17-Chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{30,35}]octatriaconta-1(37),4(38),6,11,14,16,18,20,22,29,31,3 3,35-tridecaene-23-carboxylic acid 4-1

### (Sa)-17-Chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{30,35}]octatriaconta-1(37),4(38),6,11,14,16,18,20,22,29,31,3 3,35-tridecaene-23-carboxylic acid 4-2

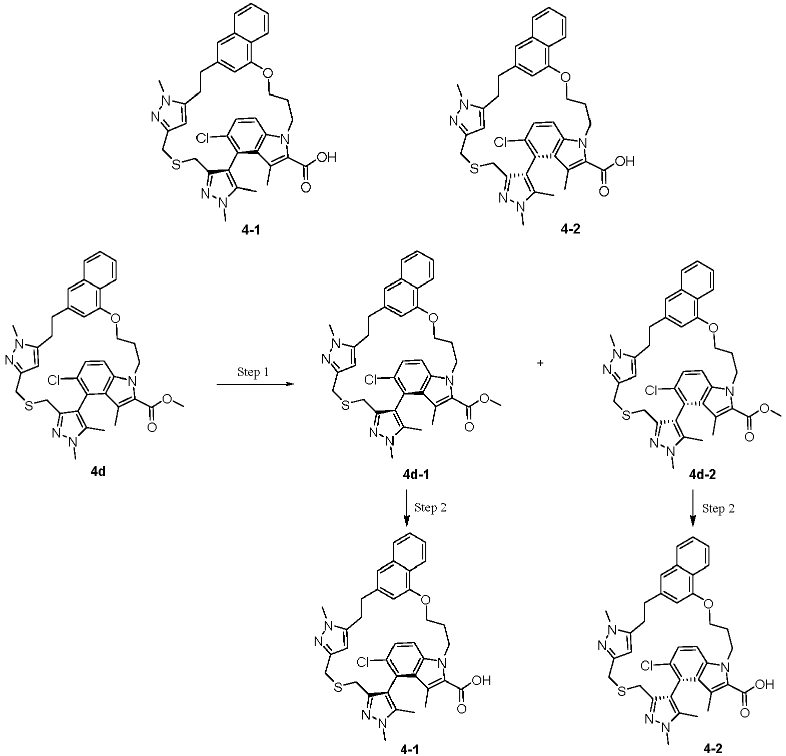

### Step 1

### Methyl (Ra)-17-chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{30,35}]octatriaconta-1(37),4(38),6,11,14,16,18,20,22,29,31,3 3,35-tridecaene-23-carboxylate 4d-1

### Methyl (Sa)-17-chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{30,35}]octatriaconta-1(37),4(38),6,11,14,16,18,20,22,29,31,3 3,35-tridecaene-23-carboxylate 4d-2

Compound **4d** (90 mg, 0.13 mmol) was separated chirally (separation conditions: AD Phenomenex Lux Amylose-1 chiral preparative column, 30 mm I.D.nex Lux Amyl; mobile phase: Hexane/EtOH(0.1%DEA)=70/30(V/V); flow rate: 60 mL/min). The corresponding fractions were collected and concentrated under reduced pressure to obtain the title products (40 mg, 40 mg).

Compound **4d-1** with single configuration (having shorter retention time):
MS m/z (ESI):668.3 [M+1].

Chiral HPLC analysis: retention time 4.397 minutes, chiral purity: 100% (chromatographic column: AD Phenomenex Lux Amylose-1 150^{∗}4.6mm, 5 µm, equipped with a guard column; mobile phase: Hexane /EtOH(0.1%DEA)=70/30(V/V)).

Compound **4d-2** with single configuration (having longer retention time):
MS m/z (ESI):668.3 [M+1].

Chiral HPLC analysis: retention time 8.515 minutes, chiral purity: 100% (chromatographic column: AD Phenomenex Lux Amylose-1 150^{∗}4.6mm, 5 µm, equipped with a guard column; mobile phase: Hexane /EtOH(0.1%DEA)=70/30(V/V)).

### Step 2

### (Ra)-17-Chloro-5,13,14,22-tetramethyl-28-oxa-9-thia-5,6,12,13,24-pentaazaheptacyclo[ 27.7.1.1^{1,7}.0^{11,15}.0^{16,21}.0^{20,24}.0^{30,35}]octatriaconta-1(37),4(38),6,11,14,16,18,20,22,29,31,3 3,35-tridecaene-23-carboxylic acid 4-1

### (Sa)-17-Chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{30,35}]octatriaconta-1(37),4(38),6,11,14,16,18,20,22,29,31,3 3,35-tridecaene-23-carboxylic acid 4-2

Compound **4d-1** (having shorter retention time)/compound **4d-2** (having longer retention time) (40 mg/40 mg, 0.06 mmol/0.06 mmol) was dissolved in 2 mL of a mixed solution of tetrahydrofuran and methanol (V:V = 1:1) at room temperature, respectively. A solution of lithium hydroxide monohydrate (42 mg, 1 mmol) in water (1 mL) was added, and the reaction solution was heated to 50°C and stirred for 1 hour. The reaction solution was cooled to room temperature, diluted with water (5 mL), and concentrated under reduced pressure to remove most of the organic solvent. Diluted hydrochloric acid (2.0 N) was added dropwise until pH = 6-7, and the solution was extracted with a mixed solvent (20 mL×2) of dichloromethane and methanol (V:V=10:1). The organic phases were combined, washed with water (10 mL) and saturated sodium chloride solution (10 mL), dried over anhydrous sodium sulfate, and filtered to remove the desiccant. The filtrate was concentrated under reduced pressure, and the resulting residues were purified by high performance liquid chromatography (Sharpsil-T C18 Column 21.2^{∗}150mm 5 µm, eluent system: water (10 mmol ammonium acetate), acetonitrile) to obtain the title products **4-1/4-2**(18 mg, 18 mg), respectively.

Compound **4-1** with single configuration (having shorter retention time):
MS m/z (ESI): 654.2 [M+1].

Chiral HPLC analysis: retention time 8.224 minutes, chiral purity: 100% (chromatographic column: CHIRALPAK IF 150^{∗}4.6 mm, 5 µm; mobile phase: n-hexane/ethanol (containing 0.1% trifluoroacetic acid) =80:20(v/v)).
¹H NMR (400 MHz, CD₃OD) δ 8.30-8.28 (m, 1H), 7.75-7.73 (m, 1H), 7.48-7.45 (m, 2H), 7.36-7.34 (d, 1H), 7.15 (s, 1H), 6.80-6.77 (d, 1H), 5.98 (s, 1H), 5.14-5.10 (m, 1H), 4.83(s, 1H), 4.69-4.63 (m, 1H), 3.87-3.81 (m, 4H), 3.53 (s, 3H), 3.42-3.28 (m, 1H), 3.19-3.17 (m, 2H), 3.07-3.02 (m, 3H) , 2.77-2.72 (m, 3H), 2.37-2.36 (m, 2H), 1.99 (s, 3H), 1.97 (s, 3H).

Compound **4-2** with single configuration (having longer retention time):
MS m/z (ESI): 654.2 [M+1].

Chiral HPLC analysis: retention time 10.998 minutes, chiral purity: 100% (chromatographic column: CHIRALPAK IF 150^{∗}4.6 mm, 5 µm; mobile phase: n-hexane/ethanol (containing 0.1% trifluoroacetic acid) =80:20(v/v)).
¹H NMR (400 MHz, CD₃OD) δ 8.30-8.27 (m, 1H), 7.75-7.73 (m, 1H), 7.48-7.45 (m, 2H), 7.36-7.34 (d, 1H), 7.15 (s, 1H), 6.80-6.77 (d, 1H), 5.98 (s, 1H), 5.14-5.10 (m, 1H), 4.83(s, 1H), 4.69-4.63 (m, 1H), 3.87-3.81 (m, 4H), 3.53 (s, 3H), 3.44-3.28 (m, 1H), 3.19-3.17 (m, 2H), 3.07-3.02 (m, 3H), 2.77-2.72 (m, 3H), 2.11 (m, 2H), 1.99 (s, 3H), 1.94 (s, 3H).

### Example 5

### 17-Chloro-5,9,13,14,22,31-hexamethyl-28-oxa-2-thia-5,6,9,12,13,24-hexaazahexacyclo [27.3.1.1^{4,7}.0^{11,15}.0^{16,21}.0^{20,24}]tetratriaconta-1(33),4(34),6,11,14,16,18,20,22,29,31-undec aene-23-carboxylic acid 5

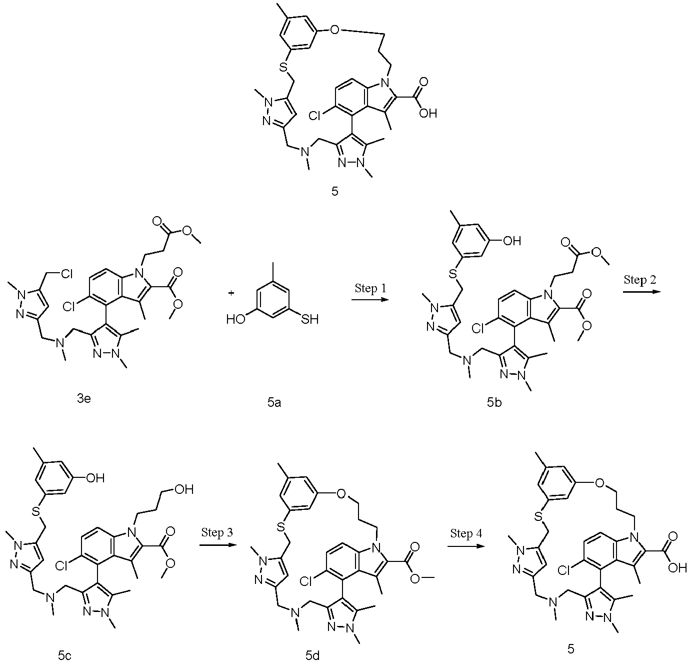

### Step 1

### Methyl 5-chloro-4-(3-((((5-(((3-hydroxy-5-methylphenyl)thio)methyl)-1-methyl-1H-pyrazol-3-yl)methyl)(methyl)amino)methyl)-1,5-dimethyl-1H-pyrazol-4-yl)-1-(3-methoxy-3-oxop ropyl)-3-methyl-1H-indole-2-carboxylate 5b

The crude product **3e** (150 mg, 0.25 mmol) was dissolved in methanol (10 mL) at room temperature. **5a** (60 mg, 427.95 µmol, prepared according to the method disclosed in Journal of Organic Chemistry, 2003, vol. 68, # 23, p. 9116 - 9118) and potassium carbonate (106 mg, 0.77 mmol) were added successively, and the reaction solution was stirred at room temperature for 2 hours. The reaction solution was diluted with ethyl acetate (50 mL), and washed with water (30 ml×3) and saturated sodium chloride solution (30 ml×2) successively. The organic phase was dried over anhydrous sodium sulfate and filtered. The filtrate was concentrated under reduced pressure, and the resulting residues were purified by silica gel column chromatography with eluent system A to obtain the title product **5b** (80 mg, yield: 45.35%).
MS m/z (ESI): 693.0 [M+1].

### Step 2

### Methyl 5-chloro-4-(3-((((5-(((3-hydroxy-5-methylphenyl)thio)methyl)-1-methyl-1H-pyrazol-3-yl)methyl)(methyl)amino)methyl)-1,5-dimethyl-1H-pyrazol-4-yl)-1-(3-hydroxypropyl)-3-methyl-1H-indole-2-carboxylate 5c

**5b** (80 mg, 0.12 mmol) was dissolved in tetrahydrofuran (2 mL) at room temperature, and cooled to 0-5°C. 1 M solution of borane in tetrahydrofuran (1.16 mL) was slowly added dropwise, and the reaction solution was warmed up to room temperature and stirred for 16 hours. The reaction solution was cooled to 0-5°C and quenched by methanol. The solution was warmed up to room temperature and stirred for 30 minutes. Hydrochloric acid (2 mL, 6.0 N) was added and stirred for 30 minutes. Saturated sodium bicarbonate solution was added to adjust pH to 7, and the solution was extracted with a mixed solvent (30 mL×3) of dichloromethane and methanol (V:V=10:1). The organic phases were combined, washed with saturated sodium chloride solution (30 mL), dried over anhydrous sodium sulfate and filtered. The filtrate was concentrated under reduced pressure, and the resulting residues were purified by silica gel column chromatography with eluent system A to obtain the title product **5c** (50 mg, yield: 65.1%).
MS m/z(ESI): 665.1 [M+1].

### Step 3

### Methyl

### 17-chloro-5,9,13,14,22,31-hexamethyl-28-oxa-2-thia-5,6,9,12,13,24-hexaazahexacyclo[ 27.3.1.1^{1,7}.0^{11,15}.0^{16,21}.0^{20,24}]tetratriaconta-1(33),4(34),6,11,14,16,18,20,22,29,31-undeca ene-23-carboxylate 5d

**5c** (20 mg, 30 µmol) was dissolved in toluene (4 mL) and tetrahydrofuran (2 mL) at room temperature, followed by the addition of tri-n-butylphosphine (40 mg, 0.15 mmol). The solution was purged with argon three times, and added dropwise with a solution (3 mL) of azodicarbonyl dipiperidine (31 mg, 0.15 mmol) in toluene. The reaction solution was heated to 60°C and stirred for 2 hours. The reaction solution was concentrated under reduced pressure, and the resulting residues were purified by silica gel column chromatography with eluent system A to obtain the title product **5d** (10 mg, yield: 51.4%).
MS m/z (ESI): 647.1 [M+1].

### Step 4

### 17-Chloro-5,9,13,14,22,31-hexamethyl-28-oxa-2-thia-5,6,9,12,13,24-hexaazahexacyclo [27.3.1.1^{4,7}.0^{11,15}.0^{16,21}.0^{20,24}]tetratriaconta-1(33),4(34),6,11,14,16,18,20,22,29,31-undec aene-23-carboxylic acid 5

**5d** (10 mg, 15 µmol) was dissolved in 6 mL of a mixed solution of tetrahydrofuran and methanol (V:V = 1:1) at room temperature. A solution of lithium hydroxide monohydrate (20 mg, 0.48 mmol) in water (2 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 6-7, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title product **5** (8 mg, yield: 85.1%).
MS m/z (ESI): 633.0 [M+1].
¹H NMR (400 MHz, DMSO-*d*₆) δ 7.66-7.69 (m, 1H), 7.30-7.32 (m, 1H), 6.64 (s, 1H), 6.47 (s, 1H), 6.40 (s, 1H), 4.85-4.90 (m, 2H), 4.69 (s, 1H), 4.30-4.34 (m, 2H), 3.92-3.94 (m, 1H), 3.70 (s, 3H), 3.67 (s, 3H), 3.54-3.57 (m, 1H), 3.33 (brs, 1H), 3.07-3.13 (m, 2H), 2.72-2.80 (m, 2H), 2.19-2.22 (m, 2H), 2.06 (s, 3H), 1.93 (s, 3H), 1.85 (s, 3H), 1.78(s, 3H).

### Example 6

### 17-Chloro-5,13,14,22,31-pentamethyl-28-oxa-2,9-dithia-5,6,12,13,24-pentaazahexacycl o[27.3.1.1^{4,7}.0^{11,15}.0^{16,21}.0^{20,24}]tetratriaconta-1(33),4(34),6,11,14,16,18,20,22,29,31-unde caene-23-carboxylic acid 6

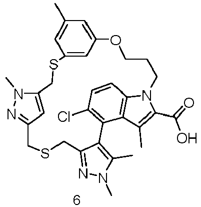

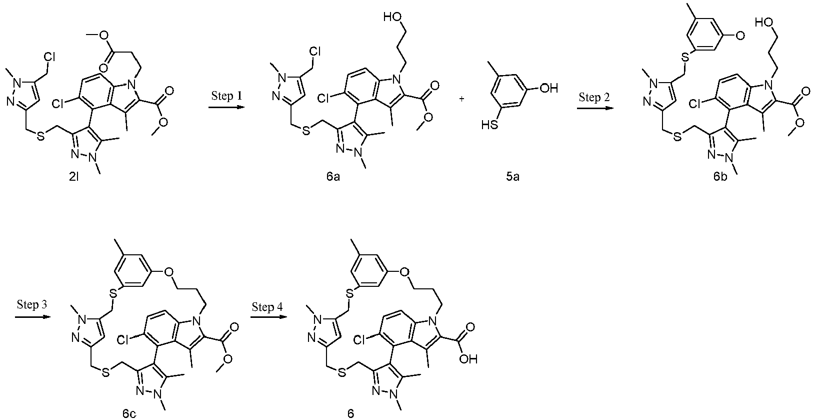

### Step 1

### Methyl 5-chloro-4-(3-((((5-(chloromethyl)-1-methyl-1H-pyrazol-3-yl)methyl)thio)methyl)-1,5-dimethyl-1H-pyrazol-4-yl)-1-(3-hydroxypropyl)-3-methyl-1H-indole-2-carboxylate 6a

**2l** (580 mg, 0.98 mmol) was dissolved in 2 mL of tetrahydrofuran. 1.0 M solution of borane in tetrahydrofuran (9.80 mL, 9.80 mmol) was added dropwise under an ice bath, and the reaction solution was stirred at room temperature for 16 hours. 5 mL of methanol and 10 mL of diluted hydrochloric acid (6M) were added dropwise under an ice bath, and the reaction solution was stirred for 1 hour. 50 mL of water was added to the reaction solution, which was extracted with dichloromethane (50 mL×2). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system A to obtain the title product **6a** (390 mg, yield: 70.57%).
MS m/z (ESI): 564.1 [M+1].

### Step 2

### Methyl 5-chloro-4-(3-((((5-(((3-hydroxy-5-methylphenyl)thio)methyl)-1-methyl-1H-pyrazol-3-yl)methyl)thio)methyl)-1,5-dimethyl-1H-pyrazol-4-yl)-1-(3-hydroxypropyl)-3-methyl-1 H-indole-2-carboxylate 6b

**6a** (100 mg, 0.18 mmol) and **5a** (30 mg, 0.55 mmol) were dissolved in 10 mL of methanol. Potassium carbonate (176 mg, 1.28 mmol) was added at room temperature, and the reaction solution was reacted at room temperature for 1 hour. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system A to obtain the title product **6b** (40 mg, yield: 33.80%).
MS m/z (ESI):668.0 [M+1].

### Step 3

### Methyl 17-chloro-5,13,14,22,31-pentamethyl-28-oxa-2,9-dithia-5,6,12,13,24-pentaazahexacycl o[27.3.1.1^{4,7}.0^{11,15}.o^{16,21}.o^{20,24}]tetratriaconta-1(33),4(34),6,11,14,16,18,20,22,29,31-unde czene-23-carboxylate 6c

Triphenylphosphine (38 mg, 0.15 mmol) and azodicarbonyl dipiperidine (31 mg, 0.15 mmol) were dissolved in 2 mL of toluene, and the solution was purged with argon three times. 6 mL of a solution of **6b** (20 mg, 0.03 mmol) in toluene and tetrahydrofuran (V:V=5:1) was added dropwise, and the reaction solution was stirred at room temperature for 16 hours. 10 mL of diluted hydrochloric acid (2M) was added, and the reaction solution was stirred for 10 minutes. 50 mL of water was added to the reaction solution, and extracted with ethyl acetate (50 mL×2). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system A to obtain the title product **6c** (10 mg, yield: 51.38%), which contained triphenylphosphine oxide and was used directly in the next step.
MS m/z (ESI): 650.0 [M+1].

### Step 4

### 17-Chloro-5,13,14,22,31-pentamethyl-28-oxa-2,9-dithia-5,6,12,13,24-pentaazahexacycl o[27.3.1.1^{4,7}.0^{11,15}.0^{16,21}.0^{20,24}]tetratriaconta-1(33),4(34),6,11,14,16,18,20,22,29,31-unde caene-23-carboxylic acid 6

**6c** (10 mg, 0.015 mmol) was dissolved in 6 mL of a mixed solvent of methanol, tetrahydrofuran and water (V:V:V=1:1:1). Lithium hydroxide monohydrate (7 mg, 0.15 mmol) was added, and the reaction solution was heated to 50°C and stirred for 0.5 hour. The reaction solution was concentrated under reduced pressure to remove most of the solvent. 2*M* hydrochloric acid was added to adjust pH to 1-2, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system A to obtain the title product **6** (4 mg, yield: 40.88%).
MS m/z (ESI): 636.2 [M+1].
¹H NMR (400 MHz, DMSO-d₆)δ 8.34-8.36 (m, 1H), 7.75-7.77 (m, 1H), 7.62 (s, 1H), 7.30-7.33 (m, 1H), 7.02-7.04 (m, 1H), 6.11 (s, 1H), 5.17-5.20 (m, 1H), 5.09 (s, 1H), 4.58-4.62 (m, 1H), 3.81-3.95 (m, 5H), 3.71-3.74 (m, 1H), 3.64 (s, 3H), 3.45-3.49 (m, 2H), 3.24-3.28 (m, 1H), 3.12-3.16 (m, 1H), 2.75-2.78 (m, 1H), 2.34-2.41 (m, 2H), 2.22 (s, 3H), 2.01 (s, 3H) 1.34 (s, 3H).

### Example 7

### 17-Chloro-5,13,14,22-tetramethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,35-un decaene-23-carboxylic acid 7

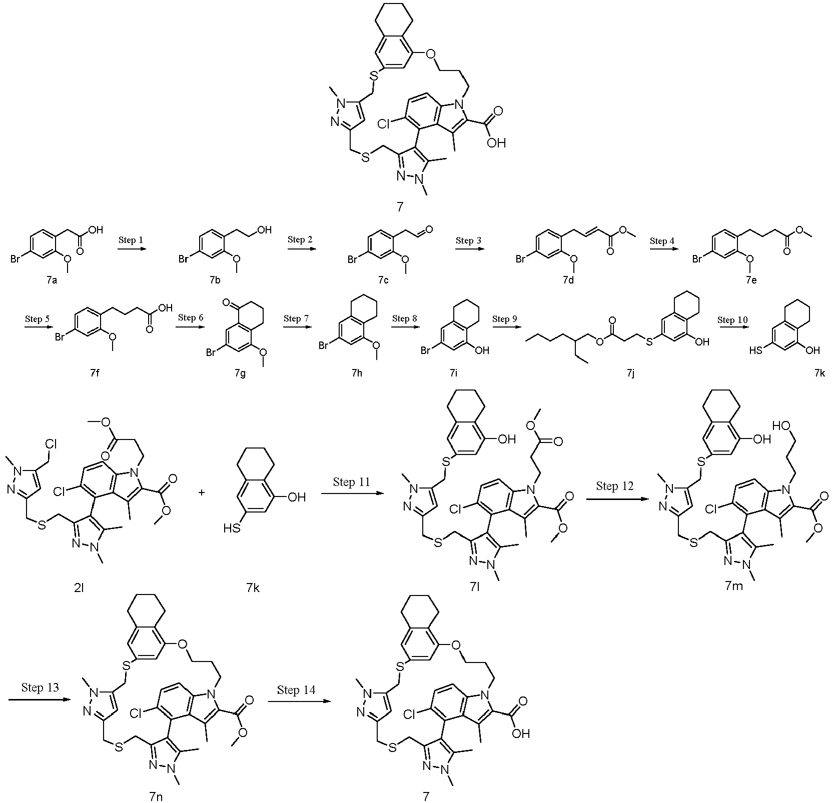

### Step 1

### 2-(4-Bromo-2-methoxyphenyl)ethanol 7b

4-Bromo-2-methoxyphenylacetic acid **7a** (5.00 g, 20.40 mmol) was dissolved in tetrahydrofuran (50 mL), and cooled to 0-5°C. 1 M solution of borane in tetrahydrofuran (27 mL) was slowly added dropwise, and the reaction solution was warmed up to room temperature and stirred for 16 hours. The reaction solution was cooled to 0-5°C and quenched by methanol (6 mL), followed by the addition of water (12 mL). The solution was stirred for 30 minutes, and extracted with ethyl acetate (50 mL×3). The organic phases were combined, washed with saturated sodium chloride solution (30 mL), dried over anhydrous sodium sulfate and filtered. The filtrate was concentrated under reduced pressure to obtain the title product **7b** (4.71 g, yield: 100%), which was used directly in the next step without purification.

### Step 2

### 2-(4-Bromo-2-methoxyphenyl)acetaldehyde 7c

The crude product **7b** (3.70 g, 16.01 mmol) was dissolved in dichloromethane (50 mL). Dess-Martin oxidant (10.19 g, 24.03 mmol) was added in batches under an ice bath, and the reaction solution was warmed to room temperature and stirred for 1 hour. 50 mL of water, 50 mL of dichloromethane, saturated sodium thiosulfate solution and saturated sodium bicarbonate solution were added under an ice bath, and the resulting solution was partitioned. The organic phase was washed with saturated sodium chloride solution three times, dried over anhydrous sodium sulfate and filtered. The filtrate was concentrated under reduced pressure, and the resulting residues were purified by silica gel column chromatography with eluent system B to obtain the title product **7c** (3.00 g, yield: 81.8%).

### Step 3

### Methyl (E)-4-(4-bromo-2-methoxyphenyl)but-2-enoate 7d

NaH (753 mg, 19.65 mmol, purity: 60%) was dissolved in tetrahydrofuran (30 mL), and the solution was purged with argon three times. Trimethylphosphonoacetate (3.58 g, 19.66 mmol) was added dropwise under an ice bath, and the reaction solution was stirred in the ice bath for 30 minutes. A solution of **7c** in tetrahydrofuran (10 mL) was added dropwise, and the reaction solution was stirred at room temperature for 1 hour. Ethyl acetate (100 mL) and water (100 mL) were added under an ice bath, and the resulting solution was partitioned. The organic phase was washed with saturated sodium chloride solution (30 ml×2), dried over anhydrous sodium sulfate and filtered. The filtrate was concentrated under reduced pressure, and the resulting residues were purified by silica gel column chromatography with eluent system B to obtain the title product **7d** (2.37 g, yield: 63.5%).
MS m/z (ESI): 285.0 287.0 [M+1].

### Step 4

### Methyl 4-(4-bromo-2-methoxyphenyl)butanoate 7e

**7d** (2.80 g, 9.82 mmol) was dissolved in methanol (50 mL) at room temperature, followed by the addition of 5% dry rhodium carbon (280 mg). The reaction solution was purged with hydrogen three times, and stirred at room temperature for 60 minutes. The reaction solution was filtered, and the filtrate was concentrated under reduced pressure to obtain the title product **7e** (2.50 g, yield: 88.7%).
MS m/z(ESI): 287.1 289.1[M+1].

### Step 5

### 4-(4-Bromo-2-methoxyphenyl)butanoic acid 7f

The crude product **7e** (3.60 g, 12.54 mmol) was dissolved in methanol (30 mL), tetrahydrofuran (30 mL) and water (30 mL) at room temperature. Lithium hydroxide monohydrate (2.63 g, 62.67 mmol) was added, and the reaction solution was heated to 50°C and stirred for 1 hour. The reaction solution was concentrated under reduced pressure, followed by the addition of water (100 mL) and dichloromethane (100 mL). The solution was adjusted to pH=2∼3 with 1M HCl, extracted with a mixed solvent (50 mL) of dichloromethane and methanol (V:V=10:1) and partitioned. The organic phase was washed with water (30 mL×3) and saturated sodium chloride solution (30 mL×2) successively, dried over anhydrous sodium sulfate and filtered. The filtrate was concentrated under reduced pressure to obtain the title product **7f** (3.40 g, yield: 99.3%).
MS m/z (ESI): 271.1 273.1[M-1].

### Step 6

### 7-Bromo-5-methoxy-3,4-dihydronaphthalen-1(2H)-one 7g

**7f** (3.40 g, 12.45 mmol) was weighed into a 100 mL reaction flask at room temperature, followed by the addition of polyphosphoric acid (60 g, 17.75 mmol). The reaction solution was heated to 95°C and stirred for 1.5 hours. The reaction solution was poured into ice water, and added with dichloromethane (100 mL) and partitioned. The organic phase was washed with sodium bicarbonate solution, water and saturated sodium chloride solution (30mL×3) successively, dried over anhydrous sodium sulfate and filtered. The filtrate was concentrated under reduced pressure, and the resulting residues were purified by silica gel column chromatography with eluent system B to obtain the title product **7g** (1.76 g, yield: 55.4%).
MS m/z(ESI): 255.1 257.1 [M+1].

### Step 7

### 7-Bromo-5-methoxy-1,2,3,4-tetrahydronaphthalene 7h

**7g** (1.76 g, 6.90 mmol) was dissolved in trifluoroacetic acid (20 ml) at room temperature, followed by the addition of triethyl silicane (1.60 g, 13.76 mmol). The reaction solution was heated to 80°C and stirred for 1.5 hours. The reaction solution was concentrated under reduced pressure, and added with ethyl acetate (50 mL) and water (50 mL) and then partitioned. The organic phase was washed with water and saturated sodium chloride solution (30 ml×3) successively, dried over anhydrous sodium sulfate and filtered. The filtrate was concentrated under reduced pressure, and the resulting residues were purified by silica gel column chromatography with eluent system B to obtain the title product **7h** (1.60 g, yield: 96.2%).

### Step 8

### 3-Bromo-5,6,7,8-tetrahydronaphthalen-1-ol 7i

**7h** (1.55 g, 6.43 mmol) was dissolved in 20 mL of dichloromethane at room temperature. 1 M solution of boron tribromide (22.5 mL, 22.5 mmol) in dichloromethane was added dropwise under an ice bath, and the reaction solution was stirred at room temperature for 2 hours. The reaction solution was poured into ice water, and extracted with dichloromethane (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **7i** (1.40 g, yield: 95.9%).
MS m/z (ESI): 225.0 227.0 [M-1].

### Step 9

### 2-Ethylhexyl 3-((4-hydroxy-5,6,7,8-tetrahydronaphthalen-2-yl)thio)propanoate 7j

**7i** (500 mg, 2.20 mmol), 2-ethylhexyl 3-mercaptopropionate (577 mg, 2.64 mmol), N,N-diisopropylethylamine (569 mg, 4.40 mmol), tris(dibenzylideneacetone)dipalladium (101 mg, 0.11 mmol) and 4,5-bis(diphenylphosphine)-9,9-dimethylxanthene (127 mg, 0.22 mmol) were dissolved in 20 mL of dioxane at room temperature. The reaction solution was purged with argon three times, heated to 95°C and stirred for 16 hours. The reaction solution was filtered through celite, and the filtrate was concentrated under reduced pressure. The resulting residues were purified by silica gel column chromatography with eluent system B to obtain the title product **7j** (800 mg, yield: 99.7%).
MS m/z (ESI): 365.3 [M+1].

### Step 10

### 3-Mercapto-5,6,7,8-tetrahydronaphthalen-1 -ol 7k

**7j** (650 mg, 1.78 mmol) was dissolved in 20 mL of tetrahydrofuran under an ice bath, and the solution was purged with argon three times. 1M solution of potassium *tert*-butoxide (5.7 mL, 5.70 mmol) in tetrahydrofuran was added dropwise, and the reaction solution was stirred at room temperature for 2 hours. The title product **7k** was obtained, which was used directly in the next step.

### Step 11

### Methyl 5-chloro-4-(3-((((5-(((4-hydroxy-5,6,7,8-tetrahydronaphthalen-2-yl)thio)methyl)-1-meth yl-1H-pyrazol-3-yl)methyl)thio)methyl)-1,5-dimethyl-1H-pyrazol-4-yl)-1-(3-methoxy-3 -oxopropyl)-3-methyl-1H-indole-2-carboxylate 7l

**2l** (150 mg, 0.25 mmol) was dissolved in methanol (10 mL) and tetrahydrofuran (3 mL) at room temperature. 0.07 M solution of the above reaction solution **7k** (5.8 mL, 0.40 mmol) in tetrahydrofuran was added dropwise under an ice bath, and the reaction solution was stirred at room temperature for 1 hour. The reaction solution was diluted with ethyl acetate (50 mL), and washed with water (30 ml×3) and saturated sodium chloride solution (30 ml×2) successively. The organic phase was dried over anhydrous sodium sulfate and filtered. The filtrate was concentrated under reduced pressure, and the resulting residues were purified by silica gel column chromatography with eluent system A to obtain the title product **7l** (60 mg, yield: 32.2%).
MS m/z(ESI): 736.0[M+1].

### Step 12

### Methyl 5-chloro-4-(3-((((5-(((4-hydroxy-5 ,6,7,8-tetrahydronaphthalen-2-yl)thio)methyl)-1-meth yl-1H-pyrazol-3-yl)methyl)thio)methyl)-1,5-dimethyl-1H-pyrazol-4-yl)-1-(3-hydroxypr opyl)-3-methyl-1H-indole-2-carboxylate 7m

**7l** (60 mg, 81 µmol) was dissolved in tetrahydrofuran (4 mL) at room temperature, and cooled to 0-5 °C. 1.0 M solution of borane in tetrahydrofuran (0.8 mL) was slowly added dropwise, and the reaction solution was warmed up to room temperature and stirred for 16 hours. The reaction solution was cooled to 0-5°C and quenched by methanol. The solution was warmed up to room temperature and stirred for 30 minutes. Hydrochloric acid (2 mL, 6.0 *N*) was added and stirred for 30 minutes. The solution was extracted with a mixed solvent (30 mL×3) of dichloromethane and methanol (V:V=10:1). The organic phases were combined, washed with saturated sodium chloride solution (30 mL), dried over anhydrous sodium sulfate and filtered. The filtrate was concentrated under reduced pressure, and the resulting residues were purified by silica gel column chromatography with eluent system A to obtain the title product **7m** (60 mg, yield: 100%).
MS m/z (ESI): 708.0 [M+1].

### Step 13

### Methyl 17-chloro-5,13,14,22-tetramethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,35-un decaene-23-carboxylate 7n

**7m** (60 mg, 84 µmol) was dissolved in toluene (10 mL) and tetrahydrofuran (5 ml) at room temperature, followed by the addition of tri-n-butylphosphine (86 mg, 0.42 mmol). The solution was purged with argon three times, and added dropwise with a solution (3 mL) of azodicarbonyl dipiperidine (107 mg, 0.42 mmol) in toluene . The reaction solution was heated to 60°C and stirred for 2 hours. The reaction solution was concentrated under reduced pressure, and the resulting residues were purified by silica gel column chromatography with eluent system A to obtain the title product **7n** (30 mg, yield: 51.3%).
MS m/z (ESI): 690.2 [M+1].

### Step 14

### 17-Chloro-5,13,14,22-tetramethyl-28-oxa-2,9-dithia-5,6,12,13,24-pentaazaheptacyclo[2 7.7.1.1^{4,7}.0^{11,15}.0^{16,21}.0^{20,24}.0^{30,35}]octatriaconta-1(37),4(38),6,11,14,16,18,20,22,29,35-un decaene-23-carboxylic acid 7

**7n** (30 mg, 43 µmol) was dissolved in 10 mL of a mixed solution of tetrahydrofuran and methanol (V:V = 1:1) at room temperature. A solution of lithium hydroxide monohydrate (18 mg, 0.43 mmol) in water (2 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 2-3, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Waters 2767-SQ Detecor2, eluent system: H₂O (10 mmol NH₄OAc), ACN) to obtain the title product **7** (15 mg, yield: 51.0%).
MS m/z (ESI): 676.0 [M+1].
¹H NMR (400 MHz, CDCl₃) δ 7.27-7.30 (m, 1H), 7.22-7.24 (m, 1H), 6.92 (s, 1H), 5.89 (s, 1H), 5.19 (s, 1H), 5.04-5.08 (m, 1H), 4.45-4.49 (m, 1H), 3.88 (s, 3H), 3.78-3.81 (m, 1H), 3.63-3.72 (m, 2H), 3.53 (s, 3H), 3.37-3.42 (m, 1H), 3.32-3.36 (m, 1H), 3.22-3.33 (m, 1H), 3.11-3.15 (m, 1H), 2.70-2.85 (m, 5H), 2.19-2.32 (m, 5H), 2.04 (s, 3H), 1.78-1.86 (m, 4H).

### Examples 7-1 and 7-2

### (Ra)-17-Chloro-5,13,14,22-tetramethyl-28-oxa-2,9-dithia-5,6,12,13,24-pentaazaheptacy clo[27.7.1.1^{4,7}.0^{11,15}.0^{16,21}.0^{20,24}.0^{30,35}]octatriaconta-1(37),4(38),6,11,14,16,18,20,22,29,3 5-undecaene-23-carboxylic acid 7-1

### (Sa)-17-Chloro-5,13,14,22-tetramethyl-28-oxa-2,9-dithia-5,6,12,13,24-pentaazaheptacy clo[27.7.1.1^{4,7}.0^{11,15}.0^{16,21}.0^{20,24}.0^{30,35}]octatriaconta-1(37),4(38),6,11,14,16,18,20,22,29,3 5-undecaene-23-carboxylic acid 7-2

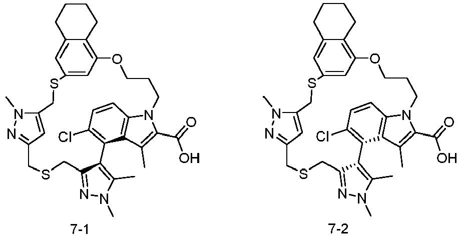

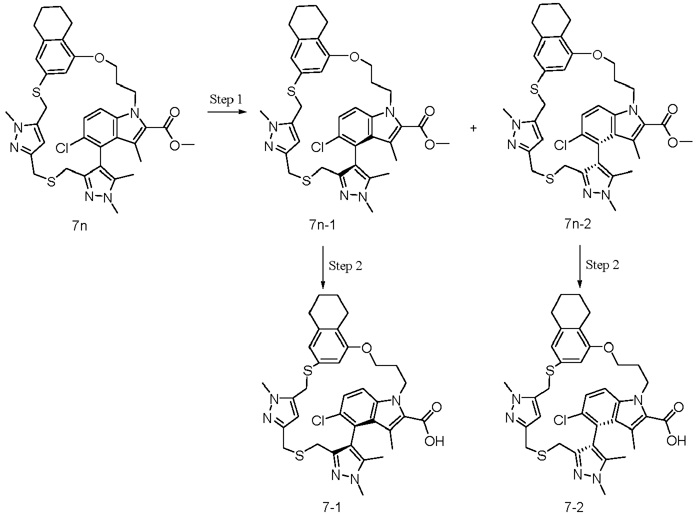

### Step 1

### Methyl (Ra)-17-chloro-5,13,14,22-tetramethyl-28-oxa-2,9-dithia-5,6,12,13,24-pentaazaheptacy clo[27.7.1.1^{1,7}.0^{11,15}.0^{16,21}.0^{20,24}.0^{30,35}]octatriaconta-1(37),4(38),6,11,14,16,18,20,22,29,3 5-undecaene-23-carboxylate 7n-1

### Methyl (Sa)-17-chloro-5,13,14,22-tetramethyl-28-oxa-2,9-dithia-5,6,12,13,24-pentaazaheptacyc lo[27.7.1.1^{4,7}.0^{11,15}.0^{16,21}.0^{20,24}.0^{30,35}]octatriaconta-1(37),4(38),6,11,14,16,18,20,22,29,35 -undecaene-23-carboxylate 7n-2

**7n** (260 mg, 0.38 mmol) was separated chirally (separation conditions: CHIRALPAK ID 250^{∗}20mm, 5 µm; mobile phase: Hexane/EtOH/DEA=85/15/0.1(V/V/V); flow rate: 20 mL/min). The corresponding fractions were collected and concentrated under reduced pressure to obtain the title products (130 mg, 130 mg).

Compound **7n-2** with single configuration (having shorter retention time):
MS m/z (ESI):690.2 [M+1].

Chiral HPLC analysis: retention time 15.351 minutes, chiral purity: 100% (chromatographic column: OD Phenomenex Lux Cellulose-1 150^{∗}4.6mm, 5 µm, equipped with a guard column; mobile phase: *n*-hexane/ethanol/diethylamine=95/5/0.1 (v/v/v)).

Compound **7n-1** with single configuration (having longer retention time):
MS m/z (ESI): 690.2 [M+1].

Chiral HPLC analysis: retention time 18.771 minutes, chiral purity: 100% (chromatographic column: OD Phenomenex Lux Cellulose-1 150^{∗}4.6mm, 5 µm, equipped with a guard column; mobile phase: *n*-hexane/ethanol/diethylamine=95/5/0.1(v/v/v)).

### Step 2

### (Ra)-17-Chloro-5,13,14,22-tetramethyl-28-oxa-2,9-dithia-5,6,12,13,24-pentaazaheptacy clo[27.7.1.1^{1,7}.0^{11,15}.0^{16,21}.0^{20,24}.0^{30,35}]octatriaconta-1(37),4(38),6,11,14,16,18,20,22,29,3 5-undecaene-23-carboxylic acid 7-1

### (Sa)-17-Chloro-5,13,14,22-tetramethyl-28-oxa-2,9-dithia-5,6,12,13,24-pentaazaheptacy clo[27.7.1.1^{4,7}.0^{11,15}.0^{16,21}.0^{20,24}.0^{30,35}]octatriaconta-1(37),4(38),6,11,14,16,18,20,22,29,3 5-undecaene-23-carboxylic acid 7-2

### Step 2(1)

Compound **7n-1** having longer retention time (130 mg, 190 µmol) was dissolved in 20 mL of a mixed solution of tetrahydrofuran and methanol (V:V = 1:1) at room temperature. A solution of lithium hydroxide monohydrate (152 mg, 3.62 mmol) in water (5 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 2-3, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title product **7-1** (60 mg, yield: 46.8%).

Compound **7-1** with single configuration (having shorter retention time):
MS m/z (ESI): 676.0 [M+1].

Chiral HPLC analysis: retention time 6.133 minutes, chiral purity: 100% (chromatographic column: CHIRALPAK IG 150^{∗}4.6mm, 5 µm, equipped with a guard column; mobile phase: Hexane/EtOH/TFA=75/25/0.1(V/V/V)).
¹H NMR (400 MHz, CDCl₃) δ 7.27-7.30 (m, 1H), 7.22-7.24 (m, 1H), 6.92 (s, 1H), 5.89 (s, 1H), 5.19 (s, 1H), 5.04-5.08 (m, 1H), 4.45-4.49 (m, 1H), 3.88 (s, 3H), 3.78-3.81 (m, 1H), 3.63-3.72 (m, 2H), 3.53 (s, 3H), 3.37-3.42 (m, 1H), 3.32-3.36 (m, 1H), 3.22-3.33 (m, 1H), 3.11-3.15 (m, 1H), 2.70-2.85 (m, 5H), 2.19-2.32 (m, 5H), 2.04 (s, 3H), 1.78-1.86 (m, 4H).

### Step 2(2)

Compound **7n-2** having shorter retention time (130 mg, 190 µmol) was dissolved in 20 mL of a mixed solution of tetrahydrofuran and methanol (V:V = 1:1) at room temperature. A solution of lithium hydroxide monohydrate (152 mg, 3.62 mmol) in water (5 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 2-3, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title product **7-2** (55 mg, yield: 42.6%).

Compound **7-2** with single configuration (having longer retention time):
MS m/z (ESI): 676.0 [M+1].

Chiral HPLC analysis: retention time 8.418 minutes, chiral purity: 96.9% (chromatographic column: CHIRALPAK IG 150^{∗}4.6mm, 5 µm, equipped with a guard column; mobile phase: Hexane/EtOH/TFA=75/25/0.1(V/V/V)).
¹H NMR (400 MHz, CDCl₃) δ 7.27-7.30 (m, 1H), 7.22-7.24 (m, 1H), 6.92 (s, 1H), 5.89 (s, 1H), 5.19 (s, 1H), 5.04-5.08 (m, 1H), 4.45-4.49 (m, 1H), 3.88 (s, 3H), 3.78-3.81 (m, 1H), 3.63-3.72 (m, 2H), 3.53 (s, 3H), 3.37-3.42 (m, 1H), 3.32-3.36 (m, 1H), 3.22-3.33 (m, 1H), 3.11-3.15 (m, 1H), 2.70-2.85 (m, 5H), 2.19-2.32 (m, 5H), 2.04 (s, 3H), 1.78-1.86 (m, 4H).

### Example 8

### 17-Chloro-5,13,14,22-tetramethyl-28-oxa-9-thia-5,6,12,13,24-pentaazaheptacyclo[27.7. 1.1^{4,7}.0^{11,15}.0^{16,21}.0^{20,24}.0^{30,35}]octatriaconta-1(37),4(38),6,11,14,16,18,20,22,29,35-undeca ene-23-carboxylic acid 8

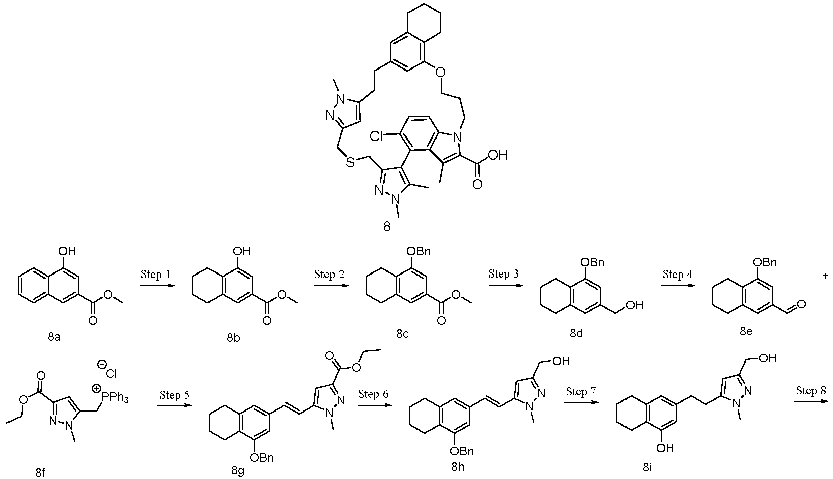

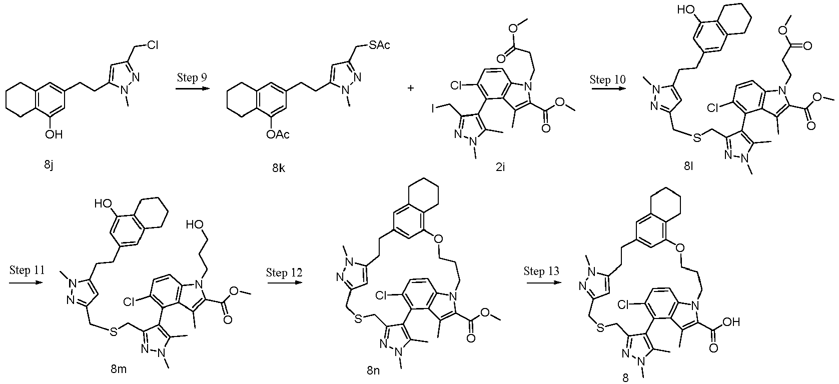

### Step 1

### Methyl 4-hydroxy-5,6,7,8-tetrahydronaphthalene-2-carboxylate 8b

**8a** (3.5 g, 17.3 mmol) was dissolved in 50 mL of methanol, followed by the addition of palladium hydroxide (1.23 g, 8.7585 mmol). The reaction solution was purged with hydrogen, pressurized to 25 atmospheres, and stirred at room temperature for 16 hours. The reaction solution was filtered, and the filtrate was concentrated under reduced pressure to obtain the title product 8**b** (3.2 g, yield: 89.2%).
MS m/z (ESI): 207.1 [M+1].

### Step 2

### Methyl 4-(benzyloxy)-5,6,7,8-tetrahydronaphthalene-2-carboxylate 8c

**8b** (3.2 g, 15.5 mmol) and benzyl bromide (3.2 g, 18.7 mmol) were dissolved in 30 mL of acetonitrile. Potassium carbonate (3.3 g, 23.9 mmol) was added, and the reaction solution was stirred at room temperature for 16 hours. 20 mL of water was added, and the solution was extracted with 20 mL of ethyl acetate twice. The organic phase was dried over anhydrous sodium sulfate and filtered. The filtrate was concentrated under reduced pressure to obtain the title product **8c** (4.2 g, yield: 91.3%).
MS m/z (ESI): 297.1 [M+1].

### Step 3

### (4-(Benzyloxy)-5,6,7,8-tetrahydronaphthalen-2-yl)methanol 8d

**8c** (3.2 g, 10.8 mmol) was dissolved in 30 mL of tetrahydrofuran. Lithium aluminum hydride (550 mg, 16.2 mmol) was added at 0°C, and the reaction solution was stirred at room temperature for 1 hour. 20 g of sodium sulfate decahydrate was added to quench the reaction, and the solution was stirred for 10 minutes. The reaction solution was filtered, and the filtrate was concentrated under reduced pressure to obtain the title product **8d** (2.8 g, yield: 96.6%).
MS m/z (ESI): 269.2 [M+1].

### Step 4

### 4-(Benzyloxy)-5,6,7,8-tetrahydronaphthalene-2-carbaldehyde 8e

**8d** (2.8 g, 10.4 mmol) was dissolved in 50 mL of dichloromethane. Manganese dioxide (9 g, 103.5 mmol) was added, and the reaction solution was reacted at room temperature for 16 hours. The reaction solution was filtered, and the filtrate was concentrated under reduced pressure. The resulting residues were purified by silica gel column chromatography with eluent system A to obtain the title product **8e** (2.2 g, yield: 78.4%).
MS m/z (ESI): 267.2 [M+1].

### Step 5

### Ethyl 5-(2-(4-(benzyloxy)-5,6,7,8-tetrahydronaphthalen-2-yl)vinyl)-1-methyl-1H-pyrazole-3-carboxylate 8g

**8e** (2.2 g, 8.1 mmol) was dissolved in 100 mL of tetrahydrofuran. Sodium hydride (356 mg, 8.9 mmol) was added at 0°C, and the reaction solution was stirred at 0°C for 40 minutes. The reaction solution was maintained at -30°C to -20°C, and added with ((3-(ethoxycarbonyl)-1-methyl-1*H*-pyrazol-5-yl)methyl)triphenylphosphonium chloride **8f** (2.2 g, 8.1 mmol, prepared according to the method disclosed in the intermediate 34 on page 68 of the description of the patent application "WO2018178226"). The reaction solution was stirred at -10°C for 2 hours. Ammonium chloride solution was added to quench the reaction, and the solution was extracted with 20 mL of ethyl acetate twice. The filtrate was concentrated under reduced pressure, and the resulting residues were purified by silica gel column chromatography with eluent system A to obtain the title product **8g** (3.2 g, yield: 93.4%).
MS m/z (ESI): 417.2 [M+1].

### Step 6

### (5-(2-(4-(Benzyloxy)-5,6,7,8-tetrahydronaphthalen-2-yl)vinyl)-1-methyl-1H-pyrazol-3-yl)methanol 8h

**8g** (3.2 g, 7.7 mmol) was dissolved in 60 mL of tetrahydrofuran. Lithium aluminum hydride (261 mg, 7.7 mmol) was added at 0°C, and the reaction solution was stirred at 0°C for 3 minutes and at room temperature for 30 minutes. 20 g of sodium sulfate decahydrate was added to quench the reaction, and the solution was stirred for 10 minutes. The reaction solution was filtered, and the filtrate was concentrated under reduced pressure. The resulting residues were purified by silica gel column chromatography with eluent system A to obtain the title product **8h** (2.7 g, yield: 93.8%).

### Step 7

### 3-(2-(3-(Hydroxymethyl)-1-methyl-1H-pyrazol-5-yl)ethyl)-5,6,7,8-tetrahydronaphthale n-1-ol 8i

**8h** (2.7 g, 7.2 mmol) was dissolved in 20 mL of a mixed solvent of methanol and tetrahydrofuran (V:V=1:1), followed by the addition of palladium on carbon (767 mg, 0.72 mmol, purity: 10%). The reaction solution was purged with hydrogen, pressurized to 3 atmospheres, and stirred at room temperature for 16 hours. The reaction solution was filtered, and the filtrate was concentrated under reduced pressure to obtain the title product **8i** (2.0 g, yield: 96.9%).
MS m/z (ESI): 287.2 [M+1].

### Step 8

### 3-(2-(3-(Chloromethyl)-1-methyl-1H-pyrazol-5-yl)ethyl)-5,6,7,8-tetrahydronaphthalen-1-ol 8j

**8i** (300 mg, 1.05 mmol) was dissolved in 20 mL of tetrahydrofuran, and the solution was purged with argon three times. Thionyl chloride (374 mg, 3.14mmol) was added dropwise at 0°C, and the reaction solution was stirred at room temperature for 2 hours. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate and filtered. The filtrate was concentrated under reduced pressure to obtain the title product **8j** (319 mg, yield: 99.9%), which was used directly in the next step without purification.
MS m/z (ESI): 305.2 [M+1].

### Step 9

### 3-(2-(3-((Acetylthio)methyl)-1-methyl-1H-pyrazol-5-yl)ethyl)-5,6,7,8-tetrahydronaphth alen-1-yl acetate 8k

**8j** (319 mg, 1.05 mmol) and potassium thioacetate (718 mg, 6.29 mmol) were dissolved in 20 mL of acetonitrile. Potassium iodide (18 mg, 0.108 mmol) was added, and the reaction solution was stirred at 40°C for 16 hours. 40 mL of water was added, and the reaction solution was stirred for 10 minutes. The solution was extracted with dichloromethane (30 mL×2), and the organic phases were combined. The filtrate was concentrated under reduced pressure, and the resulting residues were purified by silica gel column chromatography with eluent system B to obtain the title product **8k** (400 mg, yield: 98.9%).
MS m/z (ESI): 387.2 [M+1].

### Step 10

### Methyl 5-chloro-4-(3-((((5-(2-(4-hydroxy-5,6,7,8-tetrahydronaphthalen-2-yl)ethyl)-1-methyl-1 H-pyrazol-3-yl)methyl)thio)methyl)-1,5-dimethyl-1H-pyrazol-4-yl)-1-(3-methoxy-3-ox opropyl)-3-methyl-1H-indole-2-carboxylate 8l

**8k** (150 mg, 0.39 mmol) was dissolved in 10 mL of methanol. Potassium carbonate (108 mg, 0.78 mmol) and **2i** (233 mg, 0.43 mmol) were added, and the reaction solution was reacted under an argon atmosphere at room temperature for 4 hours. The reaction solution was concentrated under reduced pressure to remove the solvent, and the resulting residues were purified by silica gel column chromatography with eluent system B to obtain the title product **8l** (200 mg, yield: 71.7%).
MS m/z (ESI): 718.1 [M+1].

### Step 11

### Methyl 5-chloro-4-(3-((((5-(2-(4-hydroxy-5,6,7,8-tetrahydronaphthalen-2-yl)ethyl)-1-methyl-1 H-pyrazol-3-yl)methyl)thio)methyl)-1,5-dimethyl-1H-pyrazol-4-yl)-1-(3-hydroxypropyl )-3-methyl-1H-indole-2-carboxylate 8m

**8l** (200 mg, 0.28 mmol) was dissolved in 5 mL of tetrahydrofuran. 1.0 M solution of borane in tetrahydrofuran (2 mL, 2 mmol) was added dropwise under an ice bath, and the reaction solution was stirred at room temperature overnight. 1.6 mL of methanol and 3.2 mL of diluted hydrochloric acid (6M) were added dropwise under an ice bath, and the reaction solution was stirred for 1 hour. 50 mL of water was added to the reaction solution, which was extracted with a mixed solution of dichloromethane and methanol (V:V=10:1) (50 mL×2). The organic phases were combined, washed with water (30 mL) and saturated sodium chloride solution (30 mL) successively, dried over anhydrous sodium sulfate and filtered. The filtrate was concentrated under reduced pressure, and the resulting residues were purified by silica gel column chromatography with eluent system B to obtain the title product **8m** (20 mg, yield: 10.4%).
MS m/z (ESI):690.2 [M+1].

### Step 12

### Methyl 17-chloro-5,13,14,22-tetramethyl-28-oxa-9-thia-5,6,12,13,24-pentaazaheptacyclo[27.7. 1.1^{4,7}.0^{11,15}.0^{16,21}.0^{20,24}.0^{30,35}]octatriaconta-1(37),4(38),6,11,14,16,18,20,22,29,35-undeca ene-23-carboxylate 8n

The crude product **8m** (50 mg, 0.072 mmol) was dissolved in 7.5 mL of a mixed solvent of tetrahydrofuran and toluene (V:V=1:2). Tri-n-butylphosphine (90 mg, 0.36 mmol) and azodicarbonyl dipiperidine (90 mg, 0.36 mmol) were added, and the reaction solution was reacted at 60°C under an argon atmosphere for 2 hours. The reaction solution was concentrated under reduced pressure to remove the solvent, and the resulting residues were purified by silica gel column chromatography with eluent system B to obtain the title product **8n** (30 mg, yield: 61.6%).
MS m/z (ESI): 672.3 [M+1].

### Step 13

### 17-Chloro-5,13,14,22-tetramethyl-28-oxa-9-thia-5,6,12,13,24-pentaazaheptacyclo[27.7. 1.1^{4,7}.0^{11,15}.0^{16,21}.0^{20,24}.0^{30,35}]octatriaconta-1(37),4(38),6,11,14,16,18,20,22,29,35-undeca ene-23-carboxylic acid 8

**8n** (40 mg, 0.059 mmol) was dissolved in 9 mL of a mixed solvent of methanol, tetrahydrofuran and water (V:V:V=1:1:1). Lithium hydroxide monohydrate (25 mg, 0.60 mmol) was added, and the reaction solution was heated to 50°C and stirred for 1 hour. The reaction solution was concentrated under reduced pressure to remove most of the solvent. The resulting residues were purified by preparative liquid chromatography (Waters 2767-SQ Detecor2; eluent system: 0.1% trifluoroacetic acid, water, acetonitrile) to obtain the title product **8** (6 mg, yield: 15.3%).
MS m/z (ESI): 658.3 [M+1].
¹H NMR (400 MHz, CD₃OD) δ 7.44-7.42 (m, 1H), 7.18-7.16 (m, 1H), 6.51 (s, 1H), 5.79 (s, 1H), 5.34 (s, 1H), 4.53-4.51 (m, 2H), 3.87 (s, 3H), 3.52-3.50 (m, 5H), 3.47-3.45(m, 2H), 2.84-2.80 (m, 2H), 2.68-2.62 (m, 4H), 2.21-2.17 (m, 5H), 2.04-2.06 (m, 5H), 1.82-1.78 (m, 4H), 1.53-1.51 (m, 2H).

### Examples 8-1 and 8-2

### (Ra)-17-Chloro-5,13,14,22-tetramethyl-28-oxa-9-thia-5,6,12,13,24-pentaazaheptacyclo[ 27.7.1.1^{1,7}.0^{11,15}.0^{16,21}.0^{20,24}.0^{30,35}]octatriaconta-1(37),4(38),6,11,14,16,18,20,22,29,35-u ndecaene-23-carboxylic acid 8-1

### (Sa)-17-Chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{30,35}]octatriaconta-1(37),4(38),6,11,14,16,18,20,22,29,35-u ndecaene-23-carboxylic acid 8-2

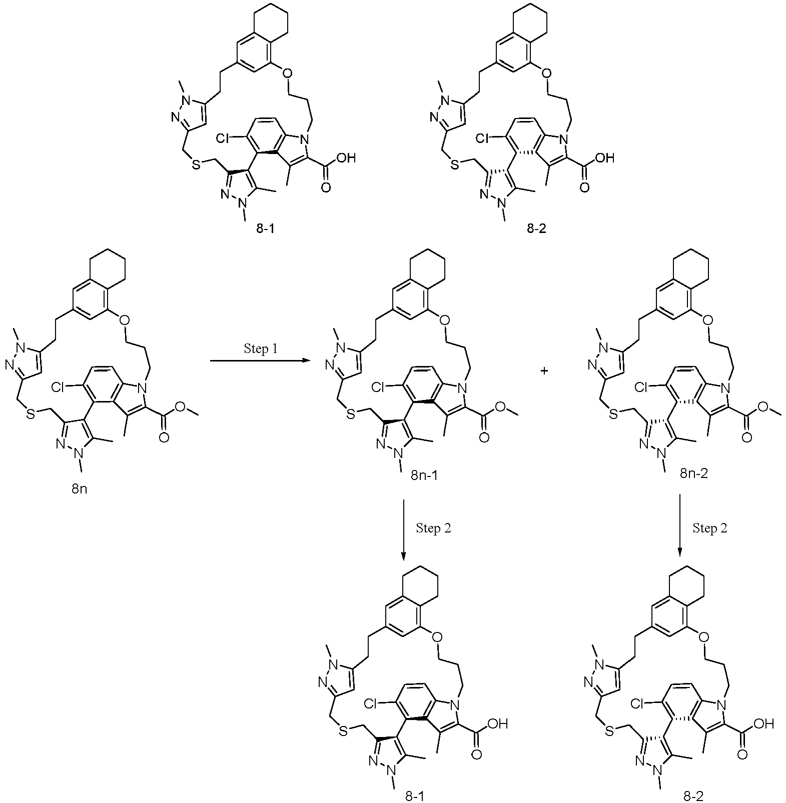

### Step 1

### Methyl (Ra)-17-chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{10,15}]octatriaconta-1(37),4(38),6,11,14,16,18,20,22,29,35-u ndecaene-23-carboxylate 8n-1

### Methyl (Sa)-17-chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{10,15}]octatriaconta-1(37),4(38),6,11,14,16,18,20,22,29,35-u ndecaene-23-carboxylate 8n-2

**8n** (400 mg, 0.13 mmol) was separated chirally (separation conditions: AD Phenomenex Lux Amylose-1 chiral preparative column, 30 mm I.D.nex Lux Amyl; mobile phase: Hexane/EtOH(0.1%DEA)=70/30(V/V); flow rate: 60 mL/min). The corresponding fractions were collected and concentrated under reduced pressure to obtain the title products (115 mg, 110 mg).

Compound **8n-1** with single configuration (having shorter retention time):
MS m/z (ESI): 672.3 [M+1].

Chiral HPLC analysis: retention time 4.064 minutes, chiral purity: 100% (chromatographic column: AD Phenomenex Lux Amylose-1 150^{∗}4.6mm, 5 µm; mobile phase: Hexane /EtOH(0.1%DEA)=70/30(V/V); flow rate: 1.0 mL/min).

Compound **8n-2** with single configuration (having longer retention time):
MS m/z (ESI): 672.3 [M+1].

Chiral HPLC analysis: retention time 6.525 minutes, chiral purity: 100% (chromatographic column: AD Phenomenex Lux Amylose-1 150^{∗}4.6mm, 5 µm; mobile phase: Hexane /EtOH(0.1%DEA)=70/30(V/V); flow rate: 1.0 mL/min).

### Step 2

### (Ra)-17-Chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{10,15}]octatriaconta-1(37),4(38),6,11,14,16,18,20,22,29,35-u ndecaene-23-carboxylic acid 8-1

### (Sa)-17-Chloro-5,13,14,22-tetramethyl-28-oxa-9-thia-5,6,12,13,24-pentaazaheptacyclo[ 27.7.1.1^{4,7}.0^{11,15}.0^{16,21}.0^{20,24}.0^{10,15}]octatriaconta-1(37),4(38),6,11,14,16,18,20,22,29,35-u ndecaene-23-carboxylic acid 8-2

Compound **8n-1** (having shorter retention time)/compound **8n-2** (having longer retention time) (40 mg/110 mg, 0.059 mmol/0.163 mmol) was dissolved in 9 mL/3 mL of a mixed solvent of methanol, tetrahydrofuran and water (V:V:V=1:1:1), respectively. Lithium hydroxide monohydrate (25 mg/ 50 mg, 0.60 mmol/1.19 mmol) was added, and the reaction solution was heated to 50°C and stirred for 1 hour. With regard to the compound having shorter retention time, the reaction solution was concentrated under reduced pressure to remove most of the solvent, and the resulting residues were purified by preparative liquid chromatography (Waters 2767-SQ Detecor2; eluent system: 0.1% trifluoroacetic acid, water, acetonitrile). With regard to the compound having longer retention time, the reaction solution was diluted with water (10 mL) and extracted with ethyl acetate (10 mL×4), the organic phase was dried over anhydrous sodium sulfate and filtered, the filtrate was concentrated under reduced pressure, and the resulting residues were purified by silica gel column chromatography with eluent system B. The title products **8-1/8-2** (10 mg, 50 mg) were obtained respectively.

Compound 8-1 with single configuration (having shorter retention time):
MS m/z (ESI): 658.3 [M+1].

Chiral HPLC analysis: retention time 7.011 minutes, chiral purity: 100% (chromatographic column: CHIRALPAK IF 150^{∗}4.6 mm, 5 µm; mobile phase: *n*-hexane/ethanol (containing 0.1% trifluoroacetic acid) =80:20(v/v)).
¹H NMR (400 MHz, CDCl₃) δ 7.31-7.22 (m, 2H), 6.54 (s, 1H), 5.38 (m, 2H), 5.05 (m, 1H), 4.46 (m, 1H), 3.92 (s, 3H), 3.74-.367(m, 1H), 3.41-3.37 (m, 1H), 3.27-3.17 (m, 6H), 2.87-2.68 (m, 9H), 2.22-2.15(m, 5H), 2.06 (s, 3H), 1.83-1.79 (m, 4H).

Compound **8-2** with single configuration (having longer retention time):
MS m/z (ESI): 658.3 [M+1].

Chiral HPLC analysis: retention time 8.189 minutes, chiral purity: 100% (chromatographic column: CHIRALPAK IF 150^{∗}4.6 mm, 5 µm; mobile phase: n-hexane/ethanol (containing 0.1% trifluoroacetic acid) =80:20(v/v)).
¹H NMR (400 MHz, CDCl₃) δ 7.31-7.22 (m, 2H), 6.56 (s, 1H), 5.40 (s, 1H), 5.35 (s, 1H), 5.07 (m, 1H), 4.46 (m, 1H), 3.90 (s, 3H), 3.72 (d, 1H), 3.40 (d, 1H), 3.26-3.16 (m, 6H), 2.85-2.69 (m, 9H), 2.25-2.13 (m, 5H), 2.06 (s, 3H), 1.85-1.80 (m, 4H).

### Example 9

### 17-Chloro-5,9,13,14,22-pentamethyl-28-oxa-2-thia-5,6,9,12,13,24-hexaazaheptacyclo[2 7.7.1.1^{4,7}.0^{11,15}.0^{16,21}.0^{20,24}.0^{30,35}]octatriaconta-1(37),4(38),6,11,14,16,18,20,22,29,35-un decaene-23-carboxylic acid 9

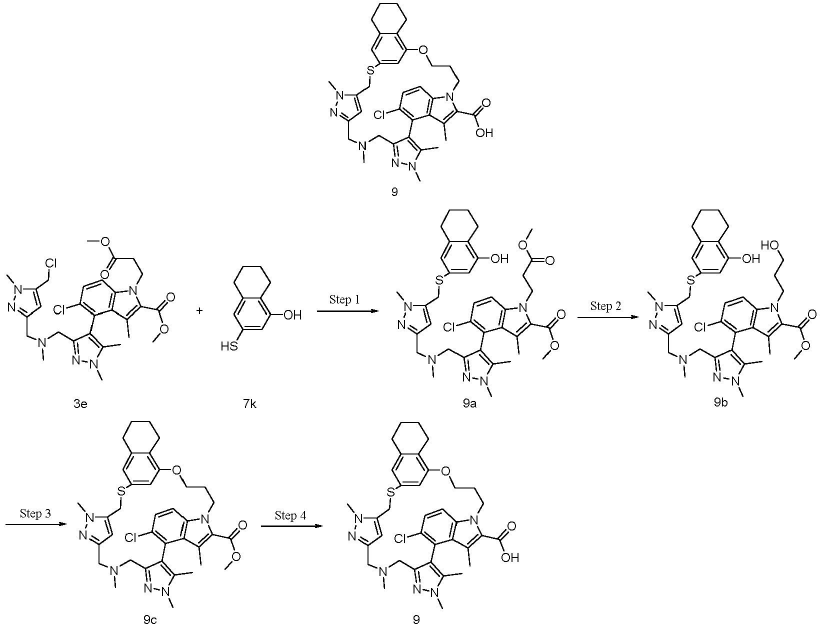

### Step 1

### Methyl 5-chloro-4-(3-((((5-(((4-hydroxy-5,6,7,8-tetrahydronaphthalen-2-yl)thio)methyl)-1-meth yl-1H-pyrazol-3-yl)methyl)(methyl)amino)methyl)-1,5-dimethyl-1H-pyrazol-4-yl)-1-(3-methoxy-3-oxopropyl)-3-methyl-1H-indole-2-carboxylate 9a

**3e** (150 mg, 0.25 mmol) was dissolved in methanol (10 mL) and tetrahydrofuran (3 mL) at room temperature. A solution of **7k** (5.8 mL, 0.40 mmol) in tetrahydrofuran was added dropwise under an ice bath, and the reaction solution was stirred at room temperature for 1 hour. The reaction solution was diluted with ethyl acetate (50 mL), and washed with water (30 mL×3) and saturated sodium chloride solution (30 ml×2) successively. The organic phase was dried over anhydrous sodium sulfate and filtered. The filtrate was concentrated under reduced pressure, and the resulting residues were purified by silica gel column chromatography with eluent system A to obtain the title product **9a** (100 mg, yield: 53.6%).
MS m/z(ESI): 733.1 [M+1].

### Step 2

### Methyl 5-chloro-4-(3-((((5-(((4-hydroxy-5 ,6,7,8-tetrahydronaphthalen-2-yl)thio)methyl)-1-meth yl-1H-pyrazol-3-yl)methyl)(methyl)amino)methyl)-1,5-dimethyl-1H-pyrazol-4-yl)-1-(3-hydroxypropyl)-3-methyl-1H-indole-2-carboxylate 9b

**9a** (100 mg, 0.14 mmol) was dissolved in tetrahydrofuran (8 mL) at room temperature, and cooled to 0-5°C. 1 M solution of borane in tetrahydrofuran (1.4 mL) was slowly added dropwise, and the reaction solution was warmed up to room temperature and stirred for 16 hours. The reaction solution was cooled to 0-5°C and quenched by methanol. The solution was warmed up to room temperature and stirred for 30 minutes. Hydrochloric acid (2.8 mL, 6.0 N) was added and stirred for 30 minutes. Saturated sodium bicarbonate solution was added to adjust pH to 7, and the solution was extracted with a mixed solvent (30 mL×3) of dichloromethane and methanol (V:V=10:1). The organic phases were combined, washed with saturated sodium chloride solution (30 mL), dried over anhydrous sodium sulfate and filtered. The filtrate was concentrated under reduced pressure, and the resulting residues were purified by silica gel column chromatography with eluent system A to obtain the title product **9b** (60 mg, yield: 62.4%).
MS m/z(ESI): 705.1 [M+1].

### Step 3

### Methyl 17-chloro-5,9,13,14,22-pentamethyl-28-oxa-2-thia-5,6,9,12,13,24-hexaazaheptacyclo[2 7.7.1.1^{4,7}.0^{11,15}.0^{16,21}.0^{20,24}.0^{30,35}]octatriaconta-1(37),4(38),6,11,14,16,18,20,22,29,35-un decaene-23-carboxylate 9c

**9b** (60 mg, 85 µmol) was dissolved in toluene (10 mL) and tetrahydrofuran (5 mL) at room temperature, followed by the addition of tri-n-butylphosphine (86 mg, 0.43 mmol). The solution was purged with argon three times, and added with a solution (3 mL) of azodicarbonyl dipiperidine (107 mg, 0.42 mmol) in toluene was added dropwise. The reaction solution was heated to 60°C and stirred for 2 hours. The reaction solution was concentrated under reduced pressure, and the resulting residues were purified by silica gel column chromatography with eluent system A to obtain the title product **9c** (60 mg, yield: 100%).
MS m/z(ESI): 687.1 [M+1].

### Step 4

### 17-Chloro-5,9,13,14,22-pentamethyl-28-oxa-2-thia-5,6,9,12,13,24-hexaazaheptacyclo[2 7.7.1.1^{4,7}.0^{11,15}.0^{16,21}.0^{20,24}.0^{30,35}]octatriaconta-1(37),4(38),6,11,14,16,18,20,22,29,35-un decaene-23-carboxylic acid 9

**9c** (60 mg, 87.3 µmol) was dissolved in 10 mL of a mixed solution of tetrahydrofuran and methanol (V:V = 1:1) at room temperature. A solution of lithium hydroxide monohydrate (18 mg, 0.43 mmol) in water (2 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 6-7, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Waters 2767-SQ Detecor2, eluent system: H₂O (10 mmol NH₄OAc), ACN) to obtain the title product **9** (10 mg, yield: 17.0%).
MS m/z (ESI): 673.0 [M+1].
¹H NMR (400 MHz, CDCl₃) δ 7.21-7.24 (m, 1H), 7.01-7.03 (m, 1H), 6.83 (s, 1H), 6.14 (s, 1H), 5.52 (s, 1H), 4.92-4.95 (m, 1H), 4.35-4.41 (m, 1H), 4.02-4.05 (m, 1H), 3.87-3.96 (m, 4H), 3.71-3.80 (m, 2H), 3.63 (s, 3H), 3.31-3.47 (m, 3H), 3.15 (s, 2H), 2.74-2.79 (m, 4H), 2.53 (s, 3H), 2.19-2.30 (m, 1H), 2.11 (s, 3H), 2.02 (s, 3H), 1.76-1.93 (m, 4H).

### Example 10

### 21-Chloro-5,26-dimethyl-32-oxa-2,9-dithia-5,6,12,13,28-pentaazaoctacyclo[31.7.1 .1^{4,7}.0^{11,19}.0^{13,18}.0^{20,25}.0^{24,28}.0^{34,39}]dotetraconta-1(41),4(42),6,11,18,20,22,24,26,33,35,37, 39-tridecaene-27-carboxylic acid 10

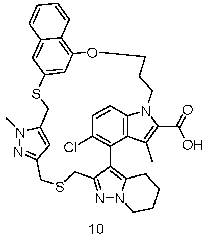

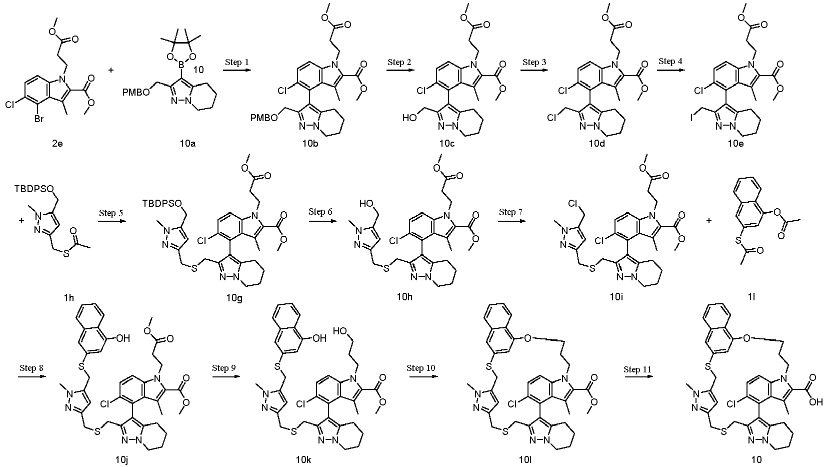

### Step 1

### Methyl 5-chloro-1-(3-methoxy-3-oxopropyl)-4-(2-(((4-methoxybenzyl)oxy)methyl)-4,5,6,7-tetr ahydropyrazolo[1,5-a]pyridin-3-yl)-3-methyl-1H-indole-2-carboxylate 10b

**2e** (0.83 g, 2.09 mmol) and 2-(((4-methoxybenzyl)oxy)methyl)-3-(4,4,5,5-tetramethyl-1,3,2-dioxaborolan-2-yl)-4,5, 6,7-tetrahydropyrazolo[1,5-a]pyridine **10a** (1.0 g, 2.5 mmol, prepared according to the method disclosed in "Bioorganic and Medicinal Chemistry Letters, 2013, vol. 23, # 16, p. 4523 - 4527") were dissolved in 24 mL of 1,4-dioxane and 6 mL of water. The solution was purged with argon three times, and added with 1,1'-bis(di-*tert*-butylphosphine)ferrocaene dichloropalladium (74 mg, 0.1 mmol) and cesium carbonate (1.36 g, 4.17 mmol). The reaction solution was purged with argon three times, heated to 95°C under an argon atmosphere, and stirred for 16 hours. The reaction solution was cooled to room temperature, and concentrated under reduced pressure to remove most of the solvent. 40 mL of water was added to the reaction solution, and extracted with ethyl acetate (20 mL×3). The aqueous phase was adjusted to pH=1-2 with 1*N* HCl, and extracted with dichloromethane (containing a small amount of methanol, 20 mL×3). The organic phase was washed with water (30 mL) and saturated sodium chloride solution (30 mL) successively, dried over anhydrous sodium sulfate, and filtered to remove the desiccant. The filtrate was concentrated under reduced pressure to obtain the title product **10b** (1.0 g, yield: 83.3%). The product was used directly in the next step without purification.
MS m/z (ESI): 580.1 [M+1].

### Step 2

### Methyl 5-chloro-4-(2-(hydroxymethyl)-4,5,6,7-tetrahydropyrazolo[1,5-a]pyridin-3-yl)-1-(3-met hoxy-3-oxopropyl)-3-methyl-1H-indole-2-carboxylate 10c

The crude product **10b** (1.0 g, 1.72 mmol) was dissolved in 30 mL of methanol. Concentrated sulfuric acid (2.00 g, 20.39 mmol) was added dropwise under an ice bath, and the reaction solution was heated to reflux and stirred for 16 hours. The reaction solution was cooled under an ice bath, and added dropwise with saturated aqueous sodium bicarbonate solution under an ice bath to adjust pH to 7-8. The solution was extracted with dichloromethane (30 mL×3). The organic phases were combined, washed with water (20 mL) and saturated sodium chloride solution (20 mL) successively, dried over anhydrous sodium sulfate and filtered. The filtrate was concentrated under reduced pressure, and the resulting residues were purified by silica gel column chromatography with eluent system A to obtain the title product **10c** (520 mg, yield: 65.58%).
MS m/z (ESI): 459.8 [M+1].

### Step 3

### Methyl 5-chloro-4-(2-(chloromethyl)-4,5,6,7-tetrahydropyrazolo[1,5-a]pyridin-3-yl)-1-(3-meth oxy-3-oxopropyl)-3-methyl-1H-indole-2-carboxylate 10d

**10c** (520 mg, 1.13 mmol) was dissolved in 15 mL of dichloromethane, and the solution was purged with argon three times. Thionyl chloride (361 mg, 3.03 mmol) was added dropwise under an ice bath, and the reaction solution was reacted at room temperature for 2 hours. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate and filtered. The filtrate was concentrated under reduced pressure to obtain the title product **10d** (500 mg, yield: 92.44%), which was used directly in the next step without purification.
MS m/z (ESI): 478.0 [M+1].

### Step 4

### Methyl 5-chloro-4-(2-(iodomethyl)-4,5,6,7-tetrahydropyrazolo[1,5-a]pyridin-3-yl)-1-(3-methox y-3-oxopropyl)-3-methyl-1H-indole-2-carboxylate 10e

The crude product **10d** (500 mg, 1.04 mmol) was dissolved in 10 mL of acetonitrile, followed by the addition of sodium iodide (313 mg, 2.08 mmol). The reaction solution was heated to 80°C and stirred for 2 hours. The reaction solution was cooled to room temperature, and added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure to obtain the title product **10e** (520 mg, yield: 87.34%), which was used directly in the next step without purification.
MS m/z (ESI): 570.0 [M+1].

### Step 5

### Methyl 4-(2-((((5-(((tert-butyldiphenylsilyl)oxy)methyl)-1-methyl-1H-pyrazol-3-yl)methyl)thio )methyl)-4,5,6,7-tetrahydropyrazolo[1,5-a]pyridin-3-yl)-5-chloro-1-(3-methoxy-3-oxop ropyl)-3-methyl-1H-indole-2-carboxylate 10g

The crude product **10e** (500 mg, 0.91 mmol) was dissolved in 10 mL of methanol and 5 mL of tetrahydrofuran, followed by the addition of potassium carbonate (313 mg, 2.27 mmol). The solution was purged with argon three times, and added dropwise with a solution of **1h** (466 mg, 1.10 mmol) in methanol (5 mL) at room temperature. The reaction solution was reacted at room temperature for 2 hours. The reaction solution was concentrated under reduced pressure to remove most of the solvent, and added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure, and the resulting residues were purified by silica gel column chromatography with eluent system A to obtain the title product **10g** (468 mg, yield: 61.16%).
MS m/z (ESI): 838.0 [M+1].

### Step 6

### Methyl 5-chloro-4-(2-((((5-(hydroxymethyl)-1-methyl-1H-pyrazol-3-yl)methyl)thio)methyl)-4, 5,6,7-tetrahydropyrazolo[1,5-a]pyridin-3-yl)-1-(3-methoxy-3-oxopropyl)-3-methyl-1H-i ndole-2-carboxylate 10h

**10g** (468 mg, 0.56 mmol) was dissolved in 10 mL of tetrahydrofuran. 1.0M tetrabutylammonium fluoride (0.673 mL, 0.673 mmol) was added dropwise, and the reaction solution was reacted at room temperature for 1 hour. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure, and the resulting residues were purified by silica gel column chromatography with eluent system A to obtain the title product **10h** (195 mg, yield: 58.21%).
MS m/z (ESI): 600.1 [M+1].

### Step 7

### Methyl 5-chloro-4-(2-((((5-(chloromethyl)-1-methyl-1H-pyrazol-3-yl)methyl)thio)methyl)-4,5, 6,7-tetrahydropyrazolo[1,5-a]pyridin-3-yl)-1-(3-methoxy-3-oxopropyl)-3-methyl-1H-in dole-2-carboxylate 10i

**10h** (195 mg, 0.32 mmol) was dissolved in 10 mL of dichloromethane, and the solution was purged with argon three times. Thionyl chloride (58 mg, 0.49 mmol) was added dropwise under an ice bath, and the reaction solution was reacted at room temperature for 2 hours. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate and filtered. The filtrate was concentrated under reduced pressure to obtain the title product **10i** (190 mg, crude product), which was used directly in the next step without purification.
MS m/z (ESI): 618.2 [M+1].

### Step 8

### Methyl 5-chloro-4-(2-((((5-(((4-hydroxynaphthalen-2-yl)thio)methyl)-1-methyl-1H-pyrazol-3-y l)methyl)thio)methyl)-4,5,6,7-tetrahydropyrazolo[1,5-a]pyridin-3-yl)-1-(3-methoxy-3-o xopropyl)-3-methyl-1H-indole-2-carboxylate 10j

The crude product **10i** (190 mg, 0.31 mmol) and **1l** (97 mg, 0.37 mmol) were dissolved in 10 mL of methanol. Potassium carbonate (128 mg, 0.93 mmol) was added at room temperature, and the reaction solution was reacted at room temperature for 2 hours. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure, and the resulting residues were purified by silica gel column chromatography with eluent system A to obtain the title product **10j** (120 mg, yield: 51.7%).
MS m/z (ESI): 758.1 [M+1].

### Step 9

### Methyl

### 5-chloro-4-(2-((((5-(((4-hydroxynaphthalen-2-yl)thio)methyl)-1-methyl-1H-pyrazol-3-y l)methyl)thio)methyl)-4,5,6,7-tetrahydropyrazolo[1,5-a]pyridin-3-yl)-1-(3-hydroxyprop yl)-3-methyl-1H-indole-2-carboxylate 10k

**10j** (120 mg, 0.16 mmol) was dissolved in 2 mL of tetrahydrofuran. 1.0 M solution of borane in tetrahydrofuran (1.58 mL, 1.58 mmol) was added dropwise under an ice bath, and the reaction solution was reacted at room temperature for 16 hours. 2 mL of methanol and 4 mL of hydrochloric acid (6M) were added dropwise under an ice bath, and the reaction solution was stirred for 1 hour. 50 mL of water was added to the reaction solution, which was extracted with a mixed solution of dichloromethane and methanol (V:V=10:1) (50 mL×2). The organic phases were combined, washed with water (30 mL) and saturated sodium chloride solution (30 mL) successively, dried over anhydrous sodium sulfate and filtered. The filtrate was concentrated under reduced pressure, and the resulting residues were purified by silica gel column chromatography with eluent system A to obtain the title product **10k** (57 mg, yield: 49.3%).
MS m/z (ESI): 730.0 [M+1].

### Step 10

### Methyl 21-chloro-5,26-dimethyl-32-oxa-2,9-dithia-5,6,12,13,28-pentaazaoctacyclo[31.7.1.1^{4,7}.0 ^{11,19}.0^{13,18}.0^{20,25}.0^{24,28}.0^{34,39}]dotetraconta-1(41),4(42),6,11,18,20,22,24,26,33,35,37,39-tri decaene-27-carboxylate 10l

Triphenylphosphine (87 mg, 0.34 mmol) and dibenzyl azodicarboxylate (70 mg, 0.34 mmol) were dissolved in 5 mL of toluene, and the solution was purged with argon three times. 6 mL of solution of **10k** (50 mg, 0.068 mmol) in toluene and tetrahydrofuran (V:V=5:1) was added dropwise, and the reaction solution was reacted at room temperature for 16 hours. 10 mL of hydrochloric acid (2M) was added, and the reaction solution was stirred for 10 minutes. 50 mL of water was added to the reaction solution, and extracted with ethyl acetate (50 mL×2). The organic phases were combined, washed with water (30 mL) and saturated sodium chloride solution (30 mL) successively, dried over anhydrous sodium sulfate and filtered. The filtrate was concentrated under reduced pressure, and the resulting residues were purified by silica gel column chromatography with eluent system A to obtain the title product **10l** (40 mg, yield: 83.33%).
MS m/z (ESI): 712.0 [M+1].

### Step 11

### 21-Chloro-5,26-dimethyl-32-oxa-2,9-dithia-5,6,12,13,28-pentaazaoctacyclo[31.7.1.1^{4,7}. 0^{11,19}.0^{13,18}.0^{20,25}.0^{24,28}.0^{34,39}]dotetraconta-1(41),4(42),6,11,18,20,22,24,26,33,35,37,39-tr idecaene-27-carboxylic acid 10

**10l** (30 mg, 0.043 mmol) was dissolved in 5 mL of a mixed solvent of methanol, tetrahydrofuran and water (V:V:V=1:1:1). Lithium hydroxide monohydrate (18 mg, 0.42 mmol) was added, and the reaction solution was heated to 50°C and reacted for 0.5 hour. The reaction solution was cooled to room temperature, and concentrated under reduced pressure to remove most of the solvent. 2M hydrochloric acid was added to adjust pH to 1-2, and the solution was extracted with a mixed solvent (50 mL×2) of dichloromethane and methanol (V:V=10:1). The organic phases were combined, washed with water (30 mL) and saturated sodium chloride solution (30 mL) successively, dried over anhydrous sodium sulfate and filtered. The filtrate was concentrated under reduced pressure, and the resulting residues were purified by silica gel column chromatography with eluent system A to obtain the title product **10** (12 mg, yield: 40.80%).
MS m/z (ESI): 698.0 [M+1].
¹H NMR (400 MHz, DMSO-*d*₆) δ 8.37-8.39 (m, 1H), 7.78-7.79 (m, 1H), 7.66 (s, 1H), 7.54-7.60 (m, 2H), 7.27-7.29 (m, 1H), 7.12 (s, 1H), 7.01-7.03 (m, 1H), 6.11 (s, 1H), 5.23-5.26 (m, 1H), 5.10 (s, 1H), 4.62-4.67 (m, 1H), 3.77-3.88 (m, 4H), 3.50-3.63 (m, 5H), 3.30 (s, 1H), 3.21 (s,3H), 3.11-3.15 (m, 1H), 2.62-2.65(m, 1H), 2.34-2.45 (m, 2H), 1.95-1.97 (m, 4H), 1.30 (s, 3H).

### Example 11

### 21-Chloro-5,9,26-trimethyl-32-oxa-2-thia-5,6,9,12,13,28-hexaazaoctacyclo[31.7. 1.1^{4,7}.0^{11,19}.0^{13,18}.0^{20,25}.0^{24,28}.0^{34,39}]dotetraconta-1(41),4(42),6,11,18,20,22,24,26,33,35,3 7,39-tridecaene-27-carboxylic acid 11

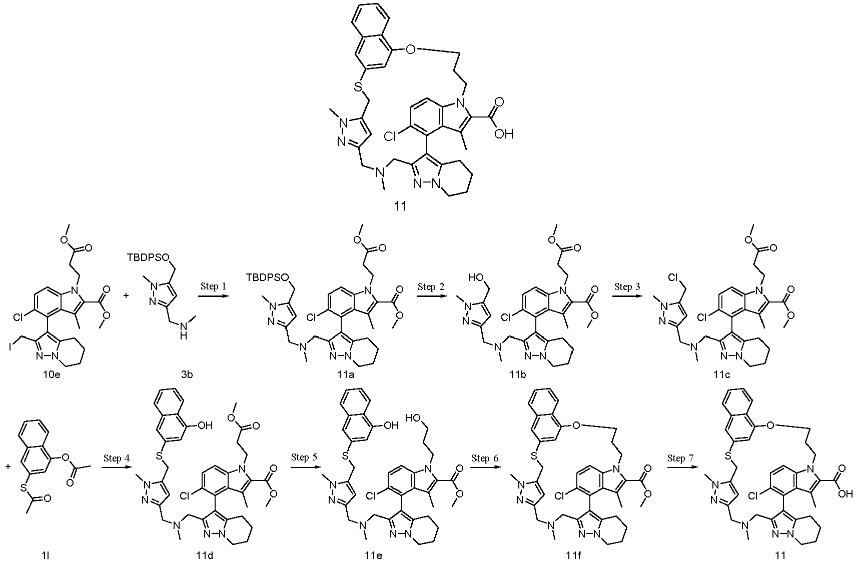

### Step 1

### Methyl 4-(2-((((5-(((tert-butyldiphenylsilyl)oxy)methyl)-1-methyl-1H-pyrazol-3-yl)methyl)(me thyl)amino)methyl)-4,5,6,7-tetrahydropyrazolo[1,5-a]pyridin-3-yl)-5-chloro-1-(3-metho xy-3-oxopropyl)-1H-indole-2-carboxylate 11a

The crude product **10e** (1.0 g, 1.75 mmol) was dissolved in 10 mL of *N*,*N*-dimethylformamide, followed by the addition of potassium carbonate (313 mg, 2.27 mmol). The solution was purged with argon three times, and added dropwise with a solution of **3b** (828 mg, 2.10 mmol) in methanol (5 mL) at room temperature. The reaction solution was reacted at 50°C for 2 hours. The reaction solution was concentrated under reduced pressure to remove most of the solvent, and added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure, and the resulting residues were purified by silica gel column chromatography with eluent system A to obtain the title product **11a** (1.0 g, yield: 68.20%).
MS m/z (ESI): 835.2 [M+1].

### Step 2

### Methyl 5-chloro-4-(2-((((5-(hydroxymethyl)-1-methyl-1H-pyrazol-3-yl)methyl)(methyl)amino) methyl)-4,5,6,7-tetrahydropyrazolo[1,5-a]pyridin-3-yl)-1-(3-methoxy-3-oxopropyl)-1H-indole-2-carboxylate 10b

**11a** (1.0 g, 1.20 mmol) was dissolved in 10 mL of tetrahydrofuran. 1.0M tetrabutylammonium fluoride (1.04 mL, 1.04 mmol) was added dropwise, and the reaction solution was reacted at room temperature for 1 hour. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure, and the resulting residues were purified by silica gel column chromatography with eluent system A to obtain the title product **10b** (520 mg, yield: 72.76%).
MS m/z (ESI): 597.1 [M+1].

### Step 3

### Methyl 5-chloro-4-(2-((((5-(chloromethyl)-1-methyl-1H-pyrazol-3-yl)methyl)(methyl)amino)m ethyl)-4,5,6,7-tetrahydropyrazolo[1,5-a]pyridin-3-yl)-1-(3-methoxy-3-oxopropyl)-1H-indole-2-carboxylate 11c

**11b** (200 mg, 0.33 mmol) was dissolved in 10 mL of dichloromethane, and the solution was purged with argon three times. Thionyl chloride (60 mg, 0.49 mmol) was added dropwise under an ice bath, and the reaction solution was reacted at room temperature for 2 hours. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate and filtered. The filtrate was concentrated under reduced pressure to obtain the title product **11c** (206 mg, yield: 99.90%), which was used directly in the next step without purification.
MS m/z (ESI): 615.1 [M+1].

### Step 4

### Methyl 5-chloro-4-(2-((((5-(((4-hydroxynaphthalen-2-yl)thio)methyl)-1-methyl-1H-pyrazol-3-y l)methyl)(methyl)amino)methyl)-4,5,6,7-tetrahydropyrazolo[1,5-a]pyridin-3-yl)-1-(3-m ethoxy-3-oxopropyl)-3-methyl-1H-indole-2-carboxylate 11d

The crude product **11c** (206 mg, 0.33 mmol) and **1l** (104 mg, 0.40 mmol) were dissolved in 10 mL of methanol. Potassium carbonate (139 mg, 1.00 mmol) was added at room temperature, and the reaction solution was reacted at room temperature for 2 hours. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure, and the resulting residues were purified by silica gel column chromatography with eluent system A to obtain the title product **11d** (160 mg, yield: 63.29%).
MS m/z (ESI): 755.0 [M+1].

### Step 5

### Methyl 5-chloro-4-(2-((((5-(((4-hydroxynaphthalen-2-yl)thio)methyl)-1-methyl-1H-pyrazol-3-y l)methyl)(methyl)amino)methyl)-4,5,6,7-tetrahydropyrazolo[1,5-a]pyridin-3-yl)-1-(3-hy droxypropyl)-3-methyl-1H-indole-2-carboxylate 11e

**11d** (160 mg, 0.211 mmol) was dissolved in 2 mL of tetrahydrofuran. 1.0 M solution of borane in tetrahydrofuran (2.12 mL, 2.12 mmol) was added dropwise under an ice bath, and the reaction solution was reacted at room temperature for 16 hours. 2 mL of methanol and 4 mL of hydrochloric acid (6M) were added dropwise under an ice bath, and the reaction solution was stirred for 1 hour. 50 mL of water was added to the reaction solution, which was extracted with a mixed solution of dichloromethane and methanol (V:V=10:1) (50 mL×2). The organic phases were combined, washed with water (30 mL) and saturated sodium chloride solution (30 mL) successively, dried over anhydrous sodium sulfate and filtered. The filtrate was concentrated under reduced pressure, and the resulting residues were purified by silica gel column chromatography with eluent system A to obtain the title product **11e** (120 mg, yield: 77.89%).
MS m/z (ESI): 727.0 [M+1]

### Step 6

### Methyl 21-chloro-5,9,26-trimethyl-32-oxa-2-thia-5,6,9,12,13,28-hexaazaoctacyclo[31.7. 1.1^{4,7}.0^{11,19}.0^{13,18}.0^{20,25}.0^{24,28}.0^{34,39}]dotetraconta-1(41),4(42),6,11,18,20,22,24,26,33,35,3 7,39-tridecaene-27-carboxylate 1f

Triphenylphosphine (260 mg, 1.03 mmol) and dibenzyl azodicarboxylate (208 mg, 1.03 mmol) were dissolved in 5 mL of toluene, and the solution was purged with argon three times. 6 mL of solution of **11e** (150 mg, 0.206 mmol) in toluene and tetrahydrofuran (V:V=5:1) was added dropwise, and the reaction solution was reacted at room temperature for 16 hours. 10 mL of hydrochloric acid (2M) was added, and the reaction solution was stirred for 10 minutes. 50 mL of water was added to the reaction solution, and extracted with ethyl acetate (50 mL×2). The organic phases were combined, washed with water (30 mL) and saturated sodium chloride solution (30 mL) successively, dried over anhydrous sodium sulfate and filtered. The filtrate was concentrated under reduced pressure, and the resulting residues were purified by silica gel column chromatography with eluent system A to obtain the title product **11f** (86 mg, yield: 58.79%).
MS m/z (ESI): 709.0 [M+1].

### Step 7

### 21-Chloro-5,9,26-trimethyl-32-oxa-2-thia-5,6,9,12,13,28-hexaazaoctacyclo[31.7. 1.1^{4,7}.0^{11,19}.0^{13,18}.0^{20,25}.0^{24,28}.0^{34,39}]dotetraconta-1(41),4(42),6,11,18,20,22,24,26,33,35,3 7,39-tridecaene-27-carboxylic acid 11

**11f** (30 mg, 0.043 mmol) was dissolved in 5 mL of a mixed solvent of methanol, tetrahydrofuran and water (V:V:V=1:1:1). Lithium hydroxide monohydrate (18 mg, 0.42 mmol) was added, and the reaction solution was heated to 50°C and reacted for 0.5 hour. The reaction solution was cooled to room temperature, and concentrated under reduced pressure to remove most of the solvent. 2M hydrochloric acid was added to adjust pH to 1-2, and the solution was extracted with a mixed solvent (50 mL×2) of dichloromethane and methanol (V:V=10:1). The organic phases were combined, washed with water (30 mL) and saturated sodium chloride solution (30 mL) successively, dried over anhydrous sodium sulfate, and filtered to remove the desiccant. The filtrate was concentrated under reduced pressure, and the resulting residues were purified by silica gel column chromatography with eluent system A to obtain the title product **11** (12 mg, yield: 40.80%).
MS m/z (ESI): 695.1 [M+1].
¹H NMR (400 MHz, CDCl₃) δ 8.37-8.39 (m, 1H), 7.78-7.79 (m, 1H), 7.66 (s, 1H), 7.54-7.60 (m, 2H), 7.27-7.29 (m, 1H), 7.12 (s, 1H), 7.01-7.03 (m, 1H), 6.11 (s, 1H), 5.23-5.26 (m, 1H), 5.10 (s, 1H), 4.62-4.67 (m, 1H), 3.77-3.88 (m, 4H), 3.50-3.63 (m, 5H), 3.21 (s, 3H), 3.11-3.15 (m, 1H), 3.92 (s, 3H), 2.62-2.65(m, 1H), 2.34-2.45 (m, 2H), 1.95-1.97 (m, 4H), 1.30 (s, 3H).

### Example 12

### 17-Chloro-5,13,14,22-tetramethyl-28-oxa-2,9-dithia-5,6,12,13,24-pentaazaheptacyclo[2 7.6.1.1^{4,7}.0^{11,15}.0^{16,21}.0^{20,24}.0^{30,35}]heptatriaconta-1(36),4(37),6,11,14,16,18,20,22,29,31(3 5)-undecaene-23-carboxylic acid 12

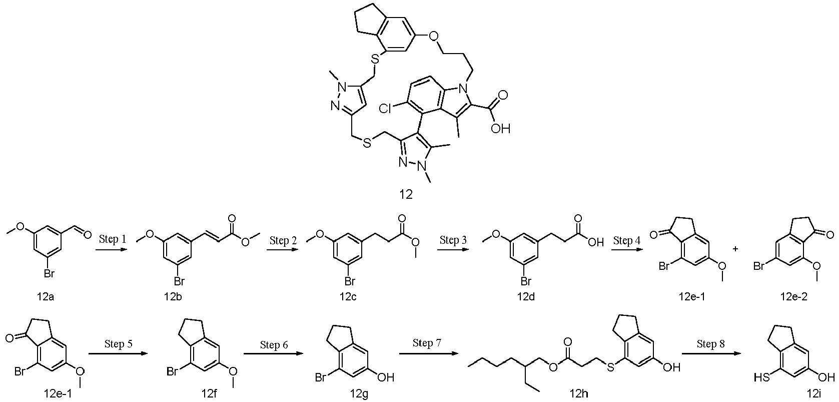

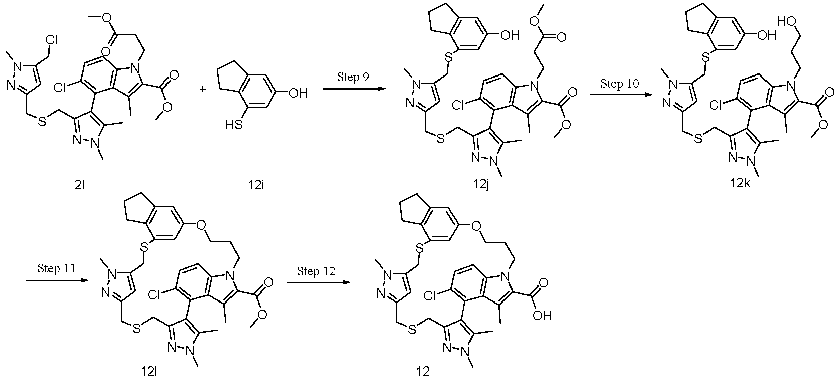

### Step 1

### Methyl (E)-3-(3-bromo-5-methoxyphenyl)acrylate 12b

NaH (1.34 g, 34.97 mmol, purity: 60%) was dissolved in tetrahydrofuran (100 mL), and the solution was purged with argon three times. Methyl 2-(dimethoxyphosphoryl)acetate (6.35 g, 34.87 mmol) was added dropwise under an ice bath, and the reaction solution was stirred in the ice bath for 30 minutes. A solution of 3-bromo-5-methoxybenzaldehyde (5.00 g, 23.25 mmol) in tetrahydrofuran (30 mL) was added dropwise, and the reaction solution was stirred at room temperature for 1 hour. Ethyl acetate (100 mL) and water (100 mL) were added under an ice bath, and the resulting solution was partitioned. The organic phase was washed with saturated sodium chloride solution (30 mL×2), dried over anhydrous sodium sulfate and filtered. The filtrate was concentrated under reduced pressure, and the resulting residues were purified by silica gel column chromatography with eluent system B to obtain the title product **12b** (6.20 g, yield: 98.4%).
MS m/z (ESI): 271.1 273.1 [M+1].

### Step 2

### Methyl 3-(3-bromo-5-methoxyphenyl)propanoate 12c

**12b** (6.00 g, 22.1 mmol) was dissolved in methanol (75 mL) and tetrahydrofuran (75 mL) at room temperature, followed by the addition of 5% dry rhodium carbon (600 mg). The reaction solution was purged with hydrogen three times, and stirred at room temperature for 90 minutes. The reaction solution was filtered, and the filtrate was concentrated under reduced pressure to obtain the title product **12c** (6.04 g, yield: 99.3%).
MS m/z (ESI): 273.0 275.0[M+1].

### Step 3

### 3-(3-Bromo-5-methoxyphenyl)propanoic acid 12d

**12c** (6.20 g, 22.7 mmol) was dissolved in methanol (30 mL), tetrahydrofuran (30 mL) and water (30 mL) at room temperature. Lithium hydroxide monohydrate (2.86 g, 68.2 mmol) was added, and the reaction solution was heated to 50°C and stirred for 1 hour. The reaction solution was concentrated under reduced pressure, followed by the addition of water (100 mL) and dichloromethane (100 mL). The solution was adjusted to pH=2-3 with 1M HCl, extracted with a mixed solvent (50 mL) of dichloromethane and methanol (V:V=10:1) and partitioned. The organic phase was washed with water (30 mL×3) and saturated sodium chloride solution (30 mL×2) successively, dried over anhydrous sodium sulfate and filtered. The filtrate was concentrated under reduced pressure to obtain the title product **12d** (5.80 g, yield: 98.6%).
MS m/z(ESI): 257.2 259.2[M-1].

### Step 4

### 7-Bromo-5-methoxy-2,3-dihydro-1H-inden-1-one 12e-1 5-Bromo-7-methoxy-2,3-dihydro-1H-inden-1-one 12e-2

**12d** (5.50 g, 21.2 mmol) was weighed into a 100 mL reaction flask at room temperature, followed by the addition of polyphosphoric acid (120 g, 35.5 mmol). The reaction solution was heated to 95°C and stirred for 1.5 hours. The reaction solution was poured into ice water, and then added with dichloromethane (200 mL) and partitioned. The organic phase was washed with sodium bicarbonate solution, water and saturated sodium chloride solution (30mL×3) successively, dried over anhydrous sodium sulfate and filtered. The filtrate was concentrated under reduced pressure, and the resulting residues were purified by silica gel column chromatography with eluent system B to obtain the title products **12e-1** (3.50 g, yield: 68.4%) and **12e-2** (1.00 g, yield: 19.5%).
MS m/z(ESI): 241.1 243.1 [M+1].

### Step 5

### 4-Bromo-6-methoxy-2,3-dihydro-1H-indene 12f

**12e-1** (3.50 g, 14.5 mmol) was dissolved in trifluoroacetic acid (30 mL) at room temperature, followed by the addition of triethyl silicane (3.37 g, 29.0 mmol). The reaction solution was heated to 80°C and stirred for 1.5 hours. The reaction solution was concentrated under reduced pressure, and then added with ethyl acetate (50 mL) and water (50 mL) and partitioned. The organic phase was washed with water and saturated sodium chloride solution (30 mL×3) successively, dried over anhydrous sodium sulfate and filtered. The filtrate was concentrated under reduced pressure, and the resulting residues were purified by silica gel column chromatography with eluent system B to obtain the title product **12f** (2.70 g, yield: 81.9%).

### Step 6

### 7-Bromo-2,3-dihydro-1H-inden-5-ol 12g

**12f** (2.70 g, 11.9 mmol) was dissolved in 30 mL of dichloromethane at room temperature. 1*M* solution of boron tribromide (40 mL, 40.0 mmol) in dichloromethane was added dropwise under an ice bath, and the reaction solution was stirred at room temperature for 2 hours. The reaction solution was poured into ice water, and extracted with dichloromethane (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **12g** (2.20 g, yield: 86.8%).
MS m/z (ESI): 211.0 213.0 [M-1].

### Step 7

### 2-Ethylhexyl 3-((6-hydroxy-2,3-dihydro-1H-inden-4-yl)thio)propanoate 12h

**12g** (800 mg, 3.75 mmol), 2-ethylhexyl 3-mercaptopropionate (984 mg, 4.51 mmol), N,N-diisopropylethylamine (971 mg, 7.51 mmol), tris(dibenzylideneacetone)dipalladium (172 mg, 0.19 mmol) and 4,5-bis(diphenylphosphine)-9,9-dimethylxanthene (217 mg, 0.38 mmol) were dissolved in 20 mL of dioxane at room temperature. The reaction solution was purged with argon three times, heated to 95 °C and stirred for 16 hours. The reaction solution was cooled to room temperature and filtered through celite. The filtrate was concentrated under reduced pressure, and the resulting residues were purified by silica gel column chromatography with eluent system B to obtain the title product **12h** (450 mg, yield: 34.2%).
MS m/z (ESI): 351.1 [M+1].

### Step 8

### 7-Mercapto-2,3-dihydro-1H-inden-5-ol 12i

**12h** (450 mg, 1.28 mmol) was dissolved in 4 mL of tetrahydrofuran under an ice bath, and the solution was purged with argon three times. 1M solution of potassium *tert*-butoxide (4.1 mL, 4.10 mmol) in tetrahydrofuran was added dropwise, and the reaction solution was stirred at room temperature for 2 hours. The title product **12i** was obtained, which was used directly in the next step.

### Step 9

### Methyl 5-chloro-4-(3-((((5-(((6-hydroxy-2,3-dihydro-1H-inden-4-yl)thio)methyl)-1-methyl-1H-pyrazol-3-yl)methyl)thio)methyl)-1,5-dimethyl-1H-pyrazol-4-yl)-1-(3-methoxy-3-oxopr opyl)-3-methyl-1H-indole-2-carboxylate 12j

**2l** (150 mg, 0.25 mmol) was dissolved in methanol (10 mL) and tetrahydrofuran (3 mL) at room temperature. 0.16M solution of the above reaction solution **12i** (2.7 mL, 0.43 mmol) in tetrahydrofuran was added dropwise under an ice bath, and the reaction solution was stirred at room temperature for 1 hour. The reaction solution was diluted with ethyl acetate (50 mL), and washed with water (30 mL×3) and saturated sodium chloride solution (30 mL×2) successively. The organic phase was dried over anhydrous sodium sulfate and filtered. The filtrate was concentrated under reduced pressure, and the resulting residues were purified by silica gel column chromatography with eluent system A to obtain the title product **12j** (200 mg, yield: 100%).
MS m/z (ESI): 722.0 [M+1].

### Step 10

### Methyl 5-chloro-4-(3-((((5-(((6-hydroxy-2,3-dihydro-1H-inden-4-yl)thio)methyl)-1-methyl-1H-pyrazol-3-yl)methyl)thio)methyl)-1,5 -dimethyl- 1H-pyrazol-4-yl)-1-(3-hydroxypropyl)-3-methyl-1H-indole-2-carboxylate 12k

**12j** (200 mg, 277 µmol) was dissolved in tetrahydrofuran (3 mL) at room temperature, and cooled to 0-5 °C. 1.0*M* solution of borane in tetrahydrofuran (2.8 mL) was slowly added dropwise, and the reaction solution was warmed up to room temperature and stirred for 16 hours. The reaction solution was cooled to 0-5°C and quenched by methanol. The solution was warmed up to room temperature and stirred for 30 minutes. Hydrochloric acid (3.2 mL, 6.0 *N*) was added and stirred for 30 minutes. The solution was extracted with a mixed solvent (30 mL×3) of dichloromethane and methanol (V:V=10:1). The organic phases were combined, washed with saturated sodium chloride solution (30 mL), dried over anhydrous sodium sulfate and filtered. The filtrate was concentrated under reduced pressure, and the resulting residues were purified by silica gel column chromatography with eluent system A to obtain the title product **12k** (50 mg, yield: 26.0%).
MS m/z(ESI): 694.0 [M+1].

### Step 11

### Methyl 17-chloro-5,13,14,22-tetramethyl-28-oxa-2,9-dithia-5,6,12,13,24-pentaazaheptacyclo[2 7.6.1.1^{4,7}.0^{11,15}.0^{16,21}.0^{20,24}.0^{31,35}]heptatriaconta-1(36),4(37),6,11,14,16,18,20,22,29,31(3 5)-undecaene-23-carboxylate 12l

**12k** (50 mg, 72 µmol) was dissolved in toluene (10 mL) and tetrahydrofuran (5 mL) at room temperature, followed by the addition of tri-n-butylphosphine (73 mg, 0.36 mmol). The solution was purged with argon three times, and added dropwise with a solution (3 mL) of azodicarbonyl dipiperidine (91 mg, 0.36 mmol) in toluene . The reaction solution was heated to 60°C and stirred for 2 hours. The reaction solution was concentrated under reduced pressure, and the resulting residues were purified by silica gel column chromatography with eluent system A to obtain the title product **12l** (30 mg, yield: 61.6%).
MS m/z(ESI): 675.9 [M+1].

### Step 12

### 17-Chloro-5,13,14,22-tetramethyl-28-oxa-2,9-dithia-5,6,12,13,24-pentaazaheptacyclo[2 7.6.1.1^{4,7}.0^{11,15}.0^{16,21}.0^{20,24}.0^{30,35}]heptatriaconta-1(36),4(37),6,11,14,16,18,20,22,29,31(3 5)-undecaene-23-carboxylic acid 12

**12l** (30 mg, 44 µmol) was dissolved in 10 mL of a mixed solution of tetrahydrofuran and methanol (V:V = 1:1) at room temperature. A solution of lithium hydroxide monohydrate (19 mg, 0.45 mmol) in water (2 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 *N*) was added dropwise until pH = 2-3, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate and filtered. The filtrate was concentrated under reduced pressure, and the resulting residues were purified by high performance liquid chromatography (Gilson GX-281, eluent system: H₂O (10 mmol NH₄OAc), ACN) to obtain the title product **7** (15 mg, yield: 51.1%).
MS m/z (ESI): 661.9 [M+1].
¹H NMR (400 MHz, CDCl₃) δ 7.38-7.41 (m, 1H), 7.23-7.25 (m, 1H), 6.82 (s, 1H), 6.09 (s, 1H), 5.00-5.12 (m, 2H), 4.47-4.51 (m, 1H), 3.88 (s, 3H), 3.72-3.84 (m, 2H), 3.68 (s, 3H), 3.51-3.65 (m, 2H), 3.38-3.41 (m, 1H), 3.22-3.31 (m, 1H), 3.17-3.20 (m, 1H), 2.75-3.01 (m, 5H), 2.17-2.36 (m, 5H), 1.96-2.15 (m, 5H).

### Examples 12-1 and 12-2

### (Ra)-17-Chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,35}]heptatriaconta-1(36),4(37),6,11,14,16,18,20,22,29, 31(35)-undecaene-23-carboxylic acid 12-1

### (Sa)-17-Chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,35}]heptatriaconta-1(36),4(37),6,11,14,16,18,20,22,29, 31(35)-undecaene-23-carboxylic acid 12-2

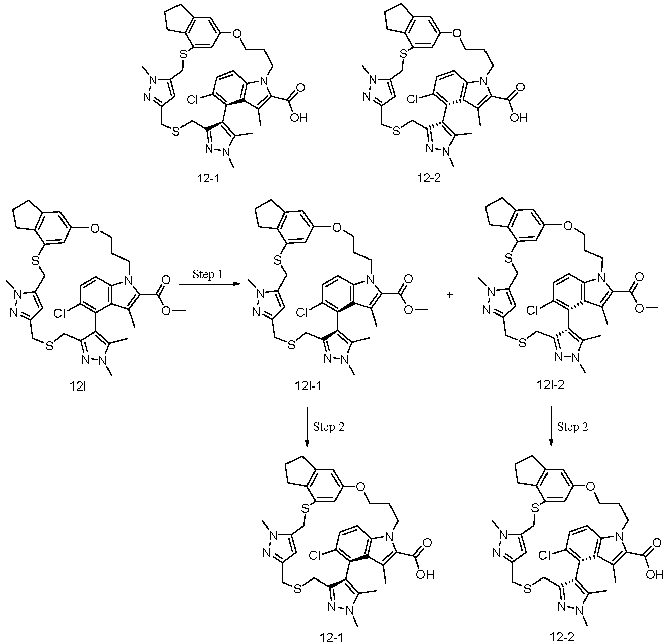

### Step 1

### Methyl (Ra)-17-chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{31,35}]heptatriaconta-1(36),4(37),6,11,14,16,18,20,22,29, 31(35)-undecaene-23-carboxylate 121-1

### Methyl (Sa)-17-chloro-5,13,14,22-tetramethyl-28-oxa-2,9-dithia-5,6,12,13,24-pentaazaheptacyc lo[27.6.1.1^{4,7}.0^{11,15}.0^{16,21}.0^{20,24}.0^{31,35}]heptatriaconta-1(36),4(37),6,11,14,16,18,20,22,29,3 1(35)-undecaene-23-carboxylate 121-2

**121** (570 mg, 0.84 mmol) was separated chirally (separation conditions: AY Phenomenex Lux Amylose-2 250^{∗}21.2mm, 5 µm; mobile phase: Hexane/EtOH/DEA=80/20/0.1(V/V/V); flow rate: 30 mL/min). The corresponding fractions were collected and concentrated under reduced pressure to obtain the title products (200 mg, 200 mg).

Compound **121-2** with single configuration (having shorter retention time):
MS m/z (ESI):676.3 [M+1].

Chiral HPLC analysis: retention time 10.787 minutes, chiral purity: 98% (chromatographic column: OZ Phenomenex Lux Cellulose-2 150^{∗}4.6mm, 5 µm (equipped with a guard column); mobile phase: *n*-hexane/ethanol/diethylamine= 70/30/0.1 (v/v/v)).

Compound **121-1** with single configuration (having longer retention time):
MS m/z (ESI): 676.3 [M+1].

Chiral HPLC analysis: retention time 14.598 minutes, chiral purity: 100% (chromatographic column: OZ Phenomenex Lux Cellulose-2 150^{∗}4.6mm, 5 µm (equipped with a guard column); mobile phase: *n*-hexane/ethanol/diethylamine= 70/30/0.1 (v/v/v)).

### Step 2

### (Ra)-17-Chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,35}]heptatriaconta-1(36),4(37),6,11,14,16,18,20,22,29, 31(35)-undecaene-23-carboxylic acid 12-1

### (Sa)-17-Chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,35}]heptatriaconta-1(36),4(37),6,11,14,16,18,20,22,29, 31(35)-undecaene-23-carboxylic acid 12-2

### Step 2(1)

Compound **121-1** having longer retention time (200 mg, 296 µmol) was dissolved in 20 mL of a mixed solution of tetrahydrofuran and methanol (V:V = 1:1) at room temperature. A solution of lithium hydroxide monohydrate (124 mg, 2.96 mmol) in water (5 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 2-3, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title product **12-1** (20 mg).

Compound **12-1** with single configuration (having shorter retention time):
MS m/z (ESI): 661.9 [M+1].

Chiral HPLC analysis: retention time 8.371 minutes, chiral purity: 100% (chromatographic column: CHIRALPAK IF 150^{∗}4.6mm, 5 µm, equipped with a guard column; mobile phase: Hexane/EtOH/TFA=80/20/0.1(V/V/V)).
¹H NMR (400 MHz, CDCl₃) δ 7.38-7.41 (m, 1H), 7.23-7.25 (m, 1H), 6.82 (s, 1H), 6.09 (s, 1H), 5.00-5.12 (m, 2H), 4.47-4.51 (m, 1H), 3.88 (s, 3H), 3.72-3.84 (m, 2H), 3.68 (s, 3H), 3.51-3.65 (m, 2H), 3.38-3.41 (m, 1H), 3.22-3.31 (m, 1H), 3.17-3.20 (m, 1H), 2.75-3.01 (m, 5H), 2.17-2.36 (m, 5H), 1.96-2.15 (m, 5H).

### Step 2(2)

Compound **121-2** having shorter retention time (200 mg, 296 µmol) was dissolved in 20 mL of a mixed solution of tetrahydrofuran and methanol (V:V = 1:1) at room temperature. A solution of lithium hydroxide monohydrate (124 mg, 2.96 mmol) in water (5 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 2-3, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title product **12-2** (20 mg).

Compound **12-2** with single configuration (having longer retention time):
MS m/z (ESI): 661.9 [M+1].

Chiral HPLC analysis: retention time 9.861 minutes, chiral purity: 95.9% (chromatographic column: CHIRALPAK IF 150^{∗}4.6mm, 5 µm, equipped with a guard column; mobile phase: Hexane/EtOH/TFA=80/20/0.1(V/V/V)).
¹H NMR (400 MHz, CDCl₃) δ 7.38-7.41 (m, 1H), 7.23-7.25 (m, 1H), 6.82 (s, 1H), 6.09 (s, 1H), 5.00-5.12 (m, 2H), 4.47-4.51 (m, 1H), 3.88 (s, 3H), 3.72-3.84 (m, 2H), 3.68 (s, 3H), 3.51-3.65 (m, 2H), 3.38-3.41 (m, 1H), 3.22-3.31 (m, 1H), 3.17-3.20 (m, 1H), 2.75-3.01 (m, 5H), 2.17-2.36 (m, 5H), 1.96-2.15 (m, 5H).

### Examples 13-1 and 13-2

### (Ra)-17-Chloro-5,13,14,22-tetramethyl-28-oxa-2,9-dithia-5,6,12,13,24-pentaazaheptacy clo[27.6.1.1^{1,7}.0^{11,15}.0^{16,21}.0^{20,24}.0^{30,34}]heptatriaconta-1(36),4(37),6,11,14,16,18,20,22,29, 34-undecaene-23-carboxylic acid 13-1

### (Sa)-17-Chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,34}]heptatriaconta-1(36),4(37),6,11,14,16,18,20,22,29, 34-undecaene-23-carboxylic acid 13-2

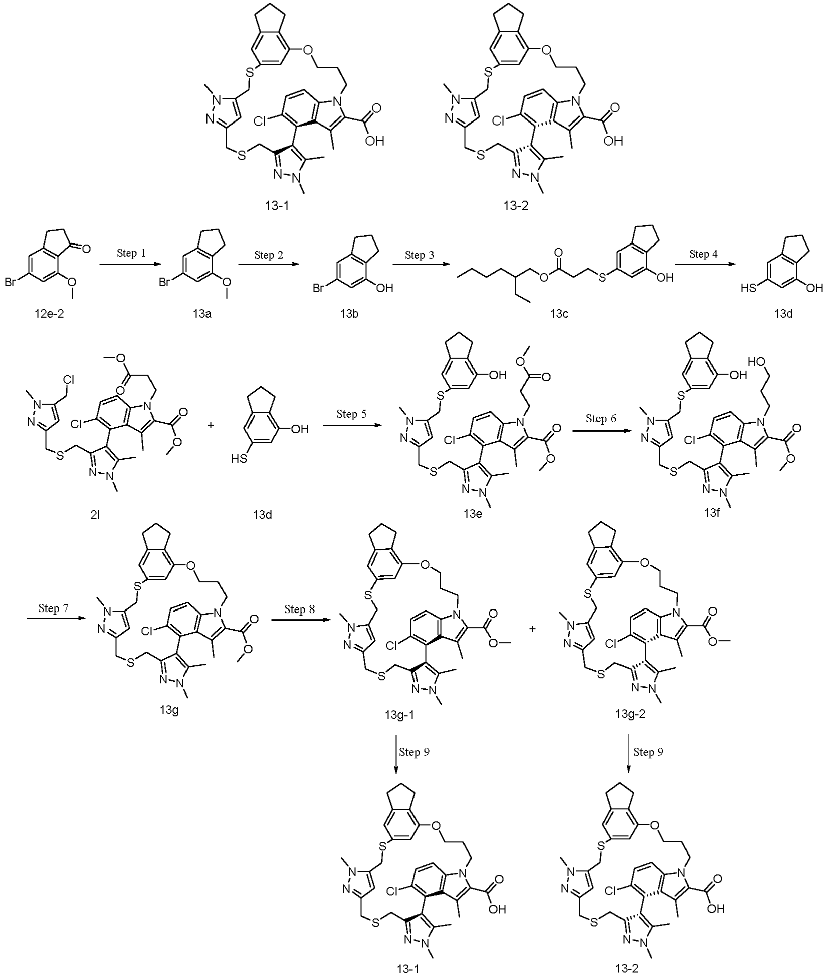

### Step 1

### 6-Bromo-4-methoxy-2,3-dihydro-1H-indene 13a

**12e-2** (1.00 g, 4.15 mmol) was dissolved in trifluoroacetic acid (10 mL) at room temperature, followed by the addition of triethyl silicane (965 mg, 8.30 mmol). The reaction solution was heated to 80°C and stirred for 1.5 hours. The reaction solution was concentrated under reduced pressure, and then added with ethyl acetate (50 mL) and water (50 mL) and partitioned. The organic phase was washed with water and saturated sodium chloride solution (30 mL×3) successively, dried over anhydrous sodium sulfate and filtered. The filtrate was concentrated under reduced pressure, and the resulting residues were purified by silica gel column chromatography with eluent system B to obtain the title product **13a** (880 mg, yield: 93.4%).

### Step 2

### 6-Bromo-2,3-dihydro-1H-inden-4-ol 13b

**13a** (900 mg, 3.96 mmol) was dissolved in 10 mL of dichloromethane at room temperature. 1*M* solution of boron tribromide (13.9 mL, 13.9 mmol) in dichloromethane was added dropwise under an ice bath, and the reaction solution was stirred at room temperature for 2 hours. The reaction solution was poured into ice water, and extracted with dichloromethane (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system B to obtain the title product **13b** (620 mg, yield: 73.4%).
MS m/z (ESI): 211.0 213.0 [M-1].

### Step 3

### 2-Ethylhexyl 3-((7-hydroxy-2,3-dihydro-1H-inden-5-yl)thio)propanoate 13c

**13b** (620 mg, 2.91 mmol), 2-ethylhexyl 3-mercaptopropionate (762 mg, 3.49 mmol), N,N-diisopropylethylamine (752 mg, 5.82 mmol), tris(dibenzylideneacetone)dipalladium (133 mg, 0.15 mmol) and 4,5-bis(diphenylphosphine)-9,9-dimethylxanthene (168 mg, 0.29 mmol) were dissolved in 20 mL of dioxane at room temperature. The reaction solution was purged with argon three times, heated to 95°C and stirred for 16 hours. The reaction solution was filtered through celite, and the filtrate was concentrated under reduced pressure. The resulting residues were purified by silica gel column chromatography with eluent system B to obtain the title product **13c** (550 mg, yield: 53.9%).
MS m/z (ESI): 351.3 [M+1].

### Step 4

### 6-Mercapto-2,3-dihydro-1H-inden-4-ol 13d

**13c** (550 mg, 1.57 mmol) was dissolved in 10 mL of tetrahydrofuran under an ice bath, and the solution was purged with argon three times. 1*M* solution of potassium *tert*-butoxide (5.0 mL, 5.0 mmol) in tetrahydrofuran was added dropwise, and the reaction solution was stirred at room temperature for 2 hours. The title product **13d** was obtained, which was used directly in the next step.

### Step 5

### Methyl 5-chloro-4-(3-((((5-(((7-hydroxy-2,3-dihydro-1H-inden-5-yl)thio)methyl)-1-methyl-1H-pyrazol-3-yl)methyl)thio)methyl)-1,5-dimethyl-1H-pyrazol-4-yl)-1-(3-methoxy-3-oxopr opyl)-3-methyl-1H-indole-2-carboxylate 13e

**2l** (250 mg, 0.42 mmol) was dissolved in methanol (10 mL) and tetrahydrofuran (3 mL) at room temperature. 0.1*M* solution of the above reaction solution **13d** (6.3 mL, 0.63 mmol) in tetrahydrofuran was added dropwise under an ice bath, and the reaction solution was stirred at room temperature for 1 hour. The reaction solution was diluted with ethyl acetate (50 mL), and washed with water (30 mL×3) and saturated sodium chloride solution (30 mL×2) successively. The organic phase was dried over anhydrous sodium sulfate and filtered. The filtrate was concentrated under reduced pressure, and the resulting residues were purified by silica gel column chromatography with eluent system A to obtain the title product **13e** (280 mg, yield: 91.9%).
MS m/z(ESI): 722.0[M+1].

### Step 6

### Methyl 5-chloro-4-(3-((((5-(((7-hydroxy-2,3-dihydro-1H-inden-5-yl)thio)methyl)-1-methyl-1H-pyrazol-3-yl)methyl)thio)methyl)-1,5-dimethyl-1H-pyrazol-4-yl)-1-(3-hydroxypropyl)-3-methyl-1H-indole-2-carboxylate 13f

**13e** (280 mg, 388 µmol) was dissolved in tetrahydrofuran (5 mL) at room temperature, and cooled to 0-5°C. 1.0M solution of borane in tetrahydrofuran (3.9 mL) was slowly added dropwise, and the reaction solution was warmed up to room temperature and stirred for 16 hours. The reaction solution was cooled to 0-5°C and quenched by methanol. The solution was warmed up to room temperature and stirred for 30 minutes. Hydrochloric acid (6.0 mL, 6.0 *N*) was added and stirred for 30 minutes. The solution was extracted with a mixed solvent (30 mL×3) of dichloromethane and methanol (V:V=10:1). The organic phases were combined, washed with saturated sodium chloride solution (30 mL), dried over anhydrous sodium sulfate and filtered. The filtrate was concentrated under reduced pressure, and the resulting residues were purified by silica gel column chromatography with eluent system A to obtain the title product **13f** (130 mg, yield: 48.3%).
MS m/z(ESI): 693.9 [M+1].

### Step 7

### Methyl 17-chloro-5,13,14,22-tetramethyl-28-oxa-2,9-dithia-5,6,12,13,24-pentaazaheptacyclo[2 7.6.1.1^{4,7}.0^{11,15}.0^{16,21}.0^{20,24}.0^{30,34}]heptatriaconta-1(36),4(37),6,11,14,16,18,20,22,29,34-u ndecaene-23-carboxylate 13g

**13f** (130 mg 187 µmol) was dissolved in toluene (10 mL) and tetrahydrofuran (5 mL) at room temperature, followed by the addition of tri-*n*-butylphosphine (189 mg, 0.94 mmol). The solution was purged with argon three times, and added dropwise with a solution (5 mL) of azodicarbonyl dipiperidine (236 mg, 0.94 mmol) in toluene. The reaction solution was heated to 60°C and stirred for 2 hours. The reaction solution was concentrated under reduced pressure, and the resulting residues were purified by silica gel column chromatography with eluent system A to obtain the title product **13g** (100 mg, yield: 79.0%).
MS m/z (ESI): 676.0 [M+1].

### Step 8

### Methyl (Ra)-17-chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,34}]heptatriaconta-1(36),4(37),6,11,14,16,18,20,22,29, 34-undecaene-23-carboxylate 13g-1

### Methyl (Sa)-17-chloro-5,13,14,22-tetramethyl-28-oxa-2,9-dithia-5,6,12,13,24-pentaazaheptacyc lo[27.6.1.1^{4,7}.0^{11,15}.0^{16,21}.0^{20,24}.0^{30,34}]heptatriaconta-1(36),4(37),6,11,14,16,18,20,22,29,3 4-undecaene-23-carboxylate 13g-2

**13g** (100 mg, 0.84 mmol) was separated chirally (separation conditions: CHIRALPAK ID 250^{∗}20mm, 5 µm; mobile phase: Hexane/EtOH/DEA=80/20/0.1(V/V/V); flow rate: 20 mL/min). The corresponding fractions were collected and concentrated under reduced pressure to obtain the title products (40 mg, 40 mg).

Compound **13g-1** with single configuration (having shorter retention time):
MS m/z (ESI): 676.0 [M+1].

Chiral HPLC analysis: retention time 7.448 minutes, chiral purity: 100% (chromatographic column: CHIRALPAK ID 150^{∗}4.6mm, 5 µm (equipped with a guard column); mobile phase: n-hexane/ethanol/diethylamine=70/30/0.1(v/v/v)).

Compound **13g-2** with single configuration (having longer retention time):
MS m/z (ESI):676.0 [M+1].

Chiral HPLC analysis: retention time 8.611 minutes, chiral purity: 100% (chromatographic column: CHIRALPAK ID 150^{∗}4.6mm, 5 µm (equipped with a guard column); mobile phase: n-hexane/ethanol/diethylamine=70/30/0.1(v/v/v)).

### Step 9 (Ra)-17-Chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,34}]heptatriaconta-1(36),4(37),6,11,14,16,18,20,22,29, 34-undecaene-23-carboxylic acid 13-1

### (Sa)-17-Chloro-5,13,14,22-tetramethyl-28-oxa-2,9-dithia-5,6,12,13,24-pentaazaheptacy clo[27.6.1.1^{4,7}.0^{11,15}.0^{16,21}.0^{20,24}.0^{30,34}]heptatriaconta-1(36),4(37),6,11,14,16,18,20,22,29, 34-undecaene-23-carboxylic acid 13-2

Compound **13g-1** (having shorter retention time)/compound **13g-2** (having longer retention time) (40 mg/40 mg, 59 µmol/59 µmol) was dissolved in 20 mL of a mixed solution of tetrahydrofuran and methanol (V:V = 1:1) at room temperature, respectively. A solution of lithium hydroxide monohydrate (25 mg, 0.60 mmol) in water (5 mL) was added, and the reaction solution was heated to 50°C and stirred for 1 hour. The reaction solution was cooled to room temperature, diluted with water (15 mL), and concentrated under reduced pressure to remove most of the organic solvent. Diluted hydrochloric acid (1.0 N) was added dropwise until pH = 2-3, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by high performance liquid chromatography (Gilson GX-281, eluent system: H₂O (10 mmol NH₄OAc), ACN) to obtain the title products **13-1/13-2** (10 mg/10 mg), respectively.

Compound **13-1** with single configuration (having shorter retention time):
MS m/z (ESI): 662.0 [M+1].

Chiral HPLC analysis: retention time 5.609 minutes, chiral purity: 100% (chromatographic column: CHIRALPAK IF 150^{∗}4.6mm, 5 µm, equipped with a guard column; mobile phase: Hexane/EtOH/TFA=70/30/0.1(V/V/V)).
¹H NMR (400 MHz, CDCl₃) δ 7.31-7.34 (m, 1H), 7.20-7.22 (m, 1H), 7.06 (s, 1H), 5.90 (s, 1H), 5.17 (s, 1H), 5.04-5.08 (m, 1H), 4.46-4.51 (m, 1H), 3.88 (s, 3H), 3.77-3.81 (m, 1H), 3.62-3.72 (m, 2H), 3.53 (s, 3H), 3.46-3.48 (m, 1H), 3.32-3.36 (m, 1H), 3.20-3.26 (m, 1H), 3.09-3.13 (m, 1H), 2.86-3.05 (m, 4H), 2.69-2.72 (m, 1H), 2.09-2.30 (m, 7H), 2.04 (s, 3H).

Compound **13-2** with single configuration (having longer retention time):
MS m/z (ESI): 662.0 [M+1].

Chiral HPLC analysis: retention time 6.807 minutes, chiral purity: 98.8% (chromatographic column: CHIRALPAK IF 150^{∗}4.6mm, 5 µm, equipped with a guard column; mobile phase: Hexane/EtOH/TFA=70/30/0.1(V/V/V)).
¹H NMR (400 MHz, CDCl₃) δ 7.31-7.34 (m, 1H), 7.20-7.22 (m, 1H), 7.06 (s, 1H), 5.90 (s, 1H), 5.17 (s, 1H), 5.04-5.08 (m, 1H), 4.46-4.51 (m, 1H), 3.88 (s, 3H), 3.77-3.81 (m, 1H), 3.62-3.72 (m, 2H), 3.53 (s, 3H), 3.46-3.48 (m, 1H), 3.32-3.36 (m, 1H), 3.20-3.26 (m, 1H), 3.09-3.13 (m, 1H), 2.86-3.05 (m, 4H), 2.69-2.72 (m, 1H), 2.09-2.30 (m, 7H), 2.04 (s, 3H).

### Examples 14-1 and 14-2

### (Ra)-17-Chloro-22-ethyl-5,13-14-trimethyl-28-oxa-2,9-dithia-5,6,12,13,24-pentaazahep tacyclo[27.7.1.1^{4,7}.0^{11,15}.0^{16,21}.0^{20,24}.0^{30,35}]octatriaconta-1(37),4(38),6,11,14,16,18,20,22, 29,31,33,35-tridecaene-23-carboxylic acid 14-1

### (Sa)-17-Chloro-22-ethyl-5,13-14-trimethyl-28-oxa-2,9-dithia-5,6,12,13,24-pentaazahept acyclo[27.7.1.1^{4,7}.0^{11,15}.0^{16,21}.0^{20,24}.0^{30,35}]octatriaconta-1(37),4(38),6,11,14,16,18,20,22,2 9,31,33,35-tridecaene-23-carboxylic acid 14-2

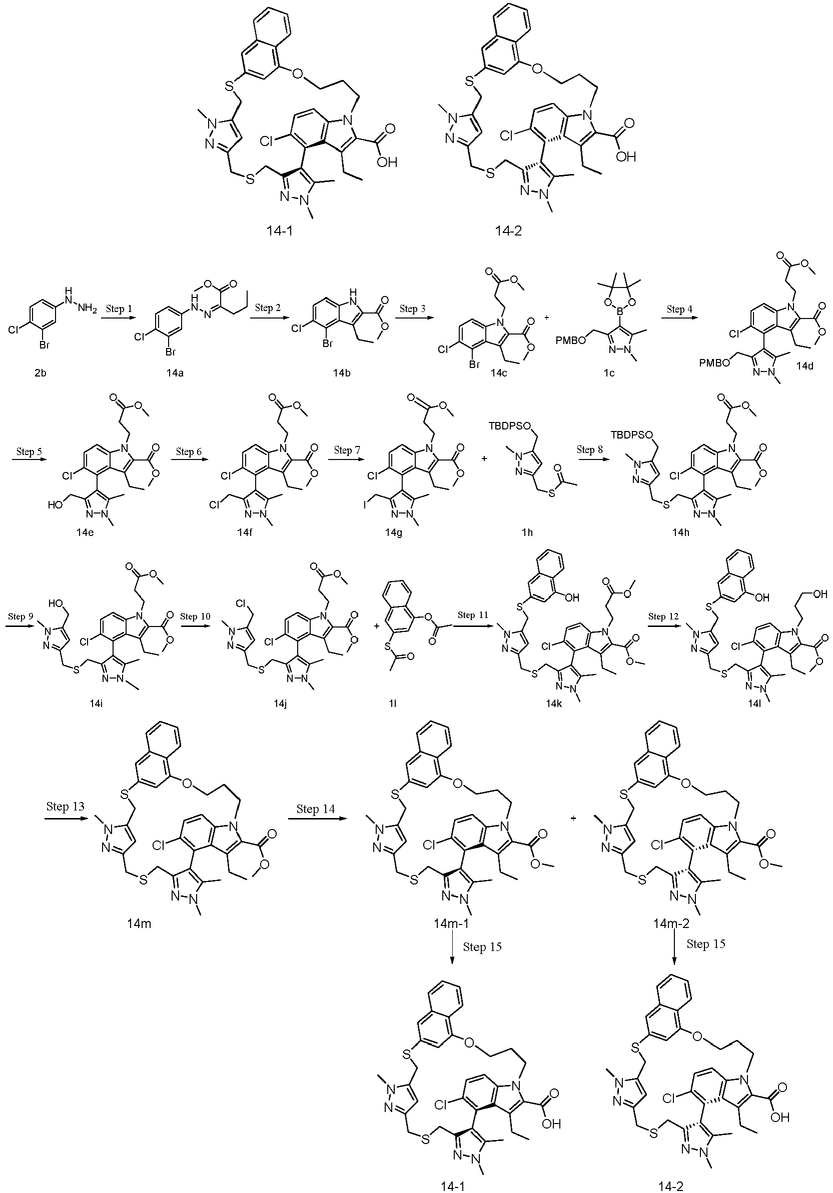

### Step 1

Methyl (Z)-2-(2-(3-bromo-4-chlorophenyl)hydrazono)pentanoate **14a** (3-Bromo-4-chlorophenyl)hydrazine **2b** (41.80 g, 188.73 mmol) was dissolved in 150 mL of ethanol. A solution of methyl 2-oxopentanoate (25.30 g, 194.40 mmol) in ethanol (20 mL) was added dropwise under an ice bath, and the reaction solution was reacted at room temperature for 1 hour. The reaction solution was concentrated under reduced pressure, and added with 80 mL of n-hexane to pulp. The mixture was filtered, and the filter cake was collected and dried under vacuum to obtain the title product **14a** (48.30 g, yield: 76.71%).
MS m/z (ESI): 333.0 335.0 [M+1].

### Step 2

### Methyl 4-bromo-5-chloro-3-ethyl-1H-indole-2-carboxylate 14b

**14a** (48.30 g, 144.78 mmol) was dissolved in 400 mL of glacial acetic acid. Zinc chloride (114.00 g, 836.41 mmol) was added, and the reaction solution was heated to 120°C and reacted for 1 hour. The reaction solution was poured into 800 mL of ice water, and a white solid was precipitated. The solid was filtered, and dried under vacuum to obtain the crude title product **2d** (42.00 g, yield: 91.63%), which was used directly in the next step without purification.
MS m/z (ESI): 314.0 316.0 [M-1].

### Step 3

### Methyl 4-bromo-5-chloro-1-(3-methoxy-3-oxopropyl)-3-ethyl-1H-indole-2-carboxylate 14c

The crude product **14b** (41.00 g, 129.51 mmol) and potassium carbonate (35.80 g, 259.03 mmol) were dissolved in 400 mL of *N*,*N*-dimethylformamide. Methyl acrylate (33.44 g, 388.44 mmol) was added at room temperature, and the reaction solution was reacted at room temperature for 16 hours. After completion of the reaction, 200 mL of water and 600 mL of ethyl acetate were added, and the resulting solution was partitioned. The organic phase was washed with saturated sodium chloride solution (100 mL×2), dried over anhydrous sodium sulfate and filtered. The filtrate was concentrated under reduced pressure, and the resulting residues were purified by silica gel column chromatography with eluent system B to obtain the title product **14c** (11.70 g, yield: 22.44%).
MS m/z (ESI): 402.0 404.0 [M+1].

### Step 4

### Methyl 5-chloro-1-(3-methoxy-3-oxopropyl)-4-(3-(((4-methoxybenzyl)oxy)methyl)-1,5-dimeth yl-1H-pyrazol-4-yl)-3-ethyl-1H-indole-2-carboxylate 14d

**14c** (5.0 g, 12.42 mmol) and **1c** (5.55 mg, 14.91 mmol) were dissolved in 100 mL of a mixed solution of 1,4-dioxane and water (V:V=4:1). The solution was purged with argon three times, and added with 1,1'-bis(di-*tert*-butylphosphine)ferrocaene dichloropalladium (440 mg, 621.41 mmol) and cesium carbonate (8.10 g, 24.86 mmol). The reaction solution was heated to 95 °C and stirred for 16 hours. The reaction solution was concentrated under reduced pressure to remove most of the solvent, and then added with 50 mL of water and extracted with ethyl acetate (150 mL×3). The organic phase was washed with water (50 mL) and saturated sodium chloride solution (50 mL) successively, dried over anhydrous sodium sulfate and filtered. The filtrate was concentrated under reduced pressure, and the resulting residues were purified by silica gel column chromatography with eluent system A to obtain the title product **14d** (6.75 g, yield: 95.69%).
MS m/z (ESI):568.2 [M+1].

### Step 5

### Methyl 5-chloro-4-(3-(hydroxymethyl)-1,5-dimethyl-1H-pyrazol-4-yl)-1-(3-methoxy-3-oxopro pyl)-3-ethyl-1H-indole-2-carboxylate 14e

**14d** (6.75 g, 11.88 mmol) was dissolved in 50 mL of methanol. 7 mL of concentrated sulfuric acid was added, and the reaction solution was heated to 80°C and reacted for 16 hours. The reaction solution was concentrated under reduced pressure to remove most of the solvent, and the resulting residues were poured into 100 mL of ice water. The solution was extracted with ethyl acetate (50 mL×3). The organic phase was washed with water (20 mL) and saturated sodium chloride solution (20 mL) successively, dried over anhydrous sodium sulfate and filtered. The filtrate was concentrated under reduced pressure, and the resulting residues were purified by silica gel column chromatography with eluent system A to obtain the title product **14e** (3.50 g, yield: 65.76%).
MS m/z (ESI): 448.2 [M+1].

### Step 6

### Methyl

### 5-chloro-4-(3-(chloromethyl)-1,5-dimethyl-1H-pyrazol-4-yl)-1-(3-methoxy-3-oxopropy l)-3-ethyl-1H-indole-2-carboxylate 14f

**14e** (260 mg, 0.58 mmol) was dissolved in 5 mL of dichloromethane, and the solution was purged with argon three times. Thionyl chloride (104 mg, 0.87 mmol) was added dropwise under an ice bath, and the reaction solution was reacted at room temperature for 30 minutes. 20 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate and filtered. The filtrate was concentrated under reduced pressure to obtain the crude title product **14f** (283 mg), which was used directly in the next step without purification.

### Step 7

### Methyl 5-chloro-4-(3-(iodomethyl)-1,5-dimethyl-1H-pyrazol-4-yl)-1-(3-methoxy-3-oxopropyl)-3 -ethyl- 1H-indole-2-carboxylate 14g

The crude product **14f** (283 mg, 0.61 mmol) was dissolved in 5 mL of acetonitrile, followed by the addition of sodium iodide (182 mg, 1.21 mmol). The reaction solution was heated to 80°C and stirred for 2 hours. The reaction solution was cooled to room temperature, and added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure to obtain the title product **14g** (338 mg, yield: 99.85%).
MS m/z (ESI): 558.0 [M+1].

### Step 8

### Methyl 4-(3-((((5-(((tert-butyldiphenylsilyl)oxy)methyl)-1-methyl-1H-pyrazol-3-yl)methyl)thio )methyl)-1,5-dimethyl-1H-pyrazol-4-yl)-5-chloro-1-(3-methoxy-3-oxopropyl)-3-ethyl-1 H-indole-2-carboxylate 14h

**14g** (338 mg, 0.61 mmol) was dissolved in 6 mL of methanol and 2 mL of tetrahydrofuran, followed by the addition of potassium carbonate (101 mg, 0.73 mmol). The solution was purged with argon three times, and added dropwise with a solution of **1h** (334 mg, 0.77 mmol) in methanol (5 mL) at room temperature. The reaction solution was reacted at room temperature for 2 hours. The reaction solution was concentrated under reduced pressure to remove most of the solvent, and added with 50 mL of water. The solution was stirred for 30 minutes, and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system A to obtain the title product **14h** (225 mg, yield: 44.92%).
MS m/z (ESI): 826.2 [M+1].

### Step 9

### Methyl 5-chloro-4-(3-((((5-(hydroxymethyl)-1-methyl-1H-pyrazol-3-yl)methyl)thio)methyl)-1, 5-dimethyl-1H-pyrazol-4-yl)-1-(3-methoxy-3-oxopropyl)-3-ethyl-1H-indole-2-carboxyl ate 14i

**14h** (225 mg, 0.27 mmol) was dissolved in 10 mL of tetrahydrofuran. 1.0 M tetrabutylammonium fluoride (0.33 mL, 0.33 mmol) was added dropwise, and the reaction solution was stirred at room temperature for 1 hour. The reaction solution was concentrated under reduced pressure to remove most of the solvent, and added with 50 mL of water was added and extracted with ethyl acetate (50 mL×2). The organic phases were combined, washed with water (30 mL) and saturated sodium chloride solution (30 mL), dried over anhydrous sodium sulfate and filtered. The filtrate was concentrated under reduced pressure, and the resulting residues were purified by silica gel column chromatography with eluent system A to obtain the title product **14i** (118 mg, yield: 73.70%).
MS m/z (ESI): 588.2 [M+1].

### Step 10

### Methyl 5-chloro-4-(3-((((5-(chloromethyl)-1-methyl-1H-pyrazol-3-yl)methyl)thio)methyl)-1,5-dimethyl-1H-pyrazol-4-yl)-1-(3-methoxy-3-oxopropyl)-3-ethyl-1H-indole-2-carboxylat e 14j

**14i** (118 mg, 0.20 mmol) was dissolved in 5 mL of dichloromethane, and the solution was purged with argon three times. Thionyl chloride (29 mg, 0.24 mmol) was added dropwise under an ice bath, and the reaction solution was reacted at room temperature for 30 minutes. 50 mL of water was added to the reaction solution, stirred for 10 minutes, and extracted with dichloromethane (30 mL×2). The organic phases were combined, washed with water (30 mL) and saturated sodium chloride solution (30 mL), dried over anhydrous sodium sulfate and filtered. The filtrate was concentrated under reduced pressure to obtain the title product **14j** (121 mg), which was used directly in the next step without purification.
MS m/z (ESI): 606.2 [M+1].

### Step 11

### Methyl 5-chloro-4-(3-((((5-(((4-hydroxynaphthalen-2-yl)thio)methyl)-1-methyl-1H-pyrazol-3-y l)methyl)thio)methyl)-1,5-dimethyl-1H-pyrazol-4-yl)-1-(3-methoxy-3-oxopropyl)-3-eth yl-1H-indole-2-carboxylate 14k

The crude product **14j** (121 mg, 0.20 mmol) and **1l** (63 mg, 0.24 mmol) were dissolved in 5 mL of methanol. Potassium carbonate (78 mg, 0.57 mmol) was added at room temperature, and the reaction solution was reacted at room temperature for 1 hour. The reaction solution was concentrated under reduced pressure to remove most of the solvent, and then added with 50 mL of water and extracted with ethyl acetate (50 mL×2). The organic phases were combined, washed with water (30 mL) and saturated sodium chloride solution (30 mL), dried over anhydrous sodium sulfate, and filtered to remove the desiccant. The filtrate was concentrated under reduced pressure, and the resulting residues were purified by silica gel column chromatography with eluent system A to obtain the title product **14k** (115 mg, yield: 77.24%).
MS m/z (ESI): 746.2 [M+1].

### Step 12

### Methyl 5-chloro-4-(3-((((5-(((4-hydroxynaphthalen-2-yl)thio)methyl)-1-methyl-1H-pyrazol-3-y l)methyl)thio)methyl)-1,5-dimethyl-1H-pyrazol-4-yl)-1-(3-hydroxypropyl)-3-ethyl-1H-i ndole-2-carboxylate 14l

**14k** (105 mg, 0.14 mmol) was dissolved in 5 mL of tetrahydrofuran. 1.0M solution of borane in tetrahydrofuran (1.4 mL, 1.41 mmol) was added dropwise under an ice bath, and the reaction solution was stirred at room temperature for 12 hours. 1 mL of methanol and 2 mL of diluted hydrochloric acid (6M) were added dropwise under an ice bath, and the reaction solution was stirred for 1 hour. 50 mL of water was added to the reaction solution, which was extracted with a mixed solution of dichloromethane and methanol (V:V=10:1) (50 mL×2). The organic phases were combined, washed with water (30 mL) and saturated sodium chloride solution (30 mL) successively, dried over anhydrous sodium sulfate and filtered. The filtrate was concentrated under reduced pressure, and the resulting residues were purified by silica gel column chromatography with eluent system A to obtain the title product **14l** (100 mg, yield: 98.95%).
MS m/z (ESI):718.2 [M+1].

### Step 13

### Methyl 17-chloro-22-ethyl-5,13-14-trimethyl-28-oxa-2,9-dithia-5,6,12,13,24-pentaazaheptacycl o[27.7.1.1^{4,7}.0^{11,15}.0^{16,21}.0^{20,24}.0^{30,35}]octatriaconta-1(37),4(38),6,11,14,16,18,20,22,29,31, 33,35-tridecaene-23-carboxylate 14m

**14l** (100 mg, 0.14 mmol) was dissolved in 10 mL of a mixed solution of toluene and tetrahydrofuran (V:V=5:1), followed by the addition of tributylphosphine (141 mg, 0.70 mmol). The solution was purged with argon three times, and added dropwise with a solution (3 mL) of azodicarbonyl dipiperidine (176 mg, 0.70 mmol) in toluene. The reaction solution was heated to 60°C and stirred for 2 hours. The reaction solution was concentrated under reduced pressure, and the resulting residues were purified by silica gel column chromatography with eluent system A to obtain the title product **14m** (40 mg, yield: 41.03%).
MS m/z (ESI): 700.1 [M+1].

### Step 14

### Methyl (Ra)-17-chloro-22-ethyl-5,13-14-trimethyl-28-oxa-2,9-dithia-5,6,12,13,24-pentaazahept acyclo[27.7.1.1^{4,7}.0^{11,15}.0^{16,21}.0^{20,24}.0^{30,35}]octatriaconta-1(37),4(38),6,11,14,16,18,20,22,2 9,31,33,35-tridecaene-23-carboxylate 14m-1

### Methyl (Sa)-17-chloro-22-ethyl-5,13-14-trimethyl-28-oxa-2,9-dithia-5,6,12,13,24-pentaazahept acyclo[27.7.1.1^{4,7}.0^{11,15}.0^{16,21}.0^{20,24}.0^{30,35}]octatriaconta-1(37),4(38),6,11,14,16,18,20,22,2 9,31,33,35-tridecaene-23-carboxylate 14m-2

**14m** (40 mg, 0.057 mmol) was separated chirally (separation conditions: CHIRALPAK IE 250^{∗}20mm, 5 µm; mobile phase: *n*-hexane/ethanol/diethylamine=60/40/0.1(V/V/V); flow rate: 15 mL/min). The corresponding fractions were collected and concentrated under reduced pressure to obtain the title products (15 mg, 10 mg).

Compound **14m-1** with single configuration (having shorter retention time):
MS m/z (ESI): 700.2 [M+1].

Chiral HPLC analysis: retention time 7.268 minutes, chiral purity: 100% (chromatographic column: CHIRALPAK IE 150^{∗}4.6mm, 5 µm, equipped with a guard column; mobile phase: *n*-hexane/ethanol/diethylamine=60/40/0.1(v/v/v)).

Compound **14m-2** with single configuration (having longer retention time):
MS m/z (ESI):700.2 [M+1].

Chiral HPLC analysis: retention time 9.573 minutes, chiral purity: 100% (chromatographic column: CHIRALPAK IE 150^{∗}4.6mm, 5 µm, equipped with a guard column; mobile phase: *n*-hexane/ethanol/diethylamine=60/40/0.1(v/v/v)).

### Step 15

### (Ra)-17-chloro-22-ethyl-5,13-14-trimethyl-28-oxa-2,9-dithia-5,6,12,13,24-pentaazahep tacyclo[27.7.1.1^{4,7}.0^{11,15}.0^{16,21}.0^{20,24}.0^{30,35}]octatriaconta-1(37),4(38),6,11,14,16,18,20,22, 29,31,33,35-tridecaene-23-carboxylic acid 14-1

### (Sa)-17-Chloro-22-ethyl-5,13-14-trimethyl-28-oxa-2,9-dithia-5,6,12,13,24-pentaazahept acyclo[27.7.1.1^{4,7}.0^{11,15}.0^{16,21}.0^{20,24}.0^{30,35}]octatriaconta-1(37),4(38),6,11,14,16,18,20,22,2 9,31,33,35-tridecaene-23-carboxylic acid 14-2

Compound **14m-1** (having shorter retention time)/compound **14m-2** (having longer retention time) (15 mg/10 mg, 21.4 µmol/14 µmol) was dissolved in 6 mL/6 mL of a mixed solvent of methanol, tetrahydrofuran and water (V:V:V=1:1:1) at room temperature, respectively. Lithium hydroxide monohydrate (9 mg/ 6 mg, 0.21 mmol/0.14 mmol) was added, and the reaction solution was heated to 50°C and stirred for 8 hours. The reaction solution was cooled to room temperature, diluted with water (15 mL/15 mL), and concentrated under reduced pressure to remove most of the organic solvent. Diluted hydrochloric acid (1.0 N) was added dropwise until pH=1∼2, and the solution was extracted with a mixed solvent (50 mL×2) of dichloromethane and methanol (V:V=10:1). The organic phases were combined, washed with water (30 mL) and saturated sodium chloride solution (30 mL), dried over anhydrous sodium sulfate and filtered. The filtrate was concentrated under reduced pressure, and the resulting residues were purified by high performance liquid chromatography (Waters 2767/MS, eluent system: H₂O (10 mmol NH₄OAc), ACN) to obtain the title products **14-1/14-2** (2.57 mg, 6.55 mg), respectively.

Compound **14-1** with single configuration (having shorter retention time):
MS m/z (ESI): 686.2 [M+1].

Chiral HPLC analysis: retention time 6.687 minutes, chiral purity: 100% (chromatographic column: CHIRALPAK IE 150^{∗}4.6 mm, 5 µm, equipped with a guard column; mobile phase: n-hexane/ethanol/trifluoroacetic acid =70/30/0.1(V/V/V)).
¹H NMR (400 MHz, CDCl₃) δ8.33 (d, 1H), 7.76 (d, 1H), 7.65-7.51 (m, 3H), 7.36 (d, 1H), 7.08 (d, 1H), 6.06 (s, 1H), 5.25-5.12 (m, 2H), 4.69-4.55 (m, 1H), 3.88 (s, 3H), 3.87-3.79 (m, 2H), 3.78-3.69 (m, 1H), 3.60 (s, 3H), 3.56-3.47 (m, 1H), 3.43-3.37 (m, 1H), 3.25-3.17 (m, 1H), 3.12-3.05 (m, 1H), 3.03-2.92 (m, 1H), 2.81-2.72 (m, 1H), 2.55-2.36 (m, 3H), 2.08 (s, 3H), 0.90 (t, 3H).

Compound **14-2** with single configuration (having longer retention time):
MS m/z (ESI): 686.2 [M+1].

Chiral HPLC analysis: retention time 9.460 minutes, chiral purity: 98.5% (chromatographic column: CHIRALPAK IE 150^{∗}4.6 mm, 5 µm, equipped with a guard column; mobile phase: n-hexane/ethanol/trifluoroacetic acid =70/30/0.1(V/V/V)).
¹H NMR (400 MHz, CDCl₃) δ 8.33 (d, 1H), 7.76 (d, 1H), 7.64-7.51 (m, 3H), 7.36 (d, 1H), 7.09 (d, 1H), 6.04 (s, 1H), 5.25-5.13 (m, 2H), 4.67-4.55 (m, 1H), 3.89 (s, 3H), 3.87-3.79 (m, 2H), 3.77-3.67 (m, 1H), 3.58 (s, 3H), 3.54-3.47 (m, 1H), 3.45-3.37 (m, 1H), 3.25-3.17 (m, 1H), 3.12-3.05 (m, 1H), 3.04-2.93 (m, 1H), 2.79-2.72 (m, 1H), 2.56-2.36 (m, 3H), 2.09 (s, 3H), 0.90 (t, 3H).

### Test examples:

### Biological Assay

Test Example 1. MCL-1 protein binding experiment of the compound of the present invention.

The following method was used to determine the binding ability of the compound of the present invention to MCL-1 protein. The experimental method is briefly described as follows.

### I. Experimental materials and instruments

1. His-MCL-1 protein (Shanghai Hengrui Pharmaceutical Co., Ltd., NA)
2. Biotin-labeled Bim protein (R&D, 3526/1)
3. Labeled europium cryptate anti-6His antibody (cisbio, 61HI2KLA)
4. XL665-labeled streptavidin (cisbio, 611SAXLA)
5. Binding buffer (cisbio, 62DLBDDF)
6. Detection buffer (cisbio, 62DB1FDG)
7. Microplate reader (BMG, PHERAsta)

### II. Experimental procedures

MCL-1 inhibitors can bind to MCL-1 protein to prevent the binding of MCL-1 to Bim protein. The experiment evaluates the binding ability of MCL-1 inhibitor to MCL-1 protein by detecting the binding of MCL-1 to Bim protein through HTRF method, and the activity of the compound was evaluated according to the Ki value.

The human recombinant protein MCL-1 (sequence 171-327; NCBI ACCESSION: AAF64255) and Bim (sequence 51-76; NCBI ACCESSION: 043521) peptides were labeled with His and biotin, respectively. 0.1 nM His-MCL-1, 2.5 nM bio-Bim and different concentrations of small molecule compounds (initial concentration: 10 µM, 11 concentrations by diluting in 3-fold gradient, diluted in the binding buffer) were mixed and incubated at room temperature for 2 hours, followed by the addition of 0.5 nM Labeled europium cryptate anti-6His antibody and 1.25 nM XL665-labeled streptavidin (diluted in the detection buffer). The mixture was incubated at room temperature for 2 hours, followed by the detection of fluorescence signals at 620 nm and 665 nm by PHERAstar. The data was processed by GraphPad software.

### III. Experimental data

The binding ability of the compound of the present invention to MCL-1 protein can be determined by the above test, and the measured Ki values are shown in Table 1.

**Table 1 Ki of the binding of the compound of the present invention to MCL-1 protein.**

| Example No. | Ki/nM | Maximum inhibition rate (%) |
|---|---|---|
| 1 | 2.39 | 100 |
| 1-1 | 0.36 | 100 |
| 1-2 | 56 | 100 |
| 2 | 0.18 | 99 |
| 2-1 | 0.11 | 100 |
| 2-2 | 65 | 95 |
| 3 | 0.6 | 100 |
| 3-1 | 0.18 | 100 |
| 3-2 | 14 | 99 |
| 4 | 1.35 | 99 |
| 4-1 | 0.15 | 100 |
| 5 | 4.8 | 99 |
| 6 | 1.9 | 98 |
| 7 | 0.71 | 99 |
| 7-1 | 0.74 | 99 |
| 7-2 | 15 | 100 |
| 8 | 0.55 | 100 |
| 8-1 | 0.46 | 100 |
| 8-2 | 57 | 98 |
| 9 | 1.5 | 100 |
| 10 | 7.9 | 101 |
| 11 | 3 | 100 |
| 12 | 0.22 | 100 |
| 12-1 | 0.24 | 100 |
| 12-2 | 5.7 | 100 |
| 13-1 | 0.16 | 100 |
| 13-2 | 4.4 | 99 |
| 14-1 | 0.32 | 100 |

Conclusion: The compound of the present invention has a strong binding ability to MCL-1 protein, and can well inhibit the binding of MCL-1 to Bim protein. The optical activity has a certain influence on the activity of the compound.

### Test Example 2: Cell proliferation experiment

The following method evaluates the inhibition effect of the compound of the present invention on the proliferation of AMO-1 and MV-4-11 cells according to IC₅₀ value by detecting intracellular ATP content. The experimental method is briefly described as follows.

### I. Experimental materials and instruments

1. AMO-1, human bone marrow plasmacytoma (Nanjing Cobioer Biosciences Co.,Ltd., CBP60242)
2. MV-4-11, human acute monocytic leukemia cells (ATCC, CRL-9591)
3. Fetal bovine serum (FBS) (GIBCO, 10099)
4. RPMI1640 (GE, SH30809.01)
5. IMDM (Gibco, 12440053)
6. 2-Mercaptoethanol (sigma, 60-24-2)
7. CellTite (Promega, G7573)
8. 96-well cell culture plate (corning, 3903)
9. Trypan blue solution (Sigma, T8154-100ML)
10. Microplate reader (BMG, PHERAsta)
11. Cell counter (Shanghai Ruiyu Biotech Co.,Ltd., IC1000)

### II. Experimental procedure

AMO-1 cells were cultured in RPMI1640 medium (containing 20% FBS), and MV-4-11 cells were cultured in IMDM medium (containing 10% FBS). The cells were passaged for 2 to 3 times a week, with a passage ratio of 1:4 or 1:6. During the passage, the cells were transferred to a centrifuge tube and centrifuged at 1200 rpm for 3 minutes. The supernatant was discarded, and fresh medium was added to resuspend the cells. 90 µL of cell suspension was added to a 96-well cell culture plate with a density of 1.33×10⁵ cells/mL. 100 µL of complete medium was added to the periphery of the 96-well plate. The plate was incubated in an incubator for 24 hours (37°C, 5% CO₂).

The test sample was diluted to 20 mM with DMSO, and then diluted to 9 concentrations by diluting in 4-fold gradient. Blank and control wells were set. 5 µL of the test compound solution formulated as gradient concentration was added to 95 µL of fresh medium. 10 µL of the above drug-containing medium solution was added to the plate. The plate was incubated in an incubator for 3 days (37°C, 5% CO₂). In the 96-well cell culture plate, 50 µL of CellTiter-Glo reagent was added to each well, and the plate was left to stand in the dark at room temperature for 5 to 10 minutes. The chemiluminescence signal value was read in PHERAstar, and the data was processed by GraphPad software.

### III. Experimental data

The inhibition effect of the compound of the present invention on the proliferation of AMO-1 and MV-4-11 cells can be determined by the above test, and the measured IC₅₀ values are shown in Table 2.

**Table 2 IC₅₀ values of the compound of the present invention for inhibiting the proliferation of AMO-1 and MV-4-11 cells.**

| Example No. | AMO-1 | MV-4-11 |
|---|---|---|
| | IC₅₀/nM | IC₅₀/nM |
| 2-1 | 76 | 42 |
| 4-1 | 37 | 39 |
| 7-1 | 70 | 71 |
| 8-1 | 100 | 136 |
| 12 | 63 | 63 |
| 12-1 | 34 | 36 |
| 13-1 | 26 | 26 |

Conclusion: The compound of the present invention has a good inhibition effect on the proliferation of AMO-1 and MV-4-11 cells.

### Test Example 3. Inhibition effect of the compound of the present invention on the enzymatic activity of human liver microsomal P450 subenzymes CYP2C9 and 2C19

The inhibition effect of the compound of the present invention on the enzymatic activity of human liver microsomal P450 subenzymes CYP2C9 and 2C19 was determined by the following experimental method.

### I. Experimental materials and instruments

1. Phosphate buffer solution (20×PBS, Sangon Biotech (Shanghai) Co., Ltd.)
2. NADPH (ACROS, A0354537)
3. Human liver microsomes (Corning Gentest, Cat No.452161, Lot No.6123001, 33 Donors)
4. ABI QTrap 4000 liquid chromatography-mass spectrometry (AB Sciex)
5. Inertsil C8-3 column, 4.6×50mm, 5 µm (Dikma Technologies Inc., USA)
6. CYP probe substrate for 2C9 (Diclofenac/4 µM, SIGMA, Cat No.D6899-10G) and CYP probe substrate for 2C19 ((S)-Mephenytoin/20 µM, J&K Scientific Ltd., Cat No.303768); positive control inhibitor for 2C9 (Sulfapyrazole, SIGMA, Cat No.526-08-9) and positive control inhibitor for 2C19 (Ticlopidine, SIGMA, Cat No. T6654-1G).

### II. Experimental procedure

100 mM PBS buffer solution was formulated. 2.5 mg/mL microsome solution, 15 mM MgCl₂ solution and 5 mM NADPH solution were formulated with the buffer solution. 30 mM stock solution was diluted with DMSO into a serial solution I with concentrations of 10 mM, 3 mM, 1 mM, 0.3 mM, 0.1 mM, 0.03 mM, 0.003 mM and 0 mM. The above serial solution I was diluted for 200 times with the phosphate buffer solution (PBS) to obtain a serial test solution II (150, 50, 15, 5, 1.5, 0.15, 0.015, 0 µM).

20 µL of 2.5 mg/mL microsome solution, 20 µL of 20 µM diclofenac (2C9) working solution or 100 µM (S)-mephenytoin (2C19) working solution, 20 µL of MgCl₂ solution and 20 µL of compound working solution (150, 50, 15, 5, 1.5, 0.15, 0.015, 0 µM, corresponding reaction system for each concentration) were mixed well. In the positive control group, the compound was replaced with the same concentration of Sulfaphenazole (2C9) or Ticlopidine (2C19). The 5 mM NADPH solution was pre-incubated at 37°C for 5 minutes. After 5 minutes, 20 µL of NADPH was added to each well to start the reaction, followed by incubation for 30 minutes. All incubation samples had duplicate samples. After 30 minutes, 250 µL of acetonitrile (containing internal standard) was added to all samples, mixed well, shaked at 800 rpm for 10 minutes, and centrifuged at 3700 rpm for 10 minutes. 165 µL of supernatant was taken out for LC-MS/MS analysis.

The data were calculated by Graphpad Prism to obtain the IC₅₀ values of the compound for the diclofenac metabolic site of CYP2C9 and (S)-mephenytoin metabolic site of 2C19, which are shown in Table 3.

**Table 3 IC₅₀ values of the compound of the present invention for the diclofenac metabolic site of CYP2C9 and (S)-Mephenytoin metabolic site of 2C19**

| Example No. | 2C9 IC₅₀(µM) | 2C19 IC₅₀(µM) |
|---|---|---|
| 12-1 | >30 | >30 |
| 13-1 | 16.6 | >30 |

Conclusion: The compound of the present invention has no inhibition effect on the Diclofenac metabolic site of human liver microsome CYP2C9 and (S)-Mephenytoin metabolic site of 2C19. The compound of the present invention shows better safety in drug interaction, suggesting that metabolic drug interaction caused by the inhibition of the Diclofenac metabolic site of CYP2C9 and (S)-Mephenytoin metabolic site of 2C19 by the compound will not occur.

## Claims

1. A compound of formula (IM) or a tautomer, mesomer, racemate, enantiomer, diastereomer thereof, or mixture thereof, or a pharmaceutically acceptable salt thereof, 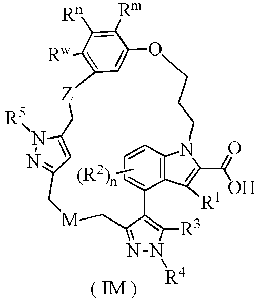 wherein:
R^{m}, Rⁿ and R^{w} are identical or different and are each independently selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or R^{m} and Rⁿ together with adjacent carbon atoms form an aryl, heteroaryl, cycloalkyl or heterocyclyl; and R^{w} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or Rⁿ and R^{w} together with adjacent carbon atoms form an aryl, heteroaryl, cycloalkyl or heterocyclyl; and R^{m} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
Z is a S atom or -CH₂-;
M is a S atom, O atom or -NR₆-;
R¹ is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
R² is selected from the group consisting of hydrogen atom, halogen, alkyl, deuterated alkyl, alkoxy, haloalkyl, hydroxy, hydroxyalkyl, cyano, amino and nitro;
R³ is selected from the group consisting of hydrogen atom, halogen, alkyl, deuterated alkyl, alkoxy, haloalkyl, hydroxy, hydroxyalkyl, cyano, amino and nitro;
R⁴ is selected from the group consisting of hydrogen atom, alkyl, deuterated alkyl and cycloalkyl;
or R³ and R⁴ together with the adjacent carbon atom and N atom form a heterocyclyl;
R⁵ is selected from the group consisting of hydrogen atom, alkyl, deuterated alkyl and cycloalkyl;
R⁶ is selected from the group consisting of hydrogen atom, alkyl and cycloalkyl;
n is 0, 1, 2 or 3.

2. The compound of formula (IM) or the tautomer, mesomer, racemate, enantiomer, diastereomer thereof, or mixture thereof, or the pharmaceutically acceptable salt thereof according to claim 1, being a compound of formula (IM-1) or (IM-2) or a tautomer, mesomer, racemate, enantiomer, diastereomer thereof, or mixture thereof, or a pharmaceutically acceptable salt thereof: 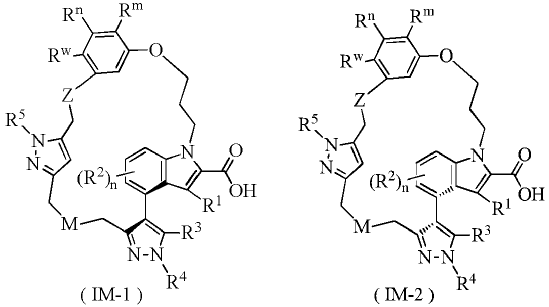 wherein R^{m}, Rⁿ, R^{w}, Z, M, R¹∼R⁵ and n are as defined in claim 1.

3. The compound of formula (IM) or the tautomer, mesomer, racemate, enantiomer, diastereomer thereof, or mixture thereof, or the pharmaceutically acceptable salt thereof according to claim 1 or 2, wherein R^{m} and Rⁿ together with adjacent carbon atoms form a phenyl or cycloalkyl; R^{w} is selected from the group consisting of hydrogen atom, halogen and alkyl; or Rⁿ and R^{w} together with adjacent carbon atoms form a cycloalkyl; and R^{m} is selected from the group consisting of hydrogen atom, halogen and alkyl.

4. The compound of formula (IM) or the tautomer, mesomer, racemate, enantiomer, diastereomer thereof, or mixture thereof, or the pharmaceutically acceptable salt thereof according to any one of claims 1 to 3, wherein 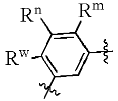 is selected from the group consisting of 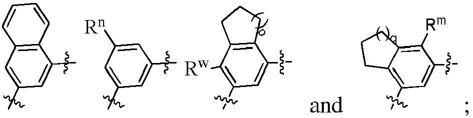 R^{m}, Rⁿ and R^{w} are identical or different and are each independently selected from the group consisting of hydrogen atom, halogen and alkyl; p is 0, 1 or 2; and q is 0, 1 or 2.

5. The compound of formula (IM) or the tautomer, mesomer, racemate, enantiomer, diastereomer thereof, or mixture thereof, or the pharmaceutically acceptable salt thereof according to any one of claims 1 to 4, being a compound of formula (IK) or (IL) or a tautomer, mesomer, racemate, enantiomer, diastereomer thereof, or mixture thereof, or a pharmaceutically acceptable salt thereof: 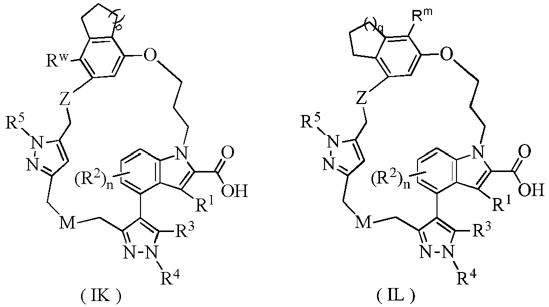 wherein:
p is 0, 1 or 2;
q is 0, 1 or 2;
R^{m} and R^{w} are identical or different and are each independently selected from the group consisting of hydrogen atom, halogen and alkyl;
Z, M, R¹∼R⁵ and n are as defined in claim 1.

6. The compound of formula (IM) or the tautomer, mesomer, racemate, enantiomer, diastereomer thereof, or mixture thereof, or the pharmaceutically acceptable salt thereof according to any one of claims 1 to 5, being a compound of formula (IK-1), (IK-2), (IL-1) or (IL-2) or a tautomer, mesomer, racemate, enantiomer, diastereomer thereof, or mixture thereof, or a pharmaceutically acceptable salt thereof: 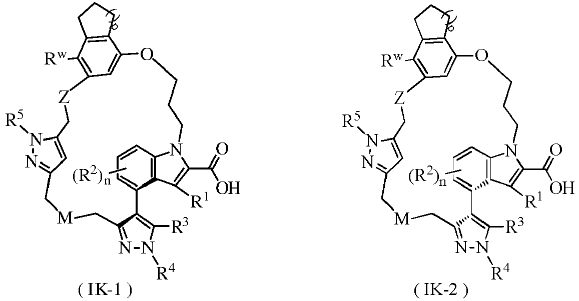 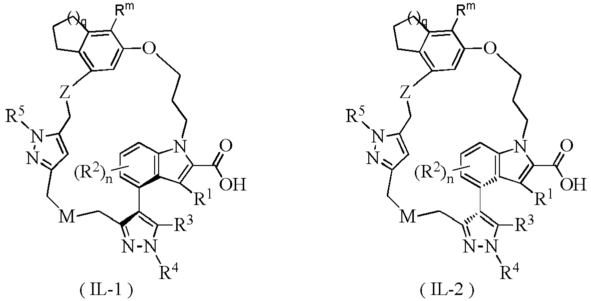 wherein:
p is 0, 1 or 2;
q is 0, 1 or 2;
R^{m} and R^{w} are identical or different and are each independently selected from the group consisting of hydrogen atom, halogen and alkyl;
Z, M, R¹∼R⁵ and n are as defined in claim 1.

7. The compound of formula (IM) or the tautomer, mesomer, racemate, enantiomer, diastereomer thereof, or mixture thereof, or the pharmaceutically acceptable salt thereof according to any one of claims 1 to 6, being a compound of formula (IIK) or (IIL) or a tautomer, mesomer, racemate, enantiomer, diastereomer thereof, or mixture thereof, or a pharmaceutically acceptable salt thereof: 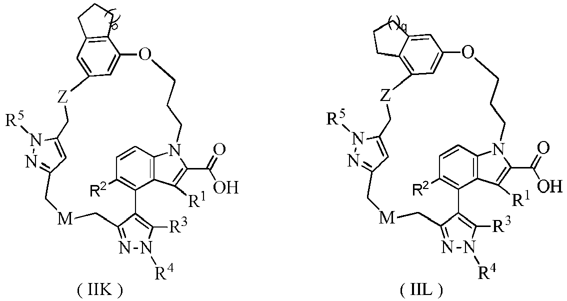 wherein:
p is 1 or 2;
q is 1 or 2;
Z, M and R¹∼R⁵ are as defined in claim 1.

8. The compound of formula (IM) or the tautomer, mesomer, racemate, enantiomer, diastereomer thereof, or mixture thereof, or the pharmaceutically acceptable salt thereof according to any one of claims 1 to 7, being a compound of formula (IIK-1), (IIK-2), (IIL-1) or (IIL-2) or a tautomer, mesomer, racemate, enantiomer, diastereomer thereof, or mixture thereof, or a pharmaceutically acceptable salt thereof: 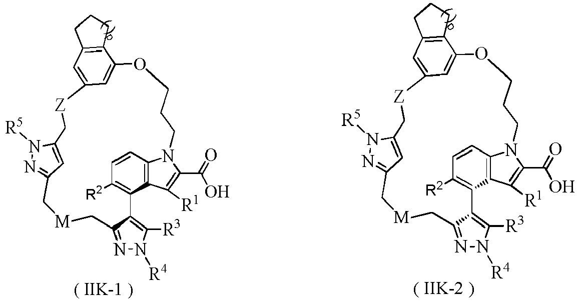 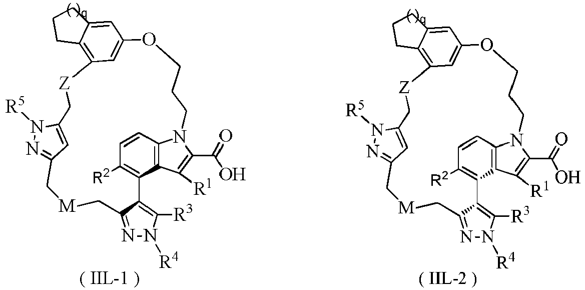 wherein:
p is 1 or 2;
q is 1 or 2;
Z, M and R¹∼R⁵ are as defined in claim 1.

9. The compound of formula (IM) or the tautomer, mesomer, racemate, enantiomer, diastereomer thereof, or mixture thereof, or the pharmaceutically acceptable salt thereof according to any one of claims 1 to 4, being a compound of formula (I) or a tautomer, mesomer, racemate, enantiomer, diastereomer thereof, or mixture thereof, or a pharmaceutically acceptable salt thereof: 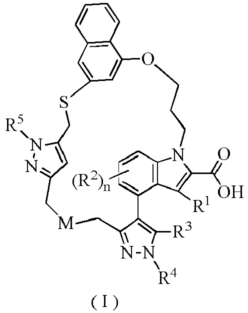 wherein:
M is a S atom, O atom or -NR₆-;
R¹ is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
R² is selected from the group consisting of hydrogen atom, halogen, alkyl, deuterated alkyl, alkoxy, haloalkyl, hydroxy, hydroxyalkyl, cyano, amino and nitro;
R³ is selected from the group consisting of hydrogen atom, halogen, alkyl, deuterated alkyl, alkoxy, haloalkyl, hydroxy, hydroxyalkyl, cyano, amino and nitro;
R⁴ is selected from the group consisting of hydrogen atom, alkyl, deuterated alkyl and cycloalkyl;
or R³ and R⁴ together with the adjacent carbon atom and N atom form a heterocyclyl;
R⁵ is selected from the group consisting of hydrogen atom, alkyl, deuterated alkyl and cycloalkyl;
R⁶ is selected from the group consisting of hydrogen atom, alkyl and cycloalkyl;
n is 0, 1, 2 or 3.

10. The compound of formula (IM) or the tautomer, mesomer, racemate, enantiomer, diastereomer thereof, or mixture thereof, or the pharmaceutically acceptable salt thereof according to any one of claims 1 to 9, wherein R³ is an alkyl.

11. The compound of formula (IM) or the tautomer, mesomer, racemate, enantiomer, diastereomer thereof, or mixture thereof, or the pharmaceutically acceptable salt thereof according to any one of claims 1 to 10, wherein R⁴ or R⁵ is an alkyl.

12. The compound of formula (IM) or the tautomer, mesomer, racemate, enantiomer, diastereomer thereof, or mixture thereof, or the pharmaceutically acceptable salt thereof according to any one of claims 1 to 4 and 9 to 11, being a compound of formula (II) or a tautomer, mesomer, racemate, enantiomer, diastereomer thereof, or mixture thereof, or a pharmaceutically acceptable salt thereof, 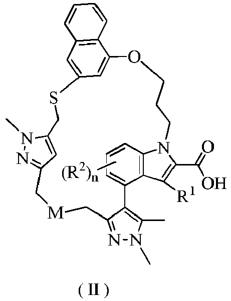 wherein
M, R¹, R² and n are as defined in claim 1.

13. The compound of formula (IM) or the tautomer, mesomer, racemate, enantiomer, diastereomer thereof, or mixture thereof, or the pharmaceutically acceptable salt thereof according to any one of claims 1 to 12, wherein M is an S atom or -N(CH₃)-, and preferably S atom.

14. The compound of formula (IM) or the tautomer, mesomer, racemate, enantiomer, diastereomer thereof, or mixture thereof, or the pharmaceutically acceptable salt thereof according to any one of claims 9 to 13, being a compound of formula (III) or a tautomer, mesomer, racemate, enantiomer, diastereomer thereof, or mixture thereof, or a pharmaceutically acceptable salt thereof, 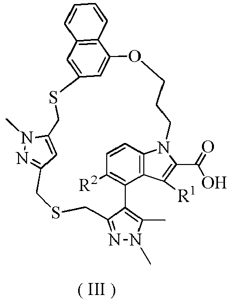 wherein
R¹ and R² are as defined in claim 1.

15. The compound of formula (IM) or the tautomer, mesomer, racemate, enantiomer, diastereomer thereof, or mixture thereof, or the pharmaceutically acceptable salt thereof according to any one of claims 1 to 14, wherein R¹ is a hydrogen atom or alkyl, and preferably alkyl.

16. The compound of formula (IM) or the tautomer, mesomer, racemate, enantiomer, diastereomer thereof, or mixture thereof, or the pharmaceutically acceptable salt thereof according to any one of claims 1 to 15, wherein R² is a halogen.

17. The compound of formula (IM) or the tautomer, mesomer, racemate, enantiomer, diastereomer thereof, or mixture thereof, or the pharmaceutically acceptable salt thereof according to any one of claims 1 to 16, wherein the compound is selected from the group consisting of: 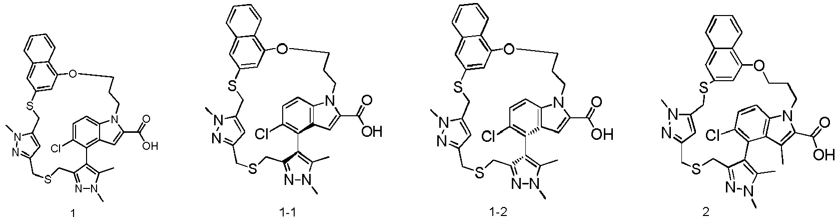 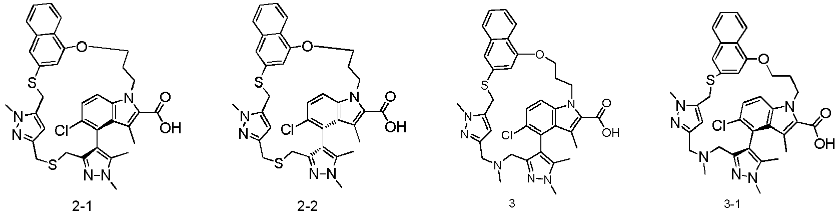 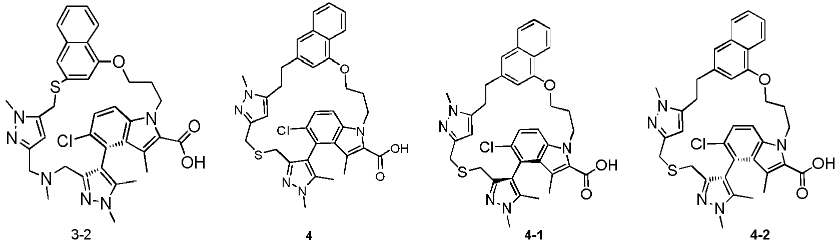 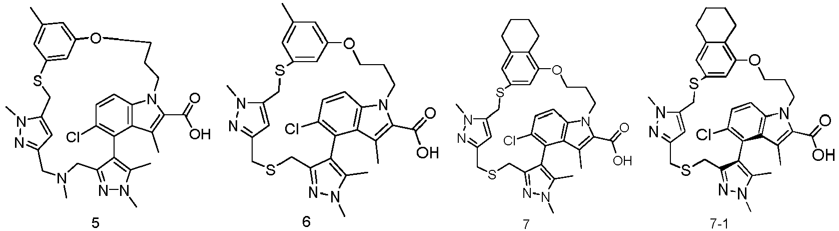 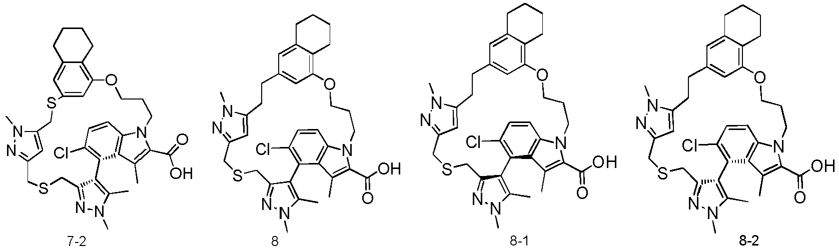 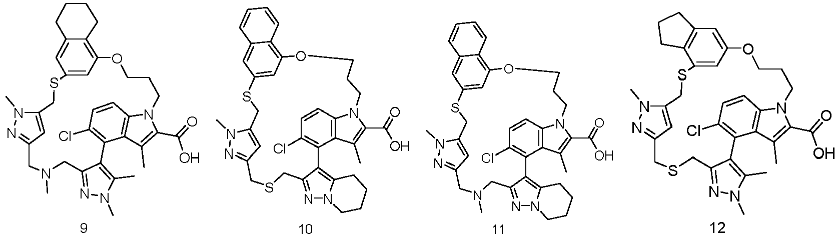 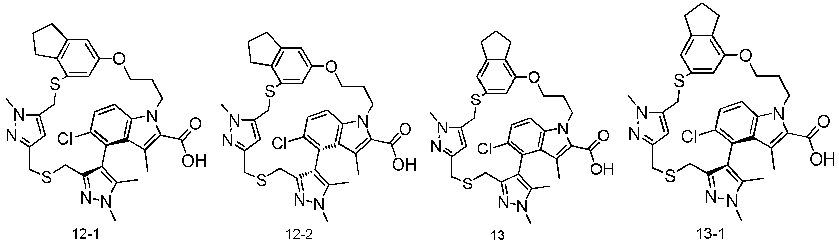 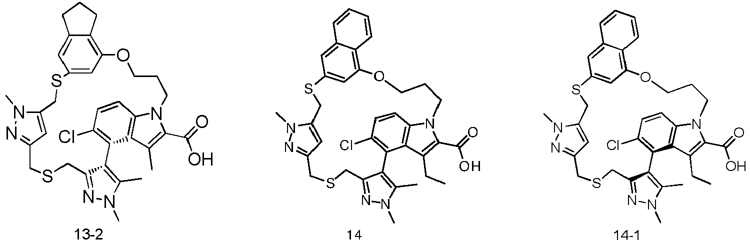 and

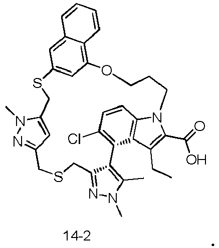

18. A compound of formula (IMA) or a tautomer, mesomer, racemate, enantiomer, diastereomer thereof, or mixture thereof, or a pharmaceutically acceptable salt thereof, 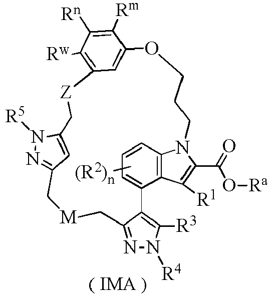 wherein:
R^{m}, Rⁿ and R^{w} are identical or different and are each independently selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or R^{m} and Rⁿ together with adjacent carbon atoms form an aryl, heteroaryl, cycloalkyl or heterocyclyl; and R^{w} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
or Rⁿ and R^{w} together with adjacent carbon atoms form an aryl, heteroaryl, cycloalkyl or heterocyclyl; and R^{m} is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
Z is a S atom or -CH₂-;
M is a S atom, O atom or -NR₆-;
R¹ is selected from the group consisting of hydrogen atom, halogen, alkyl, deuterated alkyl, alkoxy, haloalkyl, hydroxy, hydroxyalkyl, cyano, amino, nitro, cycloalkyl, cycloalkyloxy and heterocyclyl;
R² is selected from the group consisting of hydrogen atom, halogen, alkyl, deuterated alkyl, alkoxy, haloalkyl, hydroxy, hydroxyalkyl, cyano, amino and nitro;
R³ is selected from the group consisting of hydrogen atom, halogen, alkyl, deuterated alkyl, alkoxy, haloalkyl, hydroxy, hydroxyalkyl, cyano, amino and nitro;
R⁴ is selected from the group consisting of hydrogen atom, alkyl, deuterated alkyl and cycloalkyl;
or R³ and R⁴ together with the adjacent carbon atom and N atom form a heterocyclyl;
R⁵ is selected from the group consisting of hydrogen atom, alkyl, deuterated alkyl and cycloalkyl;
R⁶ is selected from the group consisting of hydrogen atom, alkyl and cycloalkyl;
R^{a} is an alkyl;
n is 0, 1, 2 or 3.

19. The compound of formula (IMA) or the tautomer, mesomer, racemate, enantiomer, diastereomer thereof, or mixture thereof, or the pharmaceutically acceptable salt thereof according to claim 18, being a compound of formula (IMA-1) or a tautomer, mesomer, racemate, enantiomer, diastereomer thereof, or mixture thereof, or a pharmaceutically acceptable salt thereof: 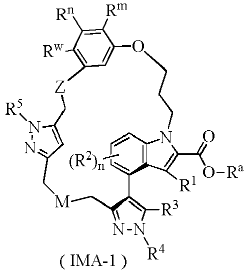 wherein:
R^{a}, R^{m}, Rⁿ, R^{w}, Z, M, R¹∼R⁵ and n are as defined in claim 18.

20. The compound of formula (IMA) or the tautomer, mesomer, racemate, enantiomer, diastereomer thereof, or mixture thereof, or the pharmaceutically acceptable salt thereof according to claim 18, being a compound of formula (IKA) or (ILA) or a tautomer, mesomer, racemate, enantiomer, diastereomer thereof, or mixture thereof, or a pharmaceutically acceptable salt thereof: 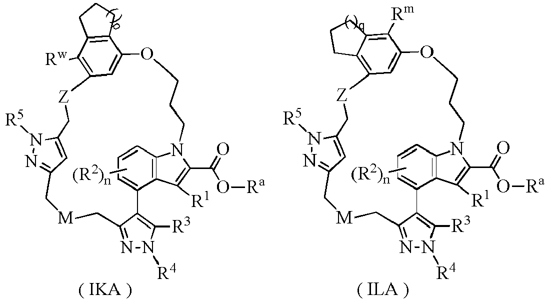 wherein:
R^{a} is an alkyl;
p is 0, 1 or 2;
q is 0, 1 or 2;
R^{m} and R^{w} are identical or different and are each independently selected from the group consisting of hydrogen atom, halogen and alkyl;
Z, M, R¹∼R⁵ and n are as defined in claim 18.

21. The compound of formula (IMA) or the tautomer, mesomer, racemate, enantiomer, diastereomer thereof, or mixture thereof, or the pharmaceutically acceptable salt thereof according to any one of claims 18 to 20, being a compound of formula (IKA-1) or a tautomer, mesomer, racemate, enantiomer, diastereomer thereof, or mixture thereof, or a pharmaceutically acceptable salt thereof: 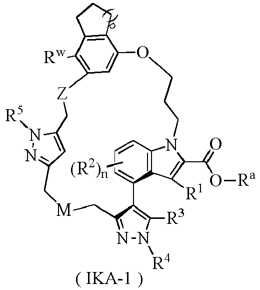 wherein:
R^{a} is an alkyl;
p is 0, 1 or 2;
R^{w} is selected from the group consisting of hydrogen atom, halogen and alkyl;
Z, M, R¹∼R⁵ and n are as defined in claim 18.

22. The compound of formula (IMA) or the tautomer, mesomer, racemate, enantiomer, diastereomer thereof, or mixture thereof, or the pharmaceutically acceptable salt thereof according to claim 18, being a compound of formula (IA) or a tautomer, mesomer, racemate, enantiomer, diastereomer thereof, or mixture thereof, or a pharmaceutically acceptable salt thereof: 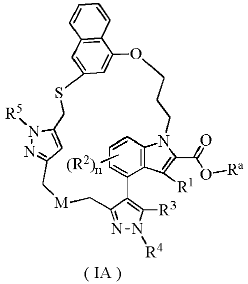 wherein:
R^{a} is an alkyl;
M, R¹∼R⁵ and n are as defined in claim 18.

23. The compound of formula (IMA) or the tautomer, mesomer, racemate, enantiomer, diastereomer thereof, or mixture thereof, or the pharmaceutically acceptable salt thereof according to any one of claims 18 to 22, wherein the compound is selected from the group consisting of: 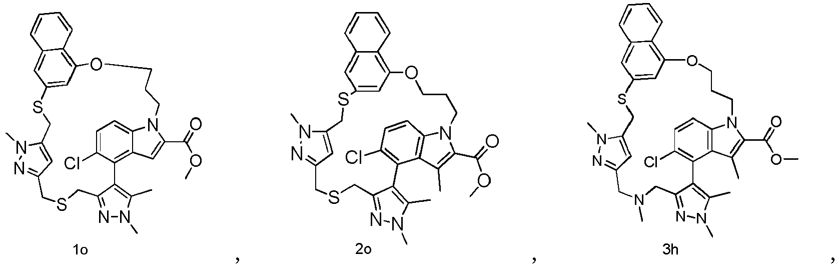 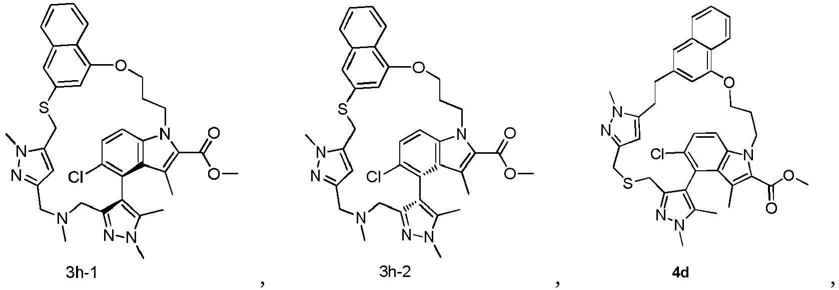 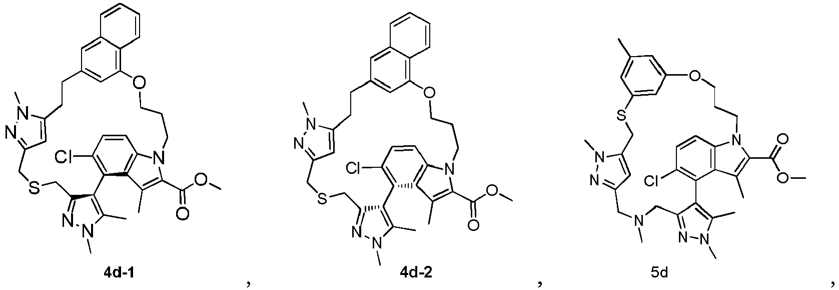 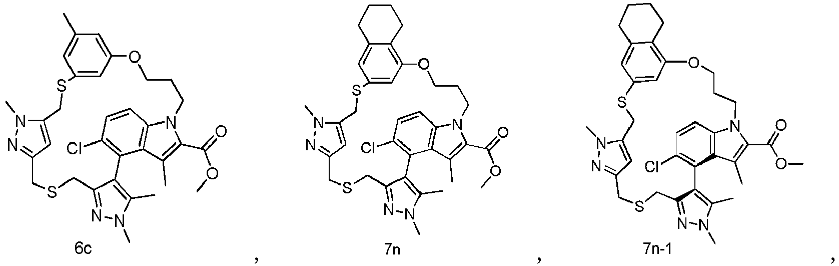 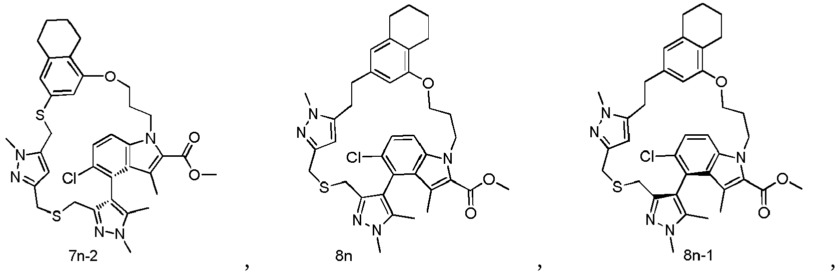 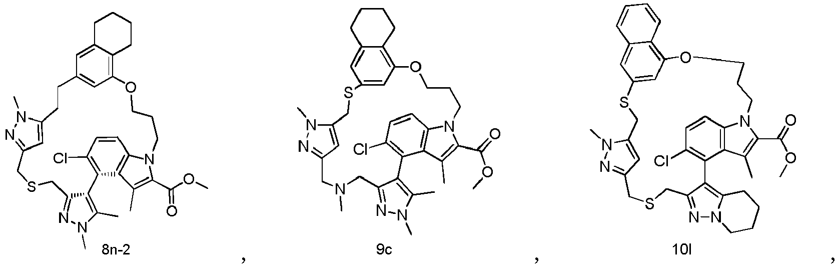 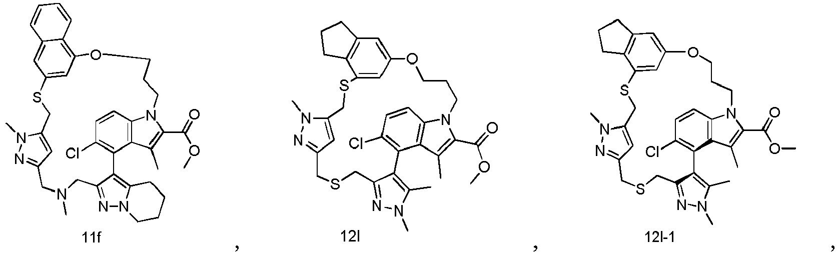 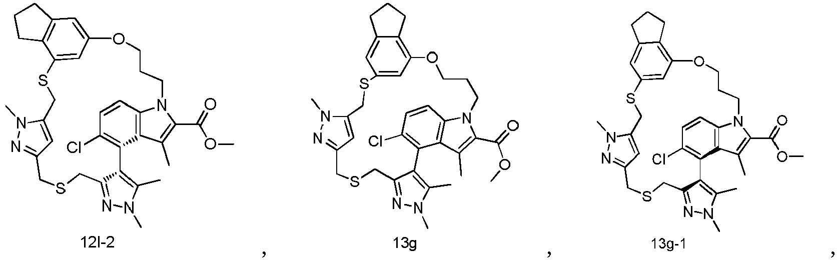 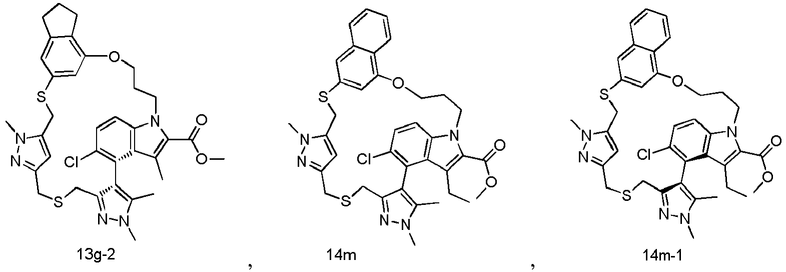 and

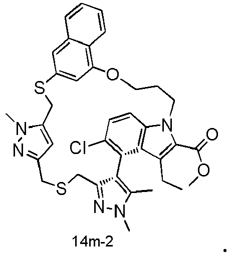

24. A method for preparing the compound of formula (IM) or the tautomer, mesomer, racemate, enantiomer, diastereomer thereof, or mixture thereof, or the pharmaceutically acceptable salt thereof according to claim 1, comprising the following step of: 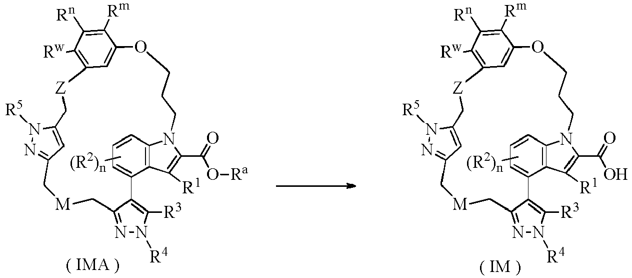 removing the protecting group R^{a} from the compound of formula (IMA) to obtain the compound of formula (IM),
wherein:
R^{a} is an alkyl;
R^{m}, Rⁿ, R^{w}, Z, M, R¹∼R⁵ and n are as defined in claim 1.

25. A method for preparing the compound of formula (IM) or the tautomer, mesomer, racemate, enantiomer, diastereomer thereof, or mixture thereof, or the pharmaceutically acceptable salt thereof according to claim 2, comprising the following step of: 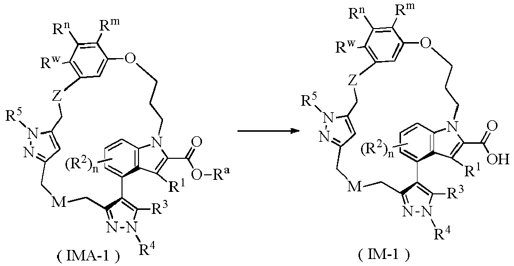 removing the protecting group R^{a} from the compound of formula (IMA-1) to obtain the compound of formula (IM-1),
wherein:
R^{a} is an alkyl;
R^{m}, Rⁿ, R^{w}, Z, M, R¹∼R⁵ and n are as defined in claim 2.

26. A method for preparing the compound of formula (IM) or the tautomer, mesomer, racemate, enantiomer, diastereomer thereof, or mixture thereof, or the pharmaceutically acceptable salt thereof according to claim 5, comprising the following step of: 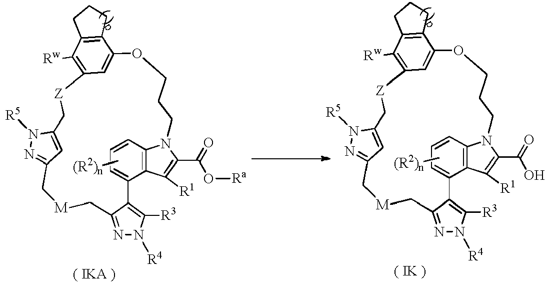 removing the protecting group R^{a} from the compound of formula (IKA) to obtain the compound of formula (IK),
wherein:
R^{a} is an alkyl;
p, R^{w}, Z, M, R¹∼R⁵ and n are as defined in claim 5.

27. A method for preparing the compound of formula (IM) or the tautomer, mesomer, racemate, enantiomer, diastereomer thereof, or mixture thereof, or the pharmaceutically acceptable salt thereof according to claim 5, comprising the following step of: 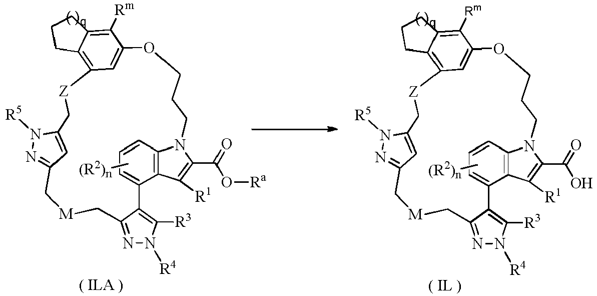 removing the protecting group R^{a} from the compound of formula (ILA) to obtain the compound of formula (IL),
wherein:
R^{a} is an alkyl;
q, R^{m}, Z, M, R¹∼R⁵ and n are as defined in claim 5.

28. A method for preparing the compound of formula (IM) or the tautomer, mesomer, racemate, enantiomer, diastereomer thereof, or mixture thereof, or the pharmaceutically acceptable salt thereof according to claim 6, comprising the following step of: 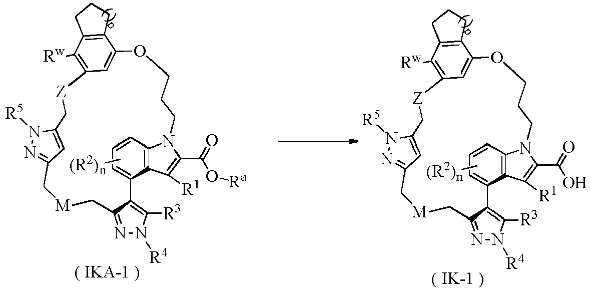 removing the protecting group R^{a} from the compound of formula (IKA-1) to obtain the compound of formula (IK-1),
wherein:
R^{a} is an alkyl;
p, R^{w}, Z, M, R¹∼R⁵ and n are as defined in claim 6.

29. A method for preparing the compound of formula (IM) or the tautomer, mesomer, racemate, enantiomer, diastereomer thereof, or mixture thereof, or the pharmaceutically acceptable salt thereof according to claim 9, comprising the following step of: 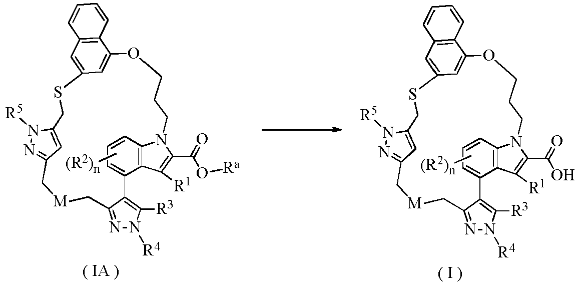 removing the protecting group R^{a} from the compound of formula (IA) to obtain the compound of formula (I),
wherein:
R^{a} is an alkyl;
M, R¹∼R⁵ and n are as defined in claim 9.

30. A pharmaceutical composition comprising the compound of formula (IM) or the tautomer, mesomer, racemate, enantiomer, diastereomer thereof, or mixture thereof, or the pharmaceutically acceptable salt thereof according to any one of claims 1 to 17, and one or more pharmaceutically acceptable carrier(s), diluent(s) or excipient(s).

31. Use of the compound of formula (IM) or the tautomer, mesomer, racemate, enantiomer, diastereomer thereof, or mixture thereof, or the pharmaceutically acceptable salt thereof according to any one of claims 1 to 17, or the pharmaceutical composition according to claim 30 in the preparation of a medicament for inhibiting MCL-1.

32. Use of the compound of formula (IM) or the tautomer, mesomer, racemate, enantiomer, diastereomer thereof, or mixture thereof, or the pharmaceutically acceptable salt thereof according to any one of claims 1 to 17, or the pharmaceutical composition according to claim 30 in the preparation of medicaments for the prevention or treatment of MCL-1 mediated diseases.

33. Use of the compound of formula (IM) or the tautomer, mesomer, racemate, enantiomer, diastereomer thereof, or mixture thereof, or the pharmaceutically acceptable salt thereof according to any one of claims 1 to 17, or the pharmaceutical composition according to claim 30 in the preparation of medicaments for the treatment of tumors, autoimmune diseases or immune system diseases.

34. The use according to claim 33, wherein the tumor is selected from the group consisting of bladder cancer, brain tumor, breast cancer, uterine cancer, cervical cancer, endometrial cancer, ovarian cancer, leukemia, kidney cancer, colon cancer, rectal cancer, colorectal cancer, esophageal cancer, liver cancer, stomach cancer, head and neck cancer, skin cancer, lymphoma, pancreatic cancer, melanoma, myeloma, bone cancer, neuroblastoma, glioma, sarcoma, lung cancer, thyroid cancer and prostate cancer.
